# EUROPEAN PATENT SPECIFICATION

(11) **EP 3 576 773 B1**
(45) Date of publication and mention of the grant of the patent: **18.10.2023**
(21) Application number: 18747362.4
(22) Date of filing: 31.01.2018
(51) Int. Cl.: A61K 38/17, A61K 47/68, A61K 39/395, A61P 35/00, A61P 37/04, A61K 39/00, C07K 14/495, C07K 14/51, C07K 16/22

(54) **TGF BETA AND ACTRII ANTAGONISTS FOR USE IN TREATING LEUKEMIA**
TGF-BETA- UND ACTRII-ANTAGONISTEN ZUR BEHANDLUNG VON LEUKÄMIE
ANTAGONISTES DU TGF BETA ET D'ACTRII À UTILISER DANS LE TRAITEMENT DE LA LEUCÉMIE

(30) Priority: 01.02.2017 US 201762453413 P
(43) Date of publication of application: 11.12.2019
(73) Proprietor: Acceleron Pharma Inc., Cambridge, MA 02139 (US)
(72) Inventor: PEARSALL, Robert Scott, North Reading, MA 01864 (US); KUMAR, Ravindra, Acton, MA 01720 (US)
(74) Representative: Merck Sharp & Dohme LLC
(86) International application number: PCT/US2018/016148
(87) International publication number: WO 2018/144542

(56) References cited:
- EP-A2- 1 486 560
- WO-A1-2012/167143
- WO-A1-2016/164501
- WO-A1-2017/147182
- WO-A2-2008/097541
- WO-A2-2011/109789
- WO-A2-2016/164089
- WO-A2-2016/201282
- CN-A- 101 780 272
- US-A1- 2009 074 768
- US-A1- 2010 047 289
- N Bhola ET AL: "PD08-04: Inhibition of the TGFb/TGFbR2 Pathway Prevents Enrichment of Drug-Resistant Breast Cancer Stem Cells by Anti-Cancer Chemotherapy. | Cancer Research", Cancer Res 2011;71(24 Suppl):Abstract nr PD08-04., 1 December 2011 (2011-12-01), XP055710729, DOI: 10.1158/0008-5472.SABCS11-PD08-04 Retrieved from the Internet: URL:https://cancerres.aacrjournals.org/con tent/71/24_Supplement/PD08-04 [retrieved on 2020-07-01]
- SARA I. CUNHA ET AL: "ALK1 as an emerging target for antiangiogenic therapy of cancer", BLOOD, vol. 117, no. 26, 30 June 2011 (2011-06-30), pages 6999-7006, XP055710393, US ISSN: 0006-4971, DOI: 10.1182/blood-2011-01-330142
- DANIEL BEDINGER ET AL: "Development and characterization of human monoclonal antibodies that neutralize multiple TGF[beta] isoforms", MABS, vol. 8, no. 2, 13 November 2015 (2015-11-13), pages 389-404, XP055710365, US ISSN: 1942-0862, DOI: 10.1080/19420862.2015.1115166

## Description

### CROSS-REFERENCE TO RELATED APPLICATIONS

This application claims the benefit of priority from U.S. Provisional Application No. 62/453,413, filed February 1, 2017.

### BACKGROUND OF THE DISCLOSURE

In cancer treatment, it has long been recognized that chemotherapy is associated with high toxicity and can lead to emergence of resistant cancer cell variants. Even with targeted therapy against overexpressed or activated oncoproteins important for tumor survival and growth, cancer cells frequently mutate and adapt to reduce dependency on the targeted pathway, such as by utilizing a redundant pathway. Cancer immunotherapy is a new paradigm in cancer treatment that, instead of targeting cancer cells, focuses on activation of the immune system. Its principle is to rearm the host's immune response, especially the adaptive T cell response, to identify and kill the cancer cells and to achieve long-lasting, protective immunity. As these therapies are directed at increasing activity of the immune system, cancer immunotherapy agents are also being investigated for the ability to improve immune responses in other disorders, particularly in infectious diseases wherein the pathogen is immune-evasive and/or compromises the host immune system.

FDA approval of the anti-CTLA-4 antibody ipilimumab for the treatment of melanoma in 2011 ushered in a new era of cancer immunotherapy. Demonstration that anti-PD-1 or anti-PD-L1 therapy induced durable responses in melanoma, kidney, and lung cancer in clinical trials further signify the potential use of immunotherapy in the treatment of a broad spectrum of cancers (Pardoll, D. M., Nat Immunol. 2012; 13:1129-32). However, many of the cancer immune therapies available or in clinical trials have limitations. For example, ipilimumab therapy has a high toxicity profile, presumably because anti-CTLA-4 treatment, by interfering with the primary T cell inhibitory checkpoint, can lead to the generation of new autoreactive T cells. While inhibiting the PD-L1/PD-1 interaction results in dis-inhibiting existing chronic immune responses in exhausted T cells that are mostly antiviral or anticancer in nature (Wherry, E. J., Nat Immunol. 2011; 12:492-9), anti-PD-1 therapy can nevertheless sometimes result in potentially fatal lung-related autoimmune adverse events.

Thus, there is still is a high unmet need for effective therapies for increasing immune responses in patients, particularly patients having cancer or an infectious disease. Accordingly, it is an object of the present disclosure to provide methods for improving increasing immune responses in patients in need thereof as well as treating cancer and infectious diseases.

### SUMMARY OF THE INVENTION

The present invention is defined in the appended claims.

In part, the data presented herein demonstrates that ActRII antagonists (inhibitors) and TGFβ antagonists (particularly inhibitors of TGFβ2) can be used alone or in combination to treat cancer. In particular, it was shown that treatment with an ActRIIA polypeptide, an ActRIIB polypeptide, or a pan-specific TGFβ antibody, separately, decreased tumor burden and increased survival time a cancer model. Moreover, it was shown that an ActRII antagonist in combination with a TGFβ antagonist can be used to synergistically increase antitumor activity compared to the effects observed with either agent alone. Accordingly, the disclosure provides, in part, methods of using an ActRII antagonist, a TGFβ antagonist, or a combination of an ActRII antagonist and a TGFβ antagonist, alone or in combination with one or more supportive therapies and/or active agents, to treat cancer, particularly treating or preventing one or more complications of a cancer (e.g., reducing tumor burden). In addition, the data indicate that efficacy of ActRII and TGFβ antagonist therapy is dependent on the immune system. Therefore, in part, the instant disclosure relates to the discovery that ActRII and TGFβ antagonists may be used as immunotherapeutics, particularly to treat a wide variety of cancers (e.g., cancers associated with immunosuppression and/or immune exhaustion). As with other known immuno-oncology agents, the ability of an ActRII and TGFβ antagonist to potentiate an immune response in a patient may have broader therapeutic implications outside the cancer field. For example, it has been proposed that immune potentiating agents may be useful in treating a wide variety of infectious diseases, particularly pathogenic agents which promote immunosuppression and/or immune exhaustion. Also, such immune potentiating agents may be useful in boosting the immunization efficacy of vaccines (e.g., infectious disease and cancer vaccines). Accordingly, the disclosure provides various ActRII and TGFβ antagonists that can be used, alone or in combination, to increase immune responses in a subject in need thereof, treat cancer, treat infectious diseases, and/or increase immunization efficacy, optionally in combination with one or more supportive therapies and/or additional active agents.

Although the ActRIIA polypeptides, ActRIIB polypeptides, and TGFβ antibody described in the examples may affect the immune system and/or cancer through a mechanisms other than inhibition of ActRII-binding and/or TGFβRII-binding ligands [e.g., inhibition of one or more of GDF 11, GDF8, activin (e.g., activin A, activin B, activin C, activin E, activin AB, and activin AE), BMP6, GDF3, BMP10, BMP9, TGFβ2, TGFβ1, and TGFβ3 may be an indicator of the tendency of an agent to inhibit the activities of a spectrum of additional agents, including, perhaps, other members of this ligand superfamily, and such collective inhibition may lead to the desired effect on, for example, cancer], other types of ActRII signaling and TGFβRII signaling pathway inhibitors [e.g., ActRII and/or TGFβRII ligand inhibitors; type I-, type II-, and/or co-receptor inhibitors (e.g., inhibitors of one or more of ALK4, ALK5, ActRIIA, ActRIIB, TGFβRII, and betaglycan); and downstream signaling inhibitors (e.g., inhibitors of one or more Smad proteins such as Smads 2 and 3)] are expected to be useful in accordance with the methods and uses of disclosure including, for example, antibody antagonists, nucleic acid antagonists, small molecule antagonists, and ligands traps (e.g., soluble ActRIIA polypeptides, ActRIIB polypeptides, TGFβRII polypeptides, ALK4:ActRIIB heterodimers, follistatin polypeptides, and FLRG polypeptides). As used herein, agents that inhibit ActRII (ActRIIA and/or ActRIIB) activity are collectively referred to as "ActRII antagonists:" or "ActRII inhibitors" and include, for example, agents that inhibit one or more of ActRIIA, ActRIIB, ALK4, and ActRII ligands [e.g., activin (e.g., activin A, activin B, activin C, activin E, activin AB, and activin AE), GDF11, GDF8, BMP6, GDF3, BMP10, and BMP9]. As used herein, agents that inhibit TGFβRII activity are collectively referred to as "TGFβ antagonists:" or "TGFβ inhibitors" and include, for example, agents that inhibit one or more of TGFβRII, ALK5, betaglycan, and TGFβRII ligands (e.g., TGFβ1, TGFβ2, and TGFβ3).

In certain aspects, the disclosure relates to methods of inducing an immune response in a patient comprising administering to a patient in need thereof an ActRII antagonist and a TGFβ antagonist wherein the ActRII antagonist and TGFβ antagonist are administering in an effective amount. In other aspects, the disclosure relates to methods of potentiating an immune response in a patient comprising administering to a patient in need thereof an ActRII antagonist and a TGFβ antagonist wherein the ActRII antagonist and TGFβ antagonist are administering in an effective amount. In some aspects, the disclosure relates to use of an ActRII antagonist in combination with a TGFβ antagonist to induce or potentiate an immune response in a patient in need thereof. In some aspects, the disclosure relates to use of a TGFβ antagonist in combination with a ActRII antagonist to induce or potentiate an immune response in a patient in need thereof. In some embodiments, the patient has a cancer. In some embodiments, the patient has a tumor. In some embodiments, the initiated or potentiated immune response is against a cancer. In some embodiments, the initiated or potentiated immune response is against a tumor. In some embodiments, the initiated or potentiated immune response inhibits growth of a cancer. In some embodiments, the initiated or potentiated immune response inhibits growth of a tumor. In some embodiments, the initiated or potentiated immune response decreases cancer cell burden in the patient. In some embodiments, the initiated or potentiated immune response decreases tumor cell burden in the patient. In some embodiments, the initiated or potentiated immune response treats or prevents cancer metastasis. In some embodiments, the initiated or potentiated immune response treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the initiated or potentiated immune response is against a pathogen. In some embodiments, the initiated or potentiated immune response treats infection by a pathogen in the patient. In some embodiments, the initiated or potentiated immune response prevents infection by a pathogen in the patient. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the pathogen is selected from the group consisting of: a bacterial, viral, fungal, or parasitic pathogen. In some embodiments, the patient is at risk for developing immune exhaustion. In some embodiments, the patient has a disease or condition associated with immune exhaustion. In some embodiments, the initiated or potentiated immune response comprises a T cell immune response. In some embodiments, the initiated or potentiated immune response vaccinates the patient against a cancer or pathogen. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of treating cancer in a patient comprising administering to a patient in need thereof an ActRII antagonist and a TGFβ antagonist wherein the ActRII antagonist and the TGFβ antagonist are administered in an effective amount. In other aspects, the disclosure relates to method of treating a tumor in a patient comprising administering to a patient in need thereof an ActRII antagonist and a TGFβ antagonist wherein the ActRII antagonist and the TGFβ antagonist are administered in an effective amount. In some aspects, the disclosure relates to use of an ActRII antagonist in combination with a TGFβ antagonist to treat cancer or a tumor in a patient in need thereof. In some aspects, the disclosure relates to use of a TGFβ antagonist in combination with an ActRII antagonist to treat cancer or a tumor in a patient in need thereof. In some embodiments, the method inhibits growth of a cancer. In some embodiments, the method inhibits growth of a tumor. In some embodiments, the method decreases cancer cell burden in the patient. In some embodiments, the method decreases tumor cell burden in the patient. In some embodiments, the method treats or prevents cancer metastasis. In some embodiments, the method treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the patient is at risk for developing immune exhaustion. In some embodiments, the patient has a disease or condition associated with immune exhaustion. In some embodiments, the patient is further administered one or more additional active agents and/or supportive therapies for treating a cancer or tumor. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of treating immune exhaustion in a patient comprising administering to a patient in need thereof an ActRII antagonist and a TGFβ antagonist wherein the ActRII antagonist and the TGFβ antagonist are administered in an effective amount. In some aspects, the disclosure relates to methods of preventing immune exhaustion in a patient comprising administering to a patient in need thereof an ActRII antagonist and a TGFβ antagonist wherein the ActRII antagonist and the TGFβ antagonist are administered in an effective amount. In some aspects, the disclosure relates to use of an ActRII antagonist in combination with a TGFβ antagonist for treating or preventing immune exhaustion. In some aspects, the disclosure relates to use of a TGFβ antagonist in combination with a ActRII antagonist for treating or preventing immune exhaustion. In some embodiments, the patient has a cancer. In some embodiments, the patient has a tumor. In some embodiments, the method inhibits growth of a cancer. In some embodiments, the method inhibits growth of a tumor. In some embodiments, the method decreases cancer cell burden in the patient. In some embodiments, the method decreases tumor cell burden in the patient. In some embodiments, the method treats or prevents cancer metastasis. In some embodiments, the method treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the patient is infected with a pathogen. In some embodiments, the method treats infection by a pathogen in the patient. In some embodiments, the method prevents infection by a pathogen in the patient. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the pathogen is selected from the group consisting of: a bacterial, viral, fungal, or parasitic pathogen. In some embodiments, immune exhaustion comprises a T cell immune exhaustion. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of inducing an immune response against an antigen in a patient comprising administering to a patient in need thereof: a TGFβ antagonist, an ActRII antagonist, and the antigen wherein the TGFβ antagonist, the ActRII antagonist, and the antigen are administered in an effective amount. In some aspects, the disclosure relates to methods of potentiating an immune response against an antigen in a patient comprising administering to a patient in need thereof: a TGFβ antagonist, an ActRII antagonist, and the antigen wherein the TGFβ antagonist, the ActRII antagonist, and the antigen are administered in an effective amount. In some aspects, the disclosure relates to use of an ActRII antagonist in combination with a TGFβ antagonist for inducing or potentiating an immune response against an antigen. In some embodiments, the antigen is a cancer antigen. In some embodiments, the antigen is a tumor antigen. In some embodiments, the cancer or tumor antigen is associated with a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the antigen is a pathogen antigen. In some embodiments the pathogen antigen is associated with a pathogen selected from the group consisting of: a bacterial pathogen, a viral pathogen, a fungal pathogen, or a parasite pathogen. In some embodiments, the cancer antigen is administered in accordance with a vaccination protocol. In some embodiments, the tumor antigen is administered in accordance with a vaccination protocol. In some embodiments, the pathogen antigen is administered in accordance with a vaccination protocol. In some embodiments, the initiated or potentiated immune response vaccinates the patient against a cancer. In some embodiments, the initiated or potentiated immune response vaccinates the patient against a tumor. In some embodiments, the initiated or potentiated immune response vaccinates the patient against a pathogen. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the patient has a disease or condition associated with immune exhaustion. In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of vaccinating a patient against a cancer comprising administering to a patient in need thereof: a TGFβ antagonist, an ActRII antagonist, and a cancer antigen, wherein the TGFβ antagonist, the ActRII antagonist, and the antigen are administered in an amount effective to vaccinate the patient. In some aspects, the disclosure relates to methods of vaccinating a patient against a pathogen comprising administering to a patient in need thereof: a TGFβ antagonist, an ActRII antagonist, and a pathogen antigen, wherein the TGFβ antagonist, the ActRII antagonist, and the antigen are administered in an amount effective to vaccinate the patient. In some aspects, the disclosure relates to use of an ActRII antagonist in combination with a TGFβ antagonist and a cancer antigen to vaccinate the patient against a cancer. In some aspects, the disclosure relates to use of a TGFβ antagonist in combination with an ActRII antagonist and a cancer antigen to vaccinate the patient against a cancer. In some aspects, the disclosure relates to use of an ActRII antagonist in combination with a TGFβ antagonist and a pathogen antigen to vaccinate the patient against a pathogen. In some aspects, the disclosure relates to use of a TGFβ antagonist in combination with an ActRII antagonist and a pathogen antigen to vaccinate the patient against a pathogen. In some embodiments, the cancer or tumor antigen is associated with a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments the pathogen antigen is associated with a pathogen selected from the group consisting of: a bacterial pathogen, a viral pathogen, a fungal pathogen, or a parasite pathogen. In some embodiments, the cancer antigen is administered in accordance with a vaccination protocol. In some embodiments, the tumor antigen is administered in accordance with a vaccination protocol. In some embodiments, the pathogen antigen is administered in accordance with a vaccination protocol. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the patient has a disease or condition associated with immune exhaustion. In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of potentiating an immune response induced by a vaccine in a patient comprising administering a TGFβ antagonist and an ActRII antagonist to a patient in an amount effective to potentiate an immune response induced by the vaccine in the patient. In some aspects, the disclosure relates to use of an ActRII antagonist in combination with a TGFβ antagonist to potentiate an immune response induced by a vaccine in a patient in need thereof. In some aspects, the disclosure relates to use of an TGFβ antagonist in combination with a ActRII antagonist to potentiate an immune response induced by a vaccine in a patient in need thereof. In some embodiments, the vaccine is a cancer vaccine. In some embodiments, the vaccine is a tumor vaccine. In some embodiments, the initiated or potentiated immune response inhibits growth of a cancer. In some embodiments, the initiated or potentiated immune response inhibits growth of a tumor. In some embodiments, the initiated or potentiated immune response decreases cancer cell burden in the patient. In some embodiments, the initiated or potentiated immune response decreases tumor cell burden in the patient. In some embodiments, the initiated or potentiated immune response treats or prevents cancer metastasis. In some embodiments, the initiated or potentiated immune response treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the vaccine is a pathogen vaccine. In some embodiments, the initiated or potentiated immune response treats infection by a pathogen in the patient. In some embodiments, the initiated or potentiated immune response prevents infection by a pathogen in the patient. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the pathogen is selected from the group consisting of: a bacterial, viral, fungal, or parasitic pathogen. In some embodiments, the patient is at risk for developing immune exhaustion. In some embodiments, the patient has a disease or condition associated with immune exhaustion. In some embodiments, the initiated or potentiated immune response comprises a T cell immune response. In some embodiments, the initiated or potentiated immune response vaccinates the patient against a cancer or pathogen. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of inducing an immune response in a patient comprising administering to a patient in need thereof and effective amount of a TGFβ antagonist. In some aspects, the disclosure relates to methods of potentiating an immune response in a patient comprising administering to a patient in need thereof and effective amount of a TGFβ antagonist. In some aspects, the disclosure relates to use of a TGFβ antagonist for inducing or potentiating an immune response in a patient in need thereof. In some embodiments, the patient has a cancer. In some embodiments, the patient has a tumor. In some embodiments, the initiated or potentiated immune response is against a cancer. In some embodiments, the initiated or potentiated immune response is against a tumor. In some embodiments, the initiated or potentiated immune response inhibits growth of a cancer. In some embodiments, the initiated or potentiated immune response inhibits growth of a tumor. In some embodiments, the initiated or potentiated immune response decreases cancer cell burden in the patient. In some embodiments, the initiated or potentiated immune response decreases tumor cell burden in the patient. In some embodiments, the initiated or potentiated immune response treats or prevents cancer metastasis. In some embodiments, the initiated or potentiated immune response treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the initiated or potentiated immune response is against a pathogen. In some embodiments, the initiated or potentiated immune response treats infection by a pathogen in the patient. In some embodiments, the initiated or potentiated immune response prevents infection by a pathogen in the patient. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the pathogen is selected from the group consisting of: a bacterial, viral, fungal, or parasitic pathogen. In some embodiments, the patient is at risk for developing immune exhaustion. In some embodiments, the patient has a disease or condition associated with immune exhaustion. In some embodiments, the initiated or potentiated immune response comprises a T cell immune response. In some embodiments, the initiated or potentiated immune response vaccinates the patient against a cancer or pathogen. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of treating cancer in a patient comprising administering to a patient in need thereof an effective amount of a TGFβ antagonist. In some aspects, the disclosure relates to methods of treating a tumor in a patient comprising administering to a patient in need thereof an effective amount of a TGFβ antagonist. In some aspects, the disclosure relates to use of a TGFβ antagonist for treating cancer or a tumor in a patient in need thereof. In some embodiments, the method inhibits growth of a cancer. In some embodiments, the method inhibits growth of a tumor. In some embodiments, the method decreases cancer cell burden in the patient. In some embodiments, the method decreases tumor cell burden in the patient. In some embodiments, the method treats or prevents cancer metastasis. In some embodiments, the method treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the patient is at risk for developing immune exhaustion. In some embodiments, the patient has a disease or condition associated with immune exhaustion. In some embodiments, the patient is further administered one or more additional active agents and/or supportive therapies for treating a cancer or tumor. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of treating immune exhaustion in a patient comprising administering to a patient in need thereof an effective amount of a TGFβ antagonist. In some aspects, the disclosure relates to methods of preventing immune exhaustion in a patient comprising administering to a patient in need thereof an effective amount of a TGFβ antagonist. In some embodiments, the disclosure relates to use of a TGFβ antagonist for treating or preventing immune exhaustion in a patient in need thereof. In some embodiments, the patient has a cancer. In some embodiments, the patient has a tumor. In some embodiments, the method inhibits growth of a cancer. In some embodiments, the method inhibits growth of a tumor. In some embodiments, the method decreases cancer cell burden in the patient. In some embodiments, the method decreases tumor cell burden in the patient. In some embodiments, the method treats or prevents cancer metastasis. In some embodiments, the method treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the patient is infected with a pathogen. In some embodiments, the method treats infection by a pathogen in the patient. In some embodiments, the method prevents infection by a pathogen in the patient. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the pathogen is selected from the group consisting of: a bacterial, viral, fungal, or parasitic pathogen. In some embodiments, immune exhaustion comprises a T cell immune exhaustion. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to methods of inducing an immune response against an antigen in a patient comprising administering to a patient in need thereof a TGFβ antagonist and the antigen wherein the TGFP antagonist and the antigen are administered in an effective amount. In certain aspects, the disclosure relates to methods of potentiating an immune response against an antigen in a patient comprising administering to a patient in need thereof a TGFβ antagonist and the antigen wherein the TGFβ antagonist and the antigen are administered in an effective amount. In some aspects, the disclosure relates to use of a TGFβ antagonist for inducing or potentiating an immune response against an antigen in a patient in need thereof. In some embodiments, the antigen is a cancer antigen. In some embodiments, the antigen is a tumor antigen. In some embodiments, the cancer or tumor antigen is associated with a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the antigen is a pathogen antigen. In some embodiments the pathogen antigen is associated with a pathogen selected from the group consisting of: a bacterial pathogen, a viral pathogen, a fungal pathogen, or a parasite pathogen. In some embodiments, the cancer antigen is administered in accordance with a vaccination protocol. In some embodiments, the tumor antigen is administered in accordance with a vaccination protocol. In some embodiments, the pathogen antigen is administered in accordance with a vaccination protocol. In some embodiments, the initiated or potentiated immune response vaccinates the patient against a cancer. In some embodiments, the initiated or potentiated immune response vaccinates the patient against a tumor. In some embodiments, the initiated or potentiated immune response vaccinates the patient against a pathogen. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the patient has a disease or condition associated with immune exhaustion. In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relate to methods of vaccinating a patient against cancer comprising administering to a patient in need thereof a TGFβ antagonist and cancer antigen wherein the TGFβ antagonist and the antigen are administered in an amount effective to vaccinate the patient. In some aspects, the disclosure relate to methods of vaccinating a patient against a pathogen comprising administering to a patient in need thereof a TGFβ antagonist and a pathogen antigen wherein the TGFβ antagonist and the antigen are administered in an amount effective to vaccinate the patient. In some aspects, the disclosure relates to use of a TGFβ antagonist in combination with a pathogen or cancer antigen for vaccinating a patient a pathogen or cancer. In some embodiments, the cancer or tumor antigen is associated with a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments the pathogen antigen is associated with a pathogen selected from the group consisting of: a bacterial pathogen, a viral pathogen, a fungal pathogen, or a parasite pathogen. In some embodiments, the cancer antigen is administered in accordance with a vaccination protocol. In some embodiments, the tumor antigen is administered in accordance with a vaccination protocol. In some embodiments, the pathogen antigen is administered in accordance with a vaccination protocol. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the patient has a disease or condition associated with immune exhaustion. In some embodiments, the patient does not have an autoimmune disease. In some embodiments, the patient is not undergoing a tissue or organ transplantation or has not received a tissue or organ transplantation. In some embodiments, the patient does not have graft vs. host disease.

In certain aspects, the disclosure relates to potentiating an immune response induced by a vaccine in a patient comprising administering a TGFβ antagonist to a patient in an amount effective to potentiate an immune response induced by the vaccine in the patient. In some aspects, the disclosure relates to use of a TGFβ antagonist for potentiating an immune response induced by a vaccine in a patient in need thereof. In some embodiments, the vaccine is a cancer vaccine. In some embodiments, the vaccine is a tumor vaccine. In some embodiments, the initiated or potentiated immune response inhibits growth of a cancer. In some embodiments, the initiated or potentiated immune response inhibits growth of a tumor. In some embodiments, the initiated or potentiated immune response decreases cancer cell burden in the patient. In some embodiments, the initiated or potentiated immune response decreases tumor cell burden in the patient. In some embodiments, the initiated or potentiated immune response treats or prevents cancer metastasis. In some embodiments, the initiated or potentiated immune response treats or prevents tumor metastasis. In some embodiments, the cancer promotes immunosuppression in the patient. In some embodiments, the tumor promotes immunosuppression in the patient. In some embodiments, the cancer promotes immune cell exhaustion in the patient. In some embodiments, the tumor promotes immune cell exhaustion in the patient. In some embodiments, the cancer promotes T cell exhaustion. In some embodiments, the tumor promotes T cell exhaustion. In some embodiments, the cancer is responsive to immunotherapy. In some embodiments, the tumor is responsive to immunotherapy. In some embodiments, the patient has a cancer or tumor selected from the group consisting of: leukemia, melanoma (e.g., metastatic melanoma), lung cancer (e.g., squamous non-small cell lung cancer), renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma). In some embodiments, the vaccine is a pathogen vaccine. In some embodiments, the initiated or potentiated immune response treats infection by a pathogen in the patient. In some embodiments, the initiated or potentiated immune response prevents infection by a pathogen in the patient. In some embodiments, the pathogen promotes immunosuppression in the patient. In some embodiments, the pathogen promotes immune cell exhaustion in the patient. In some embodiments, the pathogen promotes T cell exhaustion. In some embodiments, the pathogen is responsive to immunotherapy. In some embodiments, the pathogen is selected from the group consisting of: a bacterial, viral, fungal, or parasitic pathogen. In some embodiments, the patient is at risk for developing immune exhaustion. In some embodiments, the patient has a disease or condition associated with immune exhaustion. In some embodiments, the initiated or potentiated immune response comprises a T cell immune response. In some embodiments, the initiated or potentiated immune response vaccinates the patient against a cancer or pathogen. In some embodiments, the patient is further administered one or more additional active agents and/or supportive therapies for treating a cancer or tumor. In some embodiments, the patient is further administered one or more additional active agents and/or supportive therapies for treating a pathogen. In some embodiments, the patient is further administered one or more additional immuno-oncology agents. In some embodiments, the one or more additional immune-oncology agents is selected from the group consisting of: alemtuzumab, ipilimumab, nivolumab, ofatmumab, rituximab, pembrolizumab, atexolizumab, a programmed death-ligand 1 (PD-L1) binding agent (e.g., a PD-L1 antibody), a CD20-directed cytolytic binding agent (e.g., a CD-20 antibody), a cytotoxic T-lymphocyte antigen 4 (CTLA-4) binding agent (e.g., a CTLA-4 antibody), and a programmed death receptor-1 (PD-1) binding agent (e.g., a PD-1 antibody). In some embodiments, the patient does not have an autoimmune disease. In some embodiments, the patient is not undergoing a tissue or organ transplantation or has not received a tissue or organ transplantation. In some embodiments, the patient does not have graft vs. host disease.

In some embodiments, ActRII antagonists of the disclosure are agents that can inhibit ActRII (e.g., an ActRIIA and/or ActRIIB receptor) and/or ALK4, particularly inhibiting downstream signaling. Therefore, in some embodiments, ActRII antagonists of the disclosure are agents that can inhibit one or more ActRII and/ALK4-binding ligands [e.g., GDF11, GDF8, activin (activin A, activin B, activin AB, activin C, activin E) BMP6, GDF3, BMP10, and/or BMP9]. In some embodiments, ActRII antagonists of the disclosure are agents that can inhibit one or more intracellular mediators of the ActRII and/or ALK4 signaling pathway (e.g., Smads 2 and 3). Such ActRII antagonist agents include, for example, ActRII (ActRIIA or ActRIIB) polypeptides, or combination of ActRII polypeptides, as well as variants thereof (e.g., a GDF trap polypeptide); an antibody, or combination of antibodies, that inhibit one or more ActRII ligands, ALK4 receptor, and/or ActRII receptor; a polynucleotide, or combination of polynucleotides, that inhibits of one or more ActRII ligands, ALK4, ActRII receptor, and/or ActRII and/or ALK4 downstream signaling component (e.g., Smads); a small molecule, or combination of small molecules, that inhibits of one or more ActRII ligands, ALK4, ActRII receptor, and/or ActRII and/or ALK4 downstream signaling component (e.g., Smads), as well as combinations thereof. In certain preferred embodiments, ActRII antagonists to be used in accordance with the teachings of the disclosure inhibit at least activin, particularly activin A.

In certain aspects, an ActRII antagonist, or combination of antagonists, to be used in accordance with methods and uses described herein is an agent that inhibits at least GDF11. Effects on GDF11 inhibition may be determined, for example, using a cell-based assay including those described herein (e.g., Smad signaling reporter assay). Therefore, in some embodiments, a GDF11 antagonist, or combination of antagonist, of the disclosure may bind to at least GDF 11. Ligand binding activity may be determined, for example, using a binding affinity assay including, for example, those described herein. In some embodiments, an ActRII antagonist, or combination of antagonists, of the disclosure binds to at least GDF11 with a K_{D} of at least 1 × 10⁻⁷ M (e.g., at least 1 × 10⁻⁸ M, at least 1 × 10⁻⁹ M, at least 1 × 10⁻¹⁰ M, at least 1 × 10⁻¹¹ M, or at least 1 × 10⁻¹² M).

In certain aspects, an ActRII antagonist, or combination of antagonists, to be used in accordance with methods and uses described herein is an agent that inhibits at least GDF8. Effects on GDF8 inhibition may be determined, for example, using a cell-based assay including those described herein (e.g., Smad signaling reporter assay). Therefore, in some embodiments, a GDF8 antagonist, or combination of antagonist, of the disclosure may bind to at least GDF8. Ligand binding activity may be determined, for example, using a binding affinity assay including, for example, those described herein. In some embodiments, an ActRII antagonist, or combination of antagonists, of the disclosure binds to at least GDF8 with a K_{D} of at least 1 × 10⁻⁷ M (e.g., at least 1 × 10⁻⁸ M, at least 1 × 10⁻⁹ M, at least 1 × 10⁻¹⁰ M, at least 1 × 10⁻¹¹ M, or at least 1 × 10⁻¹² M).

In certain aspects, an ActRII antagonist, or combination of antagonists, to be used in accordance with methods and uses described herein is an agent that inhibits at least activin (e.g. activin A, activin B, activin C, activin E, activin AB, and activin AE). Effects on activin inhibition may be determined, for example, using a cell-based assay including those described herein (e.g., Smad signaling reporter assay). Therefore, in some embodiments, an activin antagonist, or combination of antagonist, of the disclosure may bind to at least activin. Ligand binding activity may be determined, for example, using a binding affinity assay including, for example, those described herein. In some embodiments, an ActRII antagonist, or combination of antagonists, of the disclosure binds to at least activin A, activin B, activin AB, activin C, and/or activin E with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M). In some embodiments, an ActRII antagonist, or combination of antagonists, of the disclosure binds to at least activin A with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M).

In certain aspects, an ActRII antagonist, or combination of antagonists, to be used in accordance with methods and uses described herein is an agent that inhibits at least BMP6. Effects on BMP6 inhibition may be determined, for example, using a cell-based assay including those described herein (e.g., Smad signaling reporter assay). Therefore, in some embodiments, a BMP6 antagonist, or combination of antagonist, of the disclosure may bind to at least BMP6. Ligand binding activity may be determined, for example, using a binding affinity assay including, for example, those described herein. In some embodiments, an ActRII antagonist, or combination of antagonists, of the disclosure binds to at least BMP6 with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M).

In certain aspects, an ActRII antagonist, or combination of antagonists, to be used in accordance with methods and uses described herein is an agent that inhibits at least GDF3. Effects on GDF3 inhibition may be determined, for example, using a cell-based assay including those described herein (e.g., Smad signaling reporter assay). Therefore, in some embodiments, a GDF3 antagonist, or combination of antagonist, of the disclosure may bind to at least GDF3. Ligand binding activity may be determined, for example, using a binding affinity assay including, for example, those described herein. In some embodiments, an ActRII antagonist, or combination of antagonists, of the disclosure binds to at least GDF3 with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M).

In certain aspects, an ActRII antagonist, or combination of antagonists, to be used in accordance with methods and uses described herein is an agent that inhibits at least BMP9. Effects on BMP9 inhibition may be determined, for example, using a cell-based assay including those described herein (e.g., Smad signaling reporter assay). Therefore, in some embodiments, a BMP9 antagonist, or combination of antagonist, of the disclosure may bind to at least BMP9. Ligand binding activity may be determined, for example, using a binding affinity assay including, for example, those described herein. In some embodiments, an ActRII antagonist, or combination of antagonists, of the disclosure binds to at least BMP9 with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M).

In certain aspects, an ActRII antagonist, or combination of antagonists, to be used in accordance with methods and uses described herein is an agent that inhibits at least BMP10. Effects on BMP10 inhibition may be determined, for example, using a cell-based assay including those described herein (e.g., Smad signaling reporter assay). Therefore, in some embodiments, a BMP 10 antagonist, or combination of antagonist, of the disclosure may bind to at least BMP10. Ligand binding activity may be determined, for example, using a binding affinity assay including, for example, those described herein. In some embodiments, an ActRII antagonist, or combination of antagonists, of the disclosure binds to at least BMP10 with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M).

In certain aspects, an ActRII antagonist, or combination of antagonists, to be used in accordance with methods and uses described herein is an agent that inhibits at least ActRIIA. Effects on ActRIIA inhibition may be determined, for example, using a cell-based assay including those described herein (e.g., Smad signaling reporter assay). Therefore, in some embodiments, an ActRII antagonist, or combination of antagonist, of the disclosure may bind to at least ActRIIA. Ligand binding activity may be determined, for example, using a binding affinity assay including, for example, those described herein. In some embodiments, an ActRII antagonist, or combination of antagonists, of the disclosure binds to at least ActRIIA with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M). In some embodiments, an ActRII antagonist that binds to and/or inhibits ActRIIA may further bind to and/or inhibit ActRIIB.

In certain aspects, an ActRII antagonist, or combination of antagonists, to be used in accordance with methods and uses described herein is an agent that inhibits at least ActRIIB. Effects on ActRIIB inhibition may be determined, for example, using a cell-based assay including those described herein (e.g., Smad signaling reporter assay). Therefore, in some embodiments, an ActRII antagonist, or combination of antagonist, of the disclosure may bind to at least ActRIIB. Ligand binding activity may be determined, for example, using a binding affinity assay including, for example, those described herein. In some embodiments, an ActRII antagonist, or combination of antagonists, of the disclosure binds to at least ActRIIB with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M). In some embodiments, an ActRII antagonist that binds to and/or inhibits ActRIIB may further bind to and/or inhibit ActRIIA.

In certain aspects, an ActRII antagonist, or combination of antagonists, to be used in accordance with methods and uses described herein is an agent that inhibits at least ALK4. Effects on ALK4 inhibition may be determined, for example, using a cell-based assay including those described herein (e.g., Smad signaling reporter assay). Therefore, in some embodiments, an ActRII antagonist, or combination of antagonist, of the disclosure may bind to at least ALK4. Ligand binding activity may be determined, for example, using a binding affinity assay including, for example, those described herein. In some embodiments, an ActRII antagonist, or combination of antagonists, of the disclosure binds to at least ALK4 with a K_{D} of at least 1 x 10⁻⁷ M (e.g., at least 1 x 10⁻⁸ M, at least 1 x 10⁻⁹ M, at least 1 x 10⁻¹⁰ M, at least 1 x 10⁻¹¹ M, or at least 1 x 10⁻¹² M).

In certain aspects, the disclosure relates to compositions comprising an ActRII polypeptide and uses thereof. The term "ActRII polypeptide" collectively refers to naturally occurring ActRIIA and ActRIIB polypeptides as well as truncations and variants thereof such as those described herein (e.g., GDF trap polypeptides). Preferably ActRII polypeptides comprise, consist essentially of, or consist of a ligand-binding domain of an ActRII polypeptide or modified (variant) form thereof. For example, in some embodiments, an ActRIIA polypeptide comprises an ActRIIA ligand-binding domain of an ActRIIA polypeptide, for example, a portion of the ActRIIA extracellular domain. Similarly, an ActRIIB polypeptide may comprise of an ActRIIB ligand-binding domain of an ActRIIB polypeptide, for example, a portion of the ActRIIB extracellular domain. Preferably, ActRII polypeptides to be used in accordance with the methods described herein are soluble polypeptides.

In certain aspects, the disclosure relates an ActRIIA polypeptide, compositions comprising the same, and uses thereof. For example, in some embodiments, an ActRIIA polypeptide of the disclosure comprises an amino acid sequence that is at least 70%, 75%, 80%, 85%, 90%, 91%, 92%, 93%, 94%, 95%, 96%, 97%, 98%, 99%, or 100% identical to the sequence of amino acids 30-110 of SEQ ID NO: 9. In other embodiments, an ActRIIA polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 9. In other embodiments, an ActRIIA polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 10. In other embodiments, an ActRIIA polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 11. In other embodiments, an ActRIIA polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 50. In still even other embodiments, an ActRIIA polypeptide comprising an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 54. In other embodiments, an ActRIIA polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 57.

In other aspects, the disclosure relates an ActRIIB polypeptide, compositions comprising the same, and uses thereof. For example, in some embodiments, an ActRIIB polypeptide of the disclosure comprises an amino acid sequence that is at least 70%, 75%, 80%, 85%, 90%, 91%, 92%, 93%, 94%, 95%, 96%, 97%, 98%, 99%, or 100% identical to the sequence of amino acids 29-109 of SEQ ID NO: 1. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the sequence of amino acids 29-109 of SEQ ID NO: 1, wherein the ActRIIB polypeptide does not comprise an acidic amino acid [naturally occurring (E or D) or artificial acidic amino acid] at position 79 with respect to SEQ ID NO: 1. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the sequence of amino acids 25-131 of SEQ ID NO: 1. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the sequence of amino acids 25-131 of SEQ ID NO: 1, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 1. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 1, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 2. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 2, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 3. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 3, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 4. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 4, wherein the ActRIIB polypeptide does not comprise an acidic amino acid at position 79 with respect to SEQ ID NO: 4. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 5. In some embodiments, an ActRIIB polypeptide may comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 5, wherein the ActRIIB polypeptide does not comprise an acidic amino acid at position 79 with respect to SEQ ID NO: 4. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 6. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 6, wherein the ActRIIB polypeptide does not comprise an acidic amino acid at position 79 with respect to SEQ ID NO: 4. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 58. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 58, wherein the ActRIIB polypeptide does not comprise an acidic amino acid at position 79 with respect to SEQ ID NO: 1. In other embodiments, an ActRIIB polypeptide comprises of an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 60. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 60, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 63. In some embodiments, an ActRIIB polypeptide comprises of an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 63, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 64. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 64, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 65. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 65, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 66. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 66, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 68. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 68, wherein the ActRIIB polypeptide does not comprise an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 69. In other embodiments, an ActRIIB polypeptide may comprise, consist essentially of, or consist of an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 69, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 70. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 70, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 123. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 123, wherein the ActRIIB polypeptide does not comprise an acidic amino acid at position 79 with respect to SEQ ID NO: 1. In still even other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 131. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 131, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 132. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 132, wherein the ActRIIB polypeptide comprises an acidic amino acid at position 79 with respect to SEQ ID NO: 1. In other embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 133. In some embodiments, an ActRIIB polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 133, wherein the ActRIIB polypeptide comprises an acidic amino acid at position 79 with respect to SEQ ID NO: 1.

In other aspects, the present disclosure relates to compositions comprising a GDF trap polypeptide and uses thereof. In some embodiments, a GDF trap comprises of an altered ActRII ligand-binding domain has a ratio of K_{d} for activin A binding to K_{d} for GDF11 and/or GDF8 binding that is at least 2-, 5-, 10-, 20, 50-, 100-, or even 1000-fold greater relative to the ratio for the wild-type ligand-binding domain. Optionally, the GDF trap comprising an altered ligand-binding domain has a ratio of IC₅₀ for inhibiting activin A to IC₅₀ for inhibiting GDF11 and/or GDF8 that is at least 2-, 5-, 10-, 20-, 25- 50-, 100-, or even 1000-fold greater relative to the wild-type ActRII ligand-binding domain. Optionally, the GDF trap comprising an altered ligand-binding domain inhibits GDF11 and/or GDF8 with an IC₅₀ at least 2, 5, 10, 20, 50, or even 100 times less than the IC₅₀ for inhibiting activin A. These GDF traps can be fusion proteins that include an immunoglobulin Fc domain (either wild-type or mutant). In certain cases, the subject soluble GDF traps are antagonists (inhibitors) of GDF8 and/or GDF11-mediated intracellular signaling (e.g., Smad 2/3 signaling).

In some embodiments, the disclosure provides GDF traps which are soluble ActRIIB polypeptides comprising an altered ligand-binding (e.g., GDF 11-binding) domain. GDF traps with altered ligand-binding domains may comprise, for example, one or more mutations at amino acid residues such as E37, E39, R40, K55, R56, Y60, A64, K74, W78, L79, D80, F82 and F101 of human ActRIIB (numbering is relative to SEQ ID NO: 1). Optionally, the altered ligand-binding domain can have increased selectivity for a ligand such as GDF8/GDF11 relative to a wild-type ligand-binding domain of an ActRIIB receptor. To illustrate, these mutations are demonstrated herein to increase the selectivity of the altered ligand-binding domain for GDF11 (and therefore, presumably, GDF8) over activin: K74Y, K74F, K74I, L79D, L79E, and D80I. The following mutations have the reverse effect, increasing the ratio of activin binding over GDF11: D54A, K55A, L79A and F82A. The overall (GDF11 and activin) binding activity can be increased by inclusion of the "tail" region or, presumably, an unstructured linker region, and also by use of a K74A mutation. Other mutations that caused an overall decrease in ligand binding affinity, include: R40A, E37A, R56A, W78A, D80K, D80R, D80A, D80G, D80F, D80M and D80N. Mutations may be combined to achieve desired effects. For example, many of the mutations that affect the ratio of GDF11 :activin binding have an overall negative effect on ligand binding, and therefore, these may be combined with mutations that generally increase ligand binding to produce an improved binding protein with ligand selectivity. In an exemplary embodiment, a GDF trap is an ActRIIB polypeptide comprising an L79D or L79E mutation, optionally in combination with additional amino acid substitutions, additions, or deletions.

In some embodiments, TGFβ antagonists of the disclosure are agents that can inhibit TGFβRII, ALK5, and/or betaglycan, particularly inhibiting downstream signaling. Therefore, in some embodiments, TGFβ antagonists of the disclosure are agents that can inhibit one or more TGFβRII, ALK5, and/or betaglycan-binding ligands [e.g., TGFβ1, TGFβ2 and/or TGFβ3]. In some embodiments, TGFβ antagonists of the disclosure are agents that can inhibit one or more intracellular mediators of the TGFβ and/or ALK5 signaling pathway (e.g., Smads). Such TGFβ antagonist agents include, for example, TGFβRII polypeptides as well as variants thereof; an antibody, or combination of antibodies, that inhibit one or more TGFβ ligands, ALK5 receptor, betaglycan, and/or TGFβRII receptor; a polynucleotide, or combination of polynucleotides, that inhibits of one or more TGFβ ligands, ALK5, betaglycan, TGFβRII receptor, and/or TGFβRII and/or ALK5 downstream signaling component (e.g., Smads); a small molecule, or combination of small molecules, that inhibits of one or more TGFβ ligands, ALK5, betaglycan, TGFβRII receptor, and/or TGFβRII and/or ALK5 downstream signaling component (e.g., Smads), as well as combinations thereof. In some embodiments, TGFβ antagonists bind to and/or inhibit TGFβ1. In some embodiments, TGFβ antagonists bind to and/or inhibit TGFβ2. In some embodiments, TGFβ antagonists bind to and/or inhibit TGFβ3. In some embodiments, TGFβ antagonists bind to and/or inhibit TGFβ1 and TGFβ3. In some embodiments, TGFβ antagonists bind to and/or inhibit TGFβ1 and TGFβ2. In some embodiments, TGFβ antagonists bind to and/or inhibit TGFβ2 and TGFβ3. In some embodiments, TGFβ antagonists bind to and/or inhibit TGFβ1, TGFβ2 and TGFβ3.

In certain aspects, the disclosure relates a TGFβRII polypeptide, compositions comprising the same, and uses thereof. For example, in some embodiments, a TGFβRII polypeptide of the disclosure comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 34. In other embodiments, a TGFβRII polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 10. In other embodiments, a TGFβRII polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 35. In other embodiments, a TGFβRII polypeptide comprises an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 148. In still even other embodiments, a TGFβRII polypeptide comprising an amino acid sequence that is at least 70%, 75%, 80%, 85%, 90%, 91%, 92%, 93%, 94%, 95%, 96%, 97%, 98%, 99%, or 100% identical to the amino acid sequence of SEQ ID NO: 150.

In some embodiments, an agent to be used in accordance with the methods and uses described herein is both an ActRII and TGFβ antagonist (ActRII:TGFβ). An ActRII:TGFβ antagonist is an agent that can inhibit one or more of ActRIIA, ActRIIB, ALK4, ActRII- and ALK4-binding ligands [e.g., GDF11, GDF8, activin (activin A, activin B, activin AB, activin C, activin E) BMP6, GDF3, BMP10, and/or BMP9], and downstream signaling mediators (e.g., Smads) as well as inhibit one or more of TGFβRII; ALK5; betaglycan; TGFβRII-, ALK5-, and betaglycan-binding ligands [e.g., TGFβ1, TGFβ2 and/or TGFβ3], and downstream signaling mediators (e.g., Smads). For example, an ActRII:TGFβ antagonist may be a bi-specific antibody that binds to and inhibits both activin A and TGFβ2. As another example, an ActRII:TGFβ antagonist may be a polynucleotide or small molecule that inhibits one or more Smads, particularly Smads 2 and 3.

As described herein, ActRII polypeptides, TGFβRII polypeptides and variants thereof be homomultimers, for example, homodimer, homotrimers, homotetramers, homopentamers, and higher order homomultimer complexes. In certain preferred embodiments, ActRII polypeptides and TGFβRII polypeptides are homodimers. In certain embodiments, ActRII polypeptide and TGFβRII polypeptides dimers described herein comprise an first ActRII or TGFβRII polypeptide covalently, or non-covalently, associated with an second ActRII or TGFβRII polypeptide wherein the first polypeptide comprises an ActRII domain or TGFβRII domain and an amino acid sequence of a first member (or second member) of an interaction pair (e.g., a constant domain of an immunoglobulin) and the second polypeptide comprises an ActRII polypeptide or TGFβRII polypeptide and an amino acid sequence of a second member (or first member) of the interaction pair.

In certain aspects, ActRII polypeptides and TGFβRII polypeptides, including variants thereof, may be fusion proteins. For example, in some embodiments, an ActRII polypeptide of TGFβRII polypeptide may be a fusion protein comprising an ActRII polypeptide domain or TGFβRII polypeptide domain and one or more heterologous (non-ActRII or non-TGFβRII) polypeptide domains. In some embodiments, an ActRII polypeptide of TGFβRII polypeptide may be a fusion protein that has, as one domain, an amino acid sequence derived from an ActRII polypeptide or TGFβRII polypeptide (e.g., a ligand-binding domain of an ActRII receptor, TGFβRII receptor or a variant thereof) and one or more heterologous domains that provide a desirable property, such as improved pharmacokinetics, easier purification, targeting to particular tissues, etc. For example, a domain of a fusion protein may enhance one or more of in vivo stability, in vivo half-life, uptake/administration, tissue localization or distribution, formation of protein complexes, multimerization of the fusion protein, and/or purification. Optionally, an ActRII polypeptide domain of TGFβRII polypeptide domain of a fusion protein is connected directly (fused) to one or more heterologous polypeptide domains, or an intervening sequence, such as a linker, may be positioned between the amino acid sequence of the ActRII polypeptide of TGFβRII polypeptide and the amino acid sequence of the one or more heterologous domains. In certain embodiments, an ActRII or TGFβRII fusion protein comprises a relatively unstructured linker positioned between the heterologous domain and the ActRII domain of TGFβRII domain. The linker may correspond to the roughly 4-15 amino acid unstructured region at the C-terminal end of the extracellular domain of ActRIIA or ActRIIB (the "tail"), or it may be an artificial sequence of between 3 and 15, 20, 30, 50 or more amino acids that are relatively free of secondary structure. A linker may be rich in glycine and proline residues and may, for example, contain repeating sequences of threonine/serine and glycines. Examples of linkers include, but are not limited to, the sequences TGGG (SEQ ID NO: 31), SGGG (SEQ ID NO: 32), TGGGG (SEQ ID NO: 29), SGGGG (SEQ ID NO: 30), GGGGS (SEQ ID NO: 33), GGGG (SEQ ID NO: 28), and GGG (SEQ ID NO: 27). In some embodiments, ActRII or TGFβRII fusion proteins may comprise a constant domain of an immunoglobulin, including, for example, the Fc portion of an immunoglobulin. For example, an amino acid sequence that is derived from an Fc domain of an IgG (IgG1, IgG2, IgG3, or IgG4), IgA (IgA1 or IgA2), IgE, or IgM immunoglobulin. For example, am Fc portion of an immunoglobulin domain may comprise, consist essentially of, or consist of an amino acid sequence that is at least 70%, 75%, 80%, 85%, 90%, 91%, 92%, 93%, 94%, 95%, 96%, 97%, 98%, 99% or 100% identical to any one of SEQ ID NOs: 22-26. Such immunoglobulin domains may comprise one or more amino acid modifications (e.g., deletions, additions, and/or substitutions) that confer an altered Fc activity, e.g., decrease of one or more Fc effector functions. In some embodiment, an ActRII of TGFβRII fusion protein comprises an amino acid sequence as set forth in the formula A-B-C. For example, the B portion is an N- and C-terminally truncated ActRII polypeptide or TGFβRII polypeptide as described herein. The A and C portions may be independently zero, one, or more than one amino acids, and both A and C portions are heterologous to B. The A and/or C portions may be attached to the B portion via a linker sequence. In certain embodiments, an ActRII of TGFβRII fusion protein comprises a leader sequence. The leader sequence may be a native ActRII or TGFβRII leader sequence (e.g., a native ActRIIA, ActRIIB, or TGFβRII leader sequence) or a heterologous leader sequence. In certain embodiments, the leader sequence is a tissue plasminogen activator (TPA) leader sequence.

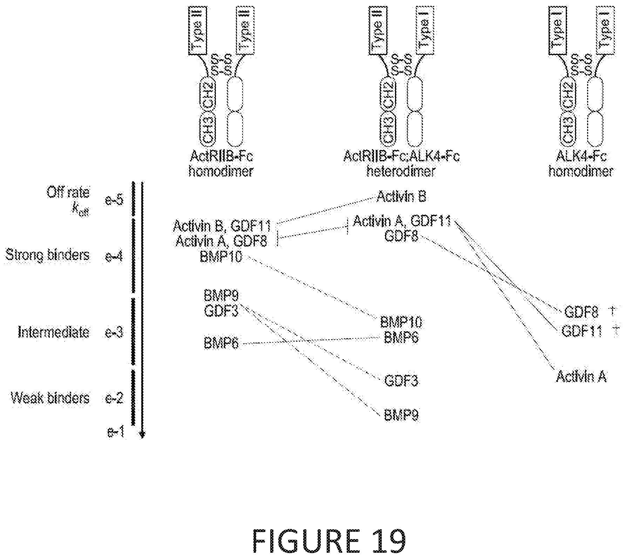

As described herein, it has been discovered that an ALK4:ActRIIB heterodimer protein complex has a different ligand-binding profile/selectivity compared to corresponding ActRIIB and ALK4 homodimers. In particular, ALK4:ActRIIB heterodimer displays enhanced binding to activin B compared to either homodimer, retains strong binding to activin A, GDF8, and GDF11 as observed with ActRIIB homodimer, and exhibits substantially reduced binding to BMP9, BMP10, and GDF3. In particular, BMP9 displays low to no observable affinity for ALK4:ActRIIB heterodimer, whereas this ligand binds strongly to ActRIIB homodimer. Like ActRIIB homodimer, ALK4:ActRIIB heterodimer retains intermediate-level binding to BMP6. See Figure 19. These results therefore demonstrate that ALK4: ActRIIB heterodimers are a more selective antagonists (inhibitors) of activin A, activin B, GDF8, and GDF11 compared to ActRIIB homodimers. Accordingly, an ALK4:ActRIIB heterodimer will be more useful than an ActRIIB homodimer in certain applications where such selective antagonism is advantageous. Examples include therapeutic applications where it is desirable to retain antagonism of one or more of activin (e.g., activin A, activin B, activin AB, activin AC), GDF8, and GDF11 but minimize antagonism of one or more of BMP9, BMP10, and GDF3. Moreover, an ALK4:ActRIIB heterodimer has been shown treat cancer in patient. Accordingly the present disclosure relates, in part, to ALK4:ActRIIB heterodimers and uses thereof, particularly in combination with a TGFβ antagonist. While not wishing to be bound to a particular mechanisms of action, it is expected that ALK4:ActRIIB heteromultimers, as well as variants thereof, that bind to at least one or more of activin (e.g., activin A, activin B, activin AB, and activin AC), GDF8, and/or GDF 11 will be useful agents for promoting beneficial effects in cancer patients.

Therefore, the present disclosure provides heteromultimer complexes (heteromultimers) comprising at least one ALK4 polypeptide and at least one ActRIIB polypeptide (ALK4:ActRIIB heteromultimers) as well as uses thereof. Preferably, ALK4 polypeptides comprise a ligand-binding domain of an ALK4 receptor, for example, a portion of the ALK4 extracellular domain. Similarly, ActRIIB polypeptides generally comprise a ligand-binding domain of an ActRIIB receptor, for example, a portion of the ActRIIB extracellular domain. Preferably, such ALK4 and ActRIIB polypeptides, as well as resultant heteromultimers thereof, are soluble.

In certain aspects, an ALK4:ActRIIB heteromultimer comprises an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to amino acids 34-101 of SEQ ID NO: 14. In other embodiments, ALK4:ActRIIB heteromultimers comprises an ALK4 amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 15. In other embodiments, ALK4:ActRIIB heteromultimers comprises an ALK4 amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 19. In other embodiments, ALK4:ActRIIB heteromultimers comprises an ALK4 amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 143. In other embodiments, ALK4:ActRIIB heteromultimers comprise an ALK4 amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 145. In other embodiments, ALK4:ActRIIB heteromultimers comprise an ALK4 amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 79. In still other embodiments, ALK4:ActRIIB heteromultimers comprises an ALK4 amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 80.

In certain aspects, an ALK4: ActRIIB heteromultimer comprises an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to amino acids 29-109 of SEQ ID NO: 1. In other embodiments, ALK4: ActRIIB heteromultimers comprises an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 2. In other embodiments, ALK4:ActRIIB heteromultimers comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 3. In other embodiments, ALK4:ActRIIB heteromultimers comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 5. In other embodiments, ALK4:ActRIIB heteromultimers comprises an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 6. In other embodiments, ALK4:ActRIIB heteromultimers comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 58. In other embodiments, ALK4:ActRIIB heteromultimers comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 60. In other embodiments, ALK4:ActRIIB heteromultimers comprises an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 63. In other embodiments, ALK4:ActRIIB heteromultimers comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 139. In still even other embodiments, ALK4:ActRIIB heteromultimers comprises an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 141 In other embodiments, ALK4:ActRIIB heteromultimers comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 77 In other embodiments, ALK4:ActRIIB heteromultimers may comprise an ActRIIB amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to SEQ ID NO: 78. In certain preferred embodiments, ALK4:ActRIIB heteromultimers do not comprise an ActRIIB polypeptide comprising an acidic amino acid (e.g., an E or D) at the position corresponding to L79 of SEQ ID NO: 1.

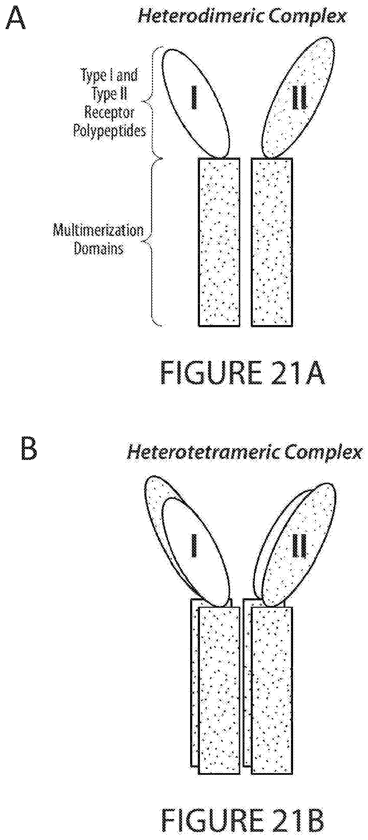

As described herein, ALK4:ActRIIB heteromultimer structures include, for example, heterodimers, heterotrimers, heterotetramers, heteropentamers, and higher order heteromultimer complexes. See, e.g., Figures 21-23. In certain preferred embodiments, ALK4:ActRIIB heteromultimers are heterodimers. In certain aspects, ALK4 and/or ActRIIB polypeptides may be fusion proteins. ALK4 and/or ActRIIB polypeptides generated as fusion proteins similar as described above for ActRII and TGFβRII fusion proteins described herein.

An ActRII, TGFβRII, and/or ALK4 polypeptide, including variants thereof, may comprise a purification subsequence, such as an epitope tag, a FLAG tag, a polyhistidine sequence, and a GST fusion. Optionally, an ActRII, TGFβRII, and/or ALK4 polypeptide includes one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent. ActRII, TGFβRII, and/or ALK4 polypeptides may comprise at least one N-linked sugar, and may include two, three or more N-linked sugars. Such polypeptides may also comprise O-linked sugars. In general, it is preferable that ActRII, TGFβRII, and/or ALK4 polypeptides be expressed in a mammalian cell line that mediates suitably natural glycosylation of the polypeptide so as to diminish the likelihood of an unfavorable immune response in a patient. ActRII, TGFβRII, and/or ALK4 polypeptides may be produced in a variety of cell lines that glycosylate the protein in a manner that is suitable for patient use, including engineered insect or yeast cells, and mammalian cells such as COS cells, CHO cells, HEK cells and NSO cells. In some embodiments, an ActRII, TGFβRII, and/or ALK4 polypeptide is glycosylated and has a glycosylation pattern obtainable from a Chinese hamster ovary cell line. In some embodiments, ActRII, TGFβRII, and/or ALK4 polypeptides of the disclosure exhibit a serum half-life of at least 4, 6, 12, 24, 36, 48, or 72 hours in a mammal (e.g., a mouse or a human). Optionally, ActRII, TGFβRII, and/or ALK4 polypeptides may exhibit a serum half-life of at least 6, 8, 10, 12, 14, 20, 25, or 30 days in a mammal (e.g., a mouse or a human).

In certain aspects, the disclosure provides pharmaceutical preparations comprising one or more ActRII antagonist and/or TGFβ antagonist and a pharmaceutically acceptable carrier. A pharmaceutical preparation may also comprise one or more additional active agents such as a compound that is used to treat or prevent a disorder or condition as described herein [e.g., leukemia, melanoma (e.g., metastatic melanoma), lung cancer (e.g., squamous non-small cell lung cancer, renal cell carcinoma, bladder cancer, mesothelioma (e.g., metastatic mesothelioma), head and neck cancer (e.g., head and neck squamous cell cancer), esophageal cancer, gastric cancer, colorectal cancer (e.g., colorectal carcinoma), liver cancer (e.g., hepatocellular carcinoma), lymphoma, multiple myeloma, myelodysplastic syndrome, breast cancer, ovarian cancer, cervical cancer, glioblastoma multiforme, and sarcoma (e.g., metastatic sarcoma)].

Any of the ActRII antagonists and/or TGFβ antagonists described herein may be formulated as a pharmaceutical preparation (compositions). In some embodiments, pharmaceutical preparations comprise a pharmaceutically acceptable carrier. A pharmaceutical preparation will preferably be pyrogen-free (meaning pyrogen free to the extent required by regulations governing the quality of products for therapeutic use). A pharmaceutical preparation may also include one or more additional compounds such as a compound that is used to treat a disorder/condition described herein. In general, ALK4:ActRIIB heteromultimer pharmaceutical preparations are substantial free of ALK4 and/or ActRIIB homomultimers. For example, in some embodiments, ALK4:ActRIIB heteromultimer pharmaceutical preparations comprise less than about 10%, 9%, 8%, 7%, 6%, 5%, 4%, 3%, 2%, or less than about 1% ALK4 homomultimers. In some embodiments, ALK4:ActRIIB heteromultimer pharmaceutical preparations comprise less than about 10%, 9%, 8%, 7%, 6%, 5%, 4%, 3%, 2%, or less than about 1% ActRIIB homomultimers. In some embodiments, ALK4: ActRIIB heteromultimer pharmaceutical preparations comprise less than about 10%, 9%, 8%, 7%, 6%, 5%, 4%, 3%, 2%, or less than about 1% ALK4 and ActRIIB homomultimers.

In certain aspects, a TGFβ antagonist of the disclosure is an antibody or combination of antibodies. In some embodiments, the TGFβ antagonist antibody binds to TGFβ1. In some embodiments, the TGFβ antagonist antibody binds to TGFβ2. In some embodiments, the TGFβ antagonist antibody binds to TGFβ3. In some embodiments, the TGFβ antagonist is a multi-specific antibody. In some embodiments, the TGFβ antagonist is a bi-specific antibody. In some embodiments, the TGFβ antagonist antibody binds to TGFβ1 and TGFβ2. In some embodiments, the TGFβ antagonist antibody binds to TGFβ1 and TGFβ3. In some embodiments, the TGFβ antagonist antibody binds to TGFβ1 and TGFβ2. In some embodiments, the TGFβ antagonist antibody binds to TGFβ2 and TGFβ3. In some embodiments, the TGFβ antagonist antibody binds to TGFβ1, TGFβ2, and TGFβ3. In some embodiments, the TGFβ antagonist antibody is fresolimumab. In some embodiments, the TGFβ antagonist antibody binds to TGFβRII. In some embodiments, at TGFβ antagonist antibody that binds to TGFβRII further binds to one or more of TGFβ1, TGFβ2, TGFβ3, ALK5, and betaglycan. In some embodiments, the TGFβ antagonist antibody binds to ALK5. In some embodiments, at TGFβ antagonist antibody that binds to ALK5 further binds to one or more of TGFβ1, TGFβ2, TGFβ3, TGFβRII, and betaglycan. In some embodiments, the TGFβ antagonist antibody binds to betaglycan. In some embodiments, at TGFβ antagonist antibody that binds to betaglycan further binds to one or more of TGFβ1, TGFβ2, TGFβ3, TGFβRII, and ALK5. In some embodiments, a TGFβ antagonist antibody of the disclosure is also an ActRII antagonist antibody, particularly in the case of multi-specific antibodies, for example, bi-specific antibodies. Therefore, in some embodiments, an antibody that binds to one or more of TGFβ1, TGFβ2, TGFβ3, TGFβRII, ALK5, and betaglycan and further binds to one or more of ActRIIA, ActRIIB, ALK4, activin A, activin B, GDF11, GDF8, GDF3, BMP6, BMP10, and BMP9. In some embodiments, a multispecific antibody of the disclosure binds to one or more of TGFβ1, TGFβ2, TGFβ3 and activin. In some embodiments, a multispecific antibody of the disclosure binds to TGFβ2 and activin A.

In certain aspects, an ActRII antagonist of the disclosure is an antibody or combination of antibodies. In some embodiments, the ActRII antagonist is a multi-specific antibody. In some embodiments, the ActRII antagonist is a bi-specific antibody. In some embodiments, the ActRII antagonist antibody binds to one or more ligands selected from the group consisting of: activin A, activin B, GDF11, GDF8, GDF3, BMP6, BMP10, and BMP9. In some embodiments, the ActRII antagonist antibody binds activin A. In some embodiments, the ActRII antagonist antibody binds to ActRIIA. In some embodiments, the ActRII antagonist antibody binds to ActRIIB. In some embodiments, the ActRII antagonist antibody binds to ActRIIA and ActRIIB. In some embodiments, the ActRII antagonist antibody binds to ALK4. In some embodiments, an ActRII antagonist antibody that binds to one or more of activin A, activin B, GDF11, GDF8, GDF3, BMP6, BMP10, and BMP9 further binds to one or more of ActRIIA, ActRIIB, and ALK4. In some embodiments, a ActRII antagonist antibody of the disclosure is also an TGFβ antagonist antibody, particularly in the case of multi-specific antibodies, for example, bi-specific antibodies. Therefore, in some embodiments, an antibody that binds to one or more of ActRIIA, ActRIIB, ALK4, activin A, activin B, GDF11, GDF8, GDF3, BMP6, BMP10, and BMP9 further binds to one or more of TGFβ1, TGFβ2, TGFβ3, TGFβRII, ALK5, and betaglycan.

### BRIEF DESCRIPTION OF THE DRAWINGS

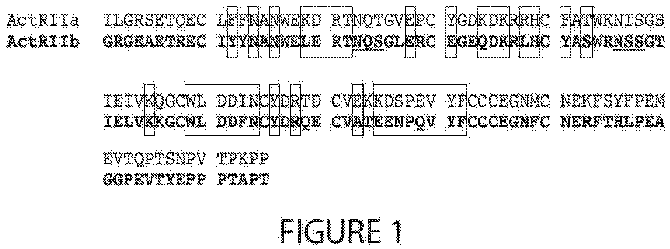

Figure 1 shows an alignment of extracellular domains of human ActRIIB and human ActRIIA with the residues that are deduced herein, based on composite analysis of multiple ActRIIB and ActRIIA crystal structures, to directly contact ligand indicated with boxes.

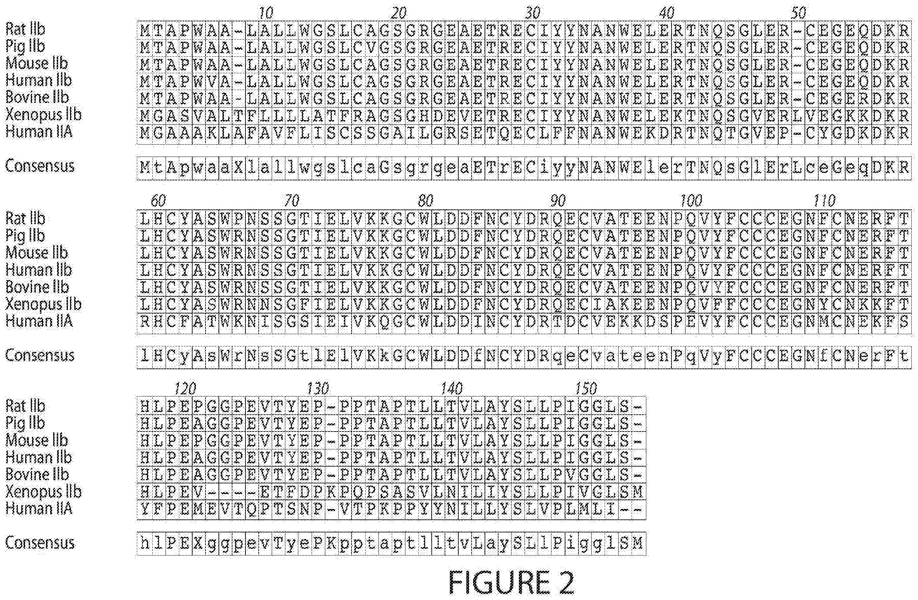

Figure 2 shows a multiple sequence alignment of various vertebrate ActRIIB proteins (SEQ ID NOs: 100-105) and human ActRIIA (SEQ ID NO: 122) as well as a consensus ActRII sequence derived from the alignment (SEQ ID NO: 106).

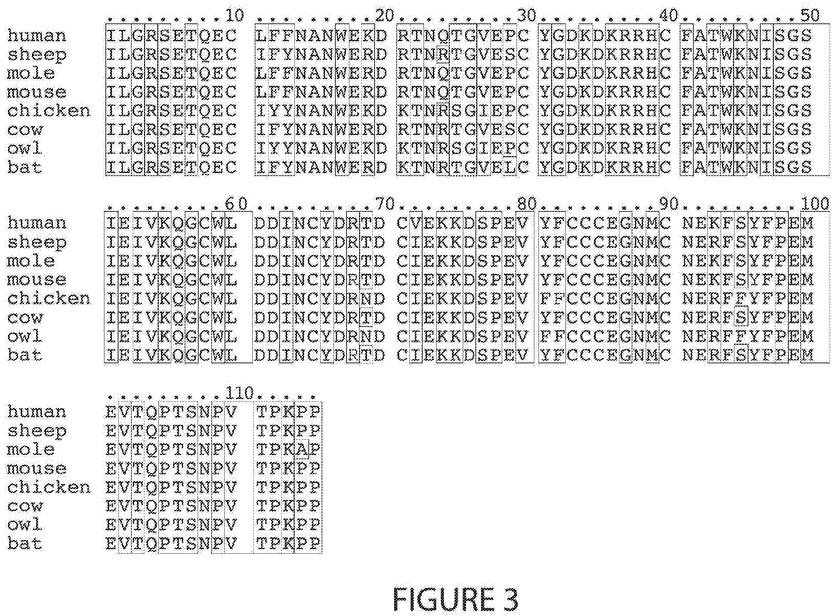

Figure 3 shows a multiple sequence alignment of various vertebrate ActRIIA proteins and human ActRIIA (SEQ ID NOs: 107-114).

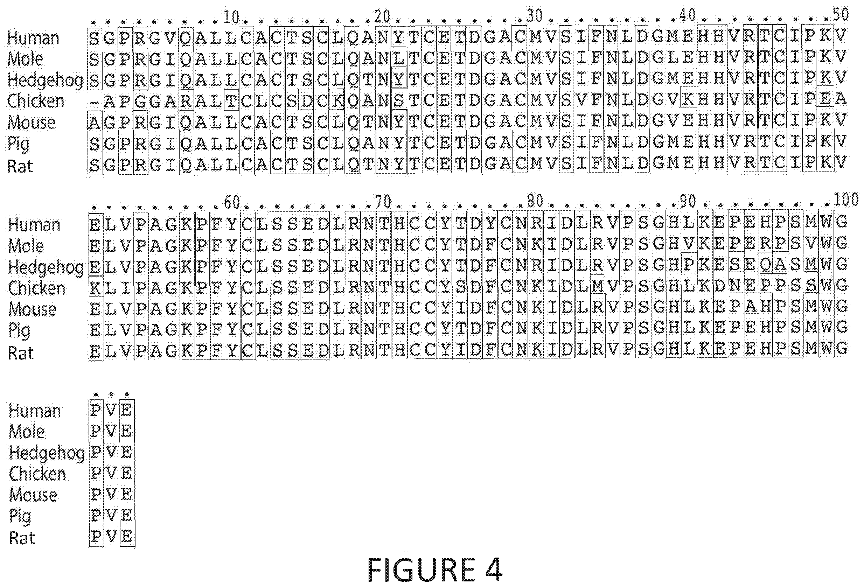

Figure 4 shows a multiple sequence alignment of various vertebrate ALK4 proteins and human ALK4 (SEQ ID NOs: 115-121).

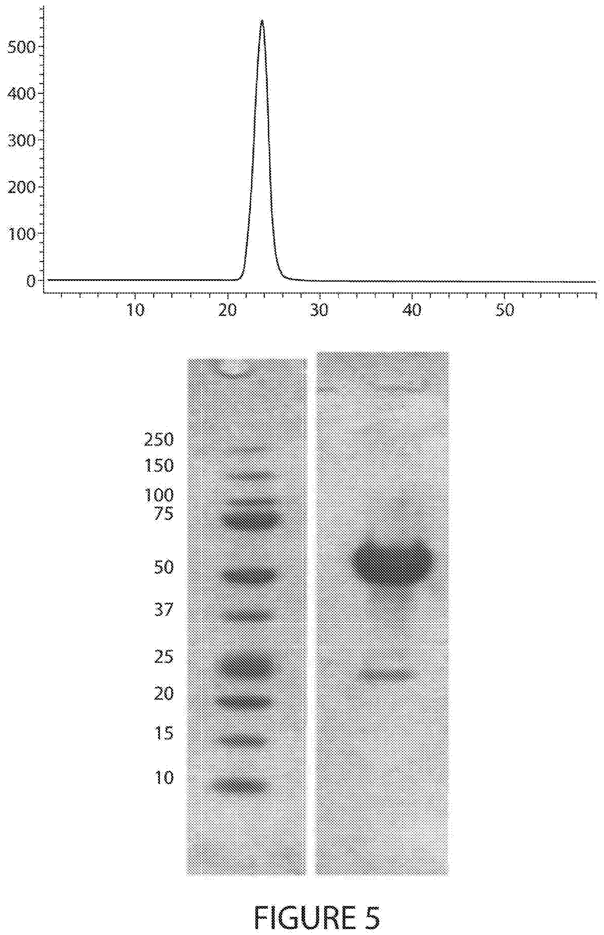

Figure 5 shows the purification of ActRIIA-hFc expressed in CHO cells. The protein purifies as a single, well-defined peak as visualized by sizing column (top panel) and Coomassie stained SDS-PAGE (bottom panel) (left lane: molecular weight standards; right lane: ActRIIA-hFc).

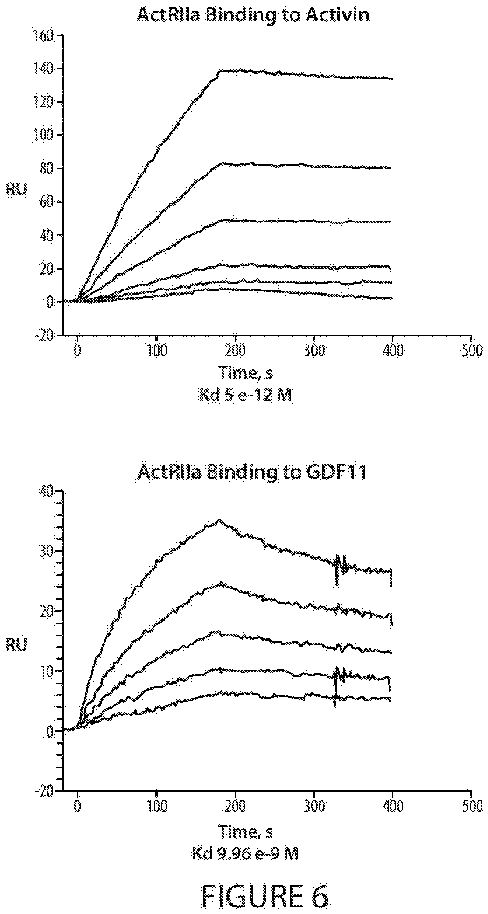

Figure 6 shows the binding of ActRIIA-hFc to activin (top panel) and GDF-11 (bottom panel), as measured by Biacore^{™} assay.

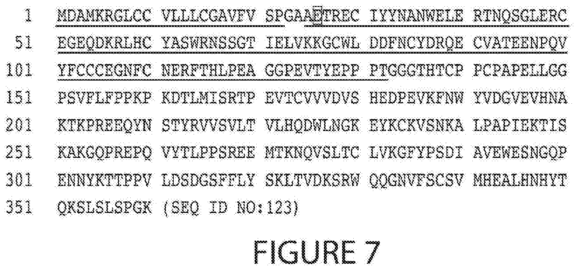

Figure 7 shows the full, unprocessed amino acid sequence for ActRIIB(25-131)-hFc (SEQ ID NO: 123). The TPA leader (residues 1-22) and double-truncated ActRIIB extracellular domain (residues 24-131, using numbering based on the native sequence in SEQ ID NO: 1) are each underlined. Highlighted is the glutamate revealed by sequencing to be the N-terminal amino acid of the mature fusion protein, which is at position 25 relative to SEQ ID NO: 1.

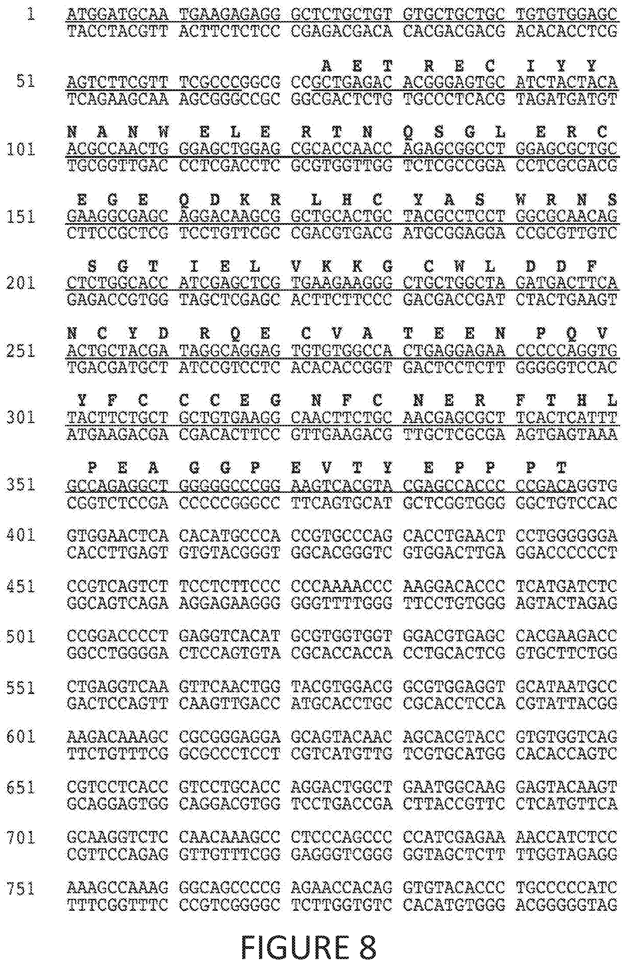

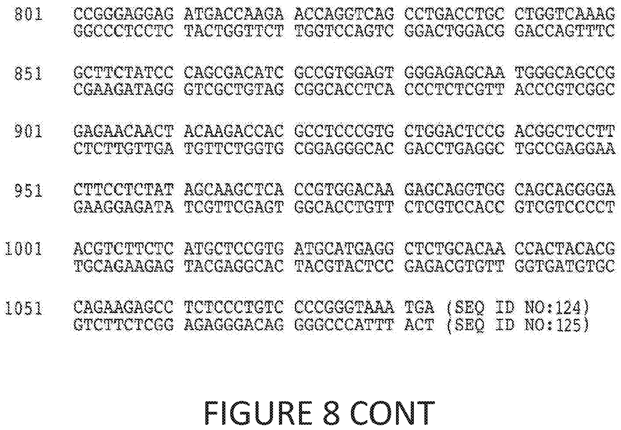

Figure 8 shows a nucleotide sequence encoding ActRIIB(25-131)-hFc (the coding strand is shown at top, SEQ ID NO: 124, and the complement shown at bottom 3'-5', SEQ ID NO: 125). Sequences encoding the TPA leader (nucleotides 1-66) and ActRIIB extracellular domain (nucleotides 73-396) are underlined. The corresponding amino acid sequence for ActRIIB(25-131) is also shown.

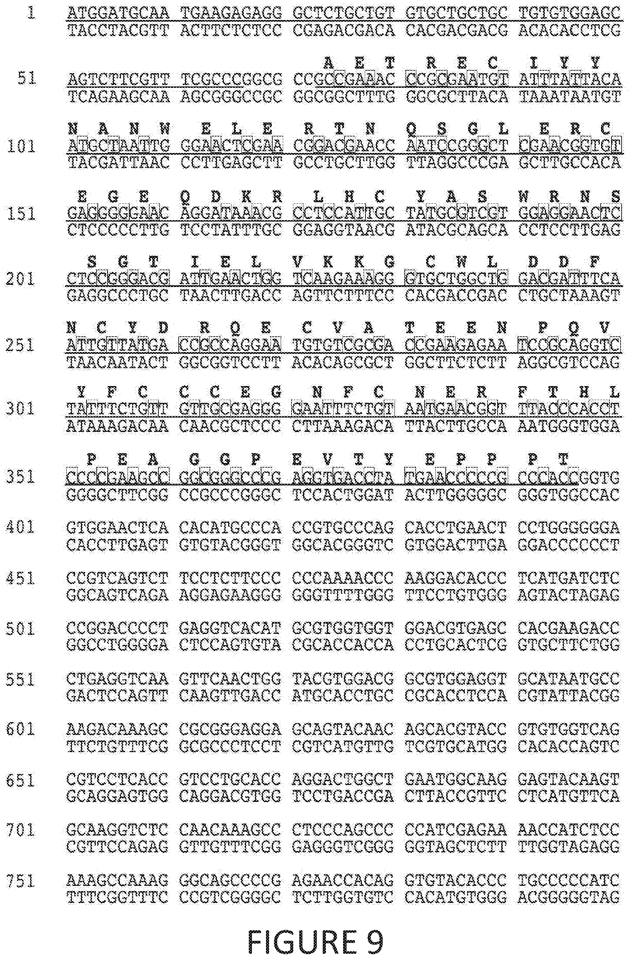

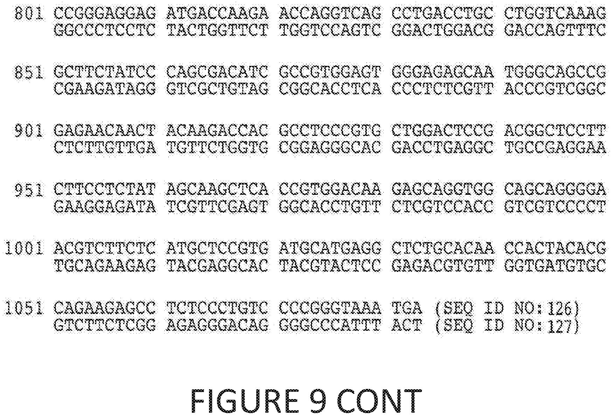

**Figure** 9 shows an alternative nucleotide sequence encoding ActRIIB(25-131)-hFc (the coding strand is shown at top, SEQ ID NO: 126, and the complement shown at bottom 3'-5', SEQ ID NO: 127). This sequence confers a greater level of protein expression in initial transformants, making cell line development a more rapid process. Sequences encoding the TPA leader (nucleotides 1-66) and ActRIIB extracellular domain (nucleotides 73-396) are underlined, and substitutions in the wild type nucleotide sequence of the ECD (see Figure 8) are highlighted. The corresponding amino acid sequence for ActRIIB(25-131) is also shown.

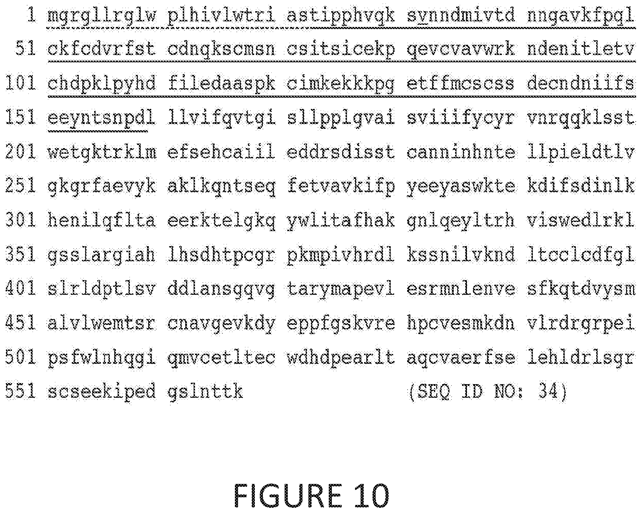

**Figure 10** shows the amino acid sequence of native precursor for the B (short) isoform of human TGFβ receptor type II (hTβRII) (NP_003233.4; SEQ ID NO: 34). Solid underline indicates the mature extracellular domain (ECD) (residues 23-159), and double underline indicates valine that is replaced in the A (long) isoform. Dotted underline denotes leader (residues 1-22).

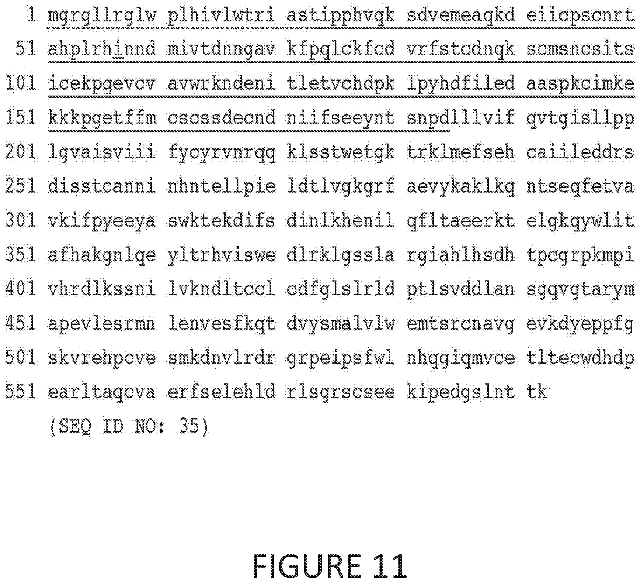

**Figure 11** shows the amino acid sequence of native precursor for the A (long) isoform of human TβRII (NP_001020018.1; SEQ ID NO: 35). Solid underline indicates the mature ECD (residues 23-184), and double underline indicates the splice-generated isoleucine substitution. Dotted underline denotes leader (residues 1-22).

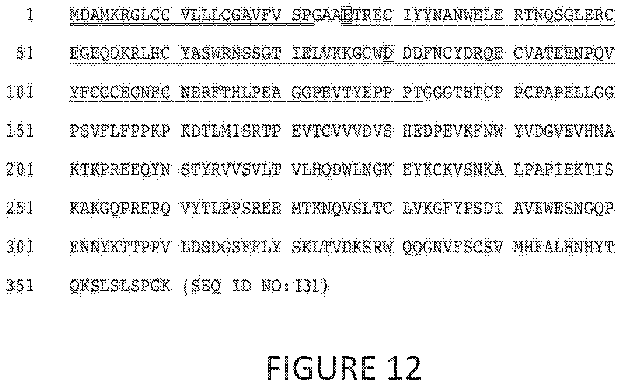

**Figure 12** shows the full amino acid sequence for the truncated GDF trap ActRIIB(L79D 25-131)-hFc (SEQ ID NO: 131), including the TPA leader (double underline), truncated ActRIIB extracellular domain (residues 25-131 in SEQ ID NO:1; single underline), and hFc domain. The aspartate substituted at position 79 in the native sequence is double underlined and highlighted, as is the glutamate revealed by sequencing to be the N-terminal residue in the mature fusion protein.

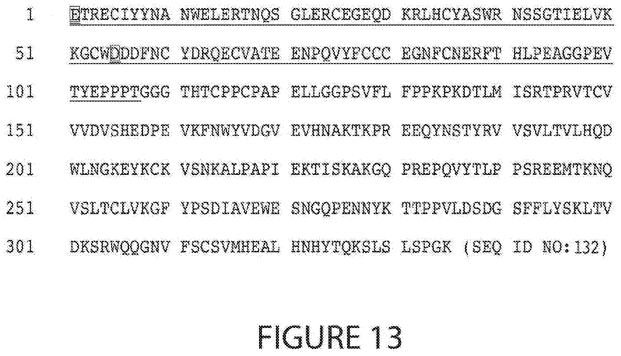

**Figure 13** shows the amino acid sequence for the truncated GDF trap ActRIIB(L79D 25-131)-hFc without a leader (SEQ ID NO: 132). The truncated ActRIIB extracellular domain (residues 25-131 in SEQ ID NO: 1) is underlined. The aspartate substituted at position 79 in the native sequence is double underlined and highlighted, as is the glutamate revealed by sequencing to be the N-terminal residue in the mature fusion protein.

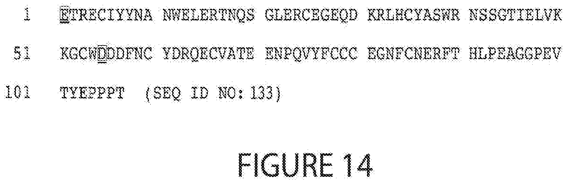

**Figure 14** shows the amino acid sequence for the truncated GDF trap ActRIIB(L79D 25-131) without the leader, hFc domain, and linker (SEQ ID NO: 133). The aspartate substituted at position 79 in the native sequence is underlined and highlighted, as is the glutamate revealed by sequencing to be the N-terminal residue in the mature fusion protein.

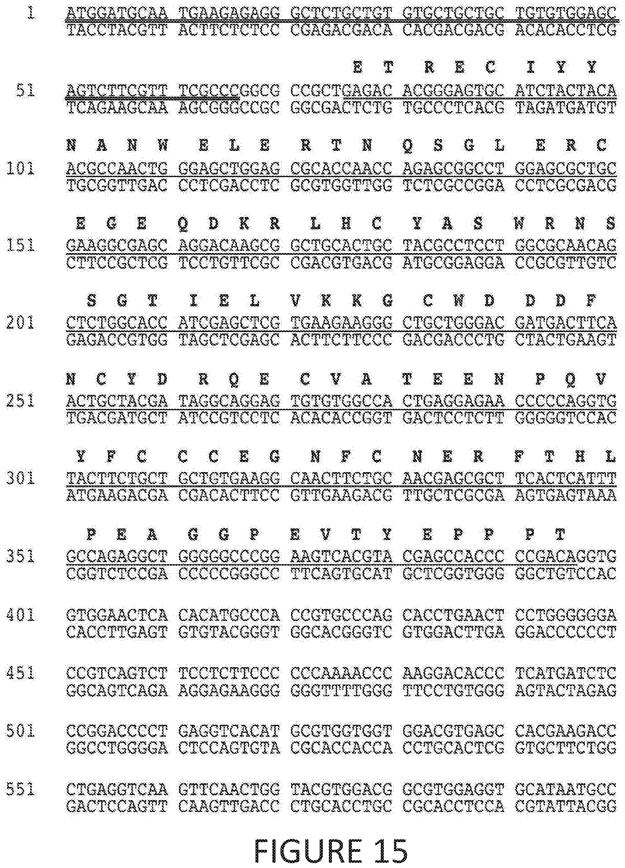

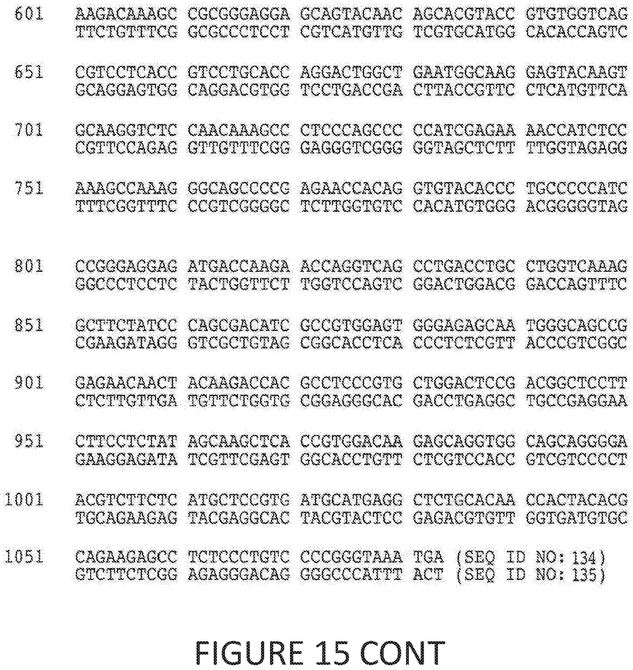

**Figure 15** shows a nucleotide sequence encoding ActRIIB(L79D 25-131)-hFc. SEQ ID NO: 134 corresponds to the sense strand, and SEQ ID NO: 135 corresponds to the antisense strand. The TPA leader (nucleotides 1-66) is double underlined, and the truncated ActRIIB extracellular domain (nucleotides 76-396) is single underlined. The amino acid sequence for the ActRIIB extracellular domain (residues 25-131 in SEQ ID NO: 1) is also shown.

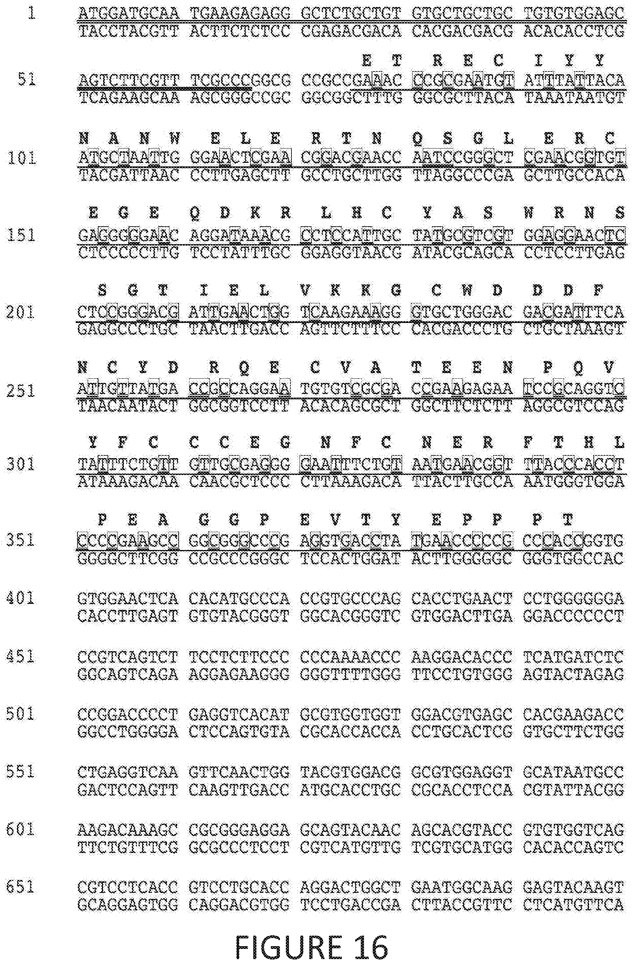

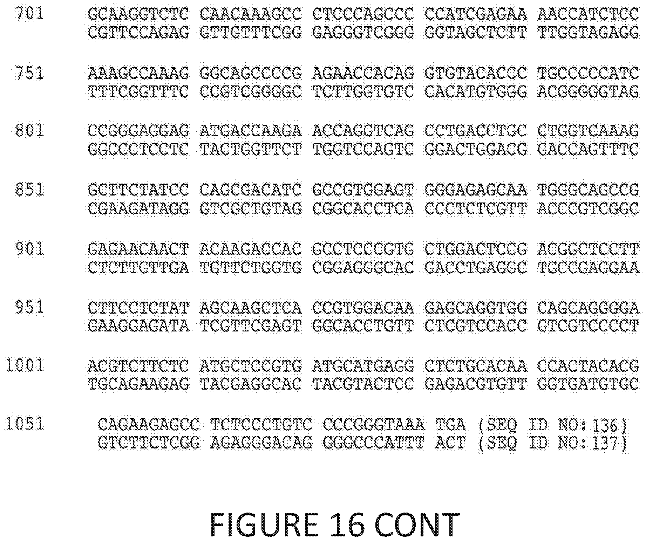

**Figure 16** shows an alternative nucleotide sequence encoding ActRIIB(L79D 25-131)-hFc. SEQ ID NO: 136 corresponds to the sense strand, and SEQ ID NO: 137 corresponds to the antisense strand. The TPA leader (nucleotides 1-66) is double underlined, the truncated ActRIIB extracellular domain (nucleotides 76-396) is underlined, and substitutions in the wild-type nucleotide sequence of the extracellular domain are double underlined and highlighted. The amino acid sequence for the ActRIIB extracellular domain (residues 25-131 in SEQ ID NO: 1) is also shown.

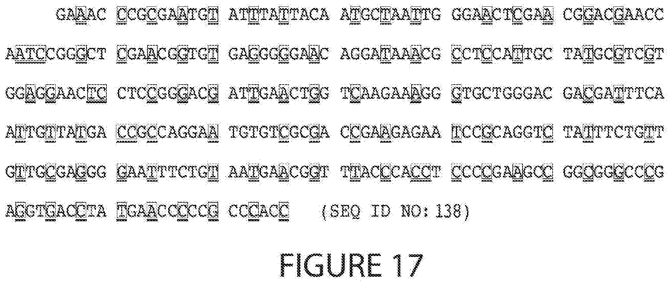

**Figure 17** shows nucleotides 76-396 (SEQ ID NO: 138) of the alternative nucleotide sequence shown in Figure 16 (SEQ ID NO: 136). The same nucleotide substitutions indicated in Figure 16 are also underlined and highlighted here. SEQ ID NO: 138 encodes only the truncated ActRIIB extracellular domain (corresponding to residues 25-131 in SEQ ID NO: 1) with a L79D substitution, e.g., ActRIIB(L79D 25-131).

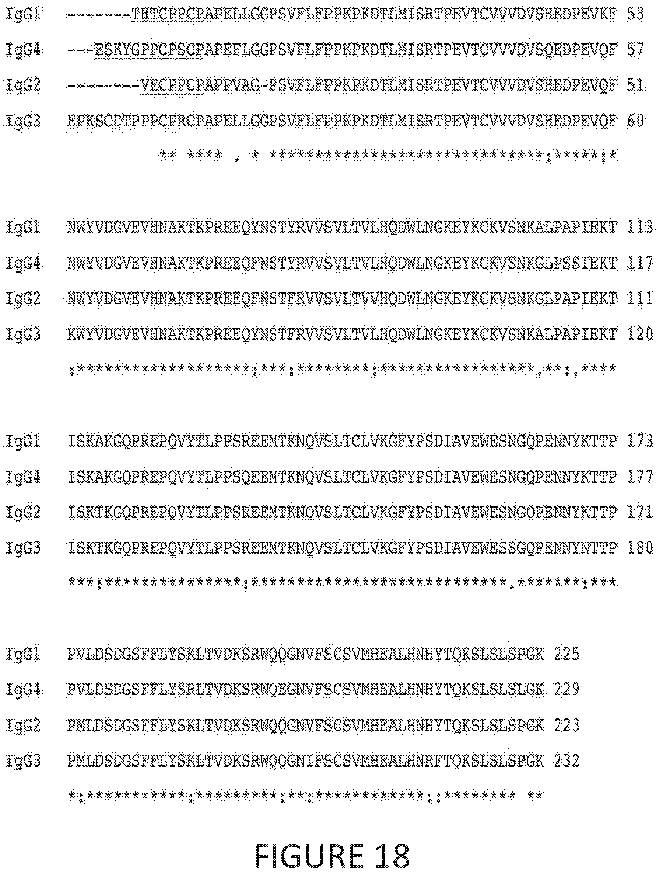

**Figure 18** shows multiple sequence alignment of Fc domains from human IgG isotypes using Clustal 2.1. Hinge regions are indicated by dotted underline. Double underline indicates examples of positions engineered in IgG1 Fc to promote asymmetric chain pairing and the corresponding positions with respect to other isotypes IgG2, IgG3 and IgG4.
**Figure 19** shows comparative ligand binding data for an ALK4-Fc:ActRIIB-Fc heterodimeric protein complex compared to ActRIIB-Fc homodimer and ALK4-Fc homodimer. For each protein complex, ligands are ranked by k_{off}, a kinetic constant that correlates well with ligand signaling inhibition, and listed in descending order of binding affinity (ligands bound most tightly are listed at the top). At left, yellow, red, green, and blue lines indicate magnitude of the off-rate constant. Solid black lines indicate ligands whose binding to heterodimer is enhanced or unchanged compared with homodimer, whereas dashed red lines indicate substantially reduced binding compared with homodimer. As shown, the ALK4-Fc:ActRIIB-Fc heterodimer displays enhanced binding to activin B compared with either homodimer, retains strong binding to activin A, GDF8, and GDF11 as observed with ActRIIB-Fc homodimer, and exhibits substantially reduced binding to BMP9, BMP10, and GDF3. Like ActRIIB-Fc homodimer, the heterodimer retains intermediate-level binding to BMP6.

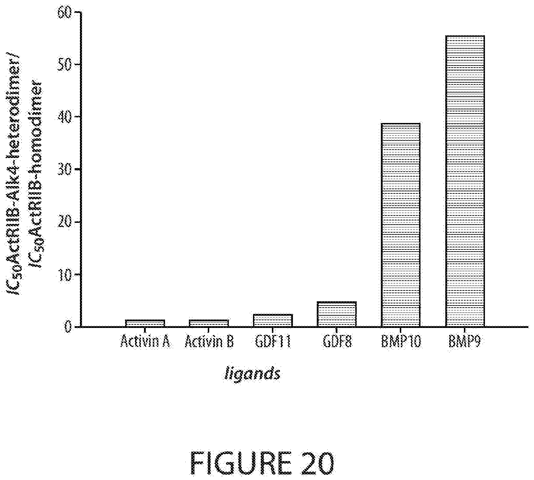

**Figure 20** shows comparative ALK4-Fc:ActRIIB-Fc heterodimer/ActRIIB-Fc:ActRIIB-Fc homodimer IC₅₀ data as determined by an A-204 Reporter Gene Assay as described herein. ALK4-Fc:ActRIIB-Fc heterodimer inhibits activin A, activin B, GDF8, and GDF11 signaling pathways similarly to the ActRIIB-Fc:ActRIIB-Fc homodimer. However, ALK4-Fc:ActRIIB-Fc heterodimer inhibition of BMP9 and BMP10 signaling pathways is significantly reduced compared to the ActRIIB-Fc:ActRIIB-Fc homodimer. These data demonstrate that ALK4:ActRIIB heterodimers are more selective antagonists of activin A, activin B, GDF8, and GDF11 compared to corresponding ActRIIB:ActRIIB homodimers.
**Figures 21A and 21B** show two schematic examples of heteromeric protein complexes comprising type I receptor and type II receptor polypeptides. Figure 21A depicts a heterodimeric protein complex comprising one type I receptor fusion polypeptide and one type II receptor fusion polypeptide, which can be assembled covalently or noncovalently via a multimerization domain contained within each polypeptide chain. Two assembled multimerization domains constitute an interaction pair, which can be either guided or unguided. Figure 21B depicts a heterotetrameric protein complex comprising two heterodimeric complexes as depicted in Figure 21A. Complexes of higher order can be envisioned.

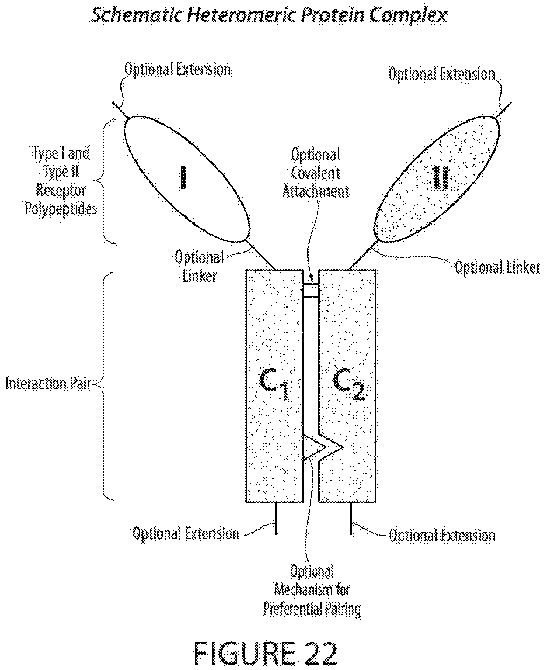

**Figures 22** show a schematic example of a heteromeric protein complex comprising a type I receptor polypeptide (indicated as "I") (e.g. a polypeptide that is at least 70%, 75%, 80%, 85%, 90%, 91%, 92%, 93%, 94%, 95%, 97%, 98%, 99% or 100% identical to an extracellular domain of an ALK4 protein from humans or other species such as those described herein) and a type II receptor polypeptide (indicated as "II") (e.g. a polypeptide that is at least 70%, 75%, 80%, 85%, 90%, 91%, 92%, 93%, 94%, 95%, 97%, 98%, 99% or 100% identical to an extracellular domain of an ActRIIB protein from humans or other species as such as those described herein). In the illustrated embodiments, the type I receptor polypeptide is part of a fusion polypeptide that comprises a first member of an interaction pair ("C₁"), and the type II receptor polypeptide is part of a fusion polypeptide that comprises a second member of an interaction pair ("C₂"). In each fusion polypeptide, a linker may be positioned between the type I or type II receptor polypeptide and the corresponding member of the interaction pair. The first and second members of the interaction pair may be a guided (asymmetric) pair, meaning that the members of the pair associate preferentially with each other rather than self-associate, or the interaction pair may be unguided, meaning that the members of the pair may associate with each other or self-associate without substantial preference and may have the same or different amino acid sequences. Traditional Fc fusion proteins and antibodies are examples of unguided interaction pairs, whereas a variety of engineered Fc domains have been designed as guided (asymmetric) interaction pairs [e.g., Spiess et al (2015) Molecular Immunology 67(2A): 95-106].

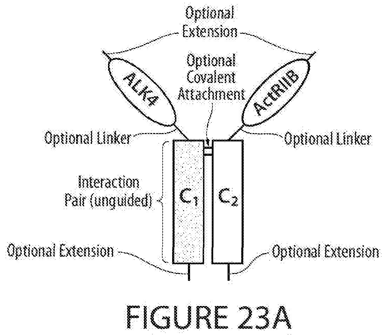

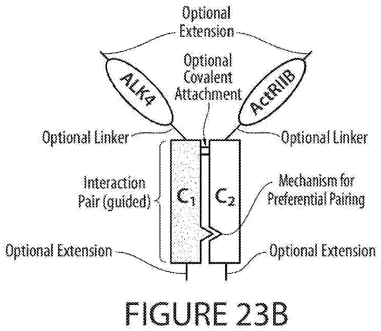

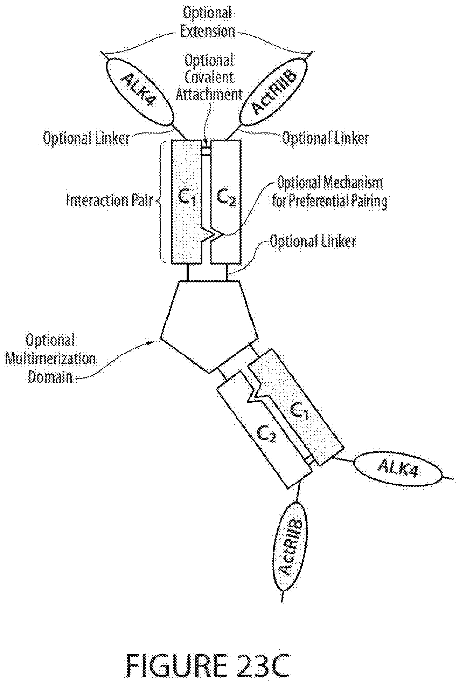

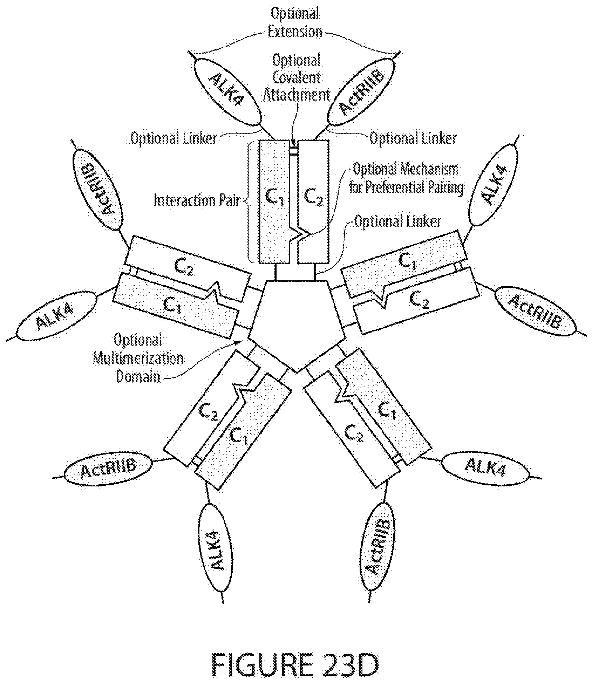

**Figures 23A-23D** show schematic examples of heteromeric protein complexes comprising an ALK4 polypeptide (e.g. a polypeptide that is at least 70%, 75%, 80%, 85%, 90%, 91%, 92%, 93%, 94%, 95%, 97%, 98%, 99% or 100% identical to an extracellular domain of an ALK4 protein from humans or other species such as those described herein) and an ActRIIB polypeptide (e.g. a polypeptide that is at least 70%, 75%, 80%, 85%, 90%, 91%, 92%, 93%, 94%, 95%, 97%, 98%, 99% or 100% identical to an extracellular domain of an ActRIIB protein from humans or other species such as those described herein). In the illustrated embodiments, the ALK4 polypeptide is part of a fusion polypeptide that comprises a first member of an interaction pair ("C₁"), and the ActRIIB polypeptide is part of a fusion polypeptide that comprises a second member of an interaction pair ("C₂"). Suitable interaction pairs included, for example, heavy chain and/or light chain immunoglobulin interaction pairs, truncations, and variants thereof such as those described herein [e.g., Spiess et al (2015) Molecular Immunology 67(2A): 95-106]. In each fusion polypeptide, a linker may be positioned between the ALK4 or ActRIIB polypeptide and the corresponding member of the interaction pair. The first and second members of the interaction pair may be unguided, meaning that the members of the pair may associate with each other or self-associate without substantial preference, and they may have the same or different amino acid sequences. See Figure 23A. Alternatively, the interaction pair may be a guided (asymmetric) pair, meaning that the members of the pair associate preferentially with each other rather than self-associate. See Figure 23B. Complexes of higher order can be envisioned. See Figure 23C and 23D.

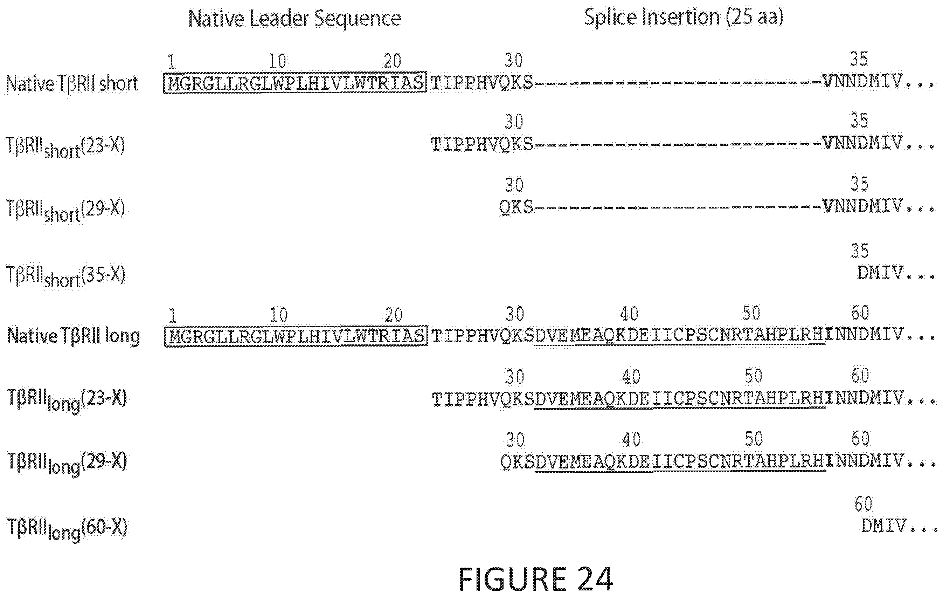

**Figure 24** shows N-terminal alignment of hTβRIIₛₕₒᵣₜ truncations and their hTβRII_{long} counterparts. The 25-amino-acid insertion present in hTβRII_{long} truncations is underlined. Note that the splicing process causes the valine flanking the insertion site in the short isoform to be replaced by an isoleucine in the long isoform. Boxed sequence denotes leader.

### DETAILED DESCRIPTION

### 1. Overview

The TGFβ superfamily is comprised of over 30 secreted factors including TGFβs, activins, nodals, bone morphogenetic proteins (BMPs), growth and differentiation factors (GDFs), and anti-Mullerian hormone (AMH) [Weiss et al. (2013) Developmental Biology, 2(1): 47-63]. Members of the superfamily, which are found in both vertebrates and invertebrates, are ubiquitously expressed in diverse tissues and function during the earliest stages of development throughout the lifetime of an animal. Indeed, TGFβ superfamily proteins are key mediators of stem cell self-renewal, gastrulation, differentiation, organ morphogenesis, and adult tissue homeostasis. Consistent with this ubiquitous activity, aberrant TGFβ superfamily signaling is associated with a wide range of human pathologies.

Ligands of the TGFβ superfamily share the same dimeric structure in which the central 3-1/2 turn helix of one monomer packs against the concave surface formed by the beta-strands of the other monomer. The maj ority of TGFβ family members are further stabilized by an intermolecular disulfide bond. This disulfide bonds traverses through a ring formed by two other disulfide bonds generating what has been termed a 'cysteine knot' motif [Lin et al. (2006) Reproduction 132: 179-190; and Hinck et al. (2012) FEBS Letters 586: 1860-1870].

TGFβ superfamily signaling is mediated by heteromeric complexes of type I and type II serine/threonine kinase receptors, which phosphorylate and activate downstream SMAD proteins (e.g., SMAD proteins 1, 2, 3, 5, and 8) upon ligand stimulation [Massagué (2000) Nat. Rev. Mol. Cell Biol. 1: 169-178]. These type I and type II receptors are transmembrane proteins, composed of a ligand-binding extracellular domain with cysteine-rich region, a transmembrane domain, and a cytoplasmic domain with predicted serine/threonine kinase specificity. In general, type I receptors mediate intracellular signaling while the type II receptors are required for binding TGFβ superfamily ligands. Type I and II receptors form a stable complex after ligand binding, resulting in phosphorylation of type I receptors by type II receptors.

The TGFβ family can be divided into two phylogenetic branches based on the type I receptors they bind and the Smad proteins they activate. One is the more recently evolved branch, which includes, *e.g.,* the TGFβs, activins, GDF8, GDF9, GDF11, BMP3 and nodal, which signal through type I receptors that activate Smads 2 and 3 [Hinck (2012) FEBS Letters 586:1860-1870]. The other branch comprises the more distantly related proteins of the superfamily and includes, e.g., BMP2, BMP4, BMP5, BMP6, BMP7, BMP8a, BMP8b, BMP9, BMP10, GDF1, GDF5, GDF6, and GDF7, which signal through Smads 1, 5, and 8.

TGFβ isoforms are the founding members of the TGFβ superfamily, of which there are 3 known isoforms in mammals designated as TGFβ1, TGFβ2, and TGFβ3. Mature bioactive TGFβ ligands function as homodimers and predominantly signal through the type I receptor ALK5, but have also been found to additionally signal through ALK1 in endothelial cells [Goumans et al. (2003) Mol Cell 12(4): 817-828]. TGFβ1 is the most abundant and ubiquitously expressed isoform. TGFβ1 is known to have an important role in wound healing, and mice expressing a constitutively active TGFβ1 transgene develop fibrosis [Clouthier et al. (1997) J Clin. Invest. 100(11): 2697-2713]. TGFβ1 expression was first described in human glioblastoma cells, and is occurs in neurons and astroglial cells of the embryonic nervous system. TGFβ3 was initially isolated from a human rhabdomyosarcoma cell line and since has been found in lung adenocarcinoma and kidney carcinoma cell lines. TGFβ3 is known to be important for palate and lung morphogenesis [Kubiczkova et al. (2012) Journal of Translational Medicine 10:183].

Activins are members of the TGFβ superfamily and were initially discovered as regulators of secretion of follicle-stimulating hormone, but subsequently various reproductive and non-reproductive roles have been characterized. There are three principal activin forms (A, B, and AB) that are homo/heterodimers of two closely related β subunits (β_{A}β_{A}, β_{B}β_{B}, and β_{A}β_{B}, respectively). The human genome also encodes an activin C and an activin E, which are primarily expressed in the liver, and heterodimeric forms containing β_{C} or β_{E} are also known. In the TGFβ superfamily, activins are unique and multifunctional factors that can stimulate hormone production in ovarian and placental cells, support neuronal cell survival, influence cell-cycle progress positively or negatively depending on cell type, and induce mesodermal differentiation at least in amphibian embryos [DePaolo et al. (1991) Proc Soc Ep Biol Med. 198:500-512; Dyson et al. (1997) Curr Biol. 7:81-84; and Woodruff (1998) Biochem Pharmacol. 55:953-963]. In several tissues, activin signaling is antagonized by its related heterodimer, inhibin. For example, in the regulation of follicle-stimulating hormone (FSH) secretion from the pituitary, activin promotes FSH synthesis and secretion, while inhibin reduces FSH synthesis and secretion. Other proteins that may regulate activin bioactivity and/or bind to activin include follistatin (FS), follistatin-related protein (FSRP, also known as FLRG or FSTL3), and α₂-macroglobulin.

As described herein, agents that bind to "activin A" are agents that specifically bind to the β_{A} subunit, whether in the context of an isolated β_{A} subunit or as a dimeric complex (e.g., a β_{A}β_{A} homodimer or a β_{A}β_{B} heterodimer). In the case of a heterodimer complex *(e.g.,* a β_{A}β_{B} heterodimer), agents that bind to "activin A" are specific for epitopes present within the β_{A} subunit, but do not bind to epitopes present within the non-β_{A} subunit of the complex (e.g., the β_{B} subunit of the complex). Similarly, agents disclosed herein that antagonize (inhibit) "activin A" are agents that inhibit one or more activities as mediated by a β_{A} subunit, whether in the context of an isolated β_{A} subunit or as a dimeric complex (e.g., a β_{A}β_{A} homodimer or a β_{A}β_{B} heterodimer). In the case of β_{A}β_{B} heterodimers, agents that inhibit "activin A" are agents that specifically inhibit one or more activities of the β_{A} subunit, but do not inhibit the activity of the non-β_{A} subunit of the complex (e.g., the β_{B} subunit of the complex). This principle applies also to agents that bind to and/or inhibit "activin B", "activin C", and "activin E". Agents disclosed herein that antagonize "activin AB" are agents that inhibit one or more activities as mediated by the β_{A} subunit and one or more activities as mediated by the β_{B} subunit.

The BMPs and GDFs together form a family of cysteine-knot cytokines sharing the characteristic fold of the TGFβ superfamily [Rider et al. (2010) Biochem J., 429(1): 1-12]. This family includes, for example, BMP2, BMP4, BMP6, BMP7, BMP2a, BMP3, BMP3b (also known as GDF10), BMP4, BMP5, BMP6, BMP7, BMP8, BMP8a, BMP8b, BMP9 (also known as GDF2), BMP10, BMP11 (also known as GDF11), BMP12 (also known as GDF7), BMP13 (also known as GDF6), BMP14 (also known as GDF5), BMP15, GDF1, GDF3 (also known as VGR2), GDF8 (also known as myostatin), GDF9, GDF15, and decapentaplegic. Besides the ability to induce bone formation, which gave the BMPs their name, the BMP/GDFs display morphogenetic activities in the development of a wide range of tissues. BMP/GDF homo- and hetero-dimers interact with combinations of type I and type II receptor dimers to produce multiple possible signaling complexes, leading to the activation of one of two competing sets of SMAD transcription factors. BMP/GDFs have highly specific and localized functions. These are regulated in a number of ways, including the developmental restriction of BMP/GDF expression and through the secretion of several specific BMP antagonist proteins that bind with high affinity to the cytokines. Curiously, a number of these antagonists resemble TGFβ superfamily ligands.

In part, the data presented herein demonstrates that ActRII antagonists (inhibitors) and TGFβ antagonists can be used alone or in combination to treat cancer. In particular, it was shown that treatment with an ActRIIA polypeptide, an ActRIIB polypeptide, or a pan-specific TGFβ antibody, separately, decreased tumor burden and increased survival time a cancer model. Moreover, it was shown that an ActRII antagonist in combination with a TGFβ antagonist can be used to synergistically increase antitumor activity compared to the effects observed with either agent alone. While the data indicate that the antitumor effects associated with inhibition of TGFβ signaling is due to TGFβ2 antagonism, it has been shown herein that an inhibitor of all three isoforms of TGFβ (TGFβ1, TGFβ2, and TGFβ3) has antitumor activities. Therefore, while it is preferably in many embodiments that TGFβ antagonists to be used in accordance with the methods and uses of the disclosure inhibit TGFβ2, it is expected that TGFβ antagonists that inhibit TGFβ1 and/or TGFβ3 will also be useful in promoting antitumor responses.

Accordingly, the disclosure provides, in part, methods of using an ActRII antagonist, a TGFβ antagonist, or a combination of an ActRII antagonist and a TGFβ antagonist, alone or in combination with one or more supportive therapies and/or additional active agents, to treat cancer, particularly treating or preventing one or more complications of a cancer (e.g., reducing tumor burden). In addition, the data indicate that efficacy of ActRII and TGFβ antagonist therapy is dependent on the immune system. Therefore, in part, the instant disclosure relates to the discovery that ActRII and TGFβ antagonists may be used as immunotherapeutics, particularly to treat a wide variety of cancers (e.g., cancers associated with immunosuppression and/or immune exhaustion). As with other known immuno-oncology agents, the ability of an ActRII and TGFβ antagonist to potentiate an immune response in a patient may have broader therapeutic implications outside the cancer field. For example, it has been proposed that immune potentiating agents may be useful in treating a wide variety of infectious diseases, particularly pathogenic agents which promote immunosuppression and/or immune exhaustion. Also, such immune potentiating agents may be useful in boosting the immunization efficacy of vaccines (e.g., infectious disease and cancer vaccines). Accordingly, the disclosure provides various ActRII and TGFβ antagonists that can be used, alone or in combination, to increase immune responses in a subject in need thereof, treat cancer, treat infectious diseases, and/or increase immunization efficacy, optionally in combination with one or more supportive therapies and/or additional active agents.

The terms used in this specification generally have their ordinary meanings in the art, within the context of this disclosure and in the specific context where each term is used. Certain terms are discussed below or elsewhere in the specification to provide additional guidance to the practitioner in describing the compositions and methods of the disclosure and how to make and use them. The scope or meaning of any use of a term will be apparent from the specific context in which it is used.

The terms "heteromer" or "heteromultimer" as used herein refer to a complex comprising at least a first polypeptide chain and a second polypeptide chain, wherein the second polypeptide chain differs in amino acid sequence from the first polypeptide chain by at least one amino acid residue. The heteromer can comprise a "heterodimer" formed by the first and second polypeptide chains or can form higher order structures where one or more polypeptide chains in addition to the first and second polypeptide chains are present. Exemplary structures for the heteromultimer include heterodimers, heterotrimers, heterotetramers and further oligomeric structures. Heterodimers are designated herein as X:Y or equivalently as X-Y, where X represents a first polypeptide chain and Y represents a second polypeptide chain. Higher-order heteromers and oligomeric structures are designated herein in a corresponding manner.

"Homologous," in all its grammatical forms and spelling variations, refers to the relationship between two proteins that possess a "common evolutionary origin," including proteins from superfamilies in the same species of organism, as well as homologous proteins from different species of organism. Such proteins (and their encoding nucleic acids) have sequence homology, as reflected by their sequence similarity, whether in terms of percent identity or by the presence of specific residues or motifs and conserved positions. However, in common usage and in the instant application, the term "homologous," when modified with an adverb such as "highly," may refer to sequence similarity and may or may not relate to a common evolutionary origin.

The term "sequence similarity," in all its grammatical forms, refers to the degree of identity or correspondence between nucleic acid or amino acid sequences that may or may not share a common evolutionary origin.

"Percent (%) sequence identity" with respect to a reference polypeptide (or nucleotide) sequence is defined as the percentage of amino acid residues (or nucleic acids) in a candidate sequence that are identical to the amino acid residues (or nucleic acids) in the reference polypeptide (nucleotide) sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid (nucleic acid) sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, Calif., or may be compiled from the source code. The ALIGN-2 program should be compiled for use on a UNIX operating system, including digital UNIX V4.0D. All sequence comparison parameters are set by the ALIGN-2 program and do not vary.

"Agonize", in all its grammatical forms, refers to the process of activating a protein and/or gene (e.g., by activating or amplifying that protein's gene expression or by inducing an inactive protein to enter an active state) or increasing a protein's and/or gene's activity.

"Antagonize", in all its grammatical forms, refers to the process of inhibiting a protein and/or gene (e.g., by inhibiting or decreasing that protein's gene expression or by inducing an active protein to enter an inactive state) or decreasing a protein's and/or gene's activity.

The terms "about" and "approximately" as used in connection with a numerical value throughout the specification and the claims denotes an interval of accuracy, familiar and acceptable to a person skilled in the art. In general, such interval of accuracy is ± 10%. Alternatively, and particularly in biological systems, the terms "about" and "approximately" may mean values that are within an order of magnitude, preferably ≤ 5-fold and more preferably ≤ 2-fold of a given value.

Numeric ranges disclosed herein are inclusive of the numbers defining the ranges.

The terms "a" and "an" include plural referents unless the context in which the term is used clearly dictates otherwise. The terms "a" (or "an"), as well as the terms "one or more," and "at least one" can be used interchangeably herein. Furthermore, "and/or" where used herein is to be taken as specific disclosure of each of the two or more specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

### 2. ActRII polypeptides, ALK4 polypeptides, TGFβRII polypeptides, and ALK4:ActRIIB heteromultimers

In certain aspects, the disclosure relates ActRII polypeptides and uses thereof (e.g., increasing an immune response in a subject in need thereof and treatment of cancer or pathogens). As used herein, the term "ActRII" refers to the family of type II activin receptors. This family includes activin receptor type IIA (ActRIIA) and activin receptor type IIB (ActRIIB). In some aspects, the disclosure relates to heteromultimers comprising at least one ActRIIB polypeptide and at least one ALK4 polypeptide, which are generally referred to herein as "ALK4:ActRIIB heteromultimers" or "ALK4:ActRIIB heteromultimer complexes" and uses thereof (e.g., increasing an immune response in a subject in need thereof and treatment of cancer or pathogens).

As used herein, the term "ActRIIB" refers to a family of activin receptor type IIB (ActRIIB) proteins from any species and variants derived from such ActRIIB proteins by mutagenesis or other modification. Reference to ActRIIB herein is understood to be a reference to any one of the currently identified forms. Members of the ActRIIB family are generally transmembrane proteins, composed of a ligand-binding extracellular domain comprising a cysteine-rich region, a transmembrane domain, and a cytoplasmic domain with predicted serine/threonine kinase activity.

The term "ActRIIB polypeptide" includes polypeptides comprising any naturally occurring polypeptide of an ActRIIB family member as well as any variants thereof (including mutants, fragments, fusions, and peptidomimetic forms) that retain a useful activity. Examples of such variant ActRIIB polypeptides are provided throughout the present disclosure as well as in International Patent Application Publication No. WO 2006/012627, WO 2008/097541, and WO 2010/151426, which are incorporated herein by reference in their entirety. Numbering of amino acids for all ActRIIB-related polypeptides described herein is based on the numbering of the human ActRIIB precursor protein sequence provided below (SEQ ID NO: 1), unless specifically designated otherwise.

A human ActRIIB precursor protein sequence is as follows:

1 
51 
101 
151 
201 
251 
301 
351 
401 
451 
501  (SEQ ID NO: 1)

The signal peptide is indicated with a single underline; the extracellular domain is indicated in **bold** font; and the potential, endogenous N-linked glycosylation sites are indicated with a double underline.

The processed extracellular ActRIIB polypeptide sequence is as follows:

GRGEAETRECIYYNANWELERTNQSGLERCEGEQDKRLHCYASWRNSSGTIELVKKGCWLDD
FNCYDRQECVATEENPQVYFCCCEGNFCNERFTHLPEAGGPEVTYEPPPTAPT (SEQ ID
NO: 2).

In some embodiments, the protein may be produced with an "SGR..." sequence at the N-terminus. The C-terminal "tail" of the extracellular domain is indicated by a single underline. The sequence with the "tail" deleted (a Δ15 sequence) is as follows:

GRGEAETRECIYYNANWELERTNQSGLERCEGEQDKRLHCYASWRNSSGTIELVKKGCWLDD
FNCYDRQECVATEENPQVYFCCCEGNFCNERFTHLPEA (SEQ ID NO: 3).

A form of ActRIIB with an alanine at position 64 of SEQ ID NO: 1 (A64) is also reported in the literature. See, *e.g.,* Hilden et al. (1994) Blood, 83(8): 2163-2170. It has been ascertained that an ActRIIB-Fc fusion protein comprising an extracellular domain of ActRIIB with the A64 substitution has a relatively low affinity for activin and GDF 11. By contrast, the same ActRIIB-Fc fusion protein with an arginine at position 64 (R64) has an affinity for activin and GDF 11 in the low nanomolar to high picomolar range. Therefore, sequences with an R64 are used as the "wild-type" reference sequence for human ActRIIB in this disclosure. The form of ActRIIB with an alanine at position 64 is as follows:

1 MTAPWVALAL LWGSLCAGSG RGEAETRECI YYNANWELER TNQSGLERCE
51 GEQDKRLHCY ASWANSSGTI ELVKKGCWLD DFNCYDRQEC VATEENPQVY
101 FCCCEGNFCN ERFTHLPEAG GPEVTYEPPP TAPTLLTVLA YSLLPIGGLS
151 LIVLLAFWMY RHRKPPYGHV DIHEDPGPPP PSPLVGLKPL QLLEIKARGR
201 FGCVWKAQLM NDFVAVKIFP LQDKQSWQSE REIFSTPGMK HENLLQFIAA
251 EKRGSNLEVE LWLITAFHDK GSLTDYLKGN IITWNELCHV AETMSRGLSY
301 LHEDVPWCRG EGHKPSIAHR DFKSKNVLLK SDLTAVLADF GLAVRFEPGK
351 PPGDTHGQVG TRRYMAPEVL EGAINFQRDA FLRIDMYAMG LVLWELVSRC
401 KAADGPVDEY MLPFEEEIGQ HPSLEELQEV VVHKKMRPTI KDHWLKHPGL
451 AQLCVTIEEC WDHDAEARLS AGCVEERVSL IRRSVNGTTS DCLVSLVTSV
501 TNVDLPPKES SI (SEQ ID NO: 4)

The signal peptide is indicated by single underline and the extracellular domain is indicated by **bold** font.

The processed extracellular ActRIIB polypeptide sequence of the alternative A64 form is as follows:

GRGEAETRECIYYNANWELERTNQSGLERCEGEQDKRLHCYASWANSSGTIELVKKGCWLDD
FNCYDRQECVATEENPQVYFCCCEGNFCNERFTHLPEAGGPEVTYEPPPTAPT (SEQ ID
NO: 5)

In some embodiments, the protein may be produced with an "SGR..." sequence at the N-terminus. The C-terminal "tail" of the extracellular domain is indicated by single underline. The sequence with the "tail" deleted (a Δ15 sequence) is as follows:

GRGEAETRECIYYNANWELERTNQSGLERCEGEQDKRLHCYASWANSSGTIELVKKGCWLDD
FNCYDRQECVATEENPQVYFCCCEGNFCNERFTHLPEA (SEQ ID NO: 6)

A nucleic acid sequence encoding the human ActRIIB precursor protein is shown below (SEQ ID NO: 7), representing nucleotides 25-1560 of Genbank Reference Sequence NM_001106.3, which encode amino acids 1-513 of the ActRIIB precursor. The sequence as shown provides an arginine at position 64 and may be modified to provide an alanine instead. The signal sequence is underlined.

1 ATGACGGCGC CCTGGGTGGC CCTCGCCCTC CTCTGGGGAT CGCTGTGCGC
51 CGGCTCTGGG CGTGGGGAGG CTGAGACACG GGAGTGCATC TACTACAACG
101 CCAACTGGGA GCTGGAGCGC ACCAACCAGA GCGGCCTGGA GCGCTGCGAA
151 GGCGAGCAGG ACAAGCGGCT GCACTGCTAC GCCTCCTGGC GCAACAGCTC
201 TGGCACCATC GAGCTCGTGA AGAAGGGCTG CTGGCTAGAT GACTTCAACT
251 GCTACGATAG GCAGGAGTGT GTGGCCACTG AGGAGAACCC CCAGGTGTAC
301 TTCTGCTGCT GTGAAGGCAA CTTCTGCAAC GAACGCTTCA CTCATTTGCC
351 AGAGGCTGGG GGCCCGGAAG TCACGTACGA GCCACCCCCG ACAGCCCCCA
401 CCCTGCTCAC GGTGCTGGCC TACTCACTGC TGCCCATCGG GGGCCTTTCC
451 CTCATCGTCC TGCTGGCCTT TTGGATGTAC CGGCATCGCA AGCCCCCCTA
501 CGGTCATGTG GACATCCATG AGGACCCTGG GCCTCCACCA CCATCCCCTC
551 TGGTGGGCCT GAAGCCACTG CAGCTGCTGG AGATCAAGGC TCGGGGGCGC
601 TTTGGCTGTG TCTGGAAGGC CCAGCTCATG AATGACTTTG TAGCTGTCAA
651 GATCTTCCCA CTCCAGGACA AGCAGTCGTG GCAGAGTGAA CGGGAGATCT
701 TCAGCACACC TGGCATGAAG CACGAGAACC TGCTACAGTT CATTGCTGCC
751 GAGAAGCGAG GCTCCAACCT CGAAGTAGAG CTGTGGCTCA TCACGGCCTT
801 CCATGACAAG GGCTCCCTCA CGGATTACCT CAAGGGGAAC ATCATCACAT
851 GGAACGAACT GTGTCATGTA GCAGAGACGA TGTCACGAGG CCTCTCATAC
901 CTGCATGAGG ATGTGCCCTG GTGCCGTGGC GAGGGCCACA AGCCGTCTAT
951 TGCCCACAGG GACTTTAAAA GTAAGAATGT ATTGCTGAAG AGCGACCTCA
1001 CAGCCGTGCT GGCTGACTTT GGCTTGGCTG TTCGATTTGA GCCAGGGAAA
1051 CCTCCAGGGG ACACCCACGG ACAGGTAGGC ACGAGACGGT ACATGGCTCC
1101 TGAGGTGCTC GAGGGAGCCA TCAACTTCCA GAGAGATGCC TTCCTGCGCA
1151 TTGACATGTA TGCCATGGGG TTGGTGCTGT GGGAGCTTGT GTCTCGCTGC
1201 AAGGCTGCAG ACGGACCCGT GGATGAGTAC ATGCTGCCCT TTGAGGAAGA
1251 GATTGGCCAG CACCCTTCGT TGGAGGAGCT GCAGGAGGTG GTGGTGCACA
1301 AGAAGATGAG GCCCACCATT AAAGATCACT GGTTGAAACA CCCGGGCCTG

1351 GCCCAGCTTT GTGTGACCAT CGAGGAGTGC TGGGACCATG ATGCAGAGGC
1401 TCGCTTGTCC GCGGGCTGTG TGGAGGAGCG GGTGTCCCTG ATTCGGAGGT
1451 CGGTCAACGG CACTACCTCG GACTGTCTCG TTTCCCTGGT GACCTCTGTC
1501 ACCAATGTGG ACCTGCCCCC TAAAGAGTCA AGCATC (SEQ ID NO: 7)

A nucleic acid sequence encoding processed extracellular human ActRIIB polypeptide is as follows (SEQ ID NO: 8). The sequence as shown provides an arginine at position 64, and may be modified to provide an alanine instead.

1 GGGCGTGGGG AGGCTGAGAC ACGGGAGTGC ATCTACTACA ACGCCAACTG
51 GGAGCTGGAG CGCACCAACC AGAGCGGCCT GGAGCGCTGC GAAGGCGAGC
101 AGGACAAGCG GCTGCACTGC TACGCCTCCT GGCGCAACAG CTCTGGCACC
151 ATCGAGCTCG TGAAGAAGGG CTGCTGGCTA GATGACTTCA ACTGCTACGA
201 TAGGCAGGAG TGTGTGGCCA CTGAGGAGAA CCCCCAGGTG TACTTCTGCT
251 GCTGTGAAGG CAACTTCTGC AACGAACGCT TCACTCATTT GCCAGAGGCT
301 GGGGGCCCGG AAGTCACGTA CGAGCCACCC CCGACAGCCC CCACC
(SEQ ID NO: 8)

An alignment of the amino acid sequences of human ActRIIB extracellular domain and human ActRIIA extracellular domain are illustrated in Figure 1. This alignment indicates amino acid residues within both receptors that are believed to directly contact ActRII ligands. For example, the composite ActRII structures indicated that the ActRIIB-ligand binding pocket is defined, in part, by residues Y31, N33, N35, L38 through T41, E47, E50, Q53 through K55, L57, H58, Y60, S62, K74, W78 through N83, Y85, R87, A92, and E94 through F101. At these positions, it is expected that conservative mutations will be tolerated.

In addition, ActRIIB is well-conserved among vertebrates, with large stretches of the extracellular domain completely conserved. For example, Figure 2 depicts a multi-sequence alignment of a human ActRIIB extracellular domain compared to various ActRIIB orthologs. Many of the ligands that bind to ActRIIB are also highly conserved. Accordingly, from these alignments, it is possible to predict key amino acid positions within the ligand-binding domain that are important for normal ActRIIB-ligand binding activities as well as to predict amino acid positions that are likely to be tolerant to substitution without significantly altering normal ActRIIB-ligand binding activities. Therefore, an active, human ActRIIB variant polypeptide useful in accordance with the presently disclosed methods may include one or more amino acids at corresponding positions from the sequence of another vertebrate ActRIIB, or may include a residue that is similar to that in the human or other vertebrate sequences. Without meaning to be limiting, the following examples illustrate this approach to defining an active ActRIIB variant. L46 in the human extracellular domain (SEQ ID NO: 103) is a valine in *Xenopus* ActRIIB (SEQ ID NO: 105), and so this position may be altered, and optionally may be altered to another hydrophobic residue, such as V, I or F, or a non-polar residue such as A. E52 in the human extracellular domain is a K in *Xenopus,* indicating that this site may be tolerant of a wide variety of changes, including polar residues, such as E, D, K, R, H, S, T, P, G, Y and probably A. T93 in the human extracellular domain is a K in *Xenopus,* indicating that a wide structural variation is tolerated at this position, with polar residues favored, such as S, K, R, E, D, H, G, P, G and Y. F108 in the human extracellular domain is a Y in *Xenopus,* and therefore Y or other hydrophobic group, such as I, V or L should be tolerated. E111 in the human extracellular domain is K in *Xenopus,* indicating that charged residues will be tolerated at this position, including D, R, K and H, as well as Q and N. R112 in the human extracellular domain is K in *Xenopus,* indicating that basic residues are tolerated at this position, including R and H. A at position 119 in the human extracellular domain is relatively poorly conserved, and appears as P in rodents and V in *Xenopus,* thus essentially any amino acid should be tolerated at this position.

Moreover, ActRII proteins have been characterized in the art in terms of structural and functional characteristics, particularly with respect to ligand binding [Attisano et al. (1992) Cell 68(1):97-108; Greenwald et al. (1999) Nature Structural Biology 6(1): 18-22; Allendorph et al. (2006) PNAS 103(20: 7643-7648; Thompson et al. (2003) The EMBO Journal 22(7): 1555-1566; as well as U.S. Patent Nos: 7,709,605, 7,612,041, and 7,842,663]. In addition to the teachings herein, these references provide amply guidance for how to generate ActRIIB variants that retain one or more normal activities (e.g., ligand-binding activity).

For example, a defining structural motif known as a three-finger toxin fold is important for ligand binding by type I and type II receptors and is formed by conserved cysteine residues located at varying positions within the extracellular domain of each monomeric receptor [Greenwald et al. (1999) Nat Struct Biol 6:18-22; and Hinck (2012) FEBS Lett 586:1860-1870]. Accordingly, the core ligand-binding domains of human ActRIIB, as demarcated by the outermost of these conserved cysteines, corresponds to positions 29-109 of SEQ ID NO: 1 (ActRIIB precursor). The structurally less-ordered amino acids flanking these cysteine-demarcated core sequences can be truncated by 1, 2, 3, 4, 5, 6, 7, 8, 9, 10, 11, 12, 13, 14, 15, 16, 17, 18, 19, 20, 21, 22, 23, 24, 25, 26, 27, or 28 residues at the N-terminus and/or 1, 2, 3, 4, 5, 6, 7, 8, 9, 10, 11, 12, 13, 14, 15, 16, 17, 18, 19, 20, 21, 22, 23, 24 or 25 residues a the C-terminus without necessarily altering ligand binding. Exemplary ActRIIB extracellular domains for N-terminal and/or C-terminal truncation include SEQ ID NOs: 2, 3, 5, and 6.

Attisano *et al.* showed that a deletion of the proline knot at the C-terminus of the extracellular domain of ActRIIB reduced the affinity of the receptor for activin. An ActRIIB-Fc fusion protein containing amino acids 20-119 of present SEQ ID NO: 1, "ActRIIB(20-119)-Fc", has reduced binding to GDF11 and activin relative to an ActRIIB(20-134)-Fc, which includes the proline knot region and the complete juxtamembrane domain (see, *e.g.,* U.S. Patent No. 7,842,663). However, an ActRIIB(20-129)-Fc protein retains similar, but somewhat reduced activity, relative to the wild-type, even though the proline knot region is disrupted.

Thus, ActRIIB extracellular domains that stop at amino acid 134, 133, 132, 131, 130 and 129 (with respect to SEQ ID NO: 1) are all expected to be active, but constructs stopping at 134 or 133 may be most active. Similarly, mutations at any of residues 129-134 (with respect to SEQ ID NO: 1) are not expected to alter ligand-binding affinity by large margins. In support of this, it is known in the art that mutations of P129 and P130 (with respect to SEQ ID NO: 1) do not substantially decrease ligand binding. Therefore, an ActRIIB polypeptide of the present disclosure may end as early as amino acid 109 (the final cysteine), however, forms ending at or between 109 and 119 (e.g., 109, 110, 111, 112, 113, 114, 115, 116, 117, 118, or 119) are expected to have reduced ligand binding. Amino acid 119 (with respect to present SEQ ID NO:1) is poorly conserved and so is readily altered or truncated. ActRIIB polypeptides and ActRIIB-based GDF traps ending at 128 (with respect to SEQ ID NO: 1) or later should retain ligand-binding activity. ActRIIB polypeptides and ActRIIB-based GDF traps ending at or between 119 and 127 (*e.g.,* 119, 120, 121, 122, 123, 124, 125, 126, or 127), with respect to SEQ ID NO: 1, will have an intermediate binding ability. Any of these forms may be desirable to use, depending on the clinical or experimental setting.

At the N-terminus of ActRIIB, it is expected that a protein beginning at amino acid 29 or before (with respect to SEQ ID NO: 1) will retain ligand-binding activity. Amino acid 29 represents the initial cysteine. An alanine-to-asparagine mutation at position 24 (with respect to SEQ ID NO: 1) introduces an N-linked glycosylation sequence without substantially affecting ligand binding [U.S. Patent No. 7,842,663]. This confirms that mutations in the region between the signal cleavage peptide and the cysteine cross-linked region, corresponding to amino acids 20-29, are well tolerated. In particular, ActRIIB polypeptides and ActRIIB-based GDF traps beginning at position 20, 21, 22, 23, and 24 (with respect to SEQ ID NO: 1) should retain general ligand-biding activity, and ActRIIB polypeptides and ActRIIB-based GDF traps beginning at positions 25, 26, 27, 28, and 29 (with respect to SEQ ID NO: 1) are also expected to retain ligand-biding activity. It has been demonstrated, *e.g.,* U.S. Patent No. 7,842,663, that, surprisingly, an ActRIIB construct beginning at 22, 23, 24, or 25 will have the most activity.

Taken together, a general formula for an active portion (*e.g.,* ligand-binding portion) of ActRIIB comprises amino acids 29-109 of SEQ ID NO: 1. Therefore ActRIIB polypeptides may, for example, comprise, consists essentially of, or consists of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to a portion of ActRIIB beginning at a residue corresponding to any one of amino acids 20-29 (*e.g.,* beginning at any one of amino acids 20, 21, 22, 23, 24, 25, 26, 27, 28, or 29) of SEQ ID NO: 1 and ending at a position corresponding to any one amino acids 109-134 (*e.g.,* ending at any one of amino acids 109, 110, 111, 112, 113, 114, 115, 116, 117, 118, 119, 120, 121, 122, 123, 124, 125, 126, 127, 128, 129, 130, 131, 132, 133, or 134) of SEQ ID NO: 1. Other examples include polypeptides that begin at a position from 20-29 (*e.g.,* any one of positions 20, 21, 22, 23, 24, 25, 26, 27, 28, or 29) or 21-29 (*e.g.,* any one of positions 21, 22, 23, 24, 25, 26, 27, 28, or 29) of SEQ ID NO: 1 and end at a position from 119-134 (e.g., any one of positions 119, 120, 121, 122, 123, 124, 125, 126, 127, 128, 129, 130, 131, 132, 133, or 134), 119-133 (*e.g.,* any one of positions 119, 120, 121, 122, 123, 124, 125, 126, 127, 128, 129, 130, 131, 132, or 133), 129-134 (*e.g.,* any one of positions 129, 130, 131, 132, 133, or 134), or 129-133 (*e.g.,* any one of positions 129, 130, 131, 132, or 133) of SEQ ID NO: 1. Other examples include constructs that begin at a position from 20-24 (e.g., any one of positions 20, 21, 22, 23, or 24), 21-24 (*e.g.,* any one of positions 21, 22, 23, or 24), or 22-25 (*e.g.,* any one of positions 22, 22, 23, or 25) of SEQ ID NO: 1 and end at a position from 109-134 (*e.g.,* any one of positions 109, 110, 111, 112, 113, 114, 115, 116, 117, 118, 119, 120, 121, 122, 123, 124, 125, 126, 127, 128, 129, 130, 131, 132, 133, or 134), 119-134 (*e.g.,* any one of positions 119, 120, 121, 122, 123, 124, 125, 126, 127, 128, 129, 130, 131, 132, 133, or 134) or 129-134 (*e.g.,* any one of positions 129, 130, 131, 132, 133, or 134) of SEQ ID NO: 1. Variants within these ranges are also contemplated, particularly those comprising, consisting essentially of, or consisting of an amino acid sequence that has at least 70%, 75%, 80%, 85%, 86%, 87%, 88%, 89%, 90%, 91%, 92%, 93%, 94%, 95%, 96%, 97%, 98%, 99%, or 100% identity to the corresponding portion of SEQ ID NO: 1.

The variations described herein may be combined in various ways. In some embodiments, ActRIIB variants comprise no more than 1, 2, 5, 6, 7, 8, 9, 10 or 15 conservative amino acid changes in the ligand-binding pocket, optionally zero, one or more non-conservative alterations at positions 40, 53, 55, 74, 79 and/or 82 in the ligand-binding pocket. Sites outside the binding pocket, at which variability may be particularly well tolerated, include the amino and carboxy termini of the extracellular domain (as noted above), and positions 42-46 and 65-73 (with respect to SEQ ID NO: 1). An asparagine-to-alanine alteration at position 65 (N65A) does not appear to decrease ligand binding in the R64 background [U.S. Patent No. 7,842,663]. This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64 [U.S. Patent No. 7,842,663]. Additionally, the results of the mutagenesis program described in the art indicate that there are amino acid positions in ActRIIB that are often beneficial to conserve. With respect to SEQ ID NO: 1, these include position 80 (acidic or hydrophobic amino acid), position 78 (hydrophobic, and particularly tryptophan), position 37 (acidic, and particularly aspartic or glutamic acid), position 56 (basic amino acid), position 60 (hydrophobic amino acid, particularly phenylalanine or tyrosine). Thus, the disclosure provides a framework of amino acids that may be conserved in ActRIIB polypeptides. Other positions that may be desirable to conserve are as follows: position 52 (acidic amino acid), position 55 (basic amino acid), position 81 (acidic), 98 (polar or charged, particularly E, D, R or K), all with respect to SEQ ID NO: 1.

It has been previously demonstrated that the addition of a further N-linked glycosylation site (N-X-S/T) into the ActRIIB extracellular domain is well-tolerated (see, e.g., U.S. Patent No. 7,842,663). Therefore, N-X-S/T sequences may be generally introduced at positions outside the ligand binding pocket defined, for example, in Figure 1 in ActRIIB polypeptide of the present disclosure. Particularly suitable sites for the introduction of non-endogenous N-X-S/T sequences include amino acids 20-29, 20-24, 22-25, 109-134, 120-134 or 129-134 (with respect to SEQ ID NO: 1). N-X-S/T sequences may also be introduced into the linker between the ActRIIB sequence and an Fc domain or other fusion component as well as optionally into the fusion component itself. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E105N, R112N, G120N, E123N, P129N, A132N, R112S and R112T (with respect to SEQ ID NO: 1). Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T (with respect to SEQ ID NO: 1) are contemplated. Likewise, in an A24N variant, an S26T alteration may be used. Accordingly, an ActRIIB polypeptide of the present disclosure may be a variant having one or more additional, non-endogenous N-linked glycosylation consensus sequences as described above.

In certain embodiments, the disclosure relates to ActRII antagonists (inhibitors) that comprise at least one ActRIIB polypeptide, which includes fragments, functional variants, and modified forms thereof as well as uses thereof (e.g., increasing an immune response in a patient in need thereof and treating cancer). Preferably, ActRIIB polypeptides are soluble (*e.g.,* an extracellular domain of ActRIIB). In some embodiments, ActRIIB polypeptides antagonize activity (*e.g.,* Smad signaling) of one or more TGFβ superfamily ligands [*e.g.,* GDF11, GDF8, activin (activin A, activin B, activin AB, activin C, activin E) BMP6, GDF3, BMP10, and/or BMP9]. Therefore, in some embodiments, ActRIIB polypeptides bind to one or more TGFβ superfamily ligands [*e.g.,* GDF11, GDF8, activin (activin A, activin B, activin AB, activin C, activin E) BMP6, GDF3, BMP10, and/or BMP9]. In some embodiments, ActRIIB polypeptides of the disclosure comprise, consist essentially of, or consist of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to a portion of ActRIIB beginning at a residue corresponding to amino acids 20-29 (*e.g.,* beginning at any one of amino acids 20, 21, 22, 23, 24, 25, 26, 27, 28, or 29) of SEQ ID NO: 1 and ending at a position corresponding to amino acids 109-134 (*e.g.,* ending at any one of amino acids 109, 110, 111, 112, 113, 114, 115, 116, 117, 118, 119, 120, 121, 122, 123, 124, 125, 126, 127, 128, 129, 130, 131, 132, 133, or 134) of SEQ ID NO: 1. In some embodiments, ActRIIB polypeptides comprise, consist, or consist essentially of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical amino acids 29-109 of SEQ ID NO: 1. In some embodiments, ActRIIB polypeptides of the disclosure comprise, consist, or consist essentially of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical amino acids 29-109 of SEQ ID NO: 1, wherein the position corresponding to L79 of SEQ ID NO: 1 is an acidic amino acid (naturally occurring acidic amino acids D and E or an artificial acidic amino acid). In certain embodiments, ActRIIB polypeptides of the disclosure comprise, consist, or consist essentially of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical amino acids 25-131 of SEQ ID NO: 1. In certain embodiments, ActRIIB polypeptides of the disclosure comprise, consist, or consist essentially of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical amino acids 25-131 of SEQ ID NO: 1, wherein the position corresponding to L79 of SEQ ID NO: 1 is an acidic amino acid. In some embodiments, ActRIIB polypeptide of disclosure comprise, consist, or consist essentially of an amino acid sequence that is at least 70%, 75%, 80%, 85%, 86%, 87%, 88%, 89%, 90%, 91%, 92%, 93%, 94%, 95%, 97%, 98%, 99%, or 100% identical to the amino acid sequence of any one of SEQ ID NOs: 1, 2, 3, 4, 5, 6, 58, 59, 60, 63, 64, 65, 66, 68, 69, 70, 73, 77, 78, 128, 131, 132, and 133. In some embodiments, ActRIIB polypeptide of disclosure comprise, consist, or consist essentially of an amino acid sequence that is at least 70%, 75%, 80%, 85%, 86%, 87%, 88%, 89%, 90%, 91%, 92%, 93%, 94%, 95%, 97%, 98%, 99%, or 100% identical to the amino acid sequence of any one of SEQ ID NOs: 1, 2, 3, 4, 5, 6, 58, 59, 60, 63, 64, 65, 66, 68, 69, 70, 73, 77, 78, 128, 131, 132, and 133, wherein the position corresponding to L79 of SEQ ID NO: 1 is an acidic amino acid. In some embodiments, ActRIIB polypeptides of the disclosure comprise, consist, or consist essentially of, at least one ActRIIB polypeptide wherein the position corresponding to L79 of SEQ ID NO: 1 is not an acidic amino acid (*i.e.,* is not naturally occurring acid amino acids D or E or an artificial acidic amino acid residue).

In certain embodiments, the present disclosure relates to ActRIIA polypeptides. As used herein, the term "ActRIIA" refers to a family of activin receptor type IIA (ActRIIA) proteins from any species and variants derived from such ActRIIA proteins by mutagenesis or other modification. Reference to ActRIIA herein is understood to be a reference to any one of the currently identified forms. Members of the ActRIIA family are generally transmembrane proteins, composed of a ligand-binding extracellular domain comprising a cysteine-rich region, a transmembrane domain, and a cytoplasmic domain with predicted serine/threonine kinase activity.

The term "ActRIIA polypeptide" includes polypeptides comprising any naturally occurring polypeptide of an ActRIIA family member as well as any variants thereof (including mutants, fragments, fusions, and peptidomimetic forms) that retain a useful activity. Examples of such variant ActRIIA polypeptides are provided throughout the present disclosure as well as in International Patent Application Publication No. WO 2006/012627 and WO 2007/062188, which are incorporated herein by reference in their entirety. Numbering of amino acids for all ActRIIA-related polypeptides described herein is based on the numbering of the human ActRIIA precursor protein sequence provided below (SEQ ID NO: 9), unless specifically designated otherwise.

The human ActRIIA precursor protein sequence is as follows:

1 MGAAAKLAFA VFLISCSSGA ILGRSETQEC LFFNANWEKD RTNQTGVEPC
51 YGDKDKRRHC FATWKNISGS IEIVKQGCWL DDINCYDRTD CVEKKDSPEV
101 YFCCCEGNMC NEKFSYFPEM EVTQPTSNPV TPKPPYYNIL LYSLVPLMLI
151 AGIVICAFWV YRHHKMAYPP VLVPTQDPGP PPPSPLLGLK PLQLLEVKAR
201 GRFGCVWKAQ LLNEYVAVKI FPIQDKQSWQ NEYEVYSLPG MKHENILQFI
251 GAEKRGTSVD VDLWLITAFH EKGSLSDFLK ANVVSWNELC HIAETMARGL
301 AYLHEDIPGL KDGHKPAISH RDIKSKNVLL KNNLTACIAD FGLALKFEAG
351 KSAGDTHGQV GTRRYMAPEV LEGAINFQRD AFLRIDMYAM GLVLWELASR
401 CTAADGPVDE YMLPFEEEIG QHPSLEDMQE VVVHKKKRPV LRDYWQKHAG
451 MAMLCETIEE CWDHDAEARL SAGCVGERIT QMQRLTNIIT TEDIVTVVTM
501 VTNVDFPPKE SSL (SEQ ID NO: 9)

The signal peptide is indicated by a single underline; the extracellular domain is indicated in **bold** font; and the potential, endogenous N-linked glycosylation sites are indicated by a double underline.

A processed extracellular human ActRIIA polypeptide sequence is as follows:

ILGRSETQECLFFNANWEKDRTNQTGVEPCYGDKDKRRHCFATWKNISGSIEIVKQGCWLDD
INCYDRTDCVEKKDSPEVYFCCCEGNMCNEKFSYFPEMEVTQPTSNPVTPKPP (SEQ ID
NO: 10)

The C-terminal "tail" of the extracellular domain is indicated by single underline. The sequence with the "tail" deleted (a Δ15 sequence) is as follows:

ILGRSETQECLFFNANWEKDRTNQTGVEPCYGDKDKRRHCFATWKNISGSIEIVKQGCWLDD
INCYDRTDCVEKKDSPEVYFCCCEGNMCNEKFSYFPEM (SEQ ID NO: 11)

A nucleic acid sequence encoding human ActRIIA precursor protein is shown below (SEQ ID NO: 12), as follows nucleotides 159-1700 of Genbank Reference Sequence NM_001616.4. The signal sequence is underlined.

1 ATGGGAGCTG CTGCAAAGTT GGCGTTTGCC GTCTTTCTTA TCTCCTGTTC
51 TTCAGGTGCT ATACTTGGTA GATCAGAAAC TCAGGAGTGT CTTTTCTTTA
101 ATGCTAATTG GGAAAAAGAC AGAACCAATC AAACTGGTGT TGAACCGTGT
151 TATGGTGACA AAGATAAACG GCGGCATTGT TTTGCTACCT GGAAGAATAT
201 TTCTGGTTCC ATTGAAATAG TGAAACAAGG TTGTTGGCTG GATGATATCA
251 ACTGCTATGA CAGGACTGAT TGTGTAGAAA AAAAAGACAG CCCTGAAGTA
301 TATTTTTGTT GCTGTGAGGG CAATATGTGT AATGAAAAGT TTTCTTATTT
351 TCCGGAGATG GAAGTCACAC AGCCCACTTC AAATCCAGTT ACACCTAAGC
401 CACCCTATTA CAACATCCTG CTCTATTCCT TGGTGCCACT TATGTTAATT
451 GCGGGGATTG TCATTTGTGC ATTTTGGGTG TACAGGCATC ACAAGATGGC
501 CTACCCTCCT GTACTTGTTC CAACTCAAGA CCCAGGACCA CCCCCACCTT
551 CTCCATTACT AGGTTTGAAA CCACTGCAGT TATTAGAAGT GAAAGCAAGG
601 GGAAGATTTG GTTGTGTCTG GAAAGCCCAG TTGCTTAACG AATATGTGGC
651 TGTCAAAATA TTTCCAATAC AGGACAAACA GTCATGGCAA AATGAATACG
701 AAGTCTACAG TTTGCCTGGA ATGAAGCATG AGAACATATT ACAGTTCATT
751 GGTGCAGAAA AACGAGGCAC CAGTGTTGAT GTGGATCTTT GGCTGATCAC
801 AGCATTTCAT GAAAAGGGTT CACTATCAGA CTTTCTTAAG GCTAATGTGG
851 TCTCTTGGAA TGAACTGTGT CATATTGCAG AAACCATGGC TAGAGGATTG
901 GCATATTTAC ATGAGGATAT ACCTGGCCTA AAAGATGGCC ACAAACCTGC
951 CATATCTCAC AGGGACATCA AAAGTAAAAA TGTGCTGTTG AAAAACAACC
1001 TGACAGCTTG CATTGCTGAC TTTGGGTTGG CCTTAAAATT TGAGGCTGGC
1051 AAGTCTGCAG GCGATACCCA TGGACAGGTT GGTACCCGGA GGTACATGGC
1101 TCCAGAGGTA TTAGAGGGTG CTATAAACTT CCAAAGGGAT GCATTTTTGA
1151 GGATAGATAT GTATGCCATG GGATTAGTCC TATGGGAACT GGCTTCTCGC
1201 TGTACTGCTG CAGATGGACC TGTAGATGAA TACATGTTGC CATTTGAGGA
1251 GGAAATTGGC CAGCATCCAT CTCTTGAAGA CATGCAGGAA GTTGTTGTGC
1301 ATAAAAAAAA GAGGCCTGTT TTAAGAGATT ATTGGCAGAA ACATGCTGGA
1351 ATGGCAATGC TCTGTGAAAC CATTGAAGAA TGTTGGGATC ACGACGCAGA

1401 AGCCAGGTTA TCAGCTGGAT GTGTAGGTGA AAGAATTACC CAGATGCAGA
1451 GACTAACAAA TATTATTACC ACAGAGGACA TTGTAACAGT GGTCACAATG
1501 GTGACAAATG TTGACTTTCC TCCCAAAGAA TCTAGTCTA (SEQ ID NO: 12)

A nucleic acid sequence encoding processed human ActRIIA polypeptide is as follows:

1 ATACTTGGTA GATCAGAAAC TCAGGAGTGT CTTTTCTTTA ATGCTAATTG
51 GGAAAAAGAC AGAACCAATC AAACTGGTGT TGAACCGTGT TATGGTGACA
101 AAGATAAACG GCGGCATTGT TTTGCTACCT GGAAGAATAT TTCTGGTTCC
151 ATTGAAATAG TGAAACAAGG TTGTTGGCTG GATGATATCA ACTGCTATGA
201 CAGGACTGAT TGTGTAGAAA AAAAAGACAG CCCTGAAGTA TATTTTTGTT
251 GCTGTGAGGG CAATATGTGT AATGAAAAGT TTTCTTATTT TCCGGAGATG
301 GAAGTCACAC AGCCCACTTC AAATCCAGTT ACACCTAAGC CACCC(SEQ ID
NO:13)

ActRIIA is well-conserved among vertebrates, with large stretches of the extracellular domain completely conserved. For example, Figure 3 depicts a multi-sequence alignment of a human ActRIIA extracellular domain compared to various ActRIIA orthologs. Many of the ligands that bind to ActRIIA are also highly conserved. Accordingly, from these alignments, it is possible to predict key amino acid positions within the ligand-binding domain that are important for normal ActRIIA-ligand binding activities as well as to predict amino acid positions that are likely to be tolerant to substitution without significantly altering normal ActRIIA-ligand binding activities. Therefore, an active, human ActRIIA variant polypeptide useful in accordance with the presently disclosed methods may include one or more amino acids at corresponding positions from the sequence of another vertebrate ActRIIA, or may include a residue that is similar to that in the human or other vertebrate sequences.

Without meaning to be limiting, the following examples illustrate this approach to defining an active ActRIIA variant. As illustrated in Figure 3, F13 in the human extracellular domain is Y in *Ovis aries* (SEQ ID NO: 108), *Gallus gallus* (SEQ ID NO: 111), *Bos Taurus* (SEQ ID NO: 112), *Tyto alba* (SEQ ID NO: 113), and *Myotis davidii* (SEQ ID NO: 114) ActRIIA, indicating that aromatic residues are tolerated at this position, including F, W, and Y. Q24 in the human extracellular domain is R in *Bos Taurus* ActRIIA, indicating that charged residues will be tolerated at this position, including D, R, K, H, and E. S95 in the human extracellular domain is F in *Gallus gallus* and *Tyto alba* ActRIIA, indicating that this site may be tolerant of a wide variety of changes, including polar residues, such as E, D, K, R, H, S, T, P, G, Y, and probably hydrophobic residue such as L, I, or F. E52 in the human extracellular domain is D in *Ovis aries* ActRIIA, indicating that acidic residues are tolerated at this position, including D and E. P29 in the human extracellular domain is relatively poorly conserved, appearing as S in *Ovis aries* ActRIIA and L in *Myotis davidii* ActRIIA, thus essentially any amino acid should be tolerated at this position.

Moreover, as discussed above, ActRII proteins have been characterized in the art in terms of structural/functional characteristics, particularly with respect to ligand binding [Attisano et al. (1992) Cell 68(1):97-108; Greenwald et al. (1999) Nature Structural Biology 6(1): 18-22; Allendorph et al. (2006) PNAS 103(20: 7643-7648; Thompson et al. (2003) The EMBO Journal 22(7): 1555-1566; as well as U.S. Patent Nos: 7,709,605, 7,612,041, and 7,842,663]. In addition to the teachings herein, these references provide amply guidance for how to generate ActRIIA variants that retain one or more desired activities (e.g., ligand-binding activity).

For example, a defining structural motif known as a three-finger toxin fold is important for ligand binding by type I and type II receptors and is formed by conserved cysteine residues located at varying positions within the extracellular domain of each monomeric receptor [Greenwald et al. (1999) Nat Struct Biol 6:18-22; and Hinck (2012) FEBS Lett 586:1860-1870]. Accordingly, the core ligand-binding domains of human ActRIIA, as demarcated by the outermost of these conserved cysteines, corresponds to positions 30-110 of SEQ ID NO: 9 (ActRIIA precursor). Therefore, the structurally less-ordered amino acids flanking these cysteine-demarcated core sequences can be truncated by about 1, 2, 3, 4, 5, 6, 7, 8, 9, 10, 11, 12, 13, 14, 15, 16, 17, 18, 19, 20, 21, 22, 23, 24, 25, 26, 27, 28, or 29 residues at the N-terminus and by about 1, 2, 3, 4, 5, 6, 7, 8, 9, 10, 11, 12, 13, 14, 15, 16, 17, 18, 19, 20, 21, 22, 23, 24, or 25 residues at the C-terminus without necessarily altering ligand binding. Exemplary ActRIIA extracellular domains truncations include SEQ ID NOs: 10 and 11.

Accordingly, a general formula for an active portion (*e.g.,* ligand binding) of ActRIIA is a polypeptide that comprises, consists essentially of, or consists of amino acids 30-110 of SEQ ID NO: 9. Therefore ActRIIA polypeptides may, for example, comprise, consists essentially of, or consists of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to a portion of ActRIIA beginning at a residue corresponding to any one of amino acids 21-30 (e.g., beginning at any one of amino acids 21, 22, 23, 24, 25, 26, 27, 28, 29, or 30) of SEQ ID NO: 9 and ending at a position corresponding to any one amino acids 110-135 (*e.g.,* ending at any one of amino acids 110, 111, 112, 113, 114, 115, 116, 117, 118, 119, 120, 121, 122, 123, 124, 125, 126, 127, 128, 129, 130, 131, 132, 133, or 135) of SEQ ID NO: 9. Other examples include constructs that begin at a position selected from 21-30 (*e.g.,* beginning at any one of amino acids 21, 22, 23, 24, 25, 26, 27, 28, 29, or 30), 22-30 (*e.g.,* beginning at any one of amino acids 22, 23, 24, 25, 26, 27, 28, 29, or 30), 23-30 (*e.g.,* beginning at any one of amino acids 23, 24, 25, 26, 27, 28, 29, or 30), 24-30 (*e.g.,* beginning at any one of amino acids 24, 25, 26, 27, 28, 29, or 30) of SEQ ID NO: 9, and end at a position selected from 111-135 (*e.g.,* ending at any one of amino acids 111, 112, 113, 114, 115, 116, 117, 118, 119, 120, 121, 122, 123, 124, 125, 126, 127, 128, 129, 130, 131, 132, 133, 134 or 135), 112-135 (*e.g.,* ending at any one of amino acids 112, 113, 114, 115, 116, 117, 118, 119, 120, 121, 122, 123, 124, 125, 126, 127, 128, 129, 130, 131, 132, 133, 134 or 135), 113-135 (*e.g.,* ending at any one of amino acids 113, 114, 115, 116, 117, 118, 119, 120, 121, 122, 123, 124, 125, 126, 127, 128, 129, 130, 131, 132, 133, 134 or 135), 120-135 (*e.g.,* ending at any one of amino acids 120, 121, 122, 123, 124, 125, 126, 127, 128, 129, 130, 131, 132, 133, 134 or 135),130-135 *(e.g.,* ending at any one of amino acids 130, 131, 132, 133, 134 or 135), 111-134 (*e.g.,* ending at any one of amino acids 110, 111, 112, 113, 114, 115, 116, 117, 118, 119, 120, 121, 122, 123, 124, 125, 126, 127, 128, 129, 130, 131, 132, 133, or 134), 111-133 (*e.g.,* ending at any one of amino acids 110, 111, 112, 113, 114, 115, 116, 117, 118, 119, 120, 121, 122, 123, 124, 125, 126, 127, 128, 129, 130, 131, 132, or 133), 111-132 (*e.g.,* ending at any one of amino acids 110, 111, 112, 113, 114, 115, 116, 117, 118, 119, 120, 121, 122, 123, 124, 125, 126, 127, 128, 129, 130, 131, or 132), or 111-131 (*e.g.,* ending at any one of amino acids 110, 111, 112, 113, 114, 115, 116, 117, 118, 119, 120, 121, 122, 123, 124, 125, 126, 127, 128, 129, 130, or 131) of SEQ ID NO: 9. Variants within these ranges are also contemplated, particularly those comprising, consisting essentially of, or consisting of an amino acid sequence that has at least 70%, 75%, 80%, 85%, 86%, 87%, 88%, 89%, 90%, 91%, 92%, 93%, 94%, 95%, 96%, 97%, 98%, 99%, or 100% identity to the corresponding portion of SEQ ID NO: 9. Thus, in some embodiments, an ActRIIA polypeptide may comprise, consists essentially of, or consist of a polypeptide that is at least 70%, 75%, 80%, 85%, 86%, 87%, 88%, 89%, 90%, 91%, 92%, 93%, 94%, 95%, 96%, 97%, 98%, 99%, or 100% identical to amino acids 30-110 of SEQ ID NO: 9. Optionally, ActRIIA polypeptides comprise a polypeptide that is at least 70%, 75%, 80%, 85%, 86%, 87%, 88%, 89%, 90%, 91%, 92%, 93%, 94%, 95%, 96%, 97%, 98%, 99%, or 100% identical to amino acids 30-110 of SEQ ID NO: 9, and comprising no more than 1, 2, 5, 10 or 15 conservative amino acid changes in the ligand-binding pocket.

In certain embodiments, the disclosure relates to ActRII antagonists (inhibitors) that comprise at least one ActRIIA polypeptide, which includes fragments, functional variants, and modified forms thereof as well as uses thereof (e.g., increasing an immune response in a patient in need thereof and treating cancer). Preferably, ActRIIA polypeptides are soluble (*e.g.,* an extracellular domain of ActRIIA). In some embodiments, ActRIIA polypeptides inhibit (*e.g.,* Smad signaling) of one or more TGFβ superfamily ligands [*e.g.,* GDF11, GDF8, activin (activin A, activin B, activin AB, activin C, activin E) BMP6, GDF3, BMP10, and/or BMP9]. In some embodiments, ActRIIA polypeptides bind to one or more TGFβ superfamily ligands [*e.g.,* GDF11, GDF8, activin (activin A, activin B, activin AB, activin C, activin E) BMP6, GDF3, BMP10, and/or BMP9]. In some embodiments, ActRIIA polypeptide of the disclosure comprise, consist essentially of, or consist of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to a portion of ActRIIA beginning at a residue corresponding to amino acids 21-30 (*e.g.,* beginning at any one of amino acids 21, 22, 23, 24, 25, 26, 27, 28, 29, or 30) of SEQ ID NO: 9 and ending at a position corresponding to any one amino acids 110-135 (*e.g.,* ending at any one of amino acids 110, 111, 112, 113, 114, 115, 116, 117, 118, 119, 120, 121, 122, 123, 124, 125, 126, 127, 128, 129, 130, 131, 132, 133, or 135) of SEQ ID NO: 9. In some embodiments, ActRIIA polypeptides comprise, consist, or consist essentially of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical amino acids 30-110 of SEQ ID NO: 9. In certain embodiments, ActRIIA polypeptides comprise, consist, or consist essentially of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical amino acids 21-135 of SEQ ID NO: 9. In some embodiments, ActRIIA polypeptides comprise, consist, or consist essentially of an amino acid sequence that is at least 70%, 75%, 80%, 85%, 86%, 87%, 88%, 89%, 90%, 91%, 92%, 93%, 94%, 95%, 97%, 98%, 99%, or 100% identical to the amino acid sequence of any one of SEQ ID NOs: 9, 10, 11, 50, 54, and 57.

In certain aspects, the present disclosure relates to GDF trap polypeptides (also referred to as "GDF traps"). In some embodiments, GDF traps of the present disclosure are variant ActRII polypeptides (*e.g.,* ActRIIA and ActRIIB polypeptides) that comprise one or more mutations (*e.g.,* amino acid additions, deletions, substitutions, and combinations thereof) in the extracellular domain (also referred to as the ligand-binding domain) of an ActRII polypeptide (*e.g.,* a "wild-type" or unmodified ActRII polypeptide) such that the variant ActRII polypeptide has one or more altered ligand-binding activities than the corresponding wild-type ActRII polypeptide. In preferred embodiments, GDF trap polypeptides of the present disclosure retain at least one similar activity as a corresponding wild-type ActRII polypeptide. For example, preferable GDF traps bind to and inhibit (*e.g.* antagonize) the function of GDF11 and/or GDF8. In some embodiments, GDF traps of the present disclosure further bind to and inhibit one or more of ligand of the TGFβ superfamily. Accordingly, the present disclosure provides GDF trap polypeptides that have an altered binding specificity for one or more ActRII ligands.

To illustrate, one or more mutations may be selected that increase the selectivity of the altered ligand-binding domain for GDF 11 and/or GDF8 over one or more ActRII-binding ligands such as activins (activin A, activin B, activin AB, activin C, and/or activin E), particularly activin A. Optionally, the altered ligand-binding domain has a ratio of K_{d} for activin binding to K_{d} for GDF 11 and/or GDF8 binding that is at least 2-, 5-, 10-, 20-, 50-, 100- or even 1000-fold greater relative to the ratio for the wild-type ligand-binding domain. Optionally, the altered ligand-binding domain has a ratio of IC₅₀ for inhibiting activin to IC₅₀ for inhibiting GDF11 and/or GDF8 that is at least 2-, 5-, 10-, 20-, 50-, 100- or even 1000-fold greater relative to the wild-type ligand-binding domain. Optionally, the altered ligand-binding domain inhibits GDF 11 and/or GDF8 with an IC₅₀ at least 2-, 5-, 10-, 20-, 50-, 100- or even 1000-times less than the IC₅₀ for inhibiting activin.

In certain preferred embodiments, GDF traps of the present disclosure are designed to preferentially bind to GDF11 and/or GDF8 (also known as myostatin). Optionally, GDF11 and/or GDF8-binding traps may further bind to activin B. Optionally, GDF11 and/or GDF8-binding traps may further bind to BMP6. Optionally, GDF11 and/or GDF8-binding traps may further bind to BMP10. Optionally, GDF11 and/or GDF8-binding traps may further bind to activin B and BMP6. In certain embodiments, GDF traps of the present disclosure have diminished binding affinity for activins (*e.g.,* activin A, activin A/B, activin B, activin C, activin E ), *e.g.,* in comparison to a wild-type ActRII polypeptide. In certain preferred embodiments, a GDF trap polypeptide of the present disclosure has diminished binding affinity for activin A.

Amino acid residues of the ActRIIB proteins (*e.g.,* E39, K55, Y60, K74, W78, L79, D80, and F101 with respect to SEQ ID NO: 1) are in the ActRIIB ligand-binding pocket and help mediated binding to its ligands including, for example, activin A, GDF11, and GDF8. Thus the present disclosure provides GDF trap polypeptides comprising an altered-ligand binding domain (*e.g.,* a GDF8/GDF11-binding domain) of an ActRIIB receptor which comprises one or more mutations at those amino acid residues.

As a specific example, the positively-charged amino acid residue Asp (D80) of the ligand-binding domain of ActRIIB can be mutated to a different amino acid residue to produce a GDF trap polypeptide that preferentially binds to GDF8, but not activin. Preferably, the D80 residue with respect to SEQ ID NO: 1 is changed to an amino acid residue selected from the group consisting of: an uncharged amino acid residue, a negative amino acid residue, and a hydrophobic amino acid residue. As a further specific example, the hydrophobic residue L79 of SEQ ID NO: 1 can be altered to confer altered activin-GDF11/GDF8 binding properties. For example, an L79P substitution reduces GDF11 binding to a greater extent than activin binding. In contrast, replacement of L79 with an acidic amino acid [an aspartic acid or glutamic acid; an L79D or an L79E substitution] greatly reduces activin A binding affinity while retaining GDF11 binding affinity. In exemplary embodiments, the methods described herein utilize a GDF trap polypeptide which is a variant ActRIIB polypeptide comprising an acidic amino acid (*e.g.,* D or E) at the position corresponding to position 79 of SEQ ID NO: 1, optionally in combination with one or more additional amino acid substitutions, additions, or deletions.

In certain aspects, the disclosure relates ALK4 polypeptides and uses thereof (e.g., increasing an immune response in a patient in need thereof and treating cancer or pathogens). As used herein, the term "ALK4" refers to a family of activin receptor-like kinase-4 proteins from any species and variants derived from such ALK4 proteins by mutagenesis or other modification. Reference to ALK4 herein is understood to be a reference to any one of the currently identified forms. Members of the ALK4 family are generally transmembrane proteins, composed of a ligand-binding extracellular domain with a cysteine-rich region, a transmembrane domain, and a cytoplasmic domain with predicted serine/threonine kinase activity.

The term "ALK4 polypeptide" includes polypeptides comprising any naturally occurring polypeptide of an ALK4 family member as well as any variants thereof (including mutants, fragments, fusions, and peptidomimetic forms) that retain a useful activity. Numbering of amino acids for all ALK4-related polypeptides described herein is based on the numbering of the human ALK4 precursor protein sequence below (SEQ ID NO: 14), unless specifically designated otherwise.

The canonical human ALK4 precursor protein sequence (NCBI Ref Seq NP_004293) is as follows:

1 MAESAGASSF FPLVVLLLAG SGGSGPRGVQ ALLCACTSCL QANYTCETDG
ACMVSIFNLD
61 GMEHHVRTCI PKVELVPAGK PFYCLSSEDL RNTHCCYTDY CNRIDLRVPS
GHLKEPEHPS
121 MWGPVELVGI IAGPVFLLFL IIIIVFLVIN YHQRVYHNRQ RLDMEDPSCE
MCLSKDKTLQ
181 DLVYDLSTSG SGSGLPLFVQ RTVARTIVLQ EIIGKGRFGE VWRGRWRGGD
VAVKIFSSRE
241 ERSWFREAEI YQTVMLRHEN ILGFIAADNK DNGTWTQLWL VSDYHEHGSL
FDYLNRYTVT
301 IEGMIKLALS AASGLAHLHM EIVGTQGKPG IAHRDLKSKN ILVKKNGMCA
IADLGLAVRH
361 DAVTDTIDIA PNQRVGTKRY MAPEVLDETI NMKHFDSFKC ADIYALGLVY
WEIARRCNSG
421 GVHEEYQLPY YDLVPSDPSI EEMRKVVCDQ KLRPNIPNWW QSYEALRVMG
KMMRECWYAN
481 GAARLTALRI KKTLSQLSVQ EDVKI (SEQ ID NO: 14)

The signal peptide is indicated by a single underline and the extracellular domain is indicated in **bold** font.

A processed extracellular human ALK4 polypeptide sequence is as follows:

SGPRGVQALLCACTSCLQANYTCETDGACMVSIFNLDGMEHHVRTCIPKVELVPAGKPFYCL
SSEDLRNTHCCYTDYCNRIDLRVPSGHLKEPEHPSMWGPVE (SEQ ID NO: 15)

A nucleic acid sequence encoding the ALK4 precursor protein is shown below (SEQ ID NO: 16), corresponding to nucleotides 78-1592 of Genbank Reference Sequence NM_004302.4. The signal sequence is underlined and the extracellular domain is indicated in **bold** font.

ATGGCGGAGTCGGCCGGAGCCTCCTCCTTCTTCCCCCTTGTTGTCCTCCTGCTCGCCGGCAG
CGGCGGGTCCGGGCCCCGGGGGGTCCAGGCTCTGCTGTGTGCGTGCACCAGCTGCCTCCAGG

CCAACTACACGTGTGAGACAGATGGGGCCTGCATGGTTTCCATTTTCAATCTGGATGGGATG
GAGCACCATGTGCGCACCTGCATCCCCAAAGTGGAGCTGGTCCCTGCCGGGAAGCCCTTCTA
CTGCCTGAGCTCGGAGGACCTGCGCAACACCCACTGCTGCTACACTGACTACTGCAACAGGA
TCGACTTGAGGGTGCCCAGTGGTCACCTCAAGGAGCCTGAGCACCCGTCCATGTGGGGCCCG
GTGGAGCTGGTAGGCATCATCGCCGGCCCGGTGTTCCTCCTGTTCCTCATCATCATCATTGT
TTTCCTTGTCATTAACTATCATCAGCGTGTCTATCACAACCGCCAGAGACTGGACATGGAAG
ATCCCTCATGTGAGATGTGTCTCTCCAAAGACAAGACGCTCCAGGATCTTGTCTACGATCTC
TCCACCTCAGGGTCTGGCTCAGGGTTACCCCTCTTTGTCCAGCGCACAGTGGCCCGAACCAT
CGTTTTACAAGAGATTATTGGCAAGGGTCGGTTTGGGGAAGTATGGCGGGGCCGCTGGAGGG
GTGGTGATGTGGCTGTGAAAATATTCTCTTCTCGTGAAGAACGGTCTTGGTTCAGGGAAGCA
GAGATATACCAGACGGTCATGCTGCGCCATGAAAACATCCTTGGATTTATTGCTGCTGACAA
TAAAGATAATGGCACCTGGACACAGCTGTGGCTTGTTTCTGACTATCATGAGCACGGGTCCC
TGTTTGATTATCTGAACCGGTACACAGTGACAATTGAGGGGATGATTAAGCTGGCCTTGTCT
GCTGCTAGTGGGCTGGCACACCTGCACATGGAGATCGTGGGCACCCAAGGGAAGCCTGGAAT
TGCTCATCGAGACTTAAAGTCAAAGAACATTCTGGTGAAGAAAAATGGCATGTGTGCCATAG
CAGACCTGGGCCTGGCTGTCCGTCATGATGCAGTCACTGACACCATTGACATTGCCCCGAAT
CAGAGGGTGGGGACCAAACGATACATGGCCCCTGAAGTACTTGATGAAACCATTAATATGAA
ACACTTTGACTCCTTTAAATGTGCTGATATTTATGCCCTCGGGCTTGTATATTGGGAGATTG
CTCGAAGATGCAATTCTGGAGGAGTCCATGAAGAATATCAGCTGCCATATTACGACTTAGTG
CCCTCTGACCCTTCCATTGAGGAAATGCGAAAGGTTGTATGTGATCAGAAGCTGCGTCCCAA
CATCCCCAACTGGTGGCAGAGTTATGAGGCACTGCGGGTGATGGGGAAGATGATGCGAGAGT
GTTGGTATGCCAACGGCGCAGCCCGCCTGACGGCCCTGCGCATCAAGAAGACCCTCTCCCAG
CTCAGCGTGCAGGAAGACGTGAAGATC (SEQ ID NO: 16)

A nucleic acid sequence encoding the extracellular ALK4 polypeptide is as follows:

TCCGGGCCCCGGGGGGTCCAGGCTCTGCTGTGTGCGTGCACCAGCTGCCTCCAGGCCAACTA
CACGTGTGAGACAGATGGGGCCTGCATGGTTTCCATTTTCAATCTGGATGGGATGGAGCACC
ATGTGCGCACCTGCATCCCCAAAGTGGAGCTGGTCCCTGCCGGGAAGCCCTTCTACTGCCTG
AGCTCGGAGGACCTGCGCAACACCCACTGCTGCTACACTGACTACTGCAACAGGATCGACTT
GAGGGTGCCCAGTGGTCACCTCAAGGAGCCTGAGCACCCGTCCATGTGGGGCCCGGTGGAG
(SEQ ID NO: 17)

An alternative isoform of human ALK4 precursor protein sequence, isoform B (NCBI Ref Seq NP_064732.3), is as follows:

1 MVSIFNLDGM EHHVRTCIPK VELVPAGKPF YCLSSEDLRN THCCYTDYCN RIDLRVPSGH

61 LKEPEHPSMW GPVELVGIIA GPVFLLFLII IIVFLVINYH QRVYHNRQRL DMEDPSCEMC

121 LSKDKTLQDL VYDLSTSGSG SGLPLFVQRT VARTIVLQEI IGKGRFGEVW RGRWRGGDVA

181 VKIFSSREER SWFREAEIYQ TVMLRHENIL GFIAADNKDN GTWTQLWLVS DYHEHGSLFD

241 YLNRYTVTIE GMIKLALSAA SGLAHLHMEI VGTQGKPGIA HRDLKSKNIL VKKNGMCAIA

301 DLGLAVRHDA VTDTIDIAPN QRVGTKRYMA PEVLDETINM KHFDSFKCAD IYALGLVYWE

361 IARRCNSGGV HEEYQLPYYD LVPSDPSIEE MRKVVCDQKL RPNIPNWWQS YEALRVMGKM

421 MRECWYANGA ARLTALRIKK TLSQLSVQED VKI (SEQ ID NO: 18)

The extracellular domain is indicated in bold font.

A processed extracellular ALK4 polypeptide sequence is as follows:

1 MVSIFNLDGM EHHVRTCIPK VELVPAGKPF YCLSSEDLRN THCCYTDYCN RIDLRVPSGH

61 LKEPEHPSMW GPVE(SEQ ID NO: 19)

A nucleic acid sequence encoding the ALK4 precursor protein (isoform B) is shown below (SEQ ID NO: 20), corresponding to nucleotides 186-1547 of Genbank Reference Sequence NM_020327.3. The nucleotides encoding the extracellular domain are indicated in **bold** font.

1 ATGGTTTCCA TTTTCAATCT GGATGGGATG GAGCACCATG TGCGCACCTG
51 CATCCCCAAA GTGGAGCTGG TCCCTGCCGG GAAGCCCTTC TACTGCCTGA
101 GCTCGGAGGA CCTGCGCAAC ACCCACTGCT GCTACACTGA CTACTGCAAC
151 AGGATCGACT TGAGGGTGCC CAGTGGTCAC CTCAAGGAGC CTGAGCACCC
201 GTCCATGTGG GGCCCGGTGG AGCTGGTAGG CATCATCGCC GGCCCGGTGT
251 TCCTCCTGTT CCTCATCATC ATCATTGTTT TCCTTGTCAT TAACTATCAT
301 CAGCGTGTCT ATCACAACCG CCAGAGACTG GACATGGAAG ATCCCTCATG
351 TGAGATGTGT CTCTCCAAAG ACAAGACGCT CCAGGATCTT GTCTACGATC
401 TCTCCACCTC AGGGTCTGGC TCAGGGTTAC CCCTCTTTGT CCAGCGCACA
451 GTGGCCCGAA CCATCGTTTT ACAAGAGATT ATTGGCAAGG GTCGGTTTGG
501 GGAAGTATGG CGGGGCCGCT GGAGGGGTGG TGATGTGGCT GTGAAAATAT
551 TCTCTTCTCG TGAAGAACGG TCTTGGTTCA GGGAAGCAGA GATATACCAG
601 ACGGTCATGC TGCGCCATGA AAACATCCTT GGATTTATTG CTGCTGACAA
651 TAAAGATAAT GGCACCTGGA CACAGCTGTG GCTTGTTTCT GACTATCATG
701 AGCACGGGTC CCTGTTTGAT TATCTGAACC GGTACACAGT GACAATTGAG
751 GGGATGATTA AGCTGGCCTT GTCTGCTGCT AGTGGGCTGG CACACCTGCA
801 CATGGAGATC GTGGGCACCC AAGGGAAGCC TGGAATTGCT CATCGAGACT
851 TAAAGTCAAA GAACATTCTG GTGAAGAAAA ATGGCATGTG TGCCATAGCA
901 GACCTGGGCC TGGCTGTCCG TCATGATGCA GTCACTGACA CCATTGACAT
951 TGCCCCGAAT CAGAGGGTGG GGACCAAACG ATACATGGCC CCTGAAGTAC
1001 TTGATGAAAC CATTAATATG AAACACTTTG ACTCCTTTAA ATGTGCTGAT
1051 ATTTATGCCC TCGGGCTTGT ATATTGGGAG ATTGCTCGAA GATGCAATTC
1101 TGGAGGAGTC CATGAAGAAT ATCAGCTGCC ATATTACGAC TTAGTGCCCT
1151 CTGACCCTTC CATTGAGGAA ATGCGAAAGG TTGTATGTGA TCAGAAGCTG
1201 CGTCCCAACA TCCCCAACTG GTGGCAGAGT TATGAGGCAC TGCGGGTGAT
1251 GGGGAAGATG ATGCGAGAGT GTTGGTATGC CAACGGCGCA GCCCGCCTGA
1301 CGGCCCTGCG CATCAAGAAG ACCCTCTCCC AGCTCAGCGT GCAGGAAGAC
1351 GTGAAGATCT AA (SEQ ID NO: 20)

A nucleic acid sequence encoding the extracellular ALK4 polypeptide (isoform B) is as follows:

1 ATGGTTTCCA TTTTCAATCT GGATGGGATG GAGCACCATG TGCGCACCTG
51 CATCCCCAAA GTGGAGCTGG TCCCTGCCGG GAAGCCCTTC TACTGCCTGA
101 GCTCGGAGGA CCTGCGCAAC ACCCACTGCT GCTACACTGA CTACTGCAAC
151 AGGATCGACT TGAGGGTGCC CAGTGGTCAC CTCAAGGAGC CTGAGCACCC
201 GTCCATGTGG GGCCCGGTGG AGCTGGTAGG (SEQ ID NO: 21)

ALK4 is well-conserved among vertebrates, with large stretches of the extracellular domain completely conserved. For example, Figure 4 depicts a multi-sequence alignment of a human ALK4 extracellular domain compared to various ALK4 orthologs. Many of the ligands that bind to ALK4 are also highly conserved. Accordingly, from these alignments, it is possible to predict key amino acid positions within the ligand-binding domain that are important for normal ALK4-ligand binding activities as well as to predict amino acid positions that are likely to be tolerant to substitution without significantly altering normal ALK4-ligand binding activities. Therefore, an active, human ALK4 variant polypeptide useful in accordance with the presently disclosed methods may include one or more amino acids at corresponding positions from the sequence of another vertebrate ALK4, or may include a residue that is similar to that in the human or other vertebrate sequences.

Without meaning to be limiting, the following examples illustrate this approach to defining an active ALK4 variant. As illustrated in Figure 4, V6 in the human ALK4 extracellular domain (SEQ ID NO: 115) is isoleucine in *Mus muculus* ALK4 (SEQ ID NO: 119), and so the position may be altered, and optionally may be altered to another hydrophobic residue such as L, I, or F, or a non-polar residue such as A, as is observed in *Gallus gallus* ALK4 (SEQ ID NO: 118). E40 in the human extracellular domain is K in *Gallus gallus* ALK4, indicating that this site may be tolerant of a wide variety of changes, including polar residues, such as E, D, K, R, H, S, T, P, G, Y, and probably a non-polar residue such as A. S15 in the human extracellular domain is D in *Gallus gallus* ALK4, indicating that a wide structural variation is tolerated at this position, with polar residues favored, such as S, T, R, E, K, H, G, P, G and Y. E40 in the human extracellular domain is K in *Gallus gallus* ALK4, indicating that charged residues will be tolerated at this position, including D, R, K, H, as well as Q and N. R80 in the human extracellular domain is K in *Condylura cristata* ALK4 (SEQ ID NO: 116), indicating that basic residues are tolerated at this position, including R, K, and H. Y77 in the human extracellular domain is F in *Sus scrofa* ALK4 (SEQ ID NO: 120), indicating that aromatic residues are tolerated at this position, including F, W, and Y. P93 in the human extracellular domain is relatively poorly conserved, appearing as S in *Erinaceus europaeus* ALK4 (SEQ ID NO: 117) and N in *Gallus gallus* ALK4, thus essentially any amino acid should be tolerated at this position.

Moreover, ALK4 proteins have been characterized in the art in terms of structural and functional characteristics, particularly with respect to ligand binding [e.g., Harrison et al. (2003) J Biol Chem 278(23):21129-21135; Romano et al. (2012) J Mol Model 18(8):3617-3625; and Calvanese et al. (2009) 15(3):175-183]. In addition to the teachings herein, these references provide amply guidance for how to generate ALK4 variants that retain one or more normal activities (e.g., ligand-binding activity).

For example, a defining structural motif known as a three-finger toxin fold is important for ligand binding by type I and type II receptors and is formed by conserved cysteine residues located at varying positions within the extracellular domain of each monomeric receptor [Greenwald et al. (1999) Nat Struct Biol 6:18-22; and Hinck (2012) FEBS Lett 586:1860-1870]. Accordingly, the core ligand-binding domains of human ALK4, as demarcated by the outermost of these conserved cysteines, corresponds to positions 34-101 of SEQ ID NO: 14 (ALK4 precursor). The structurally less-ordered amino acids flanking these cysteine-demarcated core sequences can be truncated by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 residues at the N-terminus and/or by 1, 2, 3, 4, 5, 6, 7, 8, 9, 10, 11, 12, 13, 14, 15, 16, 17, 18, 19, 20, 21, 22, 23, 24, or 25 residues at the C-terminus without necessarily altering ligand binding. Exemplary ALK4 extracellular domains for N-terminal and/or C-terminal truncation include SEQ ID NOs: 15 and 19.

Accordingly, a general formula for an active portion (*e.g.,* a ligand-binding portion) of ALK4 comprises amino acids 34-101 with respect to SEQ ID NO: 14. Therefore ALK4 polypeptides may, for example, comprise, consists essentially of, or consists of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to a portion of ALK4 beginning at a residue corresponding to any one of amino acids 24-34 (e.g., beginning at any one of amino acids 24, 25, 26, 27, 28, 29, 30, 31, 32, 33, or 34) of SEQ ID NO: 14 and ending at a position corresponding to any one amino acids 101-126 (e.g., ending at any one of amino acids 101, 102, 103, 104, 105, 106, 107, 108, 109, 110, 111, 112, 113, 114, 115, 116, 117, 118, 119, 120, 121, 122, 123, 124, 125, or 126) of SEQ ID NO: 14. Other examples include constructs that begin at a position from 24-34 (e.g., any one of positions 24, 25, 26, 27, 28, 29, 30, 31, 32, 33, or 34), 25-34 (e.g., any one of positions 25, 26, 27, 28, 29, 30, 31, 32, 33, or 34), or 26-34 (*e.g.,* any one of positions 26, 27, 28, 29, 30, 31, 32, 33, or 34) of SEQ ID NO: 14 and end at a position from 101-126 (*e.g.,* any one of positions 101, 102, 103, 104, 105, 106, 107, 108, 109, 110, 111, 112, 113, 114, 115, 116, 117, 118, 119, 120, 121, 122, 123, 124, 125, or 126), 102-126 (*e.g.,* any one of positions 102, 103, 104, 105, 106, 107, 108, 109, 110, 111, 112, 113, 114, 115, 116, 117, 118, 119, 120, 121, 122, 123, 124, 125, or 126), 101-125 (*e.g.,* any one of positions 101, 102, 103, 104, 105, 106, 107, 108, 109, 110, 111, 112, 113, 114, 115, 116, 117, 118, 119, 120, 121, 122, 123, 124, or 125), 101-124 (*e*.*g*., any one of positions 101, 102, 103, 104, 105, 106, 107, 108, 109, 110, 111, 112, 113, 114, 115, 116, 117, 118, 119, 120, 121, 122, 123, or 124), 101-121 (*e.g.,* any one of positions 101, 102, 103, 104, 105, 106, 107, 108, 109, 110, 111, 112, 113, 114, 115, 116, 117, 118, 119, 120, or 121), 111-126 (*e.g.,* any one of positions 111, 112, 113, 114, 115, 116, 117, 118, 119, 120, 121, 122, 123, 124, 125, or 126), 111-125 (*e.g.,* any one of positions 111, 112, 113, 114, 115, 116, 117, 118, 119, 120, 121, 122, 123, 124, or 125), 111-124 (*e.g.,* any one of positions 111, 112, 113, 114, 115, 116, 117, 118, 119, 120, 121, 122, 123, or 124), 121-126 (*e.g.,* any one of positions 121, 122, 123, 124, 125, or 126), 121-125 (*e.g.,* any one of positions 121, 122, 123, 124, or 125), 121-124 (*e.g.,* any one of positions 121, 122, 123, or 124), or 124-126 (*e.g.,* any one of positions 124, 125, or 126) of SEQ ID NO: 14. Variants within these ranges are also contemplated, particularly those having at least 70%, 75%, 80%, 85%, 86%, 87%, 88%, 89%, 90%, 91%, 92%, 93%, 94%, 95%, 96%, 97%, 98%, 99%, or 100% identity to the corresponding portion of SEQ ID NO: 14.

The variations described herein may be combined in various ways. In some embodiments, ALK4 variants comprise no more than 1, 2, 5, 6, 7, 8, 9, 10 or 15 conservative amino acid changes in the ligand-binding pocket. Sites outside the binding pocket, at which variability may be particularly well tolerated, include the amino and carboxy termini of the extracellular domain (as noted above).

In certain embodiments, the disclosure relates to ActRII antagonists (inhibitors) that are heteromultimers comprising at least one ALK4 polypeptide, which includes fragments, functional variants, and modified forms thereof as well as uses thereof (e.g., increasing an immune response in a patient in need thereof and treating cancer). Preferably, ALK4 polypeptides are soluble (*e.g.,* an extracellular domain of ALK4). In some embodiments, heteromultimers comprising an ALK4 polypeptide inhibit (*e.g.,* Smad signaling) of one or more TGFβ superfamily ligands [*e.g.,* GDF11, GDF8, activin (activin A, activin B, activin AB, activin C, activin E) BMP6, GDF3, BMP10, and/or BMP9]. In some embodiments, heteromultimers comprising an ALK4 polypeptide bind to one or more TGFβ superfamily ligands [*e.g.,* GDF11, GDF8, activin (activin A, activin B, activin AB, activin C, activin E) BMP6, GDF3, BMP10, and/or BMP9]. In some embodiments, heteromultimers comprise at least one ALK4 polypeptide that is at least 70%, 75%, 80%, 85%, 86%, 87%, 88%, 89%, 90%, 91%, 92%, 93%, 94%, 95%, 97%, 98%, 99%, 100% identical to amino acids 34-101 with respect to SEQ ID NO: 14. In some embodiments, heteromultimers comprise at least one ALK4 polypeptide that is at least 70%, 75%, 80%, 85%, 86%, 87%, 88%, 89%, 90%, 91%, 92%, 93%, 94%, 95%, 97%, 98%, 99%, or 100% identical to the amino acid sequence of SEQ ID NO: 14, 15, 18, 19, 73, 74, 76, 77, 79, and 80. In some embodiments, heteromultimer comprise at least one ALK4 polypeptide that consist or consist essentially of at least one ALK4 polypeptide that is at least 70%, 75%, 80%, 85%, 86%, 87%, 88%, 89%, 90%, 91%, 92%, 93%, 94%, 95%, 97%, 98%, 99%, or 100% identical to the amino acid sequence of SEQ ID NO: 14, 15, 18, 19, 74, 76, 77, 79, 80, 143, and 145.

In certain aspects, the present disclosure relates to heteromultimer complexes comprising one or more ALK4 receptor polypeptides (*e.g.,* SEQ ID Nos: 14, 15, 18, 19, 74, 76, 77, 79, 80, 143, and 145 and variants thereof) and one or more ActRIIB receptor polypeptides (*e.g.,* SEQ ID NOs: 1, 2, 3, 4, 5, 6, 58, 59, 60, 63, 64, 65, 66, 68, 69, 70, 71, 73, 77, 78, 131, 132, 133, 139, 141 and variants thereof), which are generally referred to herein as "ALK4:ActRIIB heteromultimer complexes" or "ALK4:ActRIIB heteromultimers", including uses thereof (e.g., increasing an immune response in a patient in need thereof and treating cancer). Preferably, ALK4:ActRIIB heteromultimers are soluble [*e.g.,* a heteromultimer complex comprises a soluble portion (domain) of an ALK4 receptor and a soluble portion (domain) of an ActRIIB receptor]. In general, the extracellular domains of ALK4 and ActRIIB correspond to soluble portion of these receptors. Therefore, in some embodiments, ALK4:ActRIIB heteromultimers comprise an extracellular domain of an ALK4 receptor and an extracellular domain of an ActRIIB receptor. In some embodiments, ALK4:ActRIIB heteromultimers inhibit (*e.g.,* Smad signaling) of one or more TGFβ superfamily ligands [*e.g.,* GDF11, GDF8, activin (activin A, activin B, activin AB, activin C, activin E) BMP6, GDF3, BMP10, and/or BMP9]. In some embodiments, ALK4:ActRIIB heteromultimers bind to one or more TGFβ superfamily ligands [*e.g.,* GDF11, GDF8, activin (activin A, activin B, activin AB, activin C, activin E) BMP6, GDF3, BMP10, and/or BMP9]. In some embodiments, ALK4:ActRIIB heteromultimers comprise at least one ALK4 polypeptide that comprises, consists essentially of, or consists of a sequence that is at least 70%, 75%, 80%, 85%, 86%, 87%, 88%, 89%, 90%, 91%, 92%, 93%, 94% 95%, 97%, 98%, 99%, or 100% identical to the amino acid sequence of SEQ ID NO: 14, 15, 18, 19, 74, 76, 77, 79, 80, 143, and 145. In some embodiments, ALK4:ActRIIB heteromultimer complexes of the disclosure comprise at least one ALK4 polypeptide that comprises, consists essentially of, consists of a sequence that is at least 70%, 75%, 80%, 85%, 86%, 87%, 88%, 89%, 90%, 91%, 92%, 93%, 94% 95%, 97%, 98%, 99%, or 100% identical to a portion of ALK4 beginning at a residue corresponding to any one of amino acids 24-34, 25-34, or 26-34 of SEQ ID NO: 14 and ending at a position from 101-126, 102-126, 101-125, 101-124, 101-121, 111-126, 111-125, 111-124, 121-126, 121-125, 121-124, or 124-126 of SEQ ID NO: 14. In some embodiments, ALK4:ActRIIB heteromultimers comprise at least one ALK4 polypeptide that comprises, consists essentially of, consists of a sequence that is at least 70%, 75%, 80%, 85%, 86%, 87%, 88%, 89%, 90%, 91%, 92%, 93%, 94% 95%, 97%, 98%, 99%, or 100% identical to amino acids 34-101 with respect to SEQ ID NO: 14. In some embodiments, ALK4-ActRIIB heteromultimers comprise at least one ActRIIB polypeptide that comprises, consists essentially of, consists of a sequence that is at least 70%, 75%, 80%, 85%, 86%, 87%, 88%, 89%, 90%, 91%, 92%, 93%, 94% 95%, 97%, 98%, 99%, or 100% identical to the amino acid sequence of any one of SEQ ID NOs: 1, 2, 3, 4, 5, 6, 58, 59, 60, 63, 64, 65, 66, 68, 69, 70, 71, 73, 77, 78, 131, 132, 133, 139, 141. In some embodiments, ALK4:ActRIIB heteromultimer complexes of the disclosure comprise at least one ActRIIB polypeptide that comprises, consists essentially of, consists of a sequence that is at least 70%, 75%, 80%, 85%, 86%, 87%, 88%, 89%, 90%, 91%, 92%, 93%, 94% 95%, 97%, 98%, 99%, or 100% identical to a portion of ActRIIB beginning at a residue corresponding to any one of amino acids 20-29, 20-24, 21-24, 22-25, or 21-29 and end at a position from 109-134, 119-134, 119-133, 129-134, or 129-133 of SEQ ID NO: 1. In some embodiments, ALK4:ActRIIB heteromultimers comprise at least one ActRIIB polypeptide that comprises, consists essentially of, consists of a sequence that is at least 70%, 75%, 80%, 85%, 86%, 87%, 88%, 89%, 90%, 91%, 92%, 93%, 94% 95%, 97%, 98%, 99%, or 100% identical to amino acids 29-109 of SEQ ID NO: 1. In some embodiments, ALK4:ActRIIB heteromultimers comprise at least one ActRIIB polypeptide that comprises, consists essentially of, consists of a sequence that is at least 70%, 75%, 80%, 85%, 86%, 87%, 88%, 89%, 90%, 91%, 92%, 93%, 94% 95%, 97%, 98%, 99%, or 100% identical to amino acids 25-131 of SEQ ID NO: 1. In certain embodiments, ALK4:ActRIIB heteromultimer complexes of the disclosure comprise at least one ActRIIB polypeptide wherein the position corresponding to L79 of SEQ ID NO: 1 is not an acidic amino acid (*i.e.,* not naturally occurring D or E amino acid residues or an artificial acidic amino acid residue). ALK4:ActRIIB heteromultimers of the disclosure include, *e.g.,* heterodimers, heterotrimers, heterotetramers and further higher order oligomeric structures. See, e.g., Figures 21-23. In certain preferred embodiments, heteromultimer complexes of the disclosure are ALK4:ActRIIB heterodimers.

Naturally occurring TGFβRII proteins are transmembrane proteins, with a portion of the protein positioned outside the cell (the extracellular portion) and a portion of the protein positioned inside the cell (the intracellular portion). Aspects of the present disclosure encompass variant TGFβRII polypeptides comprising mutations within the extracellular domain and/or truncated portions of the extracellular domain of TGFβRII. As described above, human TGFβRII occurs naturally in at least two isoforms - A (long) and B (short) - generated by alternative splicing in the extracellular domain (ECD) (Figures 11 and 10 and SEQ ID NOS: 35 and 34). SEQ ID NO: 148, which corresponds to residues 23-184 of SEQ ID NO: 35, depicts the native full-length extracellular domain of the long isoform of TGFβRII. Unless noted otherwise, amino acid position numbering with regard to variants based on the TGFβRII short and long isoforms refers to the corresponding position in the native precursors, SEQ ID NO: 34 and SEQ ID NO:35, respectively.

In certain embodiments, the disclosure provides variant TGFβRII polypeptides. A TGFβRII polypeptide of the disclosure may bind to and inhibit the function of a TGFβ superfamily member, such as but not limited to, TGFβ1 or TGFβ3. TGFβRII polypeptides may include a polypeptide consisting of, or comprising, an amino acid sequence at least 80% identical, and optionally at least 85%, 90%, 91%, 92%, 93%, 94% 95%, 96%, 97%, 98%, 99%, or 100% identical to a truncated ECD domain of a naturally occurring TGFβRII polypeptide, whose C-terminus occurs at any of amino acids 153-159 (e.g., 153, 154, 155, 156, 157, 158, or 159) of SEQ ID NO: 34. TGFβRII polypeptides may include a polypeptide consisting of, or comprising, an amino acid sequence at least 80% identical, and optionally at least 85%, 90%, 91%, 92%, 93%, 94% 95%, 96%, 97%, 98%, 99%, or 100% identical to a truncated ECD domain of a naturally occurring TGFβRII polypeptide, whose C-terminus occurs at any of amino acids 178-184 (e.g., 178, 179, 180, 181, 182, 183, or 184) of SEQ ID NO: 35. Optionally, a TGFβRII polypeptide does not include more than 5 consecutive amino acids, or more than 10, 20, 30, 40, 50, 52, 60, 70, 80, 90, 100, 150 or 200 or more consecutive amino acids from a sequence consisting of amino acids 160-567 of SEQ ID NO: 34 or from a sequence consisting of amino acids 185-592 of SEQ ID NO: 35. The unprocessed TGFβRII polypeptide may either include or exclude any signal sequence, as well as any sequence N-terminal to the signal sequence. As elaborated herein, the N-terminus of the mature (processed) TGFβRII polypeptide may occur at any of amino acids 23-35 of SEQ ID NO: 34 or 23-60 of SEQ ID NO: 35. It will be understood by one of skill in the art that corresponding variants based on the long isoform of TGFβRII will include nucleotide sequences encoding the 25-amino acid insertion along with a conservative Val-Ile substitution at the flanking position C-terminal to the insertion. The TGFβRII polypeptides accordingly may include isolated extracellular portions of TGFβRII polypeptides, including both the short and the long isoforms, variants thereof (including variants that comprise, for example, no more than 2, 3, 4, 5, 10, 15, 20, 25, 30, or 35 amino acid substitutions in the sequence corresponding to amino acids 23-159 of SEQ ID NO: 34 or amino acids 23-184 of SEQ ID NO: 35), fragments thereof, and fusion proteins comprising any of the foregoing, but in each case preferably any of the foregoing TGFβRII polypeptides will retain substantial affinity for at least one of TGFβ1 or TGFβ3. Generally, a TGFβRII polypeptide will be designed to be soluble in aqueous solutions at biologically relevant temperatures, pH levels, and osmolarity.

Taken together, an active portion of a TGFβRII polypeptide may comprise amino acid sequences 23-153, 23-154, 23-155, 23-156, 23-157, or 23-158 of SEQ ID NO: 34, as well as variants of these sequences starting at any of amino acids 24-35 of SEQ ID NO: 34. Similarly, an active portion of a TGFβRII polypeptide may comprise amino acid sequences 23-178, 23-179, 23-180, 23-181, 23-182, or 23-183 of SEQ ID NO: 35, as well as variants of these sequences starting at any of amino acids 24-60 of SEQ ID NO: 35. Exemplary TGFβRII polypeptides comprise amino acid sequences 29-159, 35-159, 23-153, 29-153 and 35-153 of SEQ ID NO: 34 or amino acid sequences 29-184, 60-184, 23-178, 29-178 and 60-178 of SEQ ID NO: 35. Variants within these ranges are also contemplated, particularly those having at least 85%, 90%, 91%, 92%, 93%, 94% 95%, 96%, 97%, 98%, 99%, or 100% identity to the corresponding portion of SEQ ID NO: 34 or SEQ ID NO: 35. A TGFβRII polypeptide may be selected that does not include the sequence consisting of amino acids 160-567 of SEQ ID NO: 34 or amino acids 185-592 of SEQ ID NO: 35.

TGFβRII polypeptides may additionally include any of various leader sequences at the N-terminus. Such a sequence would allow the peptides to be expressed and targeted to the secretion pathway in a eukaryotic system. See, e.g., Ernst et al., U.S. Pat. No. 5,082,783 (1992). Alternatively, a native TGFβRII signal sequence may be used to effect extrusion from the cell. Possible leader sequences include native leaders, tissue plasminogen activator (TPA) and honeybee mellitin. Processing of signal peptides may vary depending on the leader sequence chosen, the cell type used and culture conditions, among other variables, and therefore actual N-terminal start sites for mature TGFβRII polypeptides may shift by 1, 2, 3, 4 or 5 amino acids in either the N-terminal or C-terminal direction. Examples of a TGFβRII-Fc fusion proteins include SEQ ID NOs: 148 and 150, as shown herein with the TGFβRII polypeptide portion underlined. It will be understood by one of skill in the art that corresponding variants based on the long isoform of TGFβRII will include the 25-amino acid insertion along with a conservative Val-Ile substitution at the flanking position C-terminal to the insertion. In certain aspects the disclosure relates to a TGFβRII polypeptide that comprises amino acid sequence that is at least 80%, 85%, 90%, 91%, 92%, 93%, 94%, 95%, 96%, 97%, 98%, 99%, or 100% identical to the amino acid sequence of SEQ ID NO: 150 as well as uses thereof in accordance with the methods described herein. In certain aspects the disclosure relates to a TGFβRII polypeptide that comprises an amino acid sequence that is at least 80%, 85%, 90%, 91%, 92%, 93%, 94%, 95%, 96%, 97%, 98%, 99%, or 100% identical to the amino acid sequence of SEQ ID NO: 148 as well as uses thereof in accordance with the methods described herein. In certain aspects the disclosure relates to a TGFβRII polypeptide that comprises amino acid sequence that is at least 80%, 85%, 90%, 91%, 92%, 93%, 94%, 95%, 96%, 97%, 98%, 99%, or 100% identical to an amino acid sequence that begins at any one of amino acids 25-46 (e.g., 25, 26, 27, 28, 29, 30, 31, 32, 33, 34, 35, 36, 37, 38, 39, 40, 41, 42, 43, 44, 45, or 46) of SEQ ID NO: 148 and ends and any one of amino acids 170-186 (e.g., 170, 171, 172, 173, 174, 175, 176, 177, 178, 179, 180, 181, 182, 183, 184, 185, or 186) of SEQ ID NO: 148 as well as uses thereof in accordance with the methods described herein.

In some embodiments, the present disclosure contemplates making functional variants by modifying the structure of an ALK4 polypeptide, an ActRII polypeptide, and/or a TGFβRII polypeptide for such purposes as enhancing therapeutic efficacy or stability (e.g., shelf-life and resistance to proteolytic degradation *in vivo*). Variants can be produced by amino acid substitution, deletion, addition, or combinations thereof. For instance, it is reasonable to expect that an isolated replacement of a leucine with an isoleucine or valine, an aspartate with a glutamate, a threonine with a serine, or a similar replacement of an amino acid with a structurally related amino acid (*e.g.,* conservative mutations) will not have a major effect on the biological activity of the resulting molecule. Conservative replacements are those that take place within a family of amino acids that are related in their side chains. Whether a change in the amino acid sequence of a polypeptide of the disclosure results in a functional homolog can be readily determined by assessing the ability of the variant polypeptide to produce a response in cells in a fashion similar to the wild-type polypeptide, or to bind to one or more ligands including, for example, BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11BMP11, GDF15/MIC1, TGFβ1, TGFβ2, TGFβ3, activin A, activin B, activin C, activin E, activin AB, activin AC, nodal, glial cell-derived neurotrophic factor (GDNF), neurturin, artemin, persephin, MIS, and Lefty.

In certain embodiments, the present disclosure contemplates specific mutations of an ALK4 polypeptide, an ActRII polypeptide, and/or a TGFβRII polypeptide so as to alter the glycosylation of the polypeptide. Such mutations may be selected so as to introduce or eliminate one or more glycosylation sites, such as O-linked or N-linked glycosylation sites. Asparagine-linked glycosylation recognition sites generally comprise a tripeptide sequence, asparagine-X-threonine or asparagine-X-serine (where "X" is any amino acid) which is specifically recognized by appropriate cellular glycosylation enzymes. The alteration may also be made by the addition of, or substitution by, one or more serine or threonine residues to the sequence of the polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on a polypeptide is by chemical or enzymatic coupling of glycosides to the polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. Removal of one or more carbohydrate moieties present on a polypeptide may be accomplished chemically and/or enzymatically. Chemical deglycosylation may involve, for example, exposure of a polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Enzymatic cleavage of carbohydrate moieties on polypeptides can be achieved by the use of a variety of endo- and exo-glycosidases as described by Thotakura et al. [Meth. Enzymol. (1987) 138:350]. The sequence of a polypeptide may be adjusted, as appropriate, depending on the type of expression system used, as mammalian, yeast, insect, and plant cells may all introduce differing glycosylation patterns that can be affected by the amino acid sequence of the peptide. In general, ActRII polypeptides, ALK4 polypeptides, TGFβRII polypeptides, and heteromultimers of the present disclosure for use in humans may be expressed in a mammalian cell line that provides proper glycosylation, such as HEK293 or CHO cell lines, although other mammalian expression cell lines are expected to be useful as well.

The disclosure further contemplates a method of generating mutants, particularly sets of combinatorial mutants of an ALK4, ActRII, and/or TGFβRII polypeptide as well as truncation mutants. Pools of combinatorial mutants are especially useful for identifying functionally active (e.g., TGFβ superfamily ligand binding) ALK4, ActRII, and/or TGFβRII sequences. The purpose of screening such combinatorial libraries may be to generate, for example, polypeptides variants which have altered properties, such as altered pharmacokinetic or altered ligand binding. A variety of screening assays are provided below, and such assays may be used to evaluate variants. For example, ActRII polypeptide, ALK4 polypeptide, TGFβRII polypeptide and ALK4:ActRIIB heteromultimer variants may be screened for ability to bind to one or more ligands (e.g., BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11BMP11, GDF15/MIC1, TGFβ1, TGFβ2, TGFβ3, activin A, activin B, activin AB, activin AC, nodal, glial cell-derived neurotrophic factor (GDNF), neurturin, artemin, persephin, MIS, and Lefty), to prevent binding of a TGFβ superfamily ligand to a TGFβ superfamily receptor, and/or to interfere with signaling caused by a ligand.

The activity of ActRII polypeptides, ALK4 polypeptides, TGFβRII polypeptides or ALK4:ActRIIB heteromultimers also may be tested in a cell-based assay or *in vivo.* For example, the effect of an ActRII polypeptides, ALK4 polypeptides, TGFβRII polypeptide or ALK4:ActRIIB heteromultimers on the expression of genes involved in cancer growth in a cancer cell may be assessed. This may, as needed, be performed in the presence of one or more recombinant ligand proteins (*e.g.,* BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11BMP11, GDF15/MIC1, TGFβ1, TGFβ2, TGFβ3, activin A, activin B, activin C, activin E, activin AB, activin AC, nodal, glial cell-derived neurotrophic factor (GDNF), neurturin, artemin, persephin, MIS, and Lefty), and cells may be transfected so as to produce an ActRII polypeptide, ALK4 polypeptide, TGFβRII polypeptide, or ALK4:ActRIIB heteromultimes, and optionally, a TGFβ superfamily ligand. Likewise, an ActRII polypeptide, ALK4 polypeptide, TGFβRII polypeptide, or ALK4: ActRIIB heteromultimer heteromultimer may be administered to a mouse or other animal, and one or more measurements, such as muscle formation and strength may be assessed using art-recognized methods. Similarly, the activity of an ActRII polypeptide, ALK4 polypeptide, TGFβRII polypeptide, or ALK4:ActRIIB heteromultimer or variants thereof may be tested in cancer cells for any effect on growth of these cells, for example, by the assays as described herein and those of common knowledge in the art. A SMAD-responsive reporter gene may be used in such cell lines to monitor effects on downstream signaling.

Combinatorial-derived variants can be generated which have increased selectivity or generally increased potency relative to a reference ActRII polypeptide, ALK4 polypeptide, TGFβRII polypeptide, or ALK4: ActRIIB heteromultimer. Such variants, when expressed from recombinant DNA constructs, can be used in gene therapy protocols. Likewise, mutagenesis can give rise to variants which have intracellular half-lives dramatically different than the corresponding unmodified ActRII polypeptide, ALK4 polypeptide, TGFβRII polypeptide, or ALK4:ActRIIB heteromultimer. For example, the altered protein can be rendered either more stable or less stable to proteolytic degradation or other cellular processes which result in destruction, or otherwise inactivation, of an unmodified polypeptide. Such variants, and the genes which encode them, can be utilized to alter polypeptide complex levels by modulating the half-life of the polypeptide. For instance, a short half-life can give rise to more transient biological effects and, when part of an inducible expression system, can allow tighter control of recombinant polypeptide complex levels within the cell. In an Fc fusion protein, mutations may be made in the linker (if any) and/or the Fc portion to alter the half-life of the ActRII polypeptide, ALK4 polypeptide, TGFβRII polypeptide, or ALK4:ActRIIB heteromultimer.

A combinatorial library may be produced by way of a degenerate library of genes encoding a library of polypeptides which each include at least a portion of potential ALK4 TGFβRII, and/or ActRII sequences. For instance, a mixture of synthetic oligonucleotides can be enzymatically ligated into gene sequences such that the degenerate set of potential ALK4, TGFβRII, and/or ActRII encoding nucleotide sequences are expressible as individual polypeptides, or alternatively, as a set of larger fusion proteins (*e.g.,* for phage display).

There are many ways by which the library of potential homologs can be generated from a degenerate oligonucleotide sequence. Chemical synthesis of a degenerate gene sequence can be carried out in an automatic DNA synthesizer, and the synthetic genes can then be ligated into an appropriate vector for expression. The synthesis of degenerate oligonucleotides is well known in the art [Narang, SA (1983) Tetrahedron 39:3; Itakura et al. (1981) Recombinant DNA, Proc. 3rd Cleveland Sympos. Macromolecules, ed. AG Walton, Amsterdam: Elsevier pp273-289; Itakura et al. (1984) Annu. Rev. Biochem. 53:323; Itakura et al. (1984) Science 198:1056; and Ike et al. (1983) Nucleic Acid Res. 11:477]. Such techniques have been employed in the directed evolution of other proteins [Scott et al., (1990) Science 249:386-390; Roberts et al. (1992) PNAS USA 89:2429-2433; Devlin et al. (1990) Science 249: 404-406; Cwirla et al., (1990) PNAS USA 87: 6378-6382; as well as U.S. Patent Nos: 5,223,409, 5,198,346, and 5,096,815].

Alternatively, other forms of mutagenesis can be utilized to generate a combinatorial library. For example, ActRII polypeptides, ALK4 polypeptides, TGFβRII polypeptide or ALK4:ActRIIB heteromultimers of the disclosure can be generated and isolated from a library by screening using, for example, alanine scanning mutagenesis [Ruf et al. (1994) Biochemistry 33:1565-1572; Wang et al. (1994) J. Biol. Chem. 269:3095-3099; Balint et al. (1993) Gene 137:109-118; Grodberg et al. (1993) Eur. J. Biochem. 218:597-601; Nagashima et al. (1993) J. Biol. Chem. 268:2888-2892; Lowman et al. (1991) Biochemistry 30:10832-10838; and Cunningham et al. (1989) Science 244:1081-1085], by linker scanning mutagenesis [Gustin et al. (1993) Virology 193:653-660; and Brown et al. (1992) Mol. Cell Biol. 12:2644-2652; McKnight et al. (1982) Science 232:316], by saturation mutagenesis [Meyers et al., (1986) Science 232:613]; by PCR mutagenesis [Leung et al. (1989) Method Cell Mol Biol 1:11-19]; or by random mutagenesis, including chemical mutagenesis [Miller et al. (1992) A Short Course in Bacterial Genetics, CSHL Press, Cold Spring Harbor, NY; and Greener et al. (1994) Strategies in Mol Biol 7:32-34]. Linker scanning mutagenesis, particularly in a combinatorial setting, is an attractive method for identifying truncated (bioactive) forms of ALK4, TGFβRII and/or ActRII polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRII polypeptides, ALK4 polypeptides, TGFβRII polypeptides or ALK4:ActRIIB heteromultimers. The most widely used techniques for screening large gene libraries typically comprise cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include ligand (e.g., BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11/BMP11, GDF15/MIC1, TGFβ1, TGFβ2, TGFβ3, activin A, activin B, activin C, activin E, activin AB, activin AC, nodal, glial cell-derived neurotrophic factor (GDNF), neurturin, artemin, persephin, MIS, and Lefty) binding assays and/or ligand-mediated cell signaling assays.

In certain embodiments, ActRII polypeptides, ALK4 polypeptides, TGFβRII polypeptides, or ALK4:ActRIIB heteromultimers may further comprise post-translational modifications in addition to any that are naturally present in the ALK4, TGFβRII, and/or ActRII polypeptide. Such modifications include, but are not limited to, acetylation, carboxylation, glycosylation, phosphorylation, lipidation, and acylation. As a result, ActRII polypeptides, ALK4 polypeptides, TGFβRII polypeptides or ALK4:ActRIIB heteromultimers may comprise non-amino acid elements, such as polyethylene glycols, lipids, polysaccharide or monosaccharide, and phosphates. Effects of such non-amino acid elements on the functionality of a ActRII polypeptide, ALK4 polypeptide, TGFβRII polypeptide, or ALK4:ActRIIB heteromultimer may be tested as described herein for other heteromultimer complex variants. When a polypeptide of the disclosure is produced in cells by cleaving a nascent form of the polypeptide, post-translational processing may also be important for correct folding and/or function of the protein. Different cells (*e.g.,* CHO, HeLa, MDCK, 293, WI38, NIH-3T3 or HEK293) have specific cellular machinery and characteristic mechanisms for such post-translational activities and may be chosen to ensure the correct modification and processing of the ALK4, TGFβRII, and/or ActRII polypeptide.

In certain aspects, ActRII polypeptides, TGFβRII polypeptides, and/or ALK4 polypeptides of the disclosure are fusion proteins comprising at least a portion (domain) of an ActRII polypeptide (*e.g.,* an ActRIIA or ActRIIB polypeptide), TGFβRII, or ALK4 polypeptide and one or more heterologous portions (domains). Well-known examples of such fusion domains include, but are not limited to, polyhistidine, Glu-Glu, glutathione S-transferase (GST), thioredoxin, protein A, protein G, an immunoglobulin heavy-chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt- conjugated resins are used. Many of such matrices are available in "kit" form, such as the Pharmacia GST purification system and the QIAexpress^{™} system (Qiagen) useful with (HIS₆) fusion partners. As another example, a fusion domain may be selected so as to facilitate detection of the ActRII polypeptide. Examples of such detection domains include the various fluorescent proteins (*e.g.,* GFP) as well as "epitope tags," which are usually short peptide sequences for which a specific antibody is available. Well-known epitope tags for which specific monoclonal antibodies are readily available include FLAG, influenza virus haemagglutinin (HA), and c-myc tags. In some cases, the fusion domains have a protease cleavage site, such as for Factor Xa or thrombin, which allows the relevant protease to partially digest the fusion proteins and thereby liberate the recombinant proteins therefrom. The liberated proteins can then be isolated from the fusion domain by subsequent chromatographic separation. Other types of fusion domains that may be selected include multimerizing (*e.g.,* dimerizing, tetramerizing) domains and functional domains (that confer an additional biological function) including, for example constant domains from immunoglobulins (e.g., Fc domains). As described herein, in some embodiments, preferred multimerization domains are modified Fc domains that promote asymmetrical pairing to form heteromultimer structures (e.g., ALK4:ActRIIB heteromultimers)

In certain aspects, ActRII polypeptides, TGFβRII polypeptides, and/or ALK4 polypeptides of the present disclosure contain one or more modifications that are capable of "stabilizing" the polypeptides. By "stabilizing" is meant anything that increases the *in vitro* half-life, serum half-life, regardless of whether this is because of decreased destruction, decreased clearance by the kidney, or other pharmacokinetic effect of the agent. For example, such modifications enhance the shelf-life of the polypeptides, enhance circulatory half-life of the polypeptides, and/or reduce proteolytic degradation of the polypeptides. Such stabilizing modifications include, but are not limited to, fusion proteins (including, for example, fusion proteins comprising an ActRII polypeptide, TGFβRII polypeptide, or ALK4 polypeptide domain and a stabilizer domain), modifications of a glycosylation site (including, for example, addition of a glycosylation site to a polypeptide of the disclosure), and modifications of carbohydrate moiety (including, for example, removal of carbohydrate moieties from a polypeptide of the disclosure). As used herein, the term "stabilizer domain" not only refers to a fusion domain (*e.g.,* an immunoglobulin Fc domain) as in the case of fusion proteins, but also includes nonproteinaceous modifications such as a carbohydrate moiety, or nonproteinaceous moiety, such as polyethylene glycol. In certain preferred embodiments, an ActRII polypeptide, TGFβRII polypeptide, and/or ALK4 polypeptide is fused with a heterologous domain that stabilizes the polypeptide (a "stabilizer" domain), preferably a heterologous domain that increases stability of the polypeptide *in vivo.* Fusions with a constant domain of an immunoglobulin (e.g., an Fc domain) are known to confer desirable pharmacokinetic properties on a wide range of proteins. Likewise, fusions to human serum albumin can confer desirable stabilizing properties.

In some embodiments, ALK4, TGFβRII, and/or ActRII polypeptides of the disclosure are Fc fusion proteins. An example of a native amino acid sequence that may be used for the Fc portion of human IgG1 (G1Fc) is shown below (SEQ ID NO: 22). Dotted underline indicates the hinge region, and solid underline indicates positions with naturally occurring variants. In part, the disclosure provides polypeptides comprising, consisting essential of, or consisting of amino acid sequences with 70%, 75%, 80%, 85%, 86%, 87%, 88%, 89%, 90%, 91%, 92%, 93%, 94%, 95%, 96%, 97%, 98%, 99%, or 100% identity to SEQ ID NO: 22. Naturally occurring variants in G1Fc would include E134D and M136L according to the numbering system used in SEQ ID NO: 22 (see Uniprot P01857).

1 THTCPPCPAP ELLGGPSVFL FPPKPKDTLM ISRTPEVTCV VVDVSHEDPE
51 VKFNWYVDGV EVHNAKTKPR EEQYNSTYRV VSVLTVLHQD WLNGKEYKCK
101 VSNKALPAPI EKTISKAKGQ PREPQVYTLP PSREEMTKNQ VSLTCLVKGF
151 YPSDIAVEWE SNGQPENNYK TTPPVLDSDG SFFLYSKLTV DKSRWQQGNV
201 FSCSVMHEAL HNHYTQKSLS LSPGK (SEQ ID NO: 22)

Optionally, the IgG1 Fc domain has one or more mutations at residues such as Asp-265, lysine 322, and Asn-434. In certain cases, the mutant IgG1 Fc domain having one or more of these mutations (*e.g.,* Asp-265 mutation) has reduced ability of binding to the Fcγ receptor relative to a wild-type Fc domain. In other cases, the mutant Fc domain having one or more of these mutations (*e.g.,* Asn-434 mutation) has increased ability of binding to the MHC class I-related Fc-receptor (FcRN) relative to a wild-type IgG1 Fc domain.

An example of a native amino acid sequence that may be used for the Fc portion of human IgG2 (G2Fc) is shown below (SEQ ID NO: 23). Dotted underline indicates the hinge region and double underline indicates positions where there are data base conflicts in the sequence (according to UniProt P01859). In part, the disclosure provides polypeptides comprising, consisting essential of, or consisting of amino acid sequences with 70%, 75%, 80%, 85%, 86%, 87%, 88%, 89%, 90%, 91%, 92%, 93%, 94%, 95%, 96%, 97%, 98%, 99%, or 100% identity to SEQ ID NO: 23.

1 VECPPCPAPP VAGPSVFLFP PKPKDTLMIS RTPEVTCVVV DVSHEDPEVQ
51 FNWYVDGVEV HNAKTKPREE QFNSTFRVVS VLTVVHQDWL NGKEYKCKVS
101 NKGLPAPIEK TISKTKGQPR EPQVYTLPPS REEMTKNQVS LTCLVKGFYP
151 SDIAVEWESN GQPENNYKTT PPMLDSDGSF FLYSKLTVDK SRWQQGNVFS
201 CSVMHEALHN HYTQKSLSLS PGK (SEQ ID NO: 23)

Two examples of amino acid sequences that may be used for the Fc portion of human IgG3 (G3Fc) are shown below. The hinge region in G3Fc can be up to four times as long as in other Fc chains and contains three identical 15-residue segments preceded by a similar 17-residue segment. The first G3Fc sequence shown below (SEQ ID NO: 24) contains a short hinge region consisting of a single 15-residue segment, whereas the second G3Fc sequence (SEQ ID NO: 25) contains a full-length hinge region. In each case, dotted underline indicates the hinge region, and solid underline indicates positions with naturally occurring variants according to UniProt P01859. In part, the disclosure provides polypeptides comprising, consisting essential of, or consisting of amino acid sequences with 70%, 75%, 80%, 85%, 86%, 87%, 88%, 89%, 90%, 91%, 92%, 93%, 94%, 95%, 96%, 97%, 98%, 99%, or 100% identity to SEQ ID NOs: 24 or 25.

1 EPKSCDTPPP CPRCPAPELL GGPSVFLFPP KPKDTLMISR TPEVTCVVVD
51 VSHEDPEVQF KWYVDGVEVH NAKTKPREEQ YNSTFRVVSV LTVLHQDWLN
101 GKEYKCKVSN KALPAPIEKT ISKTKGQPRE PQVYTLPPSR EEMTKNQVSL
151 TCLVKGFYPS DIAVEWESSG QPENNYNTTP PMLDSDGSFF LYSKLTVDKS
201 RWQQGNIFSC SVMHEALHNR FTQKSLSLSP GK (SEQ ID NO: 24)

1 ELKTPLGDTT HTCPRCPEPK SCDTPPPCPR CPEPKSCDTP PPCPRCPEPK
51 SCDTPPPCPR CPAPELLGGP SVFLFPPKPK DTLMISRTPE VTCVVVDVSH
101 EDPEVQFKWY VDGVEVHNAK TKPREEQYNS TFRVVSVLTV LHQDWLNGKE
151 YKCKVSNKAL PAPIEKTISK TKGQPREPQV YTLPPSREEM TKNQVSLTCL
201 VKGFYPSDIA VEWESSGQPE NNYNTTPPML DSDGSFFLYS KLTVDKSRWQ
251 QGNIFSCSVM HEALHNRFTQ KSLSLSPGK (SEQ ID NO: 25)

Naturally occurring variants in G3Fc (for example, see Uniprot P01860) include E68Q, P76L, E79Q, Y81F, D97N, N100D, T124A, S169N, S169del, F221Y when converted to the numbering system used in SEQ ID NO: 24, and the present disclosure provides fusion proteins comprising G3Fc domains containing one or more of these variations. In addition, the human immunoglobulin IgG3 gene (*IGHG3*) shows a structural polymorphism characterized by different hinge lengths [see Uniprot P01859]. Specifically, variant WIS is lacking most of the V region and all of the CH1 region. It has an extra interchain disulfide bond at position 7 in addition to the 11 normally present in the hinge region. Variant ZUC lacks most of the V region, all of the CH1 region, and part of the hinge. Variant OMM may represent an allelic form or another gamma chain subclass. The present disclosure provides additional fusion proteins comprising G3Fc domains containing one or more of these variants.

An example of a native amino acid sequence that may be used for the Fc portion of human IgG4 (G4Fc) is shown below (SEQ ID NO: 26). Dotted underline indicates the hinge region. In part, the disclosure provides polypeptides comprising, consisting essential of, or consisting of amino acid sequences with 70%, 75%, 80%, 85%, 86%, 87%, 88%, 89%, 90%, 91%, 92%, 93%, 94%, 95%, 96%, 97%, 98%, 99%, or 100% identity to SEQ ID NO: 26.

1 ESKYGPPCPS CPAPEFLGGP SVFLFPPKPK DTLMISRTPE VTCVVVDVSQ
51 EDPEVQFNWY VDGVEVHNAK TKPREEQFNS TYRVVSVLTV LHQDWLNGKE
101 YKCKVSNKGL PSSIEKTISK AKGQPREPQV YTLPPSQEEM TKNQVSLTCL
151 VKGFYPSDIA VEWESNGQPE NNYKTTPPVL DSDGSFFLYS RLTVDKSRWQ
201 EGNVFSCSVM HEALHNHYTQ KSLSLSLGK (SEQ ID NO: 26)

A variety of engineered mutations in the Fc domain are presented herein with respect to the G1Fc sequence (SEQ ID NO: 22), and analogous mutations in G2Fc, G3Fc, and G4Fc can be derived from their alignment with G1Fc in Figure 18. Due to unequal hinge lengths, analogous Fc positions based on isotype alignment (Figure 18) possess different amino acid numbers in SEQ ID NOs: 22, 23, 24, 25, and 26. It can also be appreciated that a given amino acid position in an immunoglobulin sequence consisting of hinge, C_{H}2, and C_{H}3 regions (*e.g.,* SEQ ID NOs: 22, 23, 24, 25, and 26) will be identified by a different number than the same position when numbering encompasses the entire IgG1 heavy-chain constant domain (consisting of the C_{H}1, hinge, C_{H}2, and C_{H}3 regions) as in the Uniprot database. For example, correspondence between selected C_{H}3 positions in a human G1Fc sequence (SEQ ID NO: 22), the human IgG1 heavy chain constant domain (Uniprot P01857), and the human IgG1 heavy chain is as follows.

| Correspondence of C_{H}3 Positions in Different Numbering Systems | | |
|---|---|---|
| G1Fc (Numbering begins at first threonine in hinge region) | IgG1 heavy chain constant domain (Numbering begins at C_{H}1) | IgG1 heavy chain (EU numbering scheme of Kabat et al., 1991^{∗}) |
| Y127 | Y232 | Y349 |
| S132 | 5237 | 5354 |
| E134 | E239 | E356 |
| T144 | T249 | T366 |
| L146 | L251 | L368 |
| K170 | K275 | K392 |
| D177 | D282 | D399 |
| Y185 | Y290 | Y407 |
| K187 | K292 | K409 |
| ^{∗} Kabat et al. (eds) 1991; pp. 688-696 in Sequences of Proteins of Immunological Interest, 5th ed., Vol. 1, NIH, Bethesda, MD. | | |

In certain aspects, the polypeptides disclosed herein may form protein complexes comprising at least one ALK4 polypeptide associated, covalently or non-covalently, with at least one ActRIIB polypeptide. Preferably, polypeptides disclosed herein form heterodimeric complexes, although higher order heteromultimeric complexes (heteromultimers) are also included such as, but not limited to, heterotrimers, heterotetramers, and further oligomeric structures (see, e.g., Figure 21-23). In some embodiments, ALK4 and/or ActRIIB polypeptides comprise at least one multimerization domain. As disclosed herein, the term "multimerization domain" refers to an amino acid or sequence of amino acids that promote covalent or non-covalent interaction between at least a first polypeptide and at least a second polypeptide. Polypeptides disclosed herein may be joined covalently or non-covalently to a multimerization domain. Preferably, a multimerization domain promotes interaction between a first polypeptide (*e.g.,* an ALK4 polypeptide) and a second polypeptide (*e.g.,* an ActRIIB polypeptide) to promote heteromultimer formation (*e.g.,* heterodimer formation), and optionally hinders or otherwise disfavors homomultimer formation (e.g., homodimer formation), thereby increasing the yield of desired heteromultimer (see, e.g., Figure 22).

Many methods known in the art can be used to generate ALK4:ActRIIB heteromultimers. For example, non-naturally occurring disulfide bonds may be constructed by replacing on a first polypeptide (*e.g.,* an ALK4 polypeptide) a naturally occurring amino acid with a free thiol-containing residue, such as cysteine, such that the free thiol interacts with another free thiol-containing residue on a second polypeptide (*e.g.,* an ActRIIB polypeptide) such that a disulfide bond is formed between the first and second polypeptides. Additional examples of interactions to promote heteromultimer formation include, but are not limited to, ionic interactions such as described in Kjaergaard et al., WO2007147901; electrostatic steering effects such as described in Kannan et al., U.S.8,592,562; coiled-coil interactions such as described in Christensen et al., U.S.20120302737; leucine zippers such as described in Pack & Plueckthun,(1992) Biochemistry 31: 1579-1584; and helix-turn-helix motifs such as described in Pack et al., (1993) Bio/Technology 11: 1271-1277. Linkage of the various segments may be obtained via, *e.g.,* covalent binding such as by chemical crosslinking, peptide linkers, disulfide bridges, *etc.,* or affinity interactions such as by avidin-biotin or leucine zipper technology.

In certain aspects, a multimerization domain may comprise one component of an interaction pair. In some embodiments, the polypeptides disclosed herein may form protein complexes comprising a first polypeptide covalently or non-covalently associated with a second polypeptide, wherein the first polypeptide comprises the amino acid sequence of an ALK4 polypeptide and the amino acid sequence of a first member of an interaction pair; and the second polypeptide comprises the amino acid sequence of an ActRIIB polypeptide and the amino acid sequence of a second member of an interaction pair. The interaction pair may be any two polypeptide sequences that interact to form a complex, particularly a heterodimeric complex although operative embodiments may also employ an interaction pair that can form a homodimeric complex. One member of the interaction pair may be fused to an ALK4 or ActRIIB polypeptide as described herein, including for example, a polypeptide sequence comprising, consisting essentially of, or consisting of an amino acid sequence that is at least 70%, 75%, 80%, 85%, 86%, 87%, 88%, 89%, 90%, 91%, 92%, 93%, 94%, 95%, 96%, 97%, 98%, 99%, or 100% identical to the sequence of any one of SEQ ID NOs: 2, 3, 5, 6, 15, and 19. An interaction pair may be selected to confer an improved property/activity such as increased serum half-life, or to act as an adaptor on to which another moiety is attached to provide an improved property/activity. For example, a polyethylene glycol moiety may be attached to one or both components of an interaction pair to provide an improved property/activity such as improved serum half-life.

The first and second members of the interaction pair may be an asymmetric pair, meaning that the members of the pair preferentially associate with each other rather than self-associate. Accordingly, first and second members of an asymmetric interaction pair may associate to form a heterodimeric complex (see, e.g., Figure 22). Alternatively, the interaction pair may be unguided, meaning that the members of the pair may associate with each other or self-associate without substantial preference and thus may have the same or different amino acid sequences. Accordingly, first and second members of an unguided interaction pair may associate to form a homodimer complex or a heterodimeric complex. Optionally, the first member of the interaction pair (*e.g.,* an asymmetric pair or an unguided interaction pair) associates covalently with the second member of the interaction pair. Optionally, the first member of the interaction pair (*e.g.,* an asymmetric pair or an unguided interaction pair) associates non-covalently with the second member of the interaction pair.

As specific examples, the present disclosure provides fusion proteins comprising ALK4 or ActRIIB fused to a polypeptide comprising a constant domain of an immunoglobulin, such as a CH1, CH2, or CH3 domain derived from human IgG1, IgG2, IgG3, and/or IgG4, that has been modified to promote heteromultimer formation. A problem that arises in large-scale production of asymmetric immunoglobulin-based proteins from a single cell line is known as the "chain association issue". As confronted prominently in the production of bispecific antibodies, the chain association issue concerns the challenge of efficiently producing a desired multi-chain protein from among the multiple combinations that inherently result when different heavy chains and/or light chains are produced in a single cell line [Klein et al (2012) mAbs 4:653-663]. This problem is most acute when two different heavy chains and two different light chains are produced in the same cell, in which case there are a total of 16 possible chain combinations (although some of these are identical) when only one is typically desired. Nevertheless, the same principle accounts for diminished yield of a desired multi-chain fusion protein that incorporates only two different (asymmetric) heavy chains.

Various methods are known in the art that increase desired pairing of Fc-containing fusion polypeptide chains in a single cell line to produce a preferred asymmetric fusion protein at acceptable yields [Klein et al (2012) mAbs 4:653-663; and Spiess et al (2015) Molecular Immunology 67(2A): 95-106]. Methods to obtain desired pairing of Fc-containing chains include, but are not limited to, charge-based pairing (electrostatic steering), "knobs-into-holes" steric pairing, SEEDbody pairing, and leucine zipper-based pairing [Ridgway et al (1996) Protein Eng 9:617-621; Merchant et al (1998) Nat Biotech 16:677-681; Davis et al (2010) Protein Eng Des Sel 23:195-202; Gunasekaran et al (2010); 285:19637-19646; Wranik et al (2012) J Biol Chem 287:43331-43339; US5932448; WO 1993/011162; WO 2009/089004, and WO 2011/034605]. As described herein, these methods may be used to generate ALK4-Fc:ActRIIB-Fc heteromultimer complexes. See, e.g., Figure 23.

ALK4:ActRIIB heteromultimers and method of making such heteromultimers have been previously disclosed. See, for example, WO 2016/164497, the entire teachings of which are incorporated by reference herein.

It is understood that different elements of the fusion proteins (*e.g.,* immunoglobulin Fc fusion proteins) may be arranged in any manner that is consistent with desired functionality. For example, an ActRII polypeptide domain, ALK4 polypeptide domain, or TGFβRII polypeptide domain may be placed C-terminal to a heterologous domain, or alternatively, a heterologous domain may be placed C-terminal to an ActRII polypeptide domain, ALK4 polypeptide domain, or TGFβRII polypeptide domain. The ActRII polypeptide domain, ALK4 polypeptide domain, or TGFβRII polypeptide domain and the heterologous domain need not be adjacent in a fusion protein, and additional domains or amino acid sequences may be included C- or N-terminal to either domain or between the domains.

For example, an ActRII (or ALK4 or TGFβRII) receptor fusion protein may comprise an amino acid sequence as set forth in the formula A-B-C. The B portion corresponds to an ActRII (or ALK4 or TGFβRII) polypeptide domain. The A and C portions may be independently zero, one, or more than one amino acid, and both the A and C portions when present are heterologous to B. The A and/or C portions may be attached to the B portion via a linker sequence. A linker may be rich in glycine (*e.g.,* 2-10, 2-5, 2-4, 2-3 glycine residues) or glycine and proline residues and may, for example, contain a single sequence of threonine/serine and glycines or repeating sequences of threonine/serine and/or glycines, e.g., GGG (SEQ ID NO: 27), GGGG (SEQ ID NO: 28), TGGGG(SEQ ID NO: 29), SGGGG (SEQ ID NO: 30), TGGG (SEQ ID NO: 31), SGGG (SEQ ID NO: 32), or GGGGS (SEQ ID NO: 33) singlets, or repeats. In certain embodiments, an ActRII (or ALK4 or TGFβRII) fusion protein comprises an amino acid sequence as set forth in the formula A-B-C, wherein A is a leader (signal) sequence, B consists of an ActRII (or ALK4 or TGFβRII) polypeptide domain, and C is a polypeptide portion that enhances one or more of *in vivo* stability, *in vivo* half-life, uptake/administration, tissue localization or distribution, formation of protein complexes, and/or purification. In certain embodiments, an ActRII (or ALK4 or TGFβRII) fusion protein comprises an amino acid sequence as set forth in the formula A-B-C, wherein A is a TPA leader sequence, B consists of an ActRII (or ALK4 or TGFβRII) receptor polypeptide domain, and C is an immunoglobulin Fc domain. Preferred fusion proteins comprise the amino acid sequence set forth in any one of SEQ ID NOs: 50, 54 57, 58, 60, 63, 64, 66, 70, 71, 73, 74, 76, 77, 78, 79, 80, 123, 131, 132, 139, 141, 143, 145, 148, and 150.

Alternatively, ActRII antagonists may comprise one or more single-chain ligand traps, optionally which may be covalently or non-covalently associated with one or more ALK4 or ActRIIB polypeptides as well as additional ALK4: ActRIIB single chain ligand traps [US 2011/0236309 and US2009/0010879]. As described herein, single-chain ligand traps do not require fusion to any multimerization domain such as coiled-coil Fc domains to be multivalent. In general, single-chain ligand traps of the present disclosure comprise at least one ALK4 polypeptide domain and one ActRIIB polypeptide domain. The ALK4 and ActRIIB polypeptide domains, generally referred to herein as binding domains (BD), optionally may be joined by a linker region. ALK4:ActRIIB single-chain ligand traps have been previously described. See, e.g., WO 2016/164497, the entire teachings of each which are incorporated by reference herein.

In certain preferred embodiments, ActRII polypeptides, ALK4 polypeptides, TGFβRII polypeptides, and ALK4:ActRIIB heteromultimers to be used in accordance with the methods described herein are isolated complexes. As used herein, an isolated protein (or protein complex) or polypeptide (or polypeptide complex) is one which has been separated from a component of its natural environment. In some embodiments, a polypeptide or heteromultimer of the disclosure is purified to greater than 95%, 96%, 97%, 98%, or 99% purity as determined by, for example, electrophoretic (*e.g.,* SDS-PAGE, isoelectric focusing (IEF), capillary electrophoresis) or chromatographic (*e.g.,* ion exchange or reverse phase HPLC). Methods for assessment of antibody purity are well known in the art [Flatman et al., (2007) J. Chromatogr. B 848:79-87].

In certain embodiments, ActRII polypeptides, ALK4 polypeptides, TGFβRII polypeptides, and ALK4: ActRIIB heteromultimers of the disclosure can be produced by a variety of art-known techniques. For example, polypeptides of the disclosure can be synthesized using standard protein chemistry techniques such as those described in Bodansky, M. Principles of Peptide Synthesis, Springer Verlag, Berlin (1993) and Grant G. A. (ed.), Synthetic Peptides: A User's Guide, W. H. Freeman and Company, New York (1992). In addition, automated peptide synthesizers are commercially available (Advanced ChemTech Model 396; Milligen/Biosearch 9600). Alternatively, the polypeptides and complexes of the disclosure, including fragments or variants thereof, may be recombinantly produced using various expression systems [E. coli, Chinese Hamster Ovary (CHO) cells, COS cells, baculovirus] as is well known in the art. In a further embodiment, the modified or unmodified polypeptides of the disclosure may be produced by digestion of recombinantly produced full-length ALK4, TGFβRII, and/or ActRIIB polypeptides by using, for example, a protease, e.g., trypsin, thermolysin, chymotrypsin, pepsin, or paired basic amino acid converting enzyme (PACE). Computer analysis (using commercially available software, e.g., MacVector, Omega, PCGene, Molecular Simulation, Inc.) can be used to identify proteolytic cleavage sites.

### 3. Nucleic Acids Encoding ActRII, ALK4, and TGFβRII polypeptides

In certain embodiments, the present disclosure provides isolated and/or recombinant nucleic acids encoding ActRII, ALK4, and/or TGFβRII polypeptides (including fragments, functional variants, and fusion proteins thereof) disclosed herein. For example, SEQ ID NO: 16 encodes a naturally occurring human ALK4 precursor polypeptide, SEQ ID NO: 17 encodes a processed extracellular domain of ALK4. The subject nucleic acids may be single-stranded or double stranded. Such nucleic acids may be DNA or RNA molecules. These nucleic acids may be used, for example, in methods for making ActRII polypeptides, ALK4 polypeptides, TGFβRII polypeptides, and ALK4:ActRIIB heteromultimers as described herein.

As used herein, isolated nucleic acid(s) refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

In certain embodiments, nucleic acids encoding ALK4, ActRII, or TGFβRII polypeptides of the present disclosure are understood to include any one of SEQ ID NOs: 7, 8, 12, 13, 16, 17, 20, 21, 55, 61, 67, 72, 75, 124, 125, 126, 127, 134, 135, 136, 137, 138, 140, 142, 144, 146, and 149 as well as variants thereof. Variant nucleotide sequences include sequences that differ by one or more nucleotide substitutions, additions, or deletions including allelic variants, and therefore, will include coding sequences that differ from the nucleotide sequence designated in any one of SEQ ID NOs: 7, 8, 12, 13, 16, 17, 20, 21, 55, 61, 67, 72, 75, 124, 125, 126, 127, 134, 135, 136, 137, 138, 140, 142, 144, 146, and 149.

In certain embodiments, ALK4, ActRII, TGFβRII polypeptides of the present disclosure are encoded by isolated or recombinant nucleic acid sequences that comprise, consist essentially of, or consists of a sequence that is least 70%, 75%, 80%, 85%, 86%, 87%, 88%, 89%, 90%, 91%, 92%, 93%, 94%, 95%, 96%, 97%, 98%, 99% or 100% identical to SEQ ID NOs: 7, 8, 12, 13, 16, 17, 20, 21, 55, 61, 67, 72, 75, 124, 125, 126, 127, 134, 135, 136, 137, 138, 140, 142, 144, 146, and 149. One of ordinary skill in the art will appreciate that nucleic acid sequences that comprise, consist essentially of, or consists of a sequence complementary to a sequence that is least 70%, 75%, 80%, 85%, 86%, 87%, 88%, 89%, 90%, 91%, 92%, 93%, 94%, 95%, 96%, 97%, 98%, 99% or 100% identical to SEQ ID NOs: 7, 8, 12, 13, 16, 17, 20, 21, 55, 61, 67, 72, 75, 124, 125, 126, 127, 134, 135, 136, 137, 138, 140, 142, 144, 146, and 149 also within the scope of the present disclosure. In further embodiments, the nucleic acid sequences of the disclosure can be isolated, recombinant, and/or fused with a heterologous nucleotide sequence or in a DNA library.

In other embodiments, nucleic acids of the present disclosure also include nucleotide sequences that hybridize under stringent conditions to the nucleotide sequence designated in SEQ ID NOs: 7, 8, 12, 13, 16, 17, 20, 21, 55, 61, 67, 72, 75, 124, 125, 126, 127, 134, 135, 136, 137, 138, 140, 142, 144, 146, and 149, or fragments thereof. One of ordinary skill in the art will understand readily that appropriate stringency conditions which promote DNA hybridization can be varied. For example, one could perform the hybridization at 6.0 x sodium chloride/sodium citrate (SSC) at about 45 °C, followed by a wash of 2.0 x SSC at 50 °C. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In one embodiment, the disclosure provides nucleic acids which hybridize under low stringency conditions of 6 x SSC at room temperature followed by a wash at 2 x SSC at room temperature.

Isolated nucleic acids which differ from the nucleic acids as set forth in SEQ ID NOs: 7, 8, 12, 13, 16, 17, 20, 21, 55, 61, 67, 72, 75, 124, 125, 126, 127, 134, 135, 136, 137, 138, 140, 142, 144, 146, and 149 to degeneracy in the genetic code are also within the scope of the disclosure. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms are within the scope of this disclosure.

In certain embodiments, the recombinant nucleic acids of the present disclosure may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the disclosure. The promoters may be either naturally occurring promoters, or hybrid promoters that combine elements of more than one promoter. An expression construct may be present in a cell on an episome, such as a plasmid, or the expression construct may be inserted in a chromosome. In some embodiments, the expression vector contains a selectable marker gene to allow the selection of transformed host cells. Selectable marker genes are well known in the art and will vary with the host cell used.

In certain aspects of the present disclosure, the subject nucleic acid is provided in an expression vector comprising a nucleotide sequence encoding an ALK4, ActRII, and/or TGFβRII polypeptide and operably linked to at least one regulatory sequence. Regulatory sequences are art-recognized and are selected to direct expression of ALK4, ActRII, and/or TGFβRII polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding an ALK4, ActRIIB, and/or TGFβRII polypeptides. Such useful expression control sequences, include, for example, the early and late promoters of SV40, *tet* promoter, adenovirus or cytomegalovirus immediate early promoter, RSV promoters, the lac system, the *trp* system, the TAC or TRC system, T7 promoter whose expression is directed by T7 RNA polymerase, the major operator and promoter regions of phage lambda , the control regions for fd coat protein, the promoter for 3-phosphoglycerate kinase or other glycolytic enzymes, the promoters of acid phosphatase, *e.g.,* Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the present disclosure can be produced by ligating the cloned gene, or a portion thereof, into a vector suitable for expression in either prokaryotic cells, eukaryotic cells (yeast, avian, insect or mammalian), or both. Expression vehicles for production of a recombinant TGFβ superfamily type I and/or type II receptor polypeptide include plasmids and other vectors. For instance, suitable vectors include plasmids of the following types: pBR322-derived plasmids, pEMBL-derived plasmids, pEX-derived plasmids, pBTac-derived plasmids and pUC-derived plasmids for expression in prokaryotic cells, such as *E. coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures [Molecular Cloning A Laboratory Manual, 3rd Ed., ed. by Sambrook, Fritsch and Maniatis Cold Spring Harbor Laboratory Press, 2001]. In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the β-gal containing pBlueBac III).

In a preferred embodiment, a vector will be designed for production of the subject ALK4 and/or ActRII polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wisc.). As will be apparent, the subject gene constructs can be used to cause expression of the subject ALK4 and/or ActRII polypeptide in cells propagated in culture, *e.g.,* to produce proteins, including fusion proteins or variant proteins, for purification.

This disclosure also pertains to a host cell transfected with a recombinant gene including a coding sequence for one or more of the subject ALK4, ActRIIB, and/or TGFβRII polypeptides. The host cell may be any prokaryotic or eukaryotic cell. For example, an ALK4, ActRIIB, and/or TGFβRII polypeptide may be expressed in bacterial cells such as *E*. *coli,* insect cells (e.g., using a baculovirus expression system), yeast, or mammalian cells [*e.g.* a Chinese hamster ovary (CHO) cell line]. Other suitable host cells are known to those skilled in the art.

Accordingly, the present disclosure further pertains to methods of producing the subject ALK4, ActRIIB, and/or TGFβRII polypeptides. For example, a host cell transfected with an expression vector encoding an ALK4, ActRIIB, and/or TGFβRII polypeptide can be cultured under appropriate conditions to allow expression of the ALK4, ActRIIB, and/or TGFβRII polypeptide to occur. The polypeptide may be secreted and isolated from a mixture of cells and medium containing the polypeptide. Alternatively, ALK4, ActRIIB, and/or TGFβRII polypeptide may be isolated from a cytoplasmic or membrane fraction obtained from harvested and lysed cells. A cell culture includes host cells, media and other byproducts. Suitable media for cell culture are well known in the art. The subject polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of ALK4, ActRIIB, and/or TGFβRII polypeptides and affinity purification with an agent that binds to a domain fused to ALK4, ActRIIB, and/or TGFβRII polypeptide (*e.g.,* a protein A column may be used to purify ALK4-Fc, ActRIIB-Fc, and/or TGFβRII-Fc fusion proteins). In some embodiments, the ALK4 and/or ActRII polypeptide is a fusion protein containing a domain which facilitates its purification.

In some embodiments, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. An ALK4-Fc, ActRIIB-Fc, and/or TGFβRII-Fc fusion protein, as well as heteromeric complexes thereof, may be purified to a purity of >90%, >95%, >96%, >98%, or >99% as determined by size exclusion chromatography and >90%, >95%, >96%, >98%, or >99% as determined by SDS PAGE. The target level of purity should be one that is sufficient to achieve desirable results in mammalian systems, particularly non-human primates, rodents (mice), and humans.

In another embodiment, a fusion gene coding for a purification leader sequence, such as a poly-(His)/enterokinase cleavage site sequence at the N-terminus of the desired portion of the recombinant ALK4, ActRIIB, and/or TGFβRII polypeptide, can allow purification of the expressed fusion protein by affinity chromatography using a Ni²⁺ metal resin. The purification leader sequence can then be subsequently removed by treatment with enterokinase to provide the purified ALK4, ActRIIB, and/or TGFβRII polypeptide, as well as heteromeric complexes thereof [Hochuli et al. (1987) J. Chromatography 411:177; and Janknecht et al. (1991) PNAS USA 88:8972].

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence. See, *e.g.,* Current Protocols in Molecular Biology, eds. Ausubel et al., John Wiley & Sons: 1992.

### 4. Antibody Antagonists

In certain aspects, the present disclosure relates to an ActRII antagonist (inhibitor) that is antibody, or combination of antibodies. ActRII antagonist antibody, or combination of antibodies, may bind to one or more ActRII ligands *[e.g.,* GDF11, GDF8, activin (e.g., activin A, activin B, activin C, activin E, activin AB, activin AC) GDF3, BMP6, BMP 10, and BMP9] or one or more type I and/or type II receptors (e.g., ActRIIA, ActRIIB, and ALK4). In particular, the disclosure provides methods of using an ActRII antagonist antibody, or combination of ActRII antagonist antibodies, alone or in combination with one or more additional supportive therapies and/or active agents, to achieve a desired effect in a subject in need thereof (e.g., increase an immune response in a subject in need thereof and treat cancer or a pathogen). In certain preferred embodiments, an ActRII antagonist antibody may be used in combination with a TGFβRII antagonist.

In certain aspects, an ActRII antagonist antibody, or combination of antibodies, of the disclosure is an antibody that inhibits at least GDF 11. Therefore, in some embodiments, an ActRII antagonist antibody, or combination of antibodies, binds to at least GDF11. As used herein, a GDF11 antibody (anti-GDF11 antibody) generally refers to an antibody that binds to GDF11 with sufficient affinity such that the antibody is useful as a diagnostic and/or therapeutic agent in targeting GDF11. In certain embodiments, the extent of binding of an anti-GDF11 antibody to an unrelated, non-GDF11 protein is less than about 10%, 9%, 8%, 7%, 6%, 5%, 4%, 3%, 2%, or less than about 1% of the binding of the antibody to GDF11 as measured, for example, by a radioimmunoassay (RIA), Biacore, or other protein-protein interaction or binding affinity assay. In certain embodiments, an anti-GDF 11 antibody binds to an epitope of GDF 11 that is conserved among GDF 11 from different species. In certain preferred embodiments, an anti-GDF11 antibody binds to human GDF11. In other preferred embodiments, an anti-GDF11 antibody may inhibit GDF11 from binding to a cognate type I and/or type II receptor (*e.g.,* ActRIIA, ActRIIB, and ALK4) and thus inhibit GDF11-mediated signaling (e.g., Smad signaling) via these receptors. It should be noted that GDF11 has high sequence homology to GDF8 and therefore antibodies that bind to GDF11, in some cases, may also bind to and/or inhibit GDF8. In some embodiments, an anti-GDF11 antibody is a multispecific antibody (e.g., bi-specific antibody) that binds to one or more additional ligands [*e*.*g*., GDF8, activin (e.g., activin A, activin B, activin C, activin E, activin AB, activin AC) GDF3, BMP6, BMP10, BMP9, TGFβ1, TGFβ2, and TGFβ3] and/or binds to one or more type I and/or type II receptors (e.g., ActRIIA, ActRIIB, TGFβRII, ALK5, and ALK4). In some embodiments, the disclosure relates to combinations of antibodies, as well as uses thereof, wherein the combination of antibodies comprises an anti-GDF11 antibody and one or more additional antibodies that bind to, for example, different ligands [*e*.*g*., GDF8, activin (e.g., activin A, activin B, activin C, activin E, activin AB, activin AC) GDF3, BMP6, BMP10, BMP9, TGFβ1, TGFβ2, and TGFβ3] and/or bind to one or more type I and/or type II receptors (e.g., ActRIIA, ActRIIB, TGFβRII, ALK5, and ALK4).

In certain aspects, an ActRII antagonist antibody, or combination of antibodies, of the disclosure is an antibody that inhibits at least GDF8. Therefore, in some embodiments, an ActRII antagonist antibody, or combination of antibodies, binds to at least GDF8. As used herein, a GDF8 antibody (anti-GDF8 antibody) generally refers to an antibody that binds to GDF8 with sufficient affinity such that the antibody is useful as a diagnostic and/or therapeutic agent in targeting GDF8. In certain embodiments, the extent of binding of an anti-GDF8 antibody to an unrelated, non-GDF8 protein is less than about 10%, 9%, 8%, 7%, 6%, 5%, 4%, 3%, 2%, or less than about 1% of the binding of the antibody to GDF8 as measured, for example, by a radioimmunoassay (RIA), Biacore, or other protein-protein interaction or binding affinity assay. In certain embodiments, an anti-GDF8 antibody binds to an epitope of GDF8 that is conserved among GDF8 from different species. In certain preferred embodiments, an anti-GDF8 antibody binds to human GDF8. In other preferred embodiments, an anti-GDF8 antibody may inhibit GDF8 from binding to a cognate type I and/or type II receptor (e.g., ActRIIA, ActRIIB, and ALK4) and thus inhibit GDF8-mediated signaling (e.g., Smad signaling) via these receptors. It should be noted that GDF8 has high sequence homology to GDF11 and therefore antibodies that bind to GDF8, in some cases, may also bind to and/or inhibit GDF11. In some embodiments, an anti-GDF8 antibody is a multispecific antibody (e.g., bi-specific antibody) that binds to one or more additional ligands [*e.g.*, GDF11, activin (e.g., activin A, activin B, activin C, activin E, activin AB, activin AC) GDF3, BMP6, BMP10, BMP9, TGFβ1, TGFβ2, and TGFβ3] and/or binds to one or more type I and/or type II receptors (e.g., ActRIIA, ActRIIB, TGFβRII, ALK5, and ALK4 In some embodiments, the disclosure relates to combinations of antibodies, as well as uses thereof, wherein the combination of antibodies comprises an anti-GDF8 antibody and one or more additional antibodies that bind to, for example, different ligands [*e.g.*, GDF11, activin (e.g., activin A, activin B, activin C, activin E, activin AB, activin AC) GDF3, BMP6, BMP 10, BMP9, TGFβ1, TGFβ2, and TGFβ3] and/or bind to one or more type I and/or type II receptors (e.g., ActRIIA, ActRIIB, TGFβRII, ALK5, and ALK4).

In certain aspects, an ActRII antagonist antibody, or combination of antibodies, of the disclosure is an antibody that inhibits at least activin (e.g., activin A, activin B, activin C, activin E, activin AB, activin AC). Therefore, in some embodiments, an ActRII antagonist antibody, or combination of antibodies, binds to at least activin. As used herein, an activin antibody (anti-activin antibody) generally refers to an antibody that binds to activin with sufficient affinity such that the antibody is useful as a diagnostic and/or therapeutic agent in targeting activin. In certain embodiments, the extent of binding of an anti-activin antibody to an unrelated, non-activin protein is less than about 10%, 9%, 8%, 7%, 6%, 5%, 4%, 3%, 2%, or less than about 1% of the binding of the antibody to activin as measured, for example, by a radioimmunoassay (RIA), Biacore, or other protein-protein interaction or binding affinity assay. In certain embodiments, an anti-activin antibody binds to an epitope of activin that is conserved among activin from different species. In certain preferred embodiments, an anti-activin antibody binds to human activin. In other preferred embodiments, an anti-activin antibody may inhibit activin from binding to a cognate type I and/or type II receptor (*e.g.*, ActRIIA, ActRIIB, and ALK4) and thus inhibit activin-mediated signaling (e.g., Smad signaling) via these receptors. It should be noted that activins share sequence homology and therefore antibodies that bind to one activin (e.g., activin A) may bind to one or more additional activins (e.g., activin B, activin AB, activin C, activin E, activin AC). In some embodiments, an anti-activin antibody binds to at least activin A and activin B. In some embodiments, an anti-activin antibody is a multispecific antibody (e.g., bi-specific antibody) that binds to one or more additional ligands [*e.g.*, GDF11, GDF8, GDF3, BMP6, BMP10, BMP9, TGFβ1, TGFβ2, and TGFβ3] and/or binds to one or more type I and/or type II receptors (e.g., ActRIIA, ActRIIB, TGFβRII, ALK5, and ALK4). In some embodiments, the disclosure relates to combinations of antibodies, as well as uses thereof, wherein the combination of antibodies comprises an anti-activin antibody and one or more additional antibodies that bind to, for example, different ligands [e.g., GDF8, GDF11, GDF3, BMP6, BMP10, BMP9, TGFβ1, TGFβ2, and TGFβ3] and/or bind to one or more type I and/or type II receptors (e.g., ActRIIA, ActRIIB, TGFβRII, ALK5, and ALK4). In some embodiments, an antibody, or combination of antibodies, to be used in accordance with the methods and uses of the disclosure binds to activin and TGFβ. In some embodiments, an antibody, or combination of antibodies, to be used in accordance with the methods and uses of the disclosure binds to activin A and TGFβ2. In some embodiments, an antibody, or combination of antibodies, to be used in accordance with the methods and uses of the disclosure binds to ActRII (ActRIIA and/or ActRIIB) and TGFβ. In some embodiments, an antibody, or combination of antibodies, to be used in accordance with the methods and uses of the disclosure binds to ActRII (ActRIIA and/or ActRIIB) and TGFβ2.

In certain aspects, an ActRII antagonist antibody, or combination of antibodies, of the disclosure is an antibody that inhibits at least GDF3. Therefore, in some embodiments, an ActRII antagonist antibody, or combination of antibodies, binds to at least GDF3. As used herein, a GDF3 antibody (anti-GDF3 antibody) generally refers to an antibody that binds to GDF3 with sufficient affinity such that the antibody is useful as a diagnostic and/or therapeutic agent in targeting GDF3. In certain embodiments, the extent of binding of an anti-GDF3 antibody to an unrelated, non-GDF3 protein is less than about 10%, 9%, 8%, 7%, 6%, 5%, 4%, 3%, 2%, or less than about 1% of the binding of the antibody to GDF3 as measured, for example, by a radioimmunoassay (RIA), Biacore, or other protein-protein interaction or binding affinity assay. In certain embodiments, an anti-GDF3 antibody binds to an epitope of GDF3 that is conserved among GDF3 from different species. In certain preferred embodiments, an anti-GDF3 antibody binds to human GDF3. In other preferred embodiments, an anti-GDF3 antibody may inhibit GDF3 from binding to a cognate type I and/or type II receptor (e.g., ActRIIA, ActRIIB, and ALK4) and thus inhibit GDF3-mediated signaling (e.g., Smad signaling) via these receptors. In some embodiments, an anti-GDF3 antibody is a multispecific antibody (e.g., bi-specific antibody) that binds to one or more additional ligands [*e.g.*, GDF11, GDF8, activin (e.g., activin A, activin B, activin C, activin E, activin AB, activin AC), BMP6, BMP10, BMP9, TGFβ1, TGFβ2, and TGFβ3] and/or binds to one or more type I and/or type II receptors (e.g., ActRIIA, ActRIIB, TGFβRII, ALK5, and ALK4). In some embodiments, the disclosure relates to combinations of antibodies, as well as uses thereof, wherein the combination of antibodies comprises an anti-GDF3 antibody and one or more additional antibodies that bind to, for example, different ligands [*e.g.*, GDF11, GDF8, activin (e.g., activin A, activin B, activin C, activin E, activin AB, activin AC) BMP6, BMP10, BMP9, TGFβ1, TGFβ2, and TGFβ3] and/or bind to one or more type I and/or type II receptors (e.g., ActRIIA, ActRIIB, TGFβRII, ALK5, and ALK4).

In certain aspects, an ActRII antagonist antibody, or combination of antibodies, of the disclosure is an antibody that inhibits at least BMP6. Therefore, in some embodiments, an ActRII antagonist antibody, or combination of antibodies, binds to at least BMP6. As used herein, a BMP6 antibody (anti-BMP6 antibody) generally refers to an antibody that binds to BMP6 with sufficient affinity such that the antibody is useful as a diagnostic and/or therapeutic agent in targeting BMP6. In certain embodiments, the extent of binding of an anti-BMP6 antibody to an unrelated, non-BMP6 protein is less than about 10%, 9%, 8%, 7%, 6%, 5%, 4%, 3%, 2%, or less than about 1% of the binding of the antibody to BMP6 as measured, for example, by a radioimmunoassay (RIA), Biacore, or other protein-protein interaction or binding affinity assay. In certain embodiments, an anti-BMP6 antibody binds to an epitope of BMP6 that is conserved among BMP6 from different species. In certain preferred embodiments, an anti-BMP6 antibody binds to human BMP6. In other preferred embodiments, an anti-BMP6 antibody may inhibit BMP6 from binding to a cognate type I and/or type II receptor (e.g., ActRIIA, ActRIIB, and ALK4) and thus inhibit BMP6-mediated signaling (e.g., Smad signaling) via these receptors. In some embodiments, an anti-BMP6 antibody is a multispecific antibody (e.g., bi-specific antibody) that binds to one or more additional ligands [*e.g.*, GDF11, GDF8, activin (e.g., activin A, activin B, activin C, activin E, activin AB, activin AC), GDF3, BMP10, BMP9, TGFβ1, TGFβ2, and TGFβ3] and/or binds to one or more type I and/or type II receptors (e.g., ActRIIA, ActRIIB, TGFβRII, ALK5, and ALK4). In some embodiments, the disclosure relates to combinations of antibodies, as well as uses thereof, wherein the combination of antibodies comprises an anti-BMP6 antibody and one or more additional antibodies that bind to, for example, different ligands [*e.g.*, GDF11, GDF8, activin (e.g., activin A, activin B, activin C, activin E, activin AB, activin AC) GDF3, BMP10, BMP9, TGFβ1, TGFβ2, and TGFβ3] and/or bind to one or more type I and/or type II receptors (e.g., ActRIIA, ActRIIB, TGFβRII, ALK5, and ALK4).

In certain aspects, an ActRII antagonist antibody, or combination of antibodies, of the disclosure is an antibody that inhibits at least BMP9. Therefore, in some embodiments, an ActRII antagonist antibody, or combination of antibodies, binds to at least BMP9. As used herein, a BMP9 antibody (anti-BMP9 antibody) generally refers to an antibody that binds to BMP9 with sufficient affinity such that the antibody is useful as a diagnostic and/or therapeutic agent in targeting BMP9. In certain embodiments, the extent of binding of an anti-BMP9 antibody to an unrelated, non-BMP9 protein is less than about 10%, 9%, 8%, 7%, 6%, 5%, 4%, 3%, 2%, or less than about 1% of the binding of the antibody to BMP9 as measured, for example, by a radioimmunoassay (RIA), Biacore, or other protein-protein interaction or binding affinity assay. In certain embodiments, an anti-BMP9 antibody binds to an epitope of BMP9 that is conserved among BMP9 from different species. In certain preferred embodiments, an anti-BMP9 antibody binds to human BMP9. In other preferred embodiments, an anti-BMP9 antibody may inhibit BMP9 from binding to a cognate type I and/or type II receptor (e.g., ActRIIA, ActRIIB, and ALK4) and thus inhibit BMP9-mediated signaling (e.g., Smad signaling) via these receptors. In some embodiments, an anti-BMP9 antibody is a multispecific antibody (e.g., bi-specific antibody) that binds to one or more additional ligands [*e.g.*, GDF11, GDF8, activin (e.g., activin A, activin B, activin C, activin E, activin AB, activin AC), GDF3, BMP10, BMP6, TGFβ1, TGFβ2, and TGFβ3] and/or binds to one or more type I and/or type II receptors (e.g., ActRIIA, ActRIIB, TGFβRII, ALK5, and ALK4). In some embodiments, the disclosure relates to combinations of antibodies, as well as uses thereof, wherein the combination of antibodies comprises an anti-BMP9 antibody and one or more additional antibodies that bind to, for example, different ligands [*e.g.*, GDF11, GDF8, activin (e.g., activin A, activin B, activin C, activin E, activin AB, activin AC) GDF3, BMP10, BMP6, TGFβ1, TGFβ2, and TGFβ3] and/or bind to one or more type I and/or type II receptors (e.g., ActRIIA, ActRIIB, TGFβRII, ALK5, and ALK4).

In certain aspects, an ActRII antagonist antibody, or combination of antibodies, of the disclosure is an antibody that inhibits at least BMP10. Therefore, in some embodiments, an ActRII antagonist antibody, or combination of antibodies, binds to at least BMP10. As used herein, a BMP10 antibody (anti-BMP10 antibody) generally refers to an antibody that binds to BMP10 with sufficient affinity such that the antibody is useful as a diagnostic and/or therapeutic agent in targeting BMP10. In certain embodiments, the extent of binding of an anti-BMP10 antibody to an unrelated, non-BMP10 protein is less than about 10%, 9%, 8%, 7%, 6%, 5%, 4%, 3%, 2%, or less than about 1% of the binding of the antibody to BMP 10 as measured, for example, by a radioimmunoassay (RIA), Biacore, or other protein-protein interaction or binding affinity assay. In certain embodiments, an anti-BMP10 antibody binds to an epitope of BMP10 that is conserved among BMP10 from different species. In certain preferred embodiments, an anti-BMP10 antibody binds to human BMP10. In other preferred embodiments, an anti-BMP10 antibody may inhibit BMP10 from binding to a cognate type I and/or type II receptor (e.g., ActRIIA, ActRIIB, and ALK4) and thus inhibit BMP10-mediated signaling (e.g., Smad signaling) via these receptors. In some embodiments, an anti-BMP10 antibody is a multispecific antibody (e.g., bi-specific antibody) that binds to one or more additional ligands [*e.g*., GDF11, GDF8, activin (e.g., activin A, activin B, activin C, activin E, activin AB, activin AC), GDF3, BMP6, BMP9, TGFβ1, TGFβ2, and TGFβ3] and/or binds to one or more type I and/or type II receptors (e.g., ActRIIA, ActRIIB, TGFβRII, ALK5, and ALK4). In some embodiments, the disclosure relates to combinations of antibodies, as well as uses thereof, wherein the combination of antibodies comprises an anti-BMP10 antibody and one or more additional antibodies that bind to, for example, different ligands [*e.g.*, GDF11, GDF8, activin (e.g., activin A, activin B, activin C, activin E, activin AB, activin AC) GDF3, BMP6, BMP9, TGFβ1, TGFβ2, and TGFβ3] and/or bind to one or more type I and/or type II receptors (e.g., ActRIIA, ActRIIB, TGFβRII, ALK5, and ALK4).

In other aspects, an ActRII antagonist antibody, or combination of antibodies, of the disclosure is an antibody that inhibits at least an ActRII receptor (e.g., ActRIIA and/or ActRIIB). Therefore, in some embodiments, an ActRII antagonist antibody, or combination of antibodies, binds to at least ActRIIA, but does not bind or does not substantially bind to ActRIIB (e.g., binds to ActRIIB with a K_{D} of greater than 1 × 10⁻⁷ M or has relatively modest binding, e.g., about 1 × 10⁻⁸ M or about 1 × 10⁻⁹ M). In other embodiments, an ActRII antagonist antibody, or combination of antibodies, binds to at least ActRIIB, but does not bind or does not substantially bind to ActRIIA (e.g., binds to ActRIIA with a K_{D} of greater than 1 × 10⁻⁷ M or has relatively modest binding, e.g., about 1 × 10⁻⁸ M or about 1 × 10⁻⁹ M). In still other embodiments, an ActRII antagonist antibody, or combination of antibodies, binds to at least ActRIIA and ActRIIB. As used herein, an ActRII antibody (anti-ActRII antibody) generally refers to an antibody that binds to ActRII (e.g., ActRIIA and/or ActRIIB) with sufficient affinity such that the antibody is useful as a diagnostic and/or therapeutic agent in targeting ActRII. In certain embodiments, the extent of binding of an anti-ActRII antibody to an unrelated, non-ActRII protein is less than about 10%, 9%, 8%, 7%, 6%, 5%, 4%, 3%, 2%, or less than about 1% of the binding of the antibody to ActRII as measured, for example, by a radioimmunoassay (RIA), Biacore, or other protein-protein interaction or binding affinity assay. In certain embodiments, an anti-ActRII antibody binds to an epitope of ActRII (e.g., ActRIIA and/or ActRIIB) that is conserved among ActRII from different species. In certain preferred embodiments, an anti-ActRII antibody binds to human ActRII (e.g., ActRIIA and/or ActRIIB). In other preferred embodiments, an anti-ActRII antibody may inhibit one or more ligands [e.g., GDF8, activin (e.g., activin A, activin B, activin C, activin E, activin AB, activin AC) GDF3, BMP6, BMP10, and BMP9] from binding to ActRII (e.g., ActRIIA and/or ActRIIB). It should be noted that ActRIIA has sequence homology to ActRIIB and therefore antibodies that bind to ActRIIA, in some cases, may also bind to and/or inhibit ActRIIB, the reverse is also true. In some embodiments, an anti-ActRII antibody is a multispecific antibody (e.g., bi-specific antibody) that binds to ActRII (e.g., ActRIIA and/or ActRIIB) and one or more ligands [e.g., GDF8, activin (e.g., activin A, activin B, activin C, activin E, activin AB, activin AC) GDF3, BMP6, BMP10, BMP9, TGFβ1, TGFβ2, and TGFβ3]. In some embodiments, an anti-ActRII antibody is a multispecific antibody (e.g., bi-specific antibody) that binds to ActRIIA and ActRIIB. In some embodiments, the disclosure relates to combinations of antibodies, as well as uses thereof, wherein the combination of antibodies comprises at least an anti-ActRIIA antibody and at least an ActRIIB antibody. In some embodiments, the disclosure relates to combinations of antibodies, as well as uses thereof, wherein the combination of antibodies comprises an anti-ActRIIA antibody and one or more additional antibodies that bind to, for example, one or more ligands [*e.g*., GDF8, activin (e.g., activin A, activin B, activin C, activin E, activin AB, activin AC) GDF3, BMP6, BMP10, BMP9, TGFβ1, TGFβ2, and TGFβ3], TGFβRII, ALK4, and/or ALK5. In some embodiments, the disclosure relates to combinations of antibodies, as well as uses thereof, wherein the combination of antibodies comprises an anti-ActRIIB antibody and one or more additional antibodies that bind to, for example, one or more ligands [*e.g*., GDF8, activin (e.g., activin A, activin B, activin C, activin E, activin AB, activin AC) GDF3, BMP6, BMP10, BMP9, TGFβ1, TGFβ2, and TGFβ3], TGFβRII, ALK4, and/or ALK5. In some embodiments, the disclosure relates to combinations of antibodies, as well as uses thereof (e.g., increasing an immune response in a subject in need thereof and treating cancer), wherein the combination of antibodies comprises an anti-ActRIIA antibody, an anti-ActRIIB antibody, and at least one or more additional antibodies that bind to, for example, one or more ligands [*e.g*., GDF8, activin (e.g., activin A, activin B, activin C, activin E, activin AB, activin AC) GDF3, BMP6, BMP10, BMP9, TGFβ1, TGFβ2, and TGFβ3], TGFβRII, ALK4, and/or ALK5.

In certain aspects, an ActRII antagonist antibody, or combination of antibodies, of the disclosure is an antibody that inhibits at least ALK4. Therefore, in some embodiments, an ActRII antagonist antibody, or combination of antibodies, binds to at least ALK4. As used herein, an ALK4 antibody (anti-ALK4 antibody) generally refers to an antibody that binds to ALK4 with sufficient affinity such that the antibody is useful as a diagnostic and/or therapeutic agent in targeting ALK4. In certain embodiments, the extent of binding of an anti-ALK4 antibody to an unrelated, non-ALK4 protein is less than about 10%, 9%, 8%, 7%, 6%, 5%, 4%, 3%, 2%, or less than about 1% of the binding of the antibody to ALK4 as measured, for example, by a radioimmunoassay (RIA), Biacore, or other protein-protein interaction or binding affinity assay. In certain embodiments, an anti-ALK4 antibody binds to an epitope of ALK4 that is conserved among ALK4 from different species. In certain preferred embodiments, an anti-ALK4 antibody binds to human ALK4. In other preferred embodiments, an anti-ALK4 antibody may inhibit one or more ligands [*e.g*., GDF8, activin (e.g., activin A, activin B, activin C, activin E, activin AB, activin AC) GDF3, BMP6, BMP10, and BMP9] from binding to ALK4. In some embodiments, an anti-ALK4 antibody is a multispecific antibody (e.g., bi-specific antibody) that binds to ALK4 and one or more ligands [*e.g*., GDF8, activin (e.g., activin A, activin B, activin C, activin E, activin AB, activin AC) GDF3, BMP6, BMP10, BMP9, TGFβ1, TGFβ2, and TGFβ3], TGFβRII, ActRII (ActRIIA and/or ActRIIB) and/or ALK5. In some embodiments, the disclosure relates to combinations of antibodies, as well as uses thereof, wherein the combination of antibodies comprises an anti-ALK4 antibody and one or more additional antibodies that bind to, for example, one or more ligands [e.g., GDF8, activin (e.g., activin A, activin B, activin C, activin E, activin AB, activin AC) GDF3, BMP6, BMP10, and BMP9, TGFβ1, TGFβ2, and TGFβ3], TGFβRII, ActRII (ActRIIA and/or ActRIIB) and/or ALK5.

In certain aspects, a TGFβRII antagonist to be used in accordance with the methods and uses disclosed herein is a TGFβRII antagonist antibody or combination of antibodies. A TGFβRII antagonist antibody, or combination of antibodies, may inhibit and/or bind to, for example, one or more TGFβRII ligands (*e.g.*, TGFβ1, TGFβ2, and TGFβ3), TGFβRII receptor, TGFβRII-associated type I receptor (e.g., ALK5), and/or TGFβRII co-receptor (e.g., betaglycan). In some embodiments, the ability for a TGFβRII antagonist antibody, or combination of antibody, to inhibit signaling (e.g., Smad signaling) and/or bind to a target is determined in an in vitro or cell-based assay including, for example, those described herein. As described herein, a TGFβRII antagonist antibody, or combination of antagonist antibodies, may be used alone or in combination with one or more additional supportive therapies or active agents (e.g., an ActRII antagonists) to treat or prevent a disorder or condition as described herein.

In certain embodiments, a TGFβRII antagonist antibody, or combination of antibodies, is an antibody that inhibits at least TGFβ1. Therefore, in some embodiments, a TGFβRII antagonist antibody, or combination of antibodies, binds to at least TGFβ1. As used herein, a TGFβ1 antibody (anti-TGFβ1 antibody) generally refers to an antibody that binds to TGFβ1 with sufficient affinity such that the antibody is useful as a diagnostic and/or therapeutic agent in targeting TGFβ1. In certain embodiments, the extent of binding of an anti-TGFβ1 antibody to an unrelated, non-TGFβ1 protein is less than about 10%, 9%, 8%, 7%, 6%, 5%, 4%, 3%, 2%, or less than 1% of the binding of the antibody to TGFβ1 as measured, for example, by a radioimmunoassay (RIA). In certain embodiments, an anti-TGFβ1 antibody binds to an epitope of TGFβ1 that is conserved among TGFβ1 from different species. In certain preferred embodiments, an anti-TGFβ1 antibody binds to human TGFβ1. In some embodiments, a TGFβ1 antibody may inhibit TGFβ1 from binding to a type I, type II, and/or co-receptor (e.g., TGFβRII, ALK5, and/or betaglycan) and thus inhibit TGFβ1 signaling (e.g., Smad signaling). It should be noted that TGFβ1 shares some sequence homology to TGFβ2 and TGFβ3. Therefore antibodies that bind TGFβ1, in some embodiments, may also bind to TGFβ2 and/or TGFβ3. In some embodiments, the disclosure relates to a multispecific antibody (e.g., bi-specific antibody), and uses thereof, that binds to TGFβ1 and further binds to, for example, one or more additional TGFβRII ligands (e.g., TGFβ2, TGFβ3, or TGFβ2 and TGFβ3), one or more type I and/or type II receptors (e.g., TGFβRII and ALK5), and/or one or more co-receptors (e.g., betaglycan). In some embodiments, the disclosure relates to combinations of antibodies, and uses thereof, wherein the combination of antibodies comprises a TGFβ1 antibody and one or more additional antibodies that bind to, for example, one or more additional TGFβRII ligands (e.g., TGFβ2, TGFβ3, or TGFβ2 and TGFβ3), one or more type I and/or type II receptors (e.g., TGFβRII and ALK5), and/or one or more co-receptors (e.g., betaglycan). In some embodiments, an anti- TGFβ1 antibody is a multispecific antibody (e.g., bi-specific antibody) that binds to one or more ActRII ligands [*e.g.*, GDF8, activin (e.g., activin A, activin B, activin C, activin E, activin AB, activin AC) GDF3, BMP6, BMP10, and BMP9], TGFβRII, ActRII (ActRIIA and/or ActRIIB), ALK4, ALK5, and/or betaglycan. In some embodiments, the disclosure relates to combinations of antibodies, as well as uses thereof, wherein the combination of antibodies comprises an anti- TGFβ1 antibody and one or more additional antibodies that bind to, for example, one or more ActRIIB ligands [*e.g*., GDF8, activin (e.g., activin A, activin B, activin C, activin E, activin AB, activin AC) GDF3, BMP6, BMP10, and BMP9], TGFβRII, ActRII (ActRIIA and/or ActRIIB) ALK4, ALK5, and/or betaglycan.

In certain embodiments, a TGFβRII antagonist antibody, or combination of antibodies, is an antibody that inhibits at least TGFβ2. Therefore, in some embodiments, a TGFβRII antagonist antibody, or combination of antibodies, binds to at least TGFβ2. As used herein, a TGFβ2 antibody (anti-TGFβ2 antibody) generally refers to an antibody that is capable of binding to TGFβ2 with sufficient affinity such that the antibody is useful as a diagnostic and/or therapeutic agent in targeting TGFβ2. In certain embodiments, the extent of binding of an anti-TGFβ2 antibody to an unrelated, non-TGFβ2 protein is less than about 10%, 9%, 8%, 7%, 6%, 5%, 4%, 3%, 2%, or less than 1% of the binding of the antibody to TGFβ2 as measured, for example, by a radioimmunoassay (RIA). In certain embodiments, an anti-TGFβ2 antibody binds to an epitope of TGFβ2 that is conserved among TGFβ2 from different species. In certain preferred embodiments, an anti-TGFβ2 antibody binds to human TGFβ2. In some embodiments, a TGFβ2 antibody may inhibit TGFβ2 from binding to a type I, type II, and/or co-receptor (e.g., TGFβRII, ALK5, and/or betaglycan) and thus inhibit TGFβ2 signaling (e.g., Smad signaling). It should be noted that TGFβ2 shares some sequence homology to TGFβ1 and TGFβ3. Therefore antibodies that bind TGFβ2, in some embodiments, may also bind to TGFβ1 and/or TGFβ3. In some embodiments, the disclosure relates to a multispecific antibody (e.g., bi-specific antibody), and uses thereof, that binds to TGFβ2 and further binds to, for example, one or more additional TGFβRII ligands (e.g., TGFβ1, TGFβ3, or TGFβ1 and TGFβ3), one or more type I and/or type II receptors (e.g., TGFβRII and ALK5), and/or one or more co-receptors (e.g., betaglycan) In some embodiments, the disclosure relates to combinations of antibodies, and uses thereof, wherein the combination of antibodies comprises a TGFβ2 antibody and one or more additional antibodies that bind to, for example, one or more additional TGFβRII ligands (e.g., TGFβ1, TGFβ3, or TGFβ1 and TGFβ3), one or more type I and/or type II receptors (e.g., TGFβRII and ALK5), and/or one or more co-receptors (e.g., betaglycan). In some embodiments, an anti-TGFβ2 antibody is a multispecific antibody (e.g., bi-specific antibody) that binds to one or more ActRII ligands [*e.g*., GDF8, activin (e.g., activin A, activin B, activin C, activin E, activin AB, activin AC) GDF3, BMP6, BMP10, and BMP9], TGFβRII, ActRII (ActRIIA and/or ActRIIB), ALK4, ALK5, and/or betaglycan. In some embodiments, the disclosure relates to combinations of antibodies, as well as uses thereof, wherein the combination of antibodies comprises an anti-TGFβ2 antibody and one or more additional antibodies that bind to, for example, one or more ActRIIB ligands [*e.g*., GDF8, activin (e.g., activin A, activin B, activin C, activin E, activin AB, activin AC) GDF3, BMP6, BMP10, and BMP9], TGFβRII, ActRII (ActRIIA and/or ActRIIB) ALK4, ALK5, and/or betaglycan.

In certain embodiments, a TGFβRII antagonist antibody, or combination of antibodies, is an antibody that inhibits at least TGFβ3. Therefore, in some embodiments, a TGFβRII antagonist antibody, or combination of antibodies, binds to at least TGFβ3. As used herein, a TGFβ3 antibody (anti-TGFβ3 antibody) generally refers to an antibody that is capable of binding to TGFβ3 with sufficient affinity such that the antibody is useful as a diagnostic and/or therapeutic agent in targeting TGFβ3. In certain embodiments, the extent of binding of an anti-TGFβ3 antibody to an unrelated, non-TGFβ3 protein is less than about 10%, 9%, 8%, 7%, 6%, 5%, 4%, 3%, 2%, or less than 1% of the binding of the antibody to TGFβ3 as measured, for example, by a radioimmunoassay (RIA). In certain embodiments, an anti-TGFβ3 antibody binds to an epitope of TGFβ3 that is conserved among TGFβ3 from different species. In certain preferred embodiments, an anti-TGFβ3 antibody binds to human TGFβ3. In some embodiments, a TGFβ3 antibody may inhibit TGFβ3 from binding to a type I, type II, and/or co-receptor (e.g., TGFβRII, ALK5, and/or betaglycan) and thus inhibit TGFβ3 signaling (e.g., Smad signaling). It should be noted that TGFβ3 shares some sequence homology to TGFβ2 and TGFβ1. Therefore antibodies that bind TGFβ3, in some embodiments, may also bind to TGFβ2 and/or TGFβ1. In some embodiments, the disclosure relates to a multispecific antibody (e.g., bi-specific antibody), and uses thereof, that binds to TGFβ3 and further binds to, for example, one or more additional TGFβRII ligands (e.g., TGFβ2, TGFβ1, or TGFβ2 and TGFβ1), one or more type I and/or type II receptors (e.g., TGFβRII and ALK5), and/or one or more co-receptors (e.g., betaglycan). In some embodiments, an anti-TGFβ3 antibody is a multispecific antibody (e.g., bi-specific antibody) that binds to one or more ActRII ligands [*e.g*., GDF8, activin (e.g., activin A, activin B, activin C, activin E, activin AB, activin AC) GDF3, BMP6, BMP10, and BMP9], TGFβRII, ActRII (ActRIIA and/or ActRIIB), ALK4, ALK5, and/or betaglycan. In some embodiments, the disclosure relates to combinations of antibodies, as well as uses thereof, wherein the combination of antibodies comprises an anti-TGFβ3 antibody and one or more additional antibodies that bind to, for example, one or more ActRIIB ligands [*e.g*., GDF8, activin (e.g., activin A, activin B, activin C, activin E, activin AB, activin AC) GDF3, BMP6, BMP10, and BMP9], TGFβRII, ActRII (ActRIIA and/or ActRIIB) ALK4, ALK5, and/or betaglycan.

In certain aspects, a TGFβRII antagonist antibody, or combination of antibodies, is an antibody that inhibits at least TGFβRII. Therefore, in some embodiments, a TGFβRII antagonist antibody, or combination of antibodies, binds to at least TGFβRII. As used herein, a TGFβRII antibody (anti-TGFβRII antibody) generally refers to an antibody that binds to TGFβRII with sufficient affinity such that the antibody is useful as a diagnostic and/or therapeutic agent in targeting TGFβRII. In certain embodiments, the extent of binding of an anti-TGFβRII antibody to an unrelated, non-TGFβRII protein is less than about 10%, 9%, 8%, 7%, 6%, 5%, 4%, 3%, 2%, or less than about 1% of the binding of the antibody to TGFβRII as measured, for example, by a radioimmunoassay (RIA), Biacore, or other protein-protein interaction or binding affinity assay. In certain embodiments, an anti-TGFβRII antibody binds to an epitope of TGFβRII that is conserved among TGFβRII from different species. In certain preferred embodiments, an anti-TGFβRII antibody binds to human TGFβRII. In some embodiments, an anti-TGFβRII antibody may inhibit one or more TGFβRII ligands *[e.g.,* TGFβ1; TGFβ2; TGFβ3; TGFβ1 and TGFβ3; TGFβ1 and TGFβ2; TGFβ2 and TGFβ3; or TGFβ1, TGFβ2, and TGFβ3] from binding to TGFβRII. In some embodiments, an anti-TGFβRII antibody is a multispecific antibody (e.g., bi-specific antibody) that binds to TGFβRII and one or more ligands [*e.g*., GDF8, activin (e.g., activin A, activin B, activin C, activin E, activin AB, activin AC) GDF3, BMP6, BMP10, BMP9, TGFβ1, TGFβ2, and TGFβ3], ActRII (ActRIIA and/or ActRIIB) ALK5 and/or betaglycan. In some embodiments, the disclosure relates to combinations of antibodies, and uses thereof, wherein the combination of antibodies comprises an anti-TGFβRII antibody and one or more additional antibodies that bind to, for example, one or more ligands [*e.g.*, GDF8, activin (e.g., activin A, activin B, activin C, activin E, activin AB, activin AC) GDF3, BMP6, BMP10, BMP9, TGFβ1, TGFβ2, and TGFβ3], ActRII (ActRIIA and/or ActRIIB) ALK5 and/or betaglycan.

In certain aspects, a TGFβRII antagonist antibody, or combination of antibodies, is an antibody that inhibits at least ALK5. Therefore, in some embodiments, a TGFβRII antagonist antibody, or combination of antibodies, binds to at least ALK5. As used herein, an ALK5 antibody (anti-ALK5antibody) generally refers to an antibody that binds to ALK5 with sufficient affinity such that the antibody is useful as a diagnostic and/or therapeutic agent in targeting ALK5. In certain embodiments, the extent of binding of an anti-ALK5 antibody to an unrelated, non-ALK5 protein is less than about 10%, 9%, 8%, 7%, 6%, 5%, 4%, 3%, 2%, or less than about 1% of the binding of the antibody to ALK5 as measured, for example, by a radioimmunoassay (RIA), Biacore, or other protein-protein interaction or binding affinity assay. In certain embodiments, an anti-ALK5 antibody binds to an epitope of ALK5 that is conserved among ALK5 from different species. In certain preferred embodiments, an anti-ALK5 antibody binds to human ALK5. In some embodiments, an anti-ALK5 antibody may inhibit one or more TGFβRII ligands [*e.g.*, TGFβ1; TGFβ2; TGFβ3; TGFβ1 and TGFβ3; TGFβ1 and TGFβ2; TGFβ2 and TGFβ3; or TGFβ1, TGFβ2, and TGFβ3] from binding to ALK5. In some embodiments, an anti-ALK5 antibody is a multispecific antibody (e.g., bi-specific antibody) that binds to ALK5 and one or more ligands [*e.g*., GDF8, activin (e.g., activin A, activin B, activin C, activin E, activin AB, activin AC) GDF3, BMP6, BMP10, BMP9, TGFβ1, TGFβ2, and TGFβ3], TGFβRII, ActRII (ActRIIA and/or ActRIIB) and/or betaglycan. In some embodiments, the disclosure relates to combinations of antibodies, and uses thereof, wherein the combination of antibodies comprises an anti-ALK5 antibody and one or more additional antibodies that bind to, for example, one or more TGFβRII ligands [*e.g.*, GDF8, activin (e.g., activin A, activin B, activin C, activin E, activin AB, activin AC) GDF3, BMP6, BMP10, BMP9, TGFβ1, TGFβ2, and TGFβ3], TGFβRII, ActRII (ActRIIA and/or ActRIIB) and/or betaglycan.

In certain aspects, a TGFβRII antagonist antibody, or combination of antibodies, is an antibody that inhibits at least betaglycan. Therefore, in some embodiments, a TGFβRII antagonist antibody, or combination of antibodies, binds to at least betaglycan. As used herein, a betaglycan antibody (anti-betaglycan antibody) generally refers to an antibody that binds to betaglycan with sufficient affinity such that the antibody is useful as a diagnostic and/or therapeutic agent in targeting betaglycan. In certain embodiments, the extent of binding of an anti-betaglycan antibody to an unrelated, non-betaglycan protein is less than about 10%, 9%, 8%, 7%, 6%, 5%, 4%, 3%, 2%, or less than about 1% of the binding of the antibody to betaglycan as measured, for example, by a radioimmunoassay (RIA), Biacore, or other protein-protein interaction or binding affinity assay. In certain embodiments, an anti-betaglycan antibody binds to an epitope of betaglycan that is conserved among betaglycan from different species. In certain preferred embodiments, an anti-betaglycan antibody binds to human betaglycan. In some embodiments, an anti-betaglycan antibody may inhibit one or more TGFβRII ligands [*e.g.*, TGFβ1; TGFβ2; TGFβ3; TGFβ1 and TGFβ3; TGFβ1 and TGFβ2; TGFβ2 and TGFβ3; or TGFβ1, TGFβ2, and TGFβ3] from binding to betaglycan. In some embodiments, an anti-betaglycan antibody is a multispecific antibody (e.g., bi-specific antibody) that binds to betaglycan and one or more ligands [e.g., GDF8, activin (e.g., activin A, activin B, activin C, activin E, activin AB, activin AC) GDF3, BMP6, BMP10, BMP9, TGFβ1, TGFβ2, and TGFβ3], TGFβRII, ActRII (ActRIIA and/or ActRIIB) and/or ALK5. In some embodiments, the disclosure relates to combinations of antibodies, and uses thereof, wherein the combination of antibodies comprises an anti-betaglycan antibody and one or more additional antibodies that bind to, for example, one or more TGFβRII ligands [*e.g.*, GDF8, activin (e.g., activin A, activin B, activin C, activin E, activin AB, activin AC) GDF3, BMP6, BMP10, BMP9, TGFβ1, TGFβ2, and TGFβ3], TGFβRII, ActRII (ActRIIA and/or ActRIIB) and/or ALK5.

The term antibody is used herein in the broadest sense and encompasses various antibody structures, including but not limited to monoclonal antibodies, polyclonal antibodies, multispecific antibodies (*e.g.*, bispecific antibodies), and antibody fragments so long as they exhibit the desired antigen-binding activity. An antibody fragment refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g*., scFv); and multispecific antibodies formed from antibody fragments. See, *e.g.,* Hudson et al. (2003) Nat. Med. 9:129-134; Plückthun, in The Pharmacology of Monoclonal Antibodies, vol. 113, Rosenburg and Moore eds., (Springer-Verlag, New York), pp. 269-315 (1994); WO 93/16185; and U.S. Pat. Nos. 5,571,894, 5,587,458, and 5,869,046. Antibodies disclosed herein may be polyclonal antibodies or monoclonal antibodies. In certain embodiments, the antibodies of the present disclosure comprise a label attached thereto and able to be detected (*e.g.*, the label can be a radioisotope, fluorescent compound, enzyme, or enzyme co-factor). In preferred embodiments, the antibodies of the present disclosure are isolated antibodies.

Diabodies are antibody fragments with two antigen-binding sites that may be bivalent or bispecific. See, *e.g.*, EP 404,097; WO 1993/01161; Hudson et al. (2003) Nat. Med. 9:129-134 (2003); and Hollinger et al. (1993) Proc. Natl. Acad. Sci. USA 90: 6444-6448. Triabodies and tetrabodies are also described in Hudson et al. (2003) Nat. Med. 9:129-134.

Single-domain antibodies are antibody fragments comprising all or a portion of the heavy-chain variable domain or all or a portion of the light-chain variable domain of an antibody. In certain embodiments, a single-domain antibody is a human single-domain antibody. *See*, *e.g.*, U.S. Pat. No. 6,248,516.

Antibody fragments can be made by various techniques, including but not limited to proteolytic digestion of an intact antibody as well as production by recombinant host cells (e.g., E. coli or phage), as described herein.

The antibodies herein may be of any class. The class of an antibody refers to the type of constant domain or constant region possessed by its heavy chain. There are five major classes of antibodies: IgA, IgD, IgE, IgG, and IgM, and several of these may be further divided into subclasses (isotypes), for example, IgG₁, IgG₂, IgG₃, IgG₄, IgA₁, and IgA₂. The heavy-chain constant domains that correspond to the different classes of immunoglobulins are called alpha, delta, epsilon, gamma, and mu.

In general, an antibody for use in the methods disclosed herein specifically binds to its target antigen, preferably with high binding affinity. Affinity may be expressed as a K_{D} value and reflects the intrinsic binding affinity (e.g., with minimized avidity effects). Typically, binding affinity is measured *in vitro*, whether in a cell-free or cell-associated setting. Any of a number of assays known in the art, including those disclosed herein, can be used to obtain binding affinity measurements including, for example, surface plasmon resonance (Biacore^{™} assay), radiolabeled antigen binding assay (RIA), and ELISA. In some embodiments, antibodies of the present disclosure bind to their target antigens [*e.g.*, ActRIIB, ActRIIA, ALK4, GDF8, activin (e.g., activin A, activin B, activin C, activin E, activin AB, activin AC) GDF3, BMP6, BMP10, BMP9, TGFβ1, TGFβ2, TGFβ3, TGFβRII, ALK5 and/or betaglycan] with at least a K_{D} of 1x 10⁻⁷ or stronger, 1×10⁻⁸ or stronger, 1×10⁻⁹ or stronger, 1×10⁻¹⁰ or stronger, 1×10⁻¹¹ or stronger, 1×10⁻¹² or stronger, 1×10⁻¹³ or stronger, or 1×10⁻¹⁴ or stronger.

In certain embodiments, K_{D} is measured by RIA performed with the Fab version of an antibody of interest and its target antigen as described by the following assay. Solution binding affinity of Fabs for the antigen is measured by equilibrating Fab with a minimal concentration of radiolabeled antigen (*e.g*., ¹²⁵I-labeled) in the presence of a titration series of unlabeled antigen, then capturing bound antigen with an anti-Fab antibody-coated plate [see, *e.g.*, Chen et al. (1999) J. Mol. Biol. 293:865-881]. To establish conditions for the assay, multi-well plates (*e.g.*, MICROTITER^{®} from Thermo Scientific) are coated (*e.g.*, overnight) with a capturing anti-Fab antibody (*e.g.*, from Cappel Labs) and subsequently blocked with bovine serum albumin, preferably at room temperature (e.g., approximately 23°C). In a non-adsorbent plate, radiolabeled antigen are mixed with serial dilutions of a Fab of interest [e.g., consistent with assessment of the anti-VEGF antibody, Fab-12, in Presta et al., (1997) Cancer Res. 57:4593-4599]. The Fab of interest is then incubated, preferably overnight but the incubation may continue for a longer period (e.g., about 65 hours) to ensure that equilibrium is reached. Thereafter, the mixtures are transferred to the capture plate for incubation, preferably at room temperature for about one hour. The solution is then removed and the plate is washed times several times, preferably with polysorbate 20 and PBS mixture. When the plates have dried, scintillant (*e.g.*, MICROSCINT^{®} from Packard) is added, and the plates are counted on a gamma counter (*e.g.*, TOPCOUNT^{®} from Packard).

According to another embodiment, K_{D} is measured using surface plasmon resonance assays using, for example a BIACORE^{®} 2000 or a BIACORE^{®} 3000 (Biacore, Inc., Piscataway, N.J.) with immobilized antigen CM5 chips at about 10 response units (RU). Briefly, carboxymethylated dextran biosensor chips (CM5, Biacore, Inc.) are activated with N-ethyl-N'-(3-dimethylaminopropyl)-carbodiimide hydrochloride (EDC) and N-hydroxysuccinimide (NHS) according to the supplier's instructions. For example, an antigen can be diluted with 10 mM sodium acetate, pH 4.8, to 5 µg/ml (about 0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{®}) surfactant (PBST) at at a flow rate of approximately 25 µl/min. Association rates (kₒₙ) and dissociation rates (k_{off}) are calculated using, for example, a simple one-to-one Langmuir binding model (BIACORE^{®} Evaluation Software version 3.2) by simultaneously fitting the association and dissociation sensorgrams. The equilibrium dissociation constant (K_{D}) is calculated as the ratio k_{off} / kₒₙ [see, *e.g.,* Chen et al., (1999) J. Mol. Biol. 293:865-881]. If the on-rate exceeds, for example, 10⁶ M⁻¹ s⁻¹ by the surface plasmon resonance assay above, then the on-rate can be determined by using a fluorescent quenching technique that measures the increase or decrease in fluorescence emission intensity (e.g., excitation=295 nm; emission=340 nm, 16 nm bandpass) of a 20 nM anti-antigen antibody (Fab form) in PBS in the presence of increasing concentrations of antigen as measured in a spectrometer, such as a stop-flow equipped spectrophometer (Aviv Instruments) or a 8000-series SLM-AMINCO^{®} spectrophotometer (ThermoSpectronic) with a stirred cuvette.

The nucleic acid and amino acid sequences of human ActRIIB, ActRIIA, ALK4, GDF8, activin (e.g., activin A, activin B, activin C, activin E, activin AB, activin AC) GDF3, BMP6, BMP10, BMP9, TGFβ1, TGFβ2, TGFβ3, TGFβRII, ALK5 and/or betaglycan are well known in the art and thus antibody antagonists for use in accordance with this disclosure may be routinely made by the skilled artisan based on the knowledge in the art and teachings provided herein.

In certain embodiments, an antibody provided herein is a chimeric antibody. A chimeric antibody refers to an antibody in which a portion of the heavy and/or light chain is derived from a particular source or species, while the remainder of the heavy and/or light chain is derived from a different source or species. Certain chimeric antibodies are described, for example, in U.S. Pat. No. 4,816,567; and Morrison et al., (1984) Proc. Natl. Acad. Sci. USA, 81:6851-6855. In some embodiments, a chimeric antibody comprises a non-human variable region (*e.g*., a variable region derived from a mouse, rat, hamster, rabbit, or non-human primate, such as a monkey) and a human constant region. In some embodiments, a chimeric antibody is a "class switched" antibody in which the class or subclass has been changed from that of the parent antibody. In general, chimeric antibodies include antigen-binding fragments thereof.

In certain embodiments, a chimeric antibody provided herein is a humanized antibody. A humanized antibody refers to a chimeric antibody comprising amino acid residues from non-human hypervariable regions (HVRs) and amino acid residues from human framework regions (FRs). In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g.*, a non-human antibody, refers to an antibody that has undergone humanization.

Humanized antibodies and methods of making them are reviewed, for example, in Almagro and Fransson (2008) Front. Biosci. 13:1619-1633 and are further described, for example, in Riechmann et al., (1988) Nature 332:323-329; Queen et al. (1989) Proc. Nat'l Acad. Sci. USA 86:10029-10033; U.S. Pat. Nos. 5,821,337, 7,527,791, 6,982,321, and 7,087,409; Kashmiri et al., (2005) Methods 36:25-34 [describing SDR (a-CDR) grafting]; Padlan, Mol. Immunol. (1991) 28:489-498 (describing "resurfacing"); Dall'Acqua et al. (2005) Methods 36:43-60 (describing "FR shuffling"); Osbourn et al. (2005) Methods 36:61-68; and Klimka et al. Br. J. Cancer (2000) 83:252-260 (describing the "guided selection" approach to FR shuffling).

Human framework regions that may be used for humanization include but are not limited to: framework regions selected using the "best-fit" method [see, *e.g.,* Sims et al. (1993) J. Immunol. 151:2296]; framework regions derived from the consensus sequence of human antibodies of a particular subgroup of light-chain or heavy-chain variable regions [see, *e.g.,* Carter et al. (1992) Proc. Natl. Acad. Sci. USA, 89:4285; and Presta et al. (1993) J. Immunol., 151:2623]; human mature (somatically mutated) framework regions or human germline framework regions [see, e.g., Almagro and Fransson (2008) Front. Biosci. 13:1619-1633]; and framework regions derived from screening FR libraries [see, *e.g*., Baca et cd., (1997) J. Biol. Chem. 272:10678-10684; and Rosok et cd., (1996) J. Biol. Chem. 271:22611-22618].

In certain embodiments, an antibody provided herein is a human antibody. Human antibodies can be produced using various techniques known in the art. Human antibodies are described generally in van Dijk and van de Winkel (2001) Curr. Opin. Pharmacol. 5: 368-74 and Lonberg (2008) Curr. Opin. Immunol. 20:450-459.

Human antibodies may be prepared by administering an immunogen [*e.g.*, ActRIIB, ActRIIA, ALK4, GDF8, activin (e.g., activin A, activin B, activin C, activin E, activin AB, activin AC) GDF3, BMP6, BMP10, BMP9, TGFβ1, TGFβ2, TGFβ3, TGFβRII, ALK5 and/or betaglycan] to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. For a review of methods for obtaining human antibodies from transgenic animals, see, for example, Lonberg (2005) Nat. Biotechnol. 23:1117-1125; U.S. Pat. Nos. 6,075,181 and 6,150,584 (describing XENOMOUSE^{™} technology); U.S. Pat. No. 5,770,429 (describing HuMab^{®} technology); U.S. Pat. No. 7,041,870 (describing K-M MOUSE^{®} technology); and U.S. Patent Application Publication No. 2007/0061900 (describing VelociMouse^{®} technology). Human variable regions from intact antibodies generated by such animals may be further modified, for example, by combining with a different human constant region.

Human antibodies provided herein can also be made by hybridoma-based methods. Human myeloma and mouse-human heteromyeloma cell lines for the production of human monoclonal antibodies have been described [see, *e.g*., Kozbor J. Immunol., (1984) 133: 3001; Brodeur et al. (1987) Monoclonal Antibody Production Techniques and Applications, pp. 51-63, Marcel Dekker, Inc., New York; and Boerner et al. (1991) J. Immunol., 147: 86]. Human antibodies generated via human B-cell hybridoma technology are also described in Li et al., (2006) Proc. Natl. Acad. Sci. USA, 103:3557-3562. Additional methods include those described, for example, in U.S. Pat. No. 7,189,826 (describing production of monoclonal human IgM antibodies from hybridoma cell lines) and Ni, Xiandai Mianyixue (2006) 26(4):265-268 (2006) (describing human-human hybridomas). Human hybridoma technology (Trioma technology) is also described in Vollmers and Brandlein (2005) Histol. Histopathol., 20(3):927-937 (2005) and Vollmers and Brandlein (2005) Methods Find Exp. Clin. Pharmacol., 27(3):185-91.

Human antibodies provided herein may also be generated by isolating Fv clone variable-domain sequences selected from human-derived phage display libraries. Such variable-domain sequences may then be combined with a desired human constant domain. Techniques for selecting human antibodies from antibody libraries are described herein.

For example, antibodies of the present disclosure may be isolated by screening combinatorial libraries for antibodies with the desired activity or activities. A variety of methods are known in the art for generating phage-display libraries and screening such libraries for antibodies possessing the desired binding characteristics. Such methods are reviewed, for example, in Hoogenboom et al. (2001) in Methods in Molecular Biology 178:1-37, O'Brien et al., ed., Human Press, Totowa, N.J. and further described, for example, in the McCafferty et al. (1991) Nature 348:552-554; Clackson et al., (1991) Nature 352: 624-628; Marks et al. (1992) J. Mol. Biol. 222:581-597; Marks and Bradbury (2003) in Methods in Molecular Biology 248:161-175, Lo, ed., Human Press, Totowa, N.J.; Sidhu et al. (2004) J. Mol. Biol. 338(2):299-310; Lee et al. (2004) J. Mol. Biol. 340(5):1073-1093; Fellouse (2004) Proc. Natl. Acad. Sci. USA 101(34):12467-12472; and Lee et al. (2004) J. Immunol. Methods 284(1-2): 119-132.

In certain phage display methods, repertoires of VH and VL genes are separately cloned by polymerase chain reaction (PCR) and recombined randomly in phage libraries, which can then be screened for antigen-binding phage as described in Winter et al. (1994) Ann. Rev. Immunol., 12: 433-455. Phage typically display antibody fragments, either as single-chain Fv (scFv) fragments or as Fab fragments. Libraries from immunized sources provide high-affinity antibodies to the immunogen [*e.g*., ActRIIB, ActRIIA, ALK4, GDF8, activin (e.g., activin A, activin B, activin C, activin E, activin AB, activin AC) GDF3, BMP6, BMP10, BMP9, TGFβ1, TGFβ2, TGFβ3, TGFβRII, ALK5 and/or betaglycan] without the requirement of constructing hybridomas. Alternatively, the naive repertoire can be cloned (*e.g.*, from human) to provide a single source of antibodies directed against a wide range of non-self and also self-antigens without any immunization as described by Griffiths et al. (1993) EMBO J, 12: 725-734. Finally, naive libraries can also be made synthetically by cloning un-rearranged V-gene segments from stem cells and using PCR primers containing random sequence to encode the highly variable CDR3 regions and to accomplish rearrangement in vitro, as described by Hoogenboom and Winter (1992) J. Mol. Biol., 227: 381-388. Patent publications describing human antibody phage libraries include, for example: U.S. Pat. No. 5,750,373, and U.S. Patent Publication Nos. 2005/0079574, 2005/0119455, 2005/0266000, 2007/0117126, 2007/0160598, 2007/0237764, 2007/0292936, and 2009/0002360.

In certain embodiments, an antibody provided herein is a multispecific antibody, for example, a bispecific antibody. Multispecific antibodies (typically monoclonal antibodies) have binding specificities for at least two different epitopes (*e.g*., two, three, four, five, or six or more) on one or more (*e.g*., two, three, four, five, six or more) antigens.

Engineered antibodies with three or more functional antigen binding sites, including "octopus antibodies," are also included herein (see, *e.g.,* US 2006/0025576A1).

In certain embodiments, the antibodies disclosed herein are monoclonal antibodies. Monoclonal antibody refers to an antibody obtained from a population of substantially homogeneous antibodies, *i.e.*, the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. Thus, the modifier "monoclonal" indicates the character of the antibody as being obtained from a substantially homogeneous population of antibodies and is not to be construed as requiring production of the antibody by any particular method. For example, the monoclonal antibodies to be used in accordance with the present methods may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

For example, by using immunogens derived from GDF11, anti-protein/anti-peptide antisera or monoclonal antibodies can be made by standard protocols [see, *e.g*., Antibodies: A Laboratory Manual (1988) ed. by Harlow and Lane, Cold Spring Harbor Press]. A mammal, such as a mouse, hamster, or rabbit can be immunized with an immunogenic form of the GDF11 polypeptide, an antigenic fragment which is capable of eliciting an antibody response, or a fusion protein. Techniques for conferring immunogenicity on a protein or peptide include conjugation to carriers or other techniques well known in the art. An immunogenic portion of a GDF 11 polypeptide can be administered in the presence of adjuvant. The progress of immunization can be monitored by detection of antibody titers in plasma or serum. Standard ELISA or other immunoassays can be used with the immunogen as antigen to assess the levels of antibody production and/or level of binding affinity.

Following immunization of an animal with an antigenic preparation of GDF11, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique [see, *e.g.*, Kohler and Milstein (1975) Nature, 256: 495-497], the human B cell hybridoma technique [see, *e.g*., Kozbar et al. (1983) Immunology Today, 4:72], and the EBV-hybridoma technique to produce human monoclonal antibodies [Cole et al. (1985) Monoclonal Antibodies and Cancer Therapy, Alan R. Liss, Inc. pp. 77-96]. Hybridoma cells can be screened immunochemically for production of antibodies specifically reactive with a GDF11 polypeptide, and monoclonal antibodies isolated from a culture comprising such hybridoma cells.

In certain embodiments, one or more amino acid modifications may be introduced into the Fc region of an antibody provided herein thereby generating an Fc-region variant. The Fc-region variant may comprise a human Fc-region sequence (*e.g*., a human IgG1, IgG2, IgG3 or IgG4 Fc region) comprising an amino acid modification (*e.g*., a substitution, deletion, and/or addition) at one or more amino acid positions.

For example, the present disclosure contemplates an antibody variant that possesses some but not all effector functions, which make it a desirable candidate for applications in which the half-life of the antibody *in vivo* is important yet for which certain effector functions [*e.g*., complement-dependent cytotoxicity (CDC) and antibody-dependent cellular cytotoxicity (ADCC)] are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in, for example, Ravetch and Kinet (1991) Annu. Rev. Immunol. 9:457-492. Non-limiting examples of *in vitro* assays to assess ADCC activity of a molecule of interest are described in U.S. Pat. No. 5,500,362; Hellstrom, I. et al. (1986) Proc. Nat'l Acad. Sci. USA 83:7059-7063; Hellstrom, I et al. (1985) Proc. Nat'l Acad. Sci. USA 82:1499-1502; U.S. Pat. No. 5,821,337; and Bruggemann, M. et al. (1987) J. Exp. Med. 166:1351-1361. Alternatively, non-radioactive assay methods may be employed (*e.g*., ACTI^{™}, non-radioactive cytotoxicity assay for flow cytometry; CellTechnology, Inc. Mountain View, Calif.; and CytoTox 96^{®} non-radioactive cytotoxicity assay, Promega, Madison, Wis.). Useful effector cells for such assays include peripheral blood mononuclear cells (PBMC) and natural killer (NK) cells. Alternatively, or additionally, ADCC activity of the molecule of interest may be assessed *in vivo*, for example, in an animal model such as that disclosed in Clynes et al. (1998) Proc. Nat'l Acad. Sci. USA 95:652-656. C1q binding assays may also be carried out to confirm that the antibody is unable to bind C1q and hence lacks CDC activity [see, *e.g.,* C1q and C3c binding ELISA in WO 2006/029879 and WO 2005/100402]. To assess complement activation, a CDC assay may be performed [see, *e.g.,* Gazzano-Santoro et al. (1996) J. Immunol. Methods 202:163; Cragg, M. S. et al. (2003) Blood 101:1045-1052; and Cragg, M. S, and M. J. Glennie (2004) Blood 103:2738-2743]. FcRn binding and *in vivo* clearance/half-life determinations can also be performed using methods known in the art [see, *e.g.,* Petkova, S. B. et al. (2006) Int. Immunol. 18(12):1759-1769].

Antibodies of the present disclosure with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

In certain embodiments, it may be desirable to create cysteine-engineered antibodies, *e.g.*, "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy-chain Fc region. Cysteine engineered antibodies may be generated as described, for example., in U.S. Pat. No. 7,521,541.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (*e*.*g*., the Biacore^{™} binding assay, Biacore AB, Uppsala, Sweden), sandwich assays (*e.g*., the paramagnetic bead system of IGEN International, Inc., Gaithersburg, Maryland), western blots, immunoprecipitation assays, and immunohistochemistry.

In certain embodiments, amino acid sequence variants of the antibodies and/or the binding polypeptides provided herein are contemplated. For example, it may be desirable to improve the binding affinity and/or other biological properties of the antibody and/or binding polypeptide. Amino acid sequence variants of an antibody and/or binding polypeptides may be prepared by introducing appropriate modifications into the nucleotide sequence encoding the antibody and/or binding polypeptide, or by peptide synthesis. Such modifications include, for example, deletions from, and/or insertions into, and/or substitutions of residues within, the amino acid sequences of the antibody and/or binding polypeptide. Any combination of deletion, insertion, and substitution can be made to arrive at the final construct, provided that the final construct possesses the desired characteristics, *e.g.*, target-binding (ActRIIB, ActRIIA, ALK4, GDF8, activin (e.g., activin A, activin B, activin C, activin E, activin AB, activin AC) GDF3, BMP6, BMP10, BMP9, TGFβ1, TGFβ2, TGFβ3, TGFβRII, ALK5 and/or betaglycan binding).

Alterations (*e.g*., substitutions) may be made in HVRs, for example, to improve antibody affinity. Such alterations may be made in HVR "hotspots," *i.e.*, residues encoded by codons that undergo mutation at high frequency during the somatic maturation process (see, *e.g*., Chowdhury (2008) Methods Mol. Biol. 207:179-196 (2008)), and/or SDRs (a-CDRs), with the resulting variant VH or VL being tested for binding affinity. Affinity maturation by constructing and reselecting from secondary libraries has been described in the art [see, *e.g.*, Hoogenboom et al., in Methods in Molecular Biology 178:1-37, O'Brien et al., ed., Human Press, Totowa, N.J., (2001)]. In some embodiments of affinity maturation, diversity is introduced into the variable genes chosen for maturation by any of a variety of methods (*e.g.*, error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g.*, 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind to the antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two, or three amino acid substitutions.

A useful method for identification of residues or regions of the antibody and/or the binding polypeptide that may be targeted for mutagenesis is called "alanine scanning mutagenesis", as described by Cunningham and Wells (1989) Science, 244:1081-1085. In this method, a residue or group of target residues (*e.g*., charged residues such as arg, asp, his, lys, and glu) are identified and replaced by a neutral or negatively charged amino acid (*e.g.*, alanine or polyalanine) to determine whether the interaction of the antibody or binding polypeptide with antigen is affected. Further substitutions may be introduced at the amino acid locations demonstrating functional sensitivity to the initial substitutions. Alternatively, or additionally, a crystal structure of an antigen-antibody complex can be used to identify contact points between the antibody and antigen. Such contact residues and neighboring residues may be targeted or eliminated as candidates for substitution. Variants may be screened to determine whether they contain the desired properties.

Amino-acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include fusion of the N- or C-terminus of the antibody to an enzyme (*e.g*., for ADEPT) or a polypeptide which increases the serum half-life of the antibody.

In certain embodiments, an antibody and/or binding polypeptide provided herein may be further modified to contain additional non-proteinaceous moieties that are known in the art and readily available. The moieties suitable for derivatization of the antibody and/or binding polypeptide include but are not limited to water-soluble polymers. Non-limiting examples of water-soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody and/or binding polypeptide may vary, and if more than one polymer are attached, they can be the same or different molecules. In general, the number and/or type of polymers used for derivatization can be determined based on considerations including, but not limited to, the particular properties or functions of the antibody and/or binding polypeptide to be improved, whether the antibody derivative and/or binding polypeptide derivative will be used in a therapy under defined conditions.

### 5. Small Molecule Antagonists

In other aspects, the present disclosure relates to an ActRII antagonist (inhibitor) that is small molecule, or combination of small molecules. ActRII antagonist small molecules may inhibit to one or more ActRII ligands [*e.g.*, GDF11, GDF8, activin (e.g., activin A, activin B, activin C, activin E, activin AB, activin AC) GDF3, BMP6, BMP10, and BMP9], one or more type I and/or type II receptors (e.g., ActRIIA, ActRIIB, and ALK4), or one or more ActRII downstream signaling components (e.g., Smads 2 and/or 3). In particular, the disclosure provides methods of using an ActRII antagonist small molecules, or combination of ActRII antagonist small molecules, alone or in combination with one or more additional supportive therapies and/or active agents (e.g., TGFβ antagonists), to achieve a desired effect in a subject in need thereof (e.g., increase an immune response in a subject in need thereof and treat cancer or a pathogen).

In some embodiments, an ActRII antagonist is a small molecule antagonist, or combination of small molecule antagonists, that inhibits at least GDF 11. In some embodiments, a small molecule antagonist, or combination of small molecule antagonists, that inhibits GDF 11 further inhibits one or more ligand [e.g., GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least GDF8. In some embodiments, a small molecule antagonist, or combination of small molecule antagonists, that inhibits GDF8 further inhibits one or more ligand [e.g., GDF11, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least activin (e.g., activin A, activin B, activin C, activin E, activin AB, and activin AE). In some embodiments, a small molecule antagonist, or combination of small molecule antagonists, that inhibits activin further inhibits one or more ligand [e.g., GDF11, GDF8, GDF3, BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least GDF3. In some embodiments, a small molecule antagonist, or combination of small molecule antagonists, that inhibits GDF3 further inhibits one or more ligand [e.g., GDF11, GDF8, activin (e.g., activin A, activin B, activin C, activin E, activin AB, activin AC), BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least BMP6. In some embodiments, a small molecule antagonist, or combination of small molecule antagonists, that inhibits BMP6 further inhibits one or more ligand [e.g., GDF11, GDF8, activin (e.g., activin A, activin B, activin C, activin E, activin AB, activin AC), GDF3, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least BMP10. In some embodiments, a small molecule antagonist, or combination of small molecule antagonists, that inhibits BMP10 further inhibits one or more ligand [e.g., GDF11, GDF8, activin (e.g., activin A, activin B, activin C, activin E, activin AB, activin AC), GDF3, BMP6, BMP9, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least BMP9. In some embodiments, a small molecule antagonist, or combination of small molecule antagonists, that inhibits BMP9 further inhibits one or more ligand [e.g., GDF11, GDF8, activin (e.g., activin A, activin B, activin C, activin E, activin AB, activin AC), GDF3, BMP6, BMP10, TGFβ1, TGFβ2, and TGFβ3], ActRIIA, ActRIIB, ALK4, ALK5, TGFβRII, and/or betaglycan. In some embodiments, an ActRII antagonist is a small molecule antagonist, or combination of small molecule antagonists, that inhibits at least ActRIIA. In some embodiments, a small molecule antagonist, or combination of small molecule antagonists, that inhibits ActRIIA further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIB, ALK4, ALK5, TGFβRII, and/or betaglycan. In some embodiments, an ActRII antagonist is a small molecule antagonist, or combination of small molecule antagonists, that inhibits at least ActRIIB. In some embodiments, a small molecule antagonist, or combination of small molecule antagonists, that inhibits ActRIIB further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ALK4, ALK5, TGFβRII, and/or betaglycan. In some embodiments, an ActRII antagonist is a small molecule antagonist, or combination of small molecule antagonists, that inhibits at least ALK4. In some embodiments, a small molecule antagonist, or combination of small molecule antagonists, that inhibits ALK4 further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK5, TGFβRII, and/or betaglycan.

In other aspects, the present disclosure relates to a TGFβ antagonist (inhibitor) that is small molecule, or combination of small molecules. TGFβ antagonist small molecules may inhibit to one or more TGFβ ligands [*e.g.*, TGFβ1, TGFβ2, and TGFβ3], one or more type I and/or type II receptors (e.g., TGFβRII and ALK5), one or more co-receptor (e.g., betaglycan) and/or one or more TGFβ downstream signaling components (e.g., Smads 2 and/or 3). In particular, the disclosure provides methods of using an TGFβ antagonist small molecules, or combination of TGFβ antagonist small molecules, alone or in combination with one or more additional supportive therapies and/or active agents (e.g., ActRII antagonists), to achieve a desired effect in a subject in need thereof (e.g., increase an immune response in a subject in need thereof and treat cancer or a pathogen).

In some embodiments, a TGFβ antagonist is a small molecule antagonist, or combination of small molecule antagonists, that inhibits at least TGFβ1. In some embodiments, a small molecule antagonist, or combination of small molecule antagonists, that inhibits TGFβ1 further inhibits one or more ligand [e.g., GDF11, GDF8, activin (e.g., activin A, activin B, activin C, activin E, activin AB, activin AC), GDF3, BMP6, BMP10, BMP9, TGFβ2, and TGFβ3], ActRIIA, ActRIIB, ALK5, TGFβRII, and/or betaglycan. In some embodiments, a TGFβ antagonist is a small molecule antagonist, or combination of small molecule antagonists, that inhibits at least TGFβ2. In some embodiments, a small molecule antagonist, or combination of small molecule antagonists, that inhibits TGFβ2 further inhibits one or more ligand [e.g., GDF11, GDF8, activin (e.g., activin A, activin B, activin C, activin E, activin AB, activin AC), GDF3, BMP6, BMP10, BMP9, TGFβ1, and TGFβ3], ActRIIA, ActRIIB, ALK5, TGFβRII, and/or betaglycan. In some embodiments, a TGFβ antagonist is a small molecule antagonist, or combination of small molecule antagonists, that inhibits at least TGFβ3. In some embodiments, a small molecule antagonist, or combination of small molecule antagonists, that inhibits TGFβ3 further inhibits one or more ligand [e.g., GDF11, GDF8, activin (e.g., activin A, activin B, activin C, activin E, activin AB, activin AC), GDF3, BMP6, BMP10, BMP9, TGFβ1, and TGFβ2], ActRIIA, ActRIIB, ALK5, TGFβRII, and/or betaglycan. In some embodiments, a TGFβ antagonist is a small molecule antagonist, or combination of small molecule antagonists, that inhibits at least TGFβ3. In some embodiments, a small molecule antagonist, or combination of small molecule antagonists, that inhibits TGFβ3 further inhibits one or more ligand [e.g., GDF11, GDF8, activin (e.g., activin A, activin B, activin C, activin E, activin AB, activin AC), GDF3, BMP6, BMP10, BMP9, TGFβ1, and TGFβ2], ActRIIA, ActRIIB, ALK5, TGFβRII, and/or betaglycan. In some embodiments, a TGFβ antagonist is a small molecule antagonist, or combination of small molecule antagonists, that inhibits at least TGFβRII. In some embodiments, a small molecule antagonist, or combination of small molecule antagonists, that inhibits TGFβRII further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK5, and/or betaglycan. In some embodiments, a TGFβ antagonist is a small molecule antagonist, or combination of small molecule antagonists, that inhibits at least ALK5. In some embodiments, a small molecule antagonist, or combination of small molecule antagonists, that inhibits ALK5 further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, TGFβRII, and/or betaglycan. In some embodiments, a TGFβ antagonist is a small molecule antagonist, or combination of small molecule antagonists, that inhibits at least betaglycan. In some embodiments, a small molecule antagonist, or combination of small molecule antagonists, that inhibits betaglycan further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK5, and/or TGFβRII.

Small molecule antagonists can be direct or indirect inhibitors. For example, an indirect small molecule antagonist, or combination of small molecule antagonists, may inhibit the expression (*e.g*., transcription, translation, cellular secretion, or combinations thereof) of at least one or more ligands [*e.g*., GDF8, activin (e.g., activin A, activin B, activin C, activin E, activin AB, activin AC) GDF3, BMP6, BMP10, BMP9, TGFβ1, TGFβ2, abd TGFβ3], one or more type I and/or type II receptors (e.g., ActRIIA, ActRIIB, TGFβRII, ALK4, and ALK5), one or more co-receptors (betaglycan) or one or more ActRII downstream signaling components (e.g., Smads 2 and/or 3). Alternatively, a direct small molecule ActRII antagonist, or combination of small molecule antagonists, may directly bind to, for example, one or more of one or more ligands [*e.g*., GDF8, activin (e.g., activin A, activin B, activin C, activin E, activin AB, activin AC) GDF3, BMP6, BMP10, BMP9, TGFβ1, TGFβ2, abd TGFβ3], one or more type I and/or type II receptors (e.g., ActRIIA, ActRIIB, TGFβRII, ALK4, and ALK5), one or more co-receptors (betaglycan) or one or more ActRII downstream signaling components (e.g., Smads 2 and/or 3). Combinations of one or more indirect and one or more direct small molecule antagonists may be used in accordance with the methods disclosed herein.

Binding organic small molecule antagonists of the present disclosure may be identified and chemically synthesized using known methodology (*see*, *e.g.*, PCT Publication Nos. WO 00/00823 and WO 00/39585). In general, small molecule antagonists of the disclosure are usually less than about 2000 daltons in size, alternatively less than about 1500, 750, 500, 250 or 200 daltons in size, wherein such organic small molecules that are capable of binding, preferably specifically, to a polypeptide as described herein. Such small molecule antagonists may be identified without undue experimentation using well-known techniques. In this regard, it is noted that techniques for screening organic small molecule libraries for molecules that are capable of binding to a polypeptide target are well-known in the art (see, *e.g*., international patent publication Nos. WO00/00823 and WO00/39585).

Binding organic small molecules of the present disclosure may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, and acid chlorides.

### 6. Nucleotide Antagonists

In other aspects, the present disclosure relates to an ActRII antagonist (inhibitor) that is a polynucleotide, or combination of polynucleotides. ActRII antagonist polynucleotides may inhibit to one or more ActRII ligands [*e.g.*, GDF11, GDF8, activin (e.g., activin A, activin B, activin C, activin E, activin AB, activin AC) GDF3, BMP6, BMP10, and BMP9], one or more type I and/or type II receptors (e.g., ActRIIA, ActRIIB, and ALK4), or one or more ActRII downstream signaling components (e.g., Smads 2 and/or 3). In particular, the disclosure provides methods of using an ActRII antagonist polynucleotide, or combination of ActRII antagonist polynucleotides, alone or in combination with one or more additional supportive therapies and/or active agents (e.g., TGFβRII antagonists), to achieve a desired effect in a subject in need thereof (e.g., increase an immune response in a subject in need thereof and treat cancer or pathogen).

In some embodiments, an ActRII antagonist is a polynucleotide antagonist, or combination of polynucleotide antagonists, that inhibits at least GDF11. In some embodiments, a polynucleotide antagonist, or combination of polynucleotide antagonists, that inhibits GDF11 further inhibits one or more ligand [e.g., GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a polynucleotide antagonist, or combination of polynucleotide antagonists, that inhibits at least GDF8. In some embodiments, a polynucleotide antagonist, or combination of polynucleotide antagonists, that inhibits GDF8 further inhibits one or more ligand [e.g., GDF11, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a polynucleotide antagonist, or combination of polynucleotide antagonists, that inhibits at least activin (e.g., activin A, activin B, activin C, activin E, activin AB, and activin AE). In some embodiments, a polynucleotide antagonist, or combination of polynucleotide antagonists, that inhibits activin further inhibits one or more ligand [e.g., GDF11, GDF8, GDF3, BMP6, BMP10, BMP9, TGFβ1, TGFβ2, and TGFβ3], ActRIIA, ActRIIB, ALK4, ALK5, TGFβRII, and/or betaglycan. In some embodiments, an ActRII polynucleotide is a polynucleotide antagonist, or combination of polynucleotide antagonists, that inhibits at least GDF3. In some embodiments, a polynucleotide, or combination of polynucleotide antagonists, that inhibits GDF3 further inhibits one or more ligand [e.g., GDF11, GDF8, activin (e.g., activin A, activin B, activin C, activin E, activin AB, activin AC), BMP6, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a polynucleotide antagonist, or combination of polynucleotide antagonists, that inhibits at least BMP6. In some embodiments, a polynucleotide antagonist, or combination of polynucleotide antagonists, that inhibits BMP6 further inhibits one or more ligand [e.g., GDF11, GDF8, activin (e.g., activin A, activin B, activin C, activin E, activin AB, activin AC), GDF3, BMP10, BMP9, TGFβ1, TGFβ2, and TGFβ3], ActRIIA, ActRIIB, ALK4, ALK5, TGFβRII, and/or betaglycan. In some embodiments, an ActRII antagonist is a polynucleotide antagonist, or combination of polynucleotide antagonists, that inhibits at least BMP10. In some embodiments, a polynucleotide antagonist, or combination of polynucleotide antagonists, that inhibits BMP10 further inhibits one or more ligand [e.g., GDF11, GDF8, activin (e.g., activin A, activin B, activin C, activin E, activin AB, activin AC), GDF3, BMP6, BMP9, TGFβ1, TGFβ2, and TGFβ3], ActRIIA, ActRIIB, ALK4, ALK5, TGFβRII, and/or betaglycan. In some embodiments, an ActRII antagonist is a polynucleotide antagonist, or combination of polynucleotide antagonists, that inhibits at least BMP9. In some embodiments, a polynucleotide antagonist, or combination of polynucleotide antagonists, that inhibits BMP9 further inhibits one or more ligand [e.g., GDF11, GDF8, activin (e.g., activin A, activin B, activin C, activin E, activin AB, activin AC), GDF3, BMP6, BMP10, TGFβ1, TGFβ2, and TGFβ3], ActRIIA, ActRIIB, ALK4, ALK5, TGFβRII, and/or betaglycan. In some embodiments, an ActRII antagonist is a polynucleotide antagonist, or combination of polynucleotide antagonists, that inhibits at least ActRIIA. In some embodiments, a polynucleotide antagonist, or combination of polynucleotide antagonists, that inhibits ActRIIA further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIB, ALK4, ALK5, TGFβRII, and/or betaglycan. In some embodiments, an ActRII antagonist is a polynucleotide antagonist, or combination of polynucleotide antagonists, that inhibits at least ActRIIB. In some embodiments, a polynucleotide antagonist, or combination of polynucleotide antagonists, that inhibits ActRIIB further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ALK4, ALK5, TGFβRII, and/or betaglycan. In some embodiments, an ActRII antagonist is a polynucleotide antagonist, or combination of polynucleotide antagonists, that inhibits at least ALK4. In some embodiments, a polynucleotide antagonist, or combination of polynucleotide antagonists, that inhibits ALK4 further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK5, TGFβRII, and/or betaglycan.

In other aspects, the present disclosure relates to a TGFβ antagonist (inhibitor) that is polynucleotide, or combination of polynucleotides. TGFβ antagonist polynucleotides may inhibit to one or more TGFβ ligands [*e.g.*, TGFβ1, TGFβ2, and TGFβ3], one or more type I and/or type II receptors (e.g., TGFβRII and ALK5), one or more co-receptor (e.g., betaglycan) and/or one or more TGFβ downstream signaling components (e.g., Smads 2 and/or 3). In particular, the disclosure provides methods of using an TGFβ antagonist polynucleotide, or combination of TGFβ antagonist polynucleotides, alone or in combination with one or more additional supportive therapies and/or active agents (e.g., ActRII antagonists), to achieve a desired effect in a subject in need thereof (e.g., increase an immune response in a subject in need thereof and treat cancer or a pathogen).

In some embodiments, a TGFβ antagonist is a polynucleotide antagonist, or combination of polynucleotide antagonists, that inhibits at least TGFβ1. In some embodiments, a polynucleotide antagonist, or combination of polynucleotide antagonists, that inhibits TGFβ1 further inhibits one or more ligand [e.g., GDF11, GDF8, activin (e.g., activin A, activin B, activin C, activin E, activin AB, activin AC), GDF3, BMP6, BMP10, BMP9, TGFβ2, and TGFβ3], ActRIIA, ActRIIB, ALK5, TGFβRII, and/or betaglycan. In some embodiments, a TGFβ antagonist is a polynucleotide antagonist, or combination of polynucleotide antagonists, that inhibits at least TGFβ2. In some embodiments, a polynucleotide antagonist, or combination of polynucleotide antagonists, that inhibits TGFβ2 further inhibits one or more ligand [e.g., GDF11, GDF8, activin (e.g., activin A, activin B, activin C, activin E, activin AB, activin AC), GDF3, BMP6, BMP10, BMP9, TGFβ1, and TGFβ3], ActRIIA, ActRIIB, ALK5, TGFβRII, and/or betaglycan. In some embodiments, a TGFβ antagonist is a polynucleotide antagonist, or combination of polynucleotide antagonists, that inhibits at least TGFβ3. In some embodiments, a polynucleotide antagonist, or combination of polynucleotide antagonists, that inhibits TGFβ3 further inhibits one or more ligand [e.g., GDF11, GDF8, activin (e.g., activin A, activin B, activin C, activin E, activin AB, activin AC), GDF3, BMP6, BMP10, BMP9, TGFβ1, and TGFβ2], ActRIIA, ActRIIB, ALK5, TGFβRII, and/or betaglycan. In some embodiments, a TGFβ antagonist is a polynucleotide antagonist, or combination of polynucleotide antagonists, that inhibits at least TGFβ3. In some embodiments, a polynucleotide antagonist, or combination of polynucleotide antagonists, that inhibits TGFβ3 further inhibits one or more ligand [e.g., GDF11, GDF8, activin (e.g., activin A, activin B, activin C, activin E, activin AB, activin AC), GDF3, BMP6, BMP10, BMP9, TGFβ1, and TGFβ2], ActRIIA, ActRIIB, ALK5, TGFβRII, and/or betaglycan. In some embodiments, a TGFβ antagonist is a polynucleotide antagonist, or combination of polynucleotide antagonists, that inhibits at least TGFβRII. In some embodiments, a polynucleotide antagonist, or combination of polynucleotide antagonists, that inhibits TGFβRII further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK5, and/or betaglycan. In some embodiments, a TGFβ antagonist is a polynucleotide antagonist, or combination of polynucleotide antagonists, that inhibits at least ALK5. In some embodiments, a polynucleotide antagonist, or combination of polynucleotide antagonists, that inhibits ALK5 further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, TGFβRII, and/or betaglycan. In some embodiments, a TGFβ antagonist is a polynucleotide antagonist, or combination of polynucleotide antagonists, that inhibits at least betaglycan. In some embodiments, a polynucleotide antagonist, or combination of polynucleotide antagonists, that inhibits betaglycan further inhibits one or more ligand [e.g., GDF11, GDF8, activin (e.g., activin A, activin B, activin C, activin E, activin AB, activin AC), GDF3, BMP6, BMP10, BMP9, TGFβ1, TGFβ2, and TGFβ3], ActRIIA, ActRIIB, ALK5, and/or TGFβRII.

The polynucleotide antagonists of the present disclosure may be an antisense nucleic acid, an RNAi molecule [e.g., small interfering RNA (siRNA), small-hairpin RNA (shRNA), microRNA (miRNA)], an aptamer and/or a ribozyme. The nucleic acid and amino acid sequences of human ALK4, ALK5, ActRIIA, ActRIIB, TGFβRII, betaglycan, GDF11, GDF8, activin (e.g., activin A, activin B, activin C, activin E, activin AB, activin AC) GDF3, BMP6, BMP10, BMP9, TGFβ1, TGFβ2, TGFβ3, and betaglycan, are known in the art and thus polynucleotide antagonists for use in accordance with methods of the present disclosure may be routinely made by the skilled artisan based on the knowledge in the art and teachings provided herein.

For example, antisense technology can be used to control gene expression through antisense DNA or RNA, or through triple-helix formation. Antisense techniques are discussed, for example, in Okano (1991) J. Neurochem. 56:560; Oligodeoxynucleotides as Antisense Inhibitors of Gene Expression, CRC Press, Boca Raton, Fla. (1988). Triple helix formation is discussed in, for instance, Cooney et al. (1988) Science 241:456; and Dervan et al., (1991)Science 251:1300. The methods are based on binding of a polynucleotide to a complementary DNA or RNA. In some embodiments, the antisense nucleic acids comprise a single-stranded RNA or DNA sequence that is complementary to at least a portion of an RNA transcript of a desired gene. However, absolute complementarity, although preferred, is not required.

A sequence "complementary to at least a portion of an RNA," referred to herein, means a sequence having sufficient complementarity to be able to hybridize with the RNA, forming a stable duplex; in the case of double-stranded antisense nucleic acids of a gene disclosed herein, a single strand of the duplex DNA may thus be tested, or triplex formation may be assayed. The ability to hybridize will depend on both the degree of complementarity and the length of the antisense nucleic acid. Generally, the larger the hybridizing nucleic acid, the more base mismatches with an RNA it may contain and still form a stable duplex (or triplex as the case may be). One skilled in the art can ascertain a tolerable degree of mismatch by use of standard procedures to determine the melting point of the hybridized complex.

Polynucleotides that are complementary to the 5' end of the message, for example, the 5'-untranslated sequence up to and including the AUG initiation codon, should work most efficiently at inhibiting translation. However, sequences complementary to the 3'-untranslated sequences of mRNAs have been shown to be effective at inhibiting translation of mRNAs as well [see, *e.g*., Wagner, R., (1994) Nature 372:333-335]. Thus, oligonucleotides complementary to either the 5'- or 3'-untranslated, noncoding regions of a gene of the disclosure, could be used in an antisense approach to inhibit translation of an endogenous mRNA. Polynucleotides complementary to the 5'-untranslated region of the mRNA should include the complement of the AUG start codon. Antisense polynucleotides complementary to mRNA coding regions are less efficient inhibitors of translation but could be used in accordance with the methods of the present disclosure. Whether designed to hybridize to the 5'-untranslated, 3'-untranslated, or coding regions of an mRNA of the disclosure, antisense nucleic acids should be at least six nucleotides in length, and are preferably oligonucleotides ranging from 6 to about 50 nucleotides in length. In specific aspects, the oligonucleotide is at least 10 nucleotides, at least 17 nucleotides, at least 25 nucleotides, or at least 50 nucleotides.

In one embodiment, the antisense nucleic acid of the present disclosure is produced intracellularly by transcription from an exogenous sequence. For example, a vector or a portion thereof, is transcribed, producing an antisense nucleic acid (RNA) of a gene of the disclosure. Such a vector would contain a sequence encoding the desired antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the sequence encoding desired genes of the instant disclosure, or fragments thereof, can be by any promoter known in the art to act in vertebrate, preferably human cells. Such promoters can be inducible or constitutive. Such promoters include, but are not limited to, the SV40 early promoter region [see, e.g., Benoist and Chambon (1981) Nature 29:304-310], the promoter contained in the 3' long terminal repeat of Rous sarcoma virus [see, *e.g*., Yamamoto et al. (1980) Cell 22:787-797], the herpes thymidine promoter [see, *e.g*., Wagner et al. (1981) Proc. Natl. Acad. Sci. U.S.A. 78:1441-1445], and the regulatory sequences of the metallothionein gene [see, *e.g.,* Brinster, et al. (1982) Nature 296:39-42].

In some embodiments, the polynucleotide antagonists are interfering RNA or RNAi molecules that target the expression of one or more genes. RNAi refers to the expression of an RNA which interferes with the expression of the targeted mRNA. Specifically, RNAi silences a targeted gene via interacting with the specific mRNA through a siRNA (small interfering RNA). The ds RNA complex is then targeted for degradation by the cell. An siRNA molecule is a double-stranded RNA duplex of 10 to 50 nucleotides in length, which interferes with the expression of a target gene which is sufficiently complementary (*e.g*. at least 80% identity to the gene). In some embodiments, the siRNA molecule comprises a nucleotide sequence that is at least 85, 90, 95, 96, 97, 98, 99, or 100% identical to the nucleotide sequence of the target gene.

Additional RNAi molecules include short-hairpin RNA (shRNA); also short-interfering hairpin and microRNA (miRNA). The shRNA molecule contains sense and antisense sequences from a target gene connected by a loop. The shRNA is transported from the nucleus into the cytoplasm, and it is degraded along with the mRNA. Pol III or U6 promoters can be used to express RNAs for RNAi. Paddison et al. [Genes & Dev. (2002) 16:948-958, 2002] have used small RNA molecules folded into hairpins as a means to effect RNAi. Accordingly, such short hairpin RNA (shRNA) molecules are also advantageously used in the methods described herein. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the double-stranded RNA (dsRNA) products of the DICER RNase and, in any event, have the same capacity for inhibiting expression of a specific gene. The shRNA can be expressed from a lentiviral vector. An miRNA is a single-stranded RNA of about 10 to 70 nucleotides in length that are initially transcribed as pre-miRNA characterized by a "stem-loop" structure and which are subsequently processed into mature miRNA after further processing through the RISC.

Molecules that mediate RNAi, including without limitation siRNA, can be produced *in vitro* by chemical synthesis (Hohjoh, FEBS Lett 521:195-199, 2002), hydrolysis of dsRNA (Yang et al., Proc Natl Acad Sci USA 99:9942-9947, 2002), by *in vitro* transcription with T7 RNA polymerase (Donzeet et al., Nucleic Acids Res 30:e46, 2002; Yu et al., Proc Natl Acad Sci USA 99:6047-6052, 2002), and by hydrolysis of double-stranded RNA using a nuclease such as E. coli RNase III (Yang et al., Proc Natl Acad Sci USA 99:9942-9947, 2002).

According to another aspect, the disclosure provides polynucleotide antagonists including but not limited to, a decoy DNA, a double-stranded DNA, a single-stranded DNA, a complexed DNA, an encapsulated DNA, a viral DNA, a plasmid DNA, a naked RNA, an encapsulated RNA, a viral RNA, a double-stranded RNA, a molecule capable of generating RNA interference, or combinations thereof.

In some embodiments, the polynucleotide antagonists of the disclosure are aptamers. Aptamers are nucleic acid molecules, including double-stranded DNA and single-stranded RNA molecules, which bind to and form tertiary structures that specifically bind to a target molecule, such as a ALK4, ALK5, ActRIIB, ActRIIA, TGFβRII, betaglycan, GDF11, GDF8, activin (e.g., activin A, activin B, activin C, activin E, activin AB, activin AC) GDF3, BMP6, BMP10, BMP9 TGFβ1, TGFβ2, TGFβ3, TGFβRII and/or betaglycan polypeptide. The generation and therapeutic use of aptamers are well established in the art. See, *e.g.*, U.S. Pat. No. 5,475,096. Additional information on aptamers can be found in U.S. Patent Application Publication No. 20060148748. Nucleic acid aptamers are selected using methods known in the art, for example via the Systematic Evolution of Ligands by Exponential Enrichment (SELEX) process. SELEX is a method for the in vitro evolution of nucleic acid molecules with highly specific binding to target molecules as described in, *e.g.*, U.S. Pat. Nos. 5,475,096, 5,580,737, 5,567,588, 5,707,796, 5,763,177, 6,011,577, and 6,699,843. Another screening method to identify aptamers is described in U.S. Pat. No. 5,270,163. The SELEX process is based on the capacity of nucleic acids for forming a variety of two- and three-dimensional structures, as well as the chemical versatility available within the nucleotide monomers to act as ligands (form specific binding pairs) with virtually any chemical compound, whether monomeric or polymeric, including other nucleic acid molecules and polypeptides. Molecules of any size or composition can serve as targets. The SELEX method involves selection from a mixture of candidate oligonucleotides and step-wise iterations of binding, partitioning and amplification, using the same general selection scheme, to achieve desired binding affinity and selectivity. Starting from a mixture of nucleic acids, which can comprise a segment of randomized sequence, the SELEX method includes steps of contacting the mixture with the target under conditions favorable for binding; partitioning unbound nucleic acids from those nucleic acids which have bound specifically to target molecules; dissociating the nucleic acid-target complexes; amplifying the nucleic acids dissociated from the nucleic acid-target complexes to yield a ligand enriched mixture of nucleic acids. The steps of binding, partitioning, dissociating and amplifying are repeated through as many cycles as desired to yield highly specific high affinity nucleic acid ligands to the target molecule.

Typically, such binding molecules are separately administered to the animal [see, e.g., O'Connor (1991) J. Neurochem. 56:560], but such binding molecules can also be expressed in vivo from polynucleotides taken up by a host cell and expressed *in vivo* [see, *e.g.*, Oligodeoxynucleotides as Antisense Inhibitors of Gene Expression, CRC Press, Boca Raton, Fla. (1988)].

### 7. Follistatin and FLRG Antagonists

In other aspects, an ActRII antagonist (inhibitor) for use in accordance with the methods disclosed herein is a follistatin or FLRG polypeptide, which may be used alone or in combination with one or more additional supportive therapies and/or active agents as disclosed herein to achieve a desired effect (e.g., increase an immune response in a subject in need thereof and treat cancer of pathogen).

The term "follistatin polypeptide" includes polypeptides comprising any naturally occurring polypeptide of follistatin as well as any variants thereof (including mutants, fragments, fusions, and peptidomimetic forms) that retain a useful activity, and further includes any functional monomer or multimer of follistatin. In certain preferred embodiments, follistatin polypeptides of the disclosure bind to and/or inhibit activin activity, particularly activin A. Variants of follistatin polypeptides that retain activin binding properties can be identified based on previous studies involving follistatin and activin interactions. For example, WO2008/030367 discloses specific follistatin domains ("FSDs") that are shown to be important for activin binding. As shown below in SEQ ID NOs: 46-48, the follistatin N-terminal domain ("FSND" SEQ ID NO: 46), FSD2 (SEQ ID NO: 48), and to a lesser extent FSD1 (SEQ ID NO: 47) represent exemplary domains within follistatin that are important for activin binding. In addition, methods for making and testing libraries of polypeptides are described above in the context of ActRII polypeptides, and such methods also pertain to making and testing variants of follistatin. Follistatin polypeptides include polypeptides derived from the sequence of any known follistatin having a sequence at least about 80% identical to the sequence of a follistatin polypeptide, and optionally at least 85%, 90%, 95%, 96%, 97%, 98%, 99% or greater identity. Examples of follistatin polypeptides include the mature follistatin polypeptide or shorter isoforms or other variants of the human follistatin precursor polypeptide (SEQ ID NO: 44) as described, for example, in WO2005/025601.

The human follistatin precursor polypeptide isoform FST344 is as follows:

1 MVRARHQPGG LCLLLLLLCQ FMEDRSAQAG NCWLRQAKNG RCQVLYKTEL
51 SKEECCSTGR LSTSWTEEDV NDNTLFKWMI FNGGAPNCIP CKETCENVDC
101 GPGKKCRMNK KNKPRCVCAP DCSNITWKGP VCGLDGKTYR NECALLKARC
151 KEQPELEVQY QGRCKKTCRD VFCPGSSTCV VDQTNNAYCV TCNRICPEPA
201 SSEQYLCGND GVTYSSACHL RKATCLLGRS IGLAYEGKCI KAKSCEDIQC
251 TGGKKCLWDF KVGRGRCSLC DELCPDSKSD EPVCASDNAT YASECAMKEA
301 ACSSGVLLEV KHSGSCNSIS EDTEEEEEDE DQDYSFPISS ILEW
(SEQ ID NO: 44; NCBI Reference No. NP_037541.1)

The signal peptide is underlined; also underlined above are the last 27 residues which represent the C-terminal extension distinguishing this follistatin isoform from the shorter follistatin isoform FST317 shown below.

The human follistatin precursor polypeptide isoform FST317 is as follows: 
1 MVRARHQPGG LCLLLLLLCQ FMEDRSAQAG NCWLRQAKNG
RCQVLYKTEL
51 SKEECCSTGR LSTSWTEEDV NDNTLFKWMI FNGGAPNCIP CKETCENVDC
101 GPGKKCRMNK KNKPRCVCAP DCSNITWKGP VCGLDGKTYR
NECALLKARC
151 KEQPELEVQY QGRCKKTCRD VFCPGSSTCV VDQTNNAYCV TCNRICPEPA
201 SSEQYLCGND GVTYSSACHL RKATCLLGRS IGLAYEGKCI KAKSCEDIQC
 
251 TGGKKCLWDF KVGRGRCSLC DELCPDSKSD EPVCASDNAT
YASECAMKEA
301 ACSSGVLLEV KHSGSCN (SEQ ID NO: 45; NCBI Reference No. NP_006341.1)
 The signal peptide is underlined.

The follistatin N-terminal domain (FSND) sequence is as follows:

GNCWLRQAKNGRCQVLYKTELSKEECCSTGRLSTSWTEEDVNDN
TLFKWMIFNGGAPNCIPCK (SEQ ID NO: 46; FSND)

The FSD1 and FSD2 sequences are as follows:
ETCENVDCGPGKKCRMNKKNKPRCV (SEQ ID NO: 47; FSD1)
KTCRDVFCPGSSTCVVDQTNNAYCVT (SEQ ID NO: 48; FSD2)

In other aspects, an ActRII antagonist for use in accordance with the methods disclosed herein is a follistatin-like related gene (FLRG), also known as follistatin-related protein 3 (FSTL3). The term "FLRG polypeptide" includes polypeptides comprising any naturally occurring polypeptide of FLRG as well as any variants thereof (including mutants, fragments, fusions, and peptidomimetic forms) that retain a useful activity. In certain preferred embodiments, FLRG polypeptides of the disclosure bind to and/or inhibit activin activity, particularly activin A. Variants of FLRG polypeptides that retain activin binding properties can be identified using routine methods to assay FLRG and activin interactions (see, *e.g.*, US 6,537,966). In addition, methods for making and testing libraries of polypeptides are described above in the context of ActRII polypeptides and such methods also pertain to making and testing variants of FLRG. FLRG polypeptides include polypeptides derived from the sequence of any known FLRG having a sequence at least about 80% identical to the sequence of an FLRG polypeptide, and optionally at least 85%, 90%, 95%, 97%, 99% or greater identity.

The human FLRG precursor (follistatin-related protein 3 precursor) polypeptide is as follows: 
1 MRPGAPGPLW PLPWGALAWA VGFVSSMGSG NPAPGGVCWL QQGQEATCSL

51 VLQTDVTRAE CCASGNIDTA WSNLTHPGNK INLLGFLGLV HCLPCKDSCD

101 GVECGPGKAC RMLGGRPRCE CAPDCSGLPA RLQVCGSDGA TYRDECELRA

151 ARCRGHPDLS VMYRGRCRKS CEHVVCPRPQ SCVVDQTGSA HCVVCRAAPC

201 PVPSSPGQEL CGNNNVTYIS SCHMRQATCF LGRSIGVRHA GSCAGTPEEP
 251 PGGESAEEEE NFV (SEQ ID NO: 49; NCBI Reference No. NP_005851.1) The signal peptide is underlined.

In certain embodiments, functional variants or modified forms of the follistatin polypeptides and FLRG polypeptides include fusion proteins having at least a portion of the follistatin polypeptide or FLRG polypeptide and one or more fusion domains, such as, for example, domains that facilitate isolation, detection, stabilization or multimerization of the polypeptide. Suitable fusion domains are discussed in detail above with reference to the ActRII polypeptides. In some embodiment, an antagonist agent of the disclosure is a fusion protein comprising an activin-binding portion of a follistatin polypeptide fused to an Fc domain. In another embodiment, an antagonist agent of the disclosure is a fusion protein comprising an activin binding portion of an FLRG polypeptide fused to an Fc domain.

### 8. Screening Assays

In certain aspects, the present disclosure relates to the use of ActRII polypeptides, ALK4 polypeptides and/or ALK4:ActRIIB heteromultimers to identify compounds (agents) which are ActRII antagonists. In other aspects, the present disclosure relates to the use of TGFβRII polypeptides, ALK5 polypeptides and/or betaglycan polypeptides to identify compounds (agents) which are TGFβ antagonists. Compounds identified through this screening can be tested to assess their ability to modulate tissues such as bone, cartilage, muscle, fat, and/or neurons, to assess their ability to modulate tissue growth *in vivo* or *in vitro.* These compounds can be tested, for example, in animal models.

There are numerous approaches to screening for therapeutic agents for modulating tissue growth by targeting TGFβ superfamily ligand signaling (*e.g.*, SMAD signaling). In certain embodiments, high-throughput screening of compounds can be carried out to identify agents that perturb TGFβ superfamily receptor-mediated effects on a selected cell line. In certain embodiments, the assay is carried out to screen and identify compounds that specifically inhibit or reduce binding of an ActRII polypeptide, ALK4 polypeptide, ALK4:ActRIIB heteromultimer, TGFβRII polypeptides, ALK5 polypeptides and/or betaglycan to a binding partner including for example, BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11/BMP11, GDF15/MIC1, TGFβ1, TGFβ2, TGFβ3, activin A, activin B, activin AB, activin AC, nodal, glial cell-derived neurotrophic factor (GDNF), neurturin, artemin, persephin, MIS, and Lefty. Alternatively, the assay can be used to identify compounds that enhance binding of an ActRII polypeptide, ALK4 polypeptide, ALK4:ActRIIB heteromultimer, TGFβRII polypeptides, ALK5 polypeptides and/or betaglycan to a binding partner such as a ligand. In a further embodiment, the compounds can be identified by their ability to interact with an ActRII polypeptide, ALK4 polypeptide, ALK4:ActRIIB heteromultimer, TGFβRII polypeptides, ALK5 polypeptides and/or betaglycan.

A variety of assay formats will suffice and, in light of the present disclosure, those not expressly described herein will nevertheless be comprehended by one of ordinary skill in the art. As described herein, the test compounds (agents) of the disclosure may be created by any combinatorial chemical method. Alternatively, the subject compounds may be naturally occurring biomolecules synthesized *in vivo* or *in vitro.* Compounds (agents) to be tested for their ability to act as modulators of tissue growth can be produced, for example, by bacteria, yeast, plants or other organisms (*e.g.*, natural products), produced chemically (*e.g.*, small molecules, including peptidomimetics), or produced recombinantly. Test compounds contemplated by the present disclosure include non-peptidyl organic molecules, peptides, polypeptides, peptidomimetics, sugars, hormones, and nucleic acid molecules. In certain embodiments, the test agent is a small organic molecule having a molecular weight of less than about 2,000 Daltons.

The test compounds of the disclosure can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be optionally derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S-transferase (GST), photoactivatible crosslinkers or any combinations thereof.

In many drug-screening programs which test libraries of compounds and natural extracts, high-throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the *in vitro* system, the assay instead being focused primarily on the effect of the drug on the molecular target as may be manifest in an alteration of binding affinity between an ActRII polypeptide, ALK4 polypeptide, ALK4:ActRIIB heteromultimer, TGFβRII polypeptides, ALK5 polypeptides and/or betaglycan to a binding partner including for example, BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11/BMP11, GDF15/MIC1, TGFβ1, TGFβ2, TGFβ3, activin A, activin B, activin AB, activin AC, nodal, glial cell-derived neurotrophic factor (GDNF), neurturin, artemin, persephin, MIS, and Lefty.

Merely to illustrate, in an exemplary screening assay of the present disclosure, the compound of interest is contacted with an isolated and purified ALK4:ActRIIB heteromultimer which is ordinarily capable of binding to a TGFβ superfamily ligand, as appropriate for the intention of the assay. To the mixture of the compound and ALK4:ActRIIB heteromultimer is then added to a composition containing the appropriate ligand (*e.g.*, BMP2, BMP2/7, BMP3, BMP4, BMP4/7, BMP5, BMP6, BMP7, BMP8a, BMP8b, BMP9, BMP10, GDF3, GDF5, GDF6/BMP13, GDF7, GDF8, GDF9b/BMP15, GDF11/BMP11, GDF15/MIC1, TGFβ1, TGFβ2, TGFβ3, activin A, activin B, activin C, activin E, activin AB, activin AC, nodal, glial cell-derived neurotrophic factor (GDNF), neurturin, artemin, persephin, MIS, and Lefty). Detection and quantification of heteromultimer-superfamily ligand complexes provides a means for determining the compound's efficacy at inhibiting (or potentiating) complex formation between the ALK4:ActRIIB heteromultimer and its binding protein. The efficacy of the compound can be assessed by generating dose-response curves from data obtained using various concentrations of the test compound. Moreover, a control assay can also be performed to provide a baseline for comparison. For example, in a control assay, isolated and purified ligand is added to a composition containing the ALK4:ActRIIB heteromultimer, and the formation of heteromultimer-ligand complex is quantitated in the absence of the test compound. It will be understood that, in general, the order in which the reactants may be admixed can be varied, and can be admixed simultaneously. Moreover, in place of purified proteins, cellular extracts and lysates may be used to render a suitable cell-free assay system.

Binding of an ActRII polypeptide, ALK4 polypeptide, ALK4:ActRIIB heteromultimer, TGFβRII polypeptides, ALK5 polypeptides and/or betaglycan to another protein may be detected by a variety of techniques. For instance, modulation of the formation of complexes can be quantitated using, for example, detectably labeled proteins such as radiolabeled (*e.g.*, ³²P, ³⁵S, ¹⁴C or ³H), fluorescently labeled *(e.g.,* FITC), or enzymatically labeled ActRII polypeptide, ALK4 polypeptide, ALK4:ActRIIB heteromultimer, TGFβRII polypeptides, ALK5 polypeptides and/or betaglycan and/or a binding protein, by immunoassay, or by chromatographic detection.

In certain embodiments, the present disclosure contemplates the use of fluorescence polarization assays and fluorescence resonance energy transfer (FRET) assays in measuring, either directly or indirectly, the degree of interaction between an ActRII polypeptide, ALK4 polypeptide, ALK4:ActRIIB heteromultimer, TGFβRII polypeptides, ALK5 polypeptides and/or betaglycan and a binding protein. Further, other modes of detection, such as those based on optical waveguides (PCT Publication WO 96/26432 and U.S. Pat. No. 5,677,196), surface plasmon resonance (SPR), surface charge sensors, and surface force sensors, are compatible with many embodiments of the disclosure.

Moreover, the present disclosure contemplates the use of an interaction trap assay, also known as the "two-hybrid assay," for identifying agents that disrupt or potentiate interaction between an ActRII polypeptide, ALK4 polypeptide, ALK4:ActRIIB heteromultimer, TGFβRII polypeptides, ALK5 polypeptides and/or betaglycan and a binding partner. See, *e.g.,* U.S. Pat. No. 5,283,317; Zervos et al. (1993) Cell 72:223-232; Madura et al. (1993) J Biol Chem 268:12046-12054; Bartel et al. (1993) Biotechniques 14:920-924; and Iwabuchi et al. (1993) Oncogene 8:1693-1696). In a specific embodiment, the present disclosure contemplates the use of reverse two-hybrid systems to identify compounds (e.g., small molecules or peptides) that dissociate interactions between an ActRII polypeptide, ALK4 polypeptide, ALK4:ActRIIB heteromultimer, TGFβRII polypeptides, ALK5 polypeptides and/or betaglycan and a binding protein [Vidal and Legrain, (1999) Nucleic Acids Res 27:919-29; Vidal and Legrain, (1999) Trends Biotechnol 17:374-81; and U.S. Pat. Nos. 5,525,490; 5,955,280; and 5,965,368].

In certain embodiments, the subject compounds are identified by their ability to interact with an ActRII polypeptide, ALK4 polypeptide, ALK4:ActRIIB heteromultimer, TGFβRII polypeptides, ALK5 polypeptides and/or betaglycan. The interaction between the compound and the ActRII polypeptide, ALK4 polypeptide, ALK4:ActRIIB heteromultimer, TGFβRII polypeptides, ALK5 polypeptides and/or betaglycan may be covalent or noncovalent. For example, such interaction can be identified at the protein level using *in vitro* biochemical methods, including photo-crosslinking, radiolabeled ligand binding, and affinity chromatography [Jakoby WB et al. (1974) Methods in Enzymology 46: 1]. In certain cases, the compounds may be screened in a mechanism-based assay, such as an assay to detect compounds which bind to an ActRII polypeptide, ALK4 polypeptide, ALK4:ActRIIB heteromultimer, TGFβRII polypeptides, ALK5 polypeptides and/or betaglycan. This may include a solid-phase or fluid-phase binding event. Alternatively, the gene encoding an ActRII polypeptide, ALK4 polypeptide, ALK4:ActRIIB heteromultimer, TGFβRII polypeptides, ALK5 polypeptides and/or betaglycan can be transfected with a reporter system (e.g., β-galactosidase, luciferase, or green fluorescent protein) into a cell and screened against the library preferably by high-throughput screening or with individual members of the library. Other mechanism-based binding assays may be used; for example, binding assays which detect changes in free energy. Binding assays can be performed with the target fixed to a well, bead or chip or captured by an immobilized antibody or resolved by capillary electrophoresis. The bound compounds may be detected usually using colorimetric endpoints or fluorescence or surface plasmon resonance.

### 9. Exemplary Therapeutic Uses

As described herein, it was discovered that ActRII antagonists (inhibitors) and TGFβ antagonists, alone or in combination, have a surprising effect on decreasing tumor burden and increasing survival time in cancer patients. Accordingly, the disclosure provides, in part, methods of using ActRII antagonists and TGFβ antagonists, alone or in combination and optionally in combination with one or more additional supportive therapies and/or active agents, to treat cancer, particularly treating or preventing one or more complications of a cancer (e.g., reducing tumor burden). In addition, the data indicate that efficacy of ActRII and TGFβ antagonist therapy is dependent on the immune system. Therefore, in part, the instant disclosure relates to the discovery that ActRII and TGFβ antagonists, alone or in combination, may be used as an immunotherapeutic, particularly to treat a wide variety of cancers (e.g., cancers associated with immunosuppression and/or immune exhaustion). As with other known immuno-oncology agents, the ability of an ActRII and/or TGFβ antagonist to potentiate an immune response in a patient may have much broader therapeutic implications outside the cancer field. For example, it has been proposed that immune potentiating agents may be useful in treating a wide variety of infectious diseases, particularly pathogenic agents which promote immunosuppression and/or immune exhaustion. Also, such immune potentiating agents may be useful in boosting the immunization efficacy of vaccines (e.g., pathogen and cancer vaccines). Accordingly, the disclosure provides various ActRII and TGFβ antagonists that can be used, alone or in combination and optionally with one or more additional supportive therapies and/or active agents, to increase immune responses in a subject in need thereof, treat cancer, treat infectious diseases, and/or increase vaccination/immunization efficacy.

The methods and ActRII and TGFβ antagonists, alone or in combination, described and claimed herein may be used to treat malignant or premalignant conditions and to prevent progression to a neoplastic or malignant state, including but not limited to those disorders described herein. Such uses are indicated in conditions known or suspected of preceding progression to neoplasia or cancer, in particular, where non-neoplastic cell growth consisting of hyperplasia, metaplasia, or most particularly, dysplasia has occurred.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

The term "treating" as used herein includes amelioration or elimination of the condition once it has been established. In either case, prevention or treatment may be discerned in the diagnosis provided by a physician or other health care provider and the intended result of administration of the therapeutic agent.

In general, treatment or prevention of a disease or condition as described in the present disclosure is achieved by administering one or more of ActRII and/or TGFβ antagonists antagonists in an effective amount. An effective amount of an agent refers to an amount effective, at dosages and for periods of time necessary, to achieve the desired therapeutic or prophylactic result. A therapeutically effective amount of an agent of the present disclosure may vary according to factors such as the disease state, age, sex, and weight of the individual, and the ability of the agent to elicit a desired response in the individual. A prophylactically effective amount refers to an amount effective, at dosages and for periods of time necessary, to achieve the desired prophylactic result.

In general, "tumors" refers to benign and malignant cancers, as well as dormant tumors. In general, "cancer" refers to primary malignant cells or tumors (e.g., those whose cells have not migrated to sites in the subject's body other than the site of the original malignancy or tumor) and secondary malignant cells or tumors (e.g., those arising from metastasis, the migration of malignant cells or tumor cells to secondary sites that are different from the site of the original tumor). Metastasis can be local or distant. Metastases are most often detected through the sole or combined use of magnetic resonance imaging (MRI) scans, computed tomography (CT) scans, blood and platelet counts, liver function studies, chest X-rays, bone scans in addition to the monitoring of specific symptoms, and combinations thereof.

In general, an immune response refers to a response by a cell of the immune system, such as a B cell, T cell (CD4 or CD8), regulatory T cell, antigen-presenting cell, dendritic cell, monocyte, macrophage, NKT cell, NK cell, basophil, eosinophil, or neutrophil, to a stimulus. In some embodiments, the response is specific for a particular antigen (an "antigen-specific response"), and refers to a response by a CD4 T cell, CD8 T cell, or B cell via their antigen-specific receptor. In some embodiments, an immune response is a T cell response, such as a CD4+ response or a CD8+ response. Such responses by these cells can include, for example, cytotoxicity, proliferation, cytokine or chemokine production, trafficking, or phagocytosis, and can be dependent on the nature of the immune cell undergoing the response. An immune response may result in selective targeting, binding to, damage to, destruction of, and/or elimination from the body of invading pathogens, cells or tissues infected with pathogens, cancerous or other abnormal cells, or, in cases of autoimmunity or pathological inflammation, normal cells or tissues.

Immunosuppression of a host immune response plays a role in a variety of chronic immune conditions, such as in persistent infection and tumor immunosuppression. As used herein, "unresponsiveness" or "functional exhaustion", with regard to the immune system, generally refers to refractivity of immune cells to stimulation, such as stimulation via an activating receptor or a cytokine. Unresponsiveness can occur, for example, because of exposure to immunosuppressants, exposure to high or constant doses of antigen, or through the activity of inhibitor receptors, such as PD-1 or TIM-3. As used herein, the term "unresponsiveness" includes refractivity to activating stimulation. Such refractivity is generally antigen-specific and persists after exposure to the antigen has ceased. Unresponsive immune cells can have a reduction of at least 10%, 20%, 30%, 40%, 50%, 60%, 70%, 80%, 90%, 95%, or even 100% in cytotoxic activity, cytokine production, proliferation, trafficking, phagocytotic activity, or any combination thereof, relative to a corresponding control immune cell of the same type.

In general, immunotherapy refers to the treatment of a subject afflicted with, or at risk of contracting or suffering a recurrence of, a disease by a method comprising inducing, enhancing, suppressing or otherwise modifying an immune response.

In general, potentiating an immune response refers to activating or increasing the effectiveness or potency of an existing immune response in a subject. This activation or increase in effectiveness and potency may be achieved, for example, by overcoming mechanisms that suppress the endogenous host immune response or by stimulating mechanisms that activate/enhance the endogenous host immune response.

ActRII and TGFβ antagonists, alone or in combination, of the disclosure may be used in the treatment of various forms of cancer, including, but not limited to, cancer of the bladder, breast, colon, kidney, liver, lung, ovary, cervix, pancreas, rectum, prostate, stomach, epidermis; a hematopoietic tumor of lymphoid or myeloid lineage; a tumor of mesenchymal origin such as a fibrosarcoma or rhabdomyosarcoma; other tumor types such as melanoma, teratocarci-noma, neuroblastoma, glioma, adenocarcinoma and non-small lung cell carcinoma. Examples of cancers include, but are not limited to, carcinoma, lymphoma, glioblastoma, melanoma, sarcoma, and leukemia, myeloma, or lymphoid malignancies. More particular examples of such cancers are noted below and include: squamous cell cancer (e.g., epithelial squamous cell cancer), Ewing sarcoma, Wilms tumor, astrocytomas, lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma multiforme, cervical cancer, ovarian cancer, liver cancer, hepatoma, hepatocellular carcinoma, neuroendocrine tumors, medullary thyroid cancer, differentiated thyroid carcinoma, breast cancer, ovarian cancer, colon cancer, rectal cancer, endometrial cancer or uterine carcinoma, salivary gland carcinoma, kidney or renal cancer, prostate cancer, vulvar cancer, anal carcinoma, penile carcinoma, as well as head-and-neck cancer

Other examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, , Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational TROPhoblastic Tumor, Hairy Cell Leukemia, Head and Neck Cancer, Hepatocellular Cancer, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Parathyroid Cancer, Penile Cancer, Pheochromocytoma, Pituitary Tumor, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, TROPhoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms' Tumor, and any other hyperproliferative disease, besides neoplasia, located in an organ system listed above.

Dysplasia is frequently a forerunner of cancer, and is found mainly in the epithelia. It is the most disorderly form of non-neoplastic cell growth, involving a loss in individual cell uniformity and in the architectural orientation of cells. Dysplasia characteristically occurs where there exists chronic irritation or inflammation. In some embodiments, an ActRII antagonist, optionally in combination with one or more additional supportive therapies and/or active agents, may be used to treat a dysplastic disorders. Dysplastic disorders include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, opthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which may be treated with an ActRII and/or TGFβ antagonist, optionally in combination with one or more additional supportive therapies and/or active agents, include, but are not limited to, benign dysproliferative disorders (e.g., benign tumors, fibrocystic conditions, tissue hypertrophy, intestinal polyps or adenomas, and esophageal dysplasia), leukoplakia, keratoses, Bowen's disease, Farmer's Skin, solar cheilitis, and solar keratosis.

Additional hyperproliferative diseases, disorders, and/or conditions which may be treated with an ActRII and/or TGFβ antagonist, optionally in combination with one or more additional supportive therapies and/or active agents, include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, epithelial carcinoma, glioma, astrocytoma, medulloblastoma, craniopharyngioma, ependymoma, pinealoma, emangioblastoma, acoustic neuroma, oligodendroglioma, meningioma, melanoma, neuroblastoma, and retinoblastoma.

In certain aspects, therapeutic cancer agents such as cytotoxic agents, anti-angiogenic agents, pro-apoptotic agents, immunomodulator agents, antibiotics, hormones, hormone antagonists, chemokines, drugs, prodrugs, toxins, enzymes or other active agents may be used in combination with one or more ActRII and/or TGFβ antagonists. Drugs of use may possess a pharmaceutical property selected from, for example: antimitotic, anti-kinase, alkylating, antimetabolite, antibiotic, alkaloid, anti-angiogenic, pro-apoptotic agents, and combinations thereof.

As used herein, "in combination with", "conjoint administration", "conjointly" refers to any form of administration such that additional therapies (e.g., second, third, fourth, etc.) are still effective in the body (e.g., multiple compounds are simultaneously effective in the patient, which may include synergistic effects of those compounds). Effectiveness may not correlate to measurable concentration of the agent in blood, serum, or plasma. For example, the different therapeutic compounds can be administered either in the same formulation or in separate formulations, either concomitantly or sequentially, and on different schedules. Thus, an individual who receives such treatment can benefit from a combined effect of different therapies. One or more ActRII and/or TGFβ antagonists of the disclosure can be administered concurrently with, prior to, or subsequent to, one or more other additional agents and/or supportive therapies. In general, each therapeutic agent will be administered at a dose and/or on a time schedule determined for that particular agent. The particular combination to employ in a regimen will take into account compatibility of the antagonist of the present disclosure with the therapy and/or the desired.

Exemplary drugs of use may include, but are not limited to, fluorouracil, afatinib, aplidin, azaribine, anastrozole, anthracyclines, axitinib, aminoglutethimide, amsacrine, AVL-101, AVL-291, bendamustine, bleomycin, buserelin, bortezomib, bosutinib, bicalutamide, bryostatin-1, busulfan, capecitabine, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, cladribine, camptothecans, crizotinib, colchicine, cyclophosphamide, cytarabine, cyproterone, clodronate, dacarbazine, dasatinib, dienestrol, dinaciclib, docetaxel, dactinomycin, daunorubicin, diethylstilbestrol, doxorubicin, 2-pyrrolinodoxorubicine (2P-DOX), cyano-morpholino doxorubicin, doxorubicin glucuronide, epirubicin glucuronide, erlotinib, estramustine, epidophyllotoxin, erlotinib, entinostat, estrogen receptor binding agents, etoposide (VP16), etoposide glucuronide, etoposide phosphate, exemestane, filgrastim, fingolimod, floxuridine (FUdR), fluoxymesterone, 3',5'-O-dioleoyl-FudR (FUdR-dO), fludrocortisone, fludarabine, flutamide, goserelin, farnesyl-protein transferase inhibitors, flavopiridol, fostamatinib, ganetespib, GDC-0834, GS-1101, gefitinib, gemcitabine, hydroxyurea, ibrutinib, idarubicin, levamisole, idelalisib, ifosfamide, imatinib, letrozole, asparaginase, leuprolide, lapatinib, lenolidamide, leucovorin, ironotecan, LFM-A13, lomustine, mechlorethamine, melphalan, mercaptopurine, 6-mercaptopurine, megestrol, methotrexate, mitoxantrone, nilutamide, mithramycin, mitomycin, nocodazole, octreotide, mitotane, navelbine, neratinib, nilotinib, nitrosurea, olaparib, plicomycin, procarbazine, paclitaxel, oxaliplatin, PCI-32765, pentostatin, plicamycin, PSI-341, raloxifene, semustine, sorafenib, streptozocin, SU11248, sunitinib, tamoxifen, porfimer, temozolomide, mesna, temazolomide (an aqueous form of DTIC), transplatinum, thalidomide, thioguanine, raltitrexed, thiotepa, teniposide, topotecan, uracil mustard, vatalanib, vinorelbine, vinblastine, rituximab, pamidronate, vincristine, vinca alkaloids and ZD1839.

Toxins of use may include ricin, abrin, alpha toxin, saporin, ribonuclease (RNase), e.g., onconase, DNase I, Staphylococcal enterotoxin-A, pokeweed antiviral protein, gelonin, diphtheria toxin, Pseudomonas exotoxin, and Pseudomonas endotoxin.

Chemokines of use may include RANTES, MCAF, MIP 1-alpha, MIP 1-beta and IP-10.

In certain embodiments, anti-angiogenic agents, such as angiostatin, baculostatin, canstatin, maspin, anti-VEGF antibodies, anti-PlGF peptides and antibodies, anti-vascular growth factor antibodies, anti-Flk-1 antibodies, anti-Flt-1 antibodies and peptides, anti-Kras antibodies, anti-cMET antibodies, anti-MIF (macrophage migration-inhibitory factor) antibodies, laminin peptides, fibronectin peptides, plasminogen activator inhibitors, tissue metalloproteinase inhibitors, interferons, interleukin-12, IP-10, Gro-beta, thrombospondin, 2-methoxyoestradiol, proliferin-related protein, carboxiamidotriazole, CM101, Marimastat, pentosan polysulphate, angiopoietin-2, interferon-alpha, herbimycin A, PNU145156E, 16K prolactin fragment, Linomide (roquinimex), thalidomide, pentoxifylline, genistein, TNP-470, endostatin, paclitaxel, accutin, angiostatin, cidofovir, vincristine, bleomycin, AGM-1470, platelet factor 4, ALK1 polypeptides (e.g., dalantercept) or minocycline may be of use in combination with one or more ActRII antagonists.

Immunomodulators of use may be selected from a cytokine, a stem cell growth factor, a lymphotoxin, a hematopoietic factor, a colony stimulating factor (CSF), an interferon (IFN), erythropoietin, thrombopoietin and a combination thereof. Specifically useful are lymphotoxins such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF), interferon, such as interferons-alpha, -beta or -lamda, and stem cell growth factor, such as that designated "S1 factor". Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGFβ; insulin-like growth factor-I and -II; erythropoietin (EPO); osteoinductive factors; interferons such as interferon-alpha, -beta, and -gamma; colony stimulating factors (CSFs) such as macrophage-CSF (M-CSF); interleukins (ILs) such as IL-1, IL-1alpha, IL-2, IL-3, IL-4, IL-5, IL-6, IL-7, IL-8, IL-9, IL-10, IL-11, IL-12; IL-13, IL-14, IL-15, IL-16, IL-17, IL-18, IL-21, IL-25, LIF, kit-ligand or FLT-3, angiostatin, thrombospondin, endostatin, tumor necrosis factor and LT.

Radionuclides of use include, but are not limited to ¹¹¹In, ¹⁷⁷Lu, ²¹²Bi, ²¹³Bi, ²¹¹At, ⁶²Cu, ⁶⁷Cu, ⁹⁰Y, ¹²⁵I, ¹³¹ 1, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb , ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au, ²¹¹Pb, and ²²⁷Th. The therapeutic radionuclide preferably has a decay-energy in the range of 20 to 6,000 keV, preferably in the ranges 60 to 200 keV for an Auger emitter, 100-2,500 keV for a beta emitter, and 4,000-6,000 keV for an alpha emitter. Maximum decay energies of useful beta-particle-emitting nuclides are preferably 20-5,000 keV, more preferably 100-4,000 keV, and most preferably 500-2,500 keV. Also preferred are radionuclides that substantially decay with Auger-emitting particles. For example, Co-58, Ga-67, Br-80m, Tc-99m, Rh-103m, Pt-109, In-111, Sb-119, 1-125, Ho-161, Os-189m and Ir-192. Decay energies of useful beta-particle-emitting nuclides are preferably <1,000 keV, more preferably <100 keV, and most preferably <70 keV. Also preferred are radionuclides that substantially decay with generation of alpha-particles. Such radionuclides include, but are not limited to: Dy-152, At-211, Bi-212, Ra-223, Rn-219, Po-215, Bi-211, Ac-225, Fr-221, At-217, Bi-213, Th-227 and Fm-255. Decay energies of useful alpha-particle-emitting radionuclides are preferably 2,000-10,000 keV, more preferably 3,000-8,000 keV, and most preferably 4,000-7,000 keV. Additional potential radioisotopes of use include ¹¹C, ¹³N, ¹⁵O, ⁷⁵Br, ¹⁹⁸Au, ²²⁴Ac, ¹²⁶I, ¹³³I, ¹⁰³Ru, ¹⁰⁵Ru, ¹⁰⁷Hg, ²⁰³Hg, ¹²¹mTe, ¹²²mTe, ¹²⁵mTe, ¹⁶⁵Tm, ¹⁶⁷Tm, ⁷⁷Br, ¹¹³mIn, ⁹⁵Ru, ⁹⁷Ru, ¹⁶⁸Tm, ¹⁹⁷Pt, ¹⁰⁹Pd, ¹⁰⁵Rh, ¹⁴²Pr, ¹⁴³Pr, ¹⁶¹Tb, .¹⁶⁶Ho, ¹⁹⁹Au, ⁵⁷Co, ⁵⁸Co, ⁵¹Cr, ⁵⁹Fe, ⁷⁵Se, ²⁰¹Tl, ²²⁵Ac, ⁷⁶Br, and ¹⁶⁹Yb.

In some embodiments, an ActRII and/or TGFβ antagonist is for use in combination with at least one alkylating agent, a nitrosourea, an anti-metabolite, a topoisomerase inhibitor, a mitotic inhibitor, an anthracycline, a corticosteroid hormone, a sex hormone, and/or a targeted anti-tumor compound.

A targeted anti-tumor compound is a drug designed to attack cancer cells more specifically than standard chemotherapy drugs can. Most of these compounds attack cells that harbor mutations of certain genes, or cells that overexpress copies of these genes. In one embodiment, the anti-tumor compound can be imatinib (Gleevec), gefitinib (Iressa), erlotinib (Tarceva), rituximab (Rituxan), or bevacizumab (Avastin).

An alkylating agent works directly on DNA to prevent the cancer cell from propagating. These agents are not specific to any particular phase of the cell cycle. In one embodiment, alkylating agents can be selected from busulfan, cisplatin, carboplatin, chlorambucil, cyclophosphamide, ifosfamide, dacarbazine (DTIC), mechlorethamine (nitrogen mustard), melphalan, and temozolomide.

An antimetabolite makes up the class of drugs that interfere with DNA and RNA synthesis. These agents work during the S phase of the cell cycle and are commonly used to treat leukemias, tumors of the breast, ovary, and the gastrointestinal tract, as well as other cancers. In one embodiment, an antimetabolite can be 5-fluorouracil, capecitabine, 6-mercaptopurine, methotrexate, gemcitabine, cytarabine (ara-C), fludarabine, or pemetrexed.

Topoisomerase inhibitors are drugs that interfere with the topoisomerase enzymes that are important in DNA replication. Some examples of topoisomerase I inhibitors include topotecan and irinotecan while some representative examples of topoisomerase II inhibitors include etoposide (VP-16) and teniposide.

Anthracyclines are chemotherapy drugs that also interfere with enzymes involved in DNA replication. These agents work in all phases of the cell cycle and thus, are widely used as a treatment for a variety of cancers. In one embodiment, an anthracycline used with respect to the disclosure can be daunorubicin, doxorubicin (Adriamycin), epirubicin, idarubicin, or mitoxantrone.

Tumors can escape immune surveillance by co-opting certain immune-checkpoint pathways, particularly in T cells that are specific for tumor antigens (Pardoll, 2012, Nature Reviews Cancer 12:252-264). Studies with checkpoint inhibitor antibodies for cancer therapy have been successful in treating cancers previously thought to be resistant to cancer treatment (see, e.g., Ott & Bhardwaj, 2013, Frontiers in Immunology 4:346; Menzies & Long, 2013, Ther Adv Med Oncol 5:278-85; Pardoll, 2012, Nature Reviews Cancer 12:252-64; Mavilio & Lugli). In contrast to the majority of anti-cancer agents, checkpoint inhibitors do not target tumor cells directly, but rather target lymphocyte receptors or their ligands in order to enhance the endogenous antitumor activity of the immune system. (Pardoll, 2012, Nature Reviews Cancer 12:252-264). Because such inhibitors act primarily by regulating the immune response to diseased cells, tissues or pathogens, they may be used in combination with other therapeutic modalities, such as the subject ActRII and/or TGFβ antagonists, ADCs and/or interferons to enhance the anti-tumor effect of such agents.

Anti-PD1 antibodies have been used for treatment of melanoma, non-small-cell lung cancer, bladder cancer, prostate cancer, colorectal cancer, head and neck cancer, triple-negative breast cancer, leukemia, lymphoma and renal cell cancer (Topalian et al., 2012, N Engl J Med 366:2443-54; Lipson et al., 2013, Clin Cancer Res 19:462-8; Berger et al., 2008, Clin Cancer Res 14:3044-51; Gildener-Leapman et al., 2013, Oral Oncol 49:1089-96; Menzies & Long, 2013, Ther Adv Med Oncol 5:278-85). Exemplary anti-PD1 antibodies include lambrolizumab (MK-3475, MERCK), nivolumab (BMS-936558, Bristol-Myers Squibb), AMP-224 (Merck), and pidilizumab (CT-011, Curetech Ltd.). Anti-PD1 antibodies are commercially available, for example from ABCAM (AB137132), Biolegend (EH12.2H7, RMP1-14) and Affymetrix Ebioscience (J105, 1116, MIH4).

Anti-CTL4A antibodies have been used in clinical trials for treatment of melanoma, prostate cancer, small cell lung cancer, non-small cell lung cancer (Robert & Ghiringhelli, 2009, Oncologist 14:848-61; Ott et al., 2013, Clin Cancer Res 19:5300; Weber, 2007, Oncologist 12:864-72; Wada et al., 2013, J Transl Med 11:89). Exemplary anti-CTLA4 antibodies include ipilimumab (Bristol-Myers Squibb) and tremelimumab (Pfizer). Anti-PD1 antibodies are commercially available, for example from ABCAM (AB 134090), Sino Biological Inc. (11159-H03H, 11159-H08H), and Thermo Scientific Pierce (PA5-29572, PA5-23967, PA5-26465, MA1-12205, MA1-35914). Ipilimumab has recently received FDA approval for treatment of metastatic melanoma (Wada et al., 2013, J Transl Med 11:89).

Although checkpoint inhibitor against CTLA4, PD1 and PD-L1 are the most clinically advanced, other potential checkpoint antigens are known and may be used as the target of therapeutic inhibitors in combination with the subject ActRII antagonists, such as LAG3, B7-H3, B7-H4 and TIM3 (Pardoll, 2012, Nature Reviews Cancer 12:252-264). These and other known agents that stimulate immune response to tumors and/or pathogens may be used in combination with ActRIIa antagonists alone or in further combination with an interferon, such as interferon-.alpha., and/or an antibody-drug conjugate for improved cancer therapy. Other known co-stimulatory pathway modulators that may be used in combination include, but are not limited to, agatolimod, belatacept, blinatumomab, CD40 ligand, anti-B7-1 antibody, anti-B7-2 antibody, anti-B7-H4 antibody, AG4263, eritoran, anti-OX40 antibody, ISF-154, and SGN-70; B7-1, B7-2, ICAM-1, ICAM-2, ICAM-3, CD48, LFA-3, CD30 ligand, CD40 ligand, heat stable antigen, B7h, OX40 ligand, LIGHT, CD70 and CD24.

Therapeutic agents may include a photoactive agent or dye. Fluorescent compositions, such as fluorochrome, and other chromogens, or dyes, such as porphyrins sensitive to visible light, have been used to detect and to treat lesions by directing the suitable light to the lesion. In therapy, this has been termed photoradiation, phototherapy, or photodynamic therapy. See Joni et al. (eds.), PHOTODYNAMIC THERAPY OF TUMORS AND OTHER DISEASES (Libreria Progetto 1985); van den Bergh, Chem. Britain (1986), 22:430. Moreover, monoclonal antibodies have been coupled with photoactivated dyes for achieving phototherapy. See Mew et al., J. Immunol. (1983),130:1473; idem., Cancer Res. (1985), 45:4380; Oseroffet al., Proc. Natl. Acad. Sci. USA (1986), 83:8744; idem., Photochem. Photobiol. (1987), 46:83; Hasan et al., Prog. Clin. Biol. Res. (1989), 288:471; Tatsuta et al., Lasers Surg. Med. (1989), 9:422; Pelegrin et al., Cancer (1991), 67:2529.

Other useful therapeutic agents may comprise oligonucleotides, especially antisense oligonucleotides that preferably are directed against oncogenes and oncogene products, such as bcl-2 or p53. A preferred form of therapeutic oligonucleotide is siRNA. The skilled artisan will realize that any siRNA or interference RNA species may be attached to an antibody or fragment thereof for delivery to a targeted tissue. Many siRNA species against a wide variety of targets are known in the art, and any such known siRNA may be utilized in the claimed methods and compositions.

Known siRNA species of potential use include those specific for IKK-gamma (U.S. Pat. No. 7,022,828); VEGF, Flt-1 and Flk-1/KDR (U.S. Pat. No. 7,148,342); Bcl2 and EGFR (U.S. Pat. No. 7,541,453); CDC20 (U.S. Pat. No. 7,550,572); transducin (beta)-like 3 (U.S. Pat. No. 7,576,196); KRAS (U.S. Pat. No. 7,576,197); carbonic anhydrase II (U.S. Pat. No. 7,579,457); complement component 3 (U.S. Pat. No. 7,582,746); interleukin-1 receptor-associated kinase 4 (IRAK4) (U.S. Pat. No. 7,592,443); survivin (U.S. Pat. No. 7,608,7070); superoxide dismutase 1 (U.S. Pat. No. 7,632,938); MET proto-oncogene (U.S. Pat. No. 7,632,939); amyloid beta precursor protein (APP) (U.S. Pat. No. 7,635,771); IGF-1R (U.S. Pat. No. 7,638,621); ICAM1 (U.S. Pat. No. 7,642,349); complement factor B (U.S. Pat. No. 7,696,344); p53 (U.S. Pat. No. 7,781,575), and apolipoprotein B (U.S. Pat. No. 7,795,421), the Examples section of each referenced patent incorporated herein by reference.

Additional siRNA species are available from known commercial sources, such as Sigma-Aldrich (St Louis, Mo.), Invitrogen (Carlsbad, Calif.), Santa Cruz Biotechnology (Santa Cruz, Calif.), Ambion (Austin, Tex.), Dharmacon (Thermo Scientific, Lafayette, Colo.), Promega (Madison, Wis.), Mirus Bio (Madison, Wis.) and Qiagen (Valencia, Calif.), among many others. Other publicly available sources of siRNA species include the siRNAdb database at the Stockholm Bioinformatics Centre, the MIT/ICBP siRNA Database, the RNAi Consortium shRNA Library at the Broad Institute, and the Probe database at NCBI. For example, there are 30,852 siRNA species in the NCBI Probe database. The skilled artisan will realize that for any gene of interest, either a siRNA species has already been designed, or one may readily be designed using publicly available software tools. Any such siRNA species may be delivered using the subject DNL.TM. complexes.

ActRII and/or TGFβ antagonist immunotherapy may be more effective when combined with a vaccination protocol. Many experimental strategies for vaccination against tumors have been devised (see Rosenberg, S., 2000, Development of Cancer Vaccines, ASCO Educational Book Spring: 60-62; Logothetis, C., 2000, ASCO Educational Book Spring: 300-302; Khayat, D. 2000, ASCO Educational Book Spring: 414-428; Foon, K. 2000, ASCO Educational Book Spring: 730-738; see also Restifo, N. and Sznol, M., Cancer Vaccines, Ch. 61, pp. 3023-3043 in DeVita, V. et al. (eds.), 1997, Cancer: Principles and Practice of Oncology. Fifth Edition). In one of these strategies, a vaccine is prepared using autologous or allogeneic tumor cells. These cellular vaccines have been shown to have increased effectiveness when the tumor cells are transduced to express GM-CSF (Dranoff et al. (1993) Proc. Natl. Acad. Sci U.S.A. 90: 3539-43). Therefore, in some embodiments, one or more ActRII antagonists may be combined with an immunogenic agent, such as cancerous cells, purified tumor antigens (including recombinant proteins, peptides, and carbohydrate molecules), cells, and cells transfected with genes encoding immune stimulating cytokines (He et al (2004) J. Immunol. 173:4919-28). Non-limiting examples of tumor vaccines that can be used include peptides of melanoma antigens, such as peptides of gp100, MAGE antigens, Trp-2, MART1 and/or tyrosinase, or tumor cells transfected to express the cytokine GM-CSF.

The study of gene expression and large scale gene expression patterns in various tumors has led to the definition of so-called tumor specific antigens (Rosenberg, S A (1999) Immunity 10: 281-7). In many cases, these tumor specific antigens are differentiation antigens expressed in the tumors and in the cell from which the tumor arose, for example melanocyte antigens gp100, MAGE antigens, and Trp-2. More importantly, many of these antigens can be shown to be the targets of tumor specific T cells found in the host. PD-L1 blockade may be used in conjunction with a collection of recombinant proteins and/or peptides expressed in a tumor in order to generate an immune response to these proteins. These proteins are normally viewed by the immune system as self-antigens and are therefore tolerant to them. The tumor antigen may also include the protein telomerase, which is required for the synthesis of telomeres of chromosomes and which is expressed in more than 85% of human cancers and in only a limited number of somatic tissues (Kim, N et al. (1994) Science 266: 2011-2013). These somatic tissues may be protected from immune attack by various means. Tumor antigen may also be "neo-antigens" expressed in cancer cells because of somatic mutations that alter protein sequence or create fusion proteins between two unrelated sequences (e.g., ber-abl in the Philadelphia chromosome), or idiotype from B cell tumors.

Other tumor vaccines may include the proteins from viruses implicated in human cancers such a Human Papilloma Viruses (HPV), Hepatitis Viruses (HBV and HCV) and Kaposi's Herpes Sarcoma Virus (KHSV). Another form of tumor specific antigen which may be used in conjunction with ActRII antagonists thearpy is purified heat shock proteins (HSP) isolated from the tumor tissue itself. These heat shock proteins contain fragments of proteins from the tumor cells and these HSPs are highly efficient at delivery to antigen presenting cells for eliciting tumor immunity (Suot, R & Srivastava, P (1995) Science 269:1585-1588; Tamura, Y. et al. (1997) Science 278:117-120).

Dendritic cells (DC) are potent antigen presenting cells that can be used to prime antigen-specific responses. DC's can be produced ex vivo and loaded with various protein and peptide antigens as well as tumor cell extracts (Nestle, F. et al. (1998) Nature Medicine 4: 328-332). DCs may also be transduced by genetic means to express these tumor antigens as well. DCs have also been fused directly to tumor cells for the purposes of immunization (Kugler, A. et al. (2000) Nature Medicine 6:332-336). As a method of vaccination, DC immunization may be effectively combined with an ActRII antagonist to activate more potent anti-tumor responses.

Other methods of the disclosure are used to treat patients that have been exposed to particular toxins or pathogens. Accordingly, another aspect of the disclosure provides methods of treating or preventing an infectious disease (e.g., viral, bacterial or parasitic infection) in a subject comprising administering to an subject in need thereof an therapeutically effective amount of one or more ActRII and/or TGFβ antagonists, optionally further comprising administering one or more additional supportive therapies and/or active agents for treating the infectious disease. In some embodiments, the disclosure provides methods of treating an infectious disease in a subject, for example, by potentiating an endogenous immune response in a subject afflicted with an infectious disease, comprising administering to the subject a therapeutically effective amount of one or more ActRII and/or TGFβ antagonists, optionally further comprising administering one or more additional supportive therapies and/or active agents for treating the infectious disease.

Examples of infectious viruses include but are not limited to: Retroviridae; Picornaviridae (for example, polio viruses, hepatitis A virus; enteroviruses, human coxsackie viruses, rhinoviruses, echoviruses); Calciviridae (such as strains that cause gastroenteritis); Togaviridae (for example, equine encephalitis viruses, rubella viruses); Flaviridae (for example, dengue viruses, encephalitis viruses, yellow fever viruses); Coronaviridae (for example, coronaviruses); Rhabdoviridae (for example, vesicular stomatitis viruses, rabies viruses); Filoviridae (for example, ebola viruses); Paramyxoviridae (for example, parainfluenza viruses, mumps virus, measles virus, respiratory syncytial virus); Orthomyxoviridae (for example, influenza viruses); Bungaviridae (for example, Hantaan viruses, bunga viruses, phleboviruses and Nairo viruses); Arena viridae (hemorrhagic fever viruses); Reoviridae (e.g., reoviruses, orbiviurses and rotaviruses); Birnaviridae; Hepadnaviridae (Hepatitis B virus); Parvoviridae (parvoviruses); Papovaviridae (papilloma viruses, polyoma viruses); Adenoviridae (most adenoviruses); Herpesviridae (herpes simplex virus (HSV) 1 and HSV-2, varicella zoster virus, cytomegalovirus (CMV), herpes viruses); Poxyiridae (variola viruses, vaccinia viruses, pox viruses); and Iridoviridae (such as African swine fever virus); and unclassified viruses (for example, the etiological agents of Spongiform encephalopathies, the agent of delta hepatitis (thought to be a defective satellite of hepatitis B virus), the agents of non-A, non-B hepatitis (class 1 = internally transmitted; class 2 = parenterally transmitted (i.e., Hepatitis C); Norwalk and related viruses, and astroviruses). The ActRII antagonists and methods described herein are contemplated for use in treating infections with these viral agents. Therefore, in some embodiments, the disclosure provides methods of using one or more ActRII antagonists, optionally in combination with one or more supportive therapies and/or active agents, to treat or prevent a viral infection including, for example, AIDS, AIDS Related Complex, chickenpox, common cold, viral bronchitis, cytomegalovirus infection, Colorado tick fever, Dengue fever, Ebola haemorrhagic fever, epidemic parotitis, "hand, foot and mouth" disease, hepatitis, herpes simplex, herpes zoster, HPV, Influenza (Flu), Lassa fever, measles, Marburg haemorrhagic fever, infectious mononucleosis, mumps, poliomyelitis, progressive multifocal leukencephalopathy, rabies, rubella, SARS, smallpox, viral encephalitis, viral gastroenteritis, viral meningitis, viral pneumonia, West Nile disease, and Yellow fever.

Examples of infectious bacteria include but are not limited to: Helicobacter pyloris, Borelia burgdorferi, Legionella pneumophilia, Mycobacteria sps (such as M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracia, corynebacterium diphtheriae, corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, and Actinomyces israelli. The ActRII compositions and methods described herein are contemplated for use in treating infections with these bacterial agents. Therefore, in some embodiments, the disclosure provides methods of using one or more ActRII antagonists, optionally in combination with one or more supportive therapies and/or active agents, to treat or prevent a bacterial infection including, for example, anthrax, bacterial adult respiratory distress syndrome, bacterial meningitis, brucellosis, campylobacteriosis, cat scratch disease, bronchitis, cholera, diphtheria, typhus, gonorrhea, legionellosis, leprosy (Hansen's Disease), leptospirosis, listeriosis, lyme disease, melioidosis, MRSA infection, mycobacterial infection, meningitis, nocardiosis, nephritis, glomerulonephritis, periodontal disease, pertussis (Whooping Cough), plague, pneumococcal pneumonia, psittacosis, Q fever, Rocky Mountain Spotted Fever (RMSF), salmonellosis, scarlet dever, shigellosis, syphilis, septic shock, haemodynamic shock, sepsis syndrome, tetanus, trachoma, tuberculosis, tularemia, typhoid Fever.

Examples of infectious fungi include but are not limited to: Cryptococcus neoformans, Histoplasma capsulatum, Coccidioides immitis, Blastomyces dermatitidis, and Candida albicans. The ActRII antagonists and methods described herein are contemplated for use in treating infections with these fungal agents. Therefore, in some embodiments, the disclosure provides methods of using one or more ActRII antagonists, optionally in combination with one or more supportive therapies and/or active agents, to treat or prevent a fungal infection including, for example, aspergillosis; thrush, cryptococcosis, blastomycosis, coccidioidomycosis, and histoplasmosis.

Examples of infectious parasites include but are not limited to: Entamoeba histolytica, Balantidium coli, Naegleriafowleri, Acanthamoeba sp., Giardia lambia, Cryptosporidium sp., Pneumocystis carinii, Plasmodium vivax, Babesia microti, Trypanosoma brucei, Trypanosoma cruzi, Leishmania donovani, Toxoplasma gondii, Nippostrongylus brasiliensis. The ActRII antagonists and methods described herein are contemplated for use in treating infections with these agents. Therefore, in some embodiments, the disclosure provides methods of using one or more ActRII antagonists, optionally in combination with one or more supportive therapies and/or active agents, to treat or prevent a fungal infection including, for example, African trypanosomiasis, Amebiasis, Ascariasis, Babesiosis, Chagas Disease, Clonorchiasis, Cryptosporidiosis, Cysticercosis, Diphyllobothriasis, Dracunculiasis, Echinococcosis, Enterobiasis, Fascioliasis, Fasciolopsiasis, Filariasis, Free-living amebic infection, Giardiasis, Gnathostomiasis, Hymenolepiasis, Isosporiasis, Kala-azar, Leishmaniasis, Malaria, Metagonimiasis, Myiasis, Onchocerciasis, Pediculosis, Pinworm Infection, Scabies, Schistosomiasis, Taeniasis, Toxocariasis, Toxoplasmosis, Trichinellosis, Trichinosis, Trichuriasis, and Trypanosomiasis.

In some embodiments, the disclosure provides methods of treating an infectious disease by administering to a patient in need thereof an effective amount of an ActRII and/or TGFβ antagonist alone or in combination with a second therapeutic agent to treat the pathogen, for example, an antibiotic, antifungal agent, antiviral agent, or anti-parasite drug.

In general, an antibiotic refers to any compound that inhibits the growth of, or kills, bacteria. Useful, non-limiting examples of an antibiotic include lincosamides (clindomycin); chloramphenicols; tetracyclines (such as Tetracycline, Chlortetracycline, Demeclocycline, Methacycline, Doxycycline, Minocycline); aminoglycosides (such as Gentamicin, Tobramycin, Netilmicin, Amikacin, Kanamycin, Streptomycin, Neomycin); beta-lactams (such as penicillins, cephalosporins, Imipenem, Aztreonam); vancomycins; bacitracins; macrolides (erythromycins), amphotericins; sulfonamides (such as Sulfanilamide, Sulfamethoxazole, Sulfacetamide, Sulfadiazine, Sulfisoxazole, Sulfacytine, Sulfadoxine, Mafenide, p-Aminobenzoic Acid, Trimethoprim-Sulfamethoxazole); Methenamin; Nitrofurantoin; Phenazopyridine; trimethoprim; rifampicins; metronidazoles; cefazolins; Lincomycin; Spectinomycin; mupirocins; quinolones (such as Nalidixic Acid, Cinoxacin, Norfloxacin, Ciprofloxacin, Perfloxacin, Ofloxacin, Enoxacin, Fleroxacin, Levofloxacin); novobiocins; polymixins; gramicidins; and antipseudomonals (such as Carbenicillin, Carbenicillin Indanyl, Ticarcillin, Azlocillin, Mezlocillin, Piperacillin) or any salts or variants thereof. See also Physician's Desk Reference, 59.sup.th edition, (2005), Thomson P D R, Montvale N.J.; Gennaro et al., Eds. Remington's The Science and Practice of Pharmacy, 20.sup.th edition, (2000), Lippincott Williams and Wilkins, Baltimore Md.; Braunwald et al., Eds. Harrison's Principles of Internal Medicine, 15th edition, (2001), McGraw Hill, NY; Berkow et al., Eds. The Merck Manual of Diagnosis and Therapy, (1992), Merck Research Laboratories, Rahway N.J. The antibiotic used will depend on the type of bacterial infection.

In general, an anti-fungal agent refers to any compound that inhibits the growth of, or kills, fungi. Non-limiting examples include imidazoles (such as griseofulvin, miconazole, terbinafine, fluconazole, ketoconazole, voriconazole, and itraconizole); polyenes (such as amphotericin B and nystatin); Flucytosines; and candicidin or any salts or variants thereof. S ee also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J. The anti-fungal used will depend on the type of fungal infection.

An anti-viral drug refers to any compound that inhibits action of a virus. Non-limiting examples include interferon alpha, beta or gamma, didanosine, lamivudine, zanamavir, lopanivir, nelfinavir, efavirenz, indinavir, valacyclovir, zidovudine, amantadine, rimantidine, ribavirin, ganciclovir, foscarnet, and acyclovir or any salts or variants thereof. See also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J. The anti-viral used will depend on the type of viral infection.

An anti-parasitic agent refers to any compound that inhibits the growth of, or kills, parasites. Useful, non-limiting examples of an anti-parasitic agent include chloroquine, mefloquine, quinine, primaquine, atovaquone, sulfasoxine, and pyrimethamine or any salts or variants thereof. See also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J. The anti-parasitic agent used will depend on the type of parasite infection.

Similar to its application to tumors discussed above, ActRII and/or TGFβ antagonists described herein can be used alone, or as an adjuvant, in combination with vaccines, to stimulate the immune response to pathogens, toxins, and self-antigens. These approaches can be combined with other forms of immunotherapy such as cytokine treatment (e.g., administration of interferons, GM-CSF, G-CSF or IL-2). Examples of pathogens for which this therapeutic approach may be particularly useful, include pathogens for which there is currently no effective vaccine, or pathogens for which conventional vaccines are less than completely effective. These include, but are not limited to HIV, Hepatitis (A, B, & C), Influenza, Herpes, Giardia, Malaria, Leishmania, Staphylococcus aureus, and Pseudomonas Aeruginosa.

In some embodiments, ActRII and/or TGFβ antagonists of the disclosure are not administered to patients having breast cancer. In some embodiments, ActRII and/or TGFβ antagonists of the disclosure are not administered to patients having multiple myeloma. In some embodiments, ActRII and/or TGFβ antagonists of the disclosure are not administered to patients having breast cancer. In some embodiments, ActRII and/or TGFβ antagonists of the disclosure are not administered to patients having myelodysplastic syndrome. In some embodiments, ActRII and/or TGFβ antagonists of the disclosure are not administered to patients having an FSH-secreting pituitary tumor. In some embodiments, ActRII and/or TGFβ antagonists of the disclosure are not administered to patients having prostate cancer. In some embodiments, ActRII and/or TGFβ antagonists of the disclosure are not administered to patients having undesirably elevated immune activity (e.g., increased T cell activity), as compared to normal, healthy patients. In some embodiments, ActRII and/or TGFβ antagonists of the disclosure are not administered to patients having an autoimmune disorder, or autoimmune-related disorder. In some embodiments, ActRII and/or TGFβ antagonists of the disclosure are not administered to patients having an autoimmune disorder, or autoimmune-related disorder, that is mediated by undesirably elevated T cell activity. For example, in some embodiments, ActRII and/or TGFβ antagonists of the disclosure are not administered to patients having one or more of: acute disseminated encephalomyelitis, acute necrotizing hemorrhagic leukoencephalitis, Addison's disease, agammaglobulinemia, alopecia areata, amyloidosis, ankylosing spondylitis, anti-GBM/Anti-TBM nephritis, antiphospholipid syndrome (APS), autoimmune angioedema, autoimmune aplastic anemia, autoimmune dysautonomia, autoimmune hepatitis, autoimmune hyperlipidemia, autoimmune immunodeficiency, autoimmune inner ear disease, autoimmune myocarditis, autoimmune oophoritis, autoimmune pancreatitis, autoimmune retinopathy, autoimmune thrombocytopenic purpura (ATP), autoimmune thyroid disease, autoimmune urticarial, axonal & neuronal neuropathies, Balo disease, Behcet's disease, bullous pemphigoid, castleman disease, celiac disease, Chagas disease, chronic inflammatory demyelinating polyneuropathy, chronic recurrent multifocal osteomyelitis, Churg-Strauss syndrome, cicatricial pemphigoid/benign mucosal pemphigoid, Crohn's disease, Cogans syndrome, cold agglutinin disease, coxsackie myocarditis, CREST disease, essential mixed cryoglobulinemia, demyelinating neuropathies, dermatitis herpetiformis, dermatomyositis, Devic's disease (neuromyelitis optica), discoid lupus, Dressler's syndrome, endometriosis, eosinophilic esophagitis, eosinophilic fasciitis, erythema nodosum, experimental allergic encephalomyelitis, Evans syndrome, fibrosing alveolitis, giant cell arteritis, giant cell myocarditis, glomerulonephritis, Goodpasture's syndrome, granulomatosis with polyangiitis, Graves' disease, Guillain-Barre syndrome, Hashimoto's encephalitis, Hashimoto's thyroiditis, Henoch-Schonlein purpura, herpes gestationis, hypogammaglobulinemia, IgA nephropathy, IgG4-related sclerosing disease, immunoregulatory lipoproteins, inclusion body myositis, interstitial cystitis, juvenile arthritis, juvenile myositis, Kawasaki syndrome, Lambert-Eaton syndrome, leukocytoclastic vasculitis, lichen planus, lichen sclerosus, ligneous conjunctivitis, linear IgA disease (LAD), lupus (SLE), lyme disease (chronic), Meniere's disease, microscopic polyangiitis, mixed connective tissue disease (MCTD), Mooren's ulcer, Mucha-Habermann disease, multiple sclerosis, myasthenia gravis, myositis, neuromyelitis optica (Devic's), ocular cicatricial pemphigoid, optic neuritis, palindromic rheumatism, PANDAS (Pediatric Autoimmune Neuropsychiatric Disorders Associated with Streptococcus), paraneoplastic cerebellar degeneration, paroxysmal nocturnal hemoglobinuria (PNH), Parry Romberg syndrome, Parsonnage-Turner syndrome, pars planitis (peripheral uveitis), pemphigus, perivenous encephalomyelitis, POEMS syndrome, polyarteritis nodosa, Type I, II, & III autoimmune polyglandular syndromes, polymyalgia rheumatic, polymyositis, progesterone dermatitis, primary biliary cirrhosis, primary sclerosing cholangitis, psoriasis, psoriatic arthritis, pyoderma gangrenosum, Raynauds phenomenon, reactive arthritis, reflex sympathetic dystrophy, Reiter's syndrome, relapsing polychondritis, rheumatic fever, rheumatoid arthritis, sarcoidosis, Schmidt syndrome, scleritis, scleroderma, Sjogren's syndrome, sperm & testicular autoimmunity, stiff person syndrome, Susac's syndrome, sympathetic ophthalmia, Takayasu's arteritis, Tolosa-Hunt syndrome, transverse myelitis, ulcerative colitis, undifferentiated connective tissue disease (UCTD), vesiculobullous dermatosis, Wegener's granulomatosis. In some embodiments, ActRII and/or TGFβ antagonists of the disclosure are not administered to patients undergoing tissue or organ transplantation. In some embodiments, ActRII and/or TGFβ antagonists of the disclosure are not administered to patients who have had tissue or organ transplantation. In some embodiments, ActRII and/or TGFβ antagonists of the disclosure are not administered to patients who have graft-versus-host disease. In some embodiments, ActRII and/or TGFβ antagonists of the disclosure are not administered to patients who are being treated with one or more immunosuppressant agents and/or therapies.

In certain embodiments, the present disclosure provides methods for managing a patient that has been treated with, or is a candidate to be treated with, one or more one or more ActRII antagonists of the disclosure by measuring one or more hematologic parameters in the patient. The hematologic parameters may be used to evaluate appropriate dosing for a patient who is a candidate to be treated with the antagonist of the present disclosure, to monitor the hematologic parameters during treatment, to evaluate whether to adjust the dosage during treatment with one or more antagonist of the disclosure, and/or to evaluate an appropriate maintenance dose of one or more antagonists of the disclosure. If one or more of the hematologic parameters are outside the normal level, dosing with one or more ActRII antagonists may be reduced, delayed, or terminated.

Hematologic parameters that may be measured in accordance with the methods provided herein include, for example, red blood cell levels, blood pressure, iron stores, and other agents found in bodily fluids that correlate with increased red blood cell levels, using art recognized methods. Such parameters may be determined using a blood sample from a patient. Increases in red blood cell levels, hemoglobin levels, and/or hematocrit levels may cause increases in blood pressure.

In one embodiment, if one or more hematologic parameters are outside the normal range or on the high side of normal in a patient who is a candidate to be treated with one or more ActRII antagonists, then onset of administration of the one or more antagonists may be delayed until the hematologic parameters have returned to a normal or acceptable level either naturally or via therapeutic intervention. For example, if a candidate patient is hypertensive or pre-hypertensive, then the patient may be treated with a blood pressure lowering agent in order to reduce the patient's blood pressure. Any blood pressure lowering agent appropriate for the individual patient's condition may be used including, for example, diuretics, adrenergic inhibitors (including alpha blockers and beta blockers), vasodilators, calcium channel blockers, angiotensin-converting enzyme (ACE) inhibitors, or angiotensin II receptor blockers. Blood pressure may alternatively be treated using a diet and exercise regimen. Similarly, if a candidate patient has iron stores that are lower than normal, or on the low side of normal, then the patient may be treated with an appropriate regimen of diet and/or iron supplements until the patient's iron stores have returned to a normal or acceptable level. For patients having higher than normal red blood cell levels and/or hemoglobin levels, then administration of the one or more antagonists of the disclosure may be delayed until the levels have returned to a normal or acceptable level.

In certain embodiments, if one or more hematologic parameters are outside the normal range or on the high side of normal in a patient who is a candidate to be treated with one or more ActRII antagonists agents, then the onset of administration may not be delayed. However, the dosage amount or frequency of dosing of the one or more antagonists may be set at an amount that would reduce the risk of an unacceptable increase in the hematologic parameters arising upon administration of the one or more antagonists of the disclosure. Alternatively, a therapeutic regimen may be developed for the patient that combines one or more ActRII antagonists with a therapeutic agent that addresses the undesirable level of the hematologic parameter. For example, if the patient has elevated blood pressure, then a therapeutic regimen may be designed involving administration of one or more ActRII antagonists and a blood pressure lowering agent. For a patient having lower than desired iron stores, a therapeutic regimen may be developed involving one or more ActRII antagonists and iron supplementation.

In one embodiment, baseline parameter(s) for one or more hematologic parameters may be established for a patient who is a candidate to be treated with one or more ActRII antagonists and an appropriate dosing regimen established for that patient based on the baseline value(s). Alternatively, established baseline parameters based on a patient's medical history could be used to inform an appropriate antagonist dosing regimen for a patient. For example, if a healthy patient has an established baseline blood pressure reading that is above the defined normal range it may not be necessary to bring the patient's blood pressure into the range that is considered normal for the general population prior to treatment with the one or more antagonist of the disclosure. A patient's baseline values for one or more hematologic parameters prior to treatment with one or more ActRII antagonists may also be used as the relevant comparative values for monitoring any changes to the hematologic parameters during treatment with the one or more antagonists described herein.

In certain embodiments, one or more hematologic parameters are measured in patients who are being treated with one or more ActRII antagonists. The hematologic parameters may be used to monitor the patient during treatment and permit adjustment or termination of the dosing with the one or more antagonists of the disclosure or additional dosing with another therapeutic agent. For example, if administration of one or more ActRII antagonists results in an increase in blood pressure, red blood cell level, or hemoglobin level, or a reduction in iron stores, then the dose of the one or more antagonists of the disclosure may be reduced in amount or frequency in order to decrease the effects of the one or more antagonists of the disclosure on the one or more hematologic parameters. If administration of one or more ActRII antagonists results in a change in one or more hematologic parameters that is adverse to the patient, then the dosing of the one or more antagonists described herein may be terminated either temporarily, until the hematologic parameter(s) return to an acceptable level, or permanently. Similarly, if one or more hematologic parameters are not brought within an acceptable range after reducing the dose or frequency of administration of the one or more antagonists described herein, then the dosing may be terminated. As an alternative, or in addition to, reducing or terminating the dosing with the one or more antagonists described herein, the patient may be dosed with an additional therapeutic agent that addresses the undesirable level in the hematologic parameter(s), such as, for example, a blood pressure lowering agent or an iron supplement. For example, if a patient being treated with one or more ActRII antagonists has elevated blood pressure, then dosing with the one or more antagonists of the disclosure may continue at the same level and a blood-pressure-lowering agent is added to the treatment regimen, dosing with the one or more antagonist (e.g., in amount and/or frequency) and a blood-pressure-lowering agent is added to the treatment regimen, or dosing with the one or more antagonist may be terminated and the patient may be treated with a blood-pressure-lowering agent.

### 10. Pharmaceutical Compositions

In certain aspects, ActRII and/or TGFβ antagonists, or combinations of such antagonists, of the present disclosure can be administered alone or as a component of a pharmaceutical formulation (also referred to as a therapeutic composition or pharmaceutical composition). A pharmaceutical formation refers to a preparation which is in such form as to permit the biological activity of an active ingredient (e.g., an agent of the present disclosure) contained therein to be effective and which contains no additional components which are unacceptably toxic to a subject to which the formulation would be administered. The subject compounds may be formulated for administration in any convenient way for use in human or veterinary medicine. For example, one or more agents of the present disclosure may be formulated with a pharmaceutically acceptable carrier. A pharmaceutically acceptable carrier refers to an ingredient in a pharmaceutical formulation, other than an active ingredient, which is generally nontoxic to a subject. A pharmaceutically acceptable carrier includes, but is not limited to, a buffer, excipient, stabilizer, and/or preservative. In general, pharmaceutical formulations for use in the present disclosure are in a pyrogen-free, physiologically-acceptable form when administered to a subject. Therapeutically useful agents other than those described herein, which may optionally be included in the formulation as described above, may be administered in combination with the subject agents in the methods of the present disclosure.

In certain embodiments, compositions will be administered parenterally [e.g., by intravenous (I.V.) injection, intraarterial injection, intraosseous injection, intramuscular injection, intrathecal injection, subcutaneous injection, or intradermal injection]. Pharmaceutical compositions suitable for parenteral administration may comprise one or more agents of the disclosure in combination with one or more pharmaceutically acceptable sterile isotonic aqueous or nonaqueous solutions, dispersions, suspensions or emulsions, or sterile powders which may be reconstituted into sterile injectable solutions or dispersions just prior to use. Injectable solutions or dispersions may contain antioxidants, buffers, bacteriostats, suspending agents, thickening agents, or solutes which render the formulation isotonic with the blood of the intended recipient. Examples of suitable aqueous and nonaqueous carriers which may be employed in the pharmaceutical formulations of the present disclosure include water, ethanol, polyols (e.g., glycerol, propylene glycol, polyethylene glycol, *etc.*)*,* vegetable oils (*e.g.,* olive oil), injectable organic esters (*e.g.,* ethyl oleate), and suitable mixtures thereof. Proper fluidity can be maintained, for example, by the use of coating materials (*e.g.,* lecithin), by the maintenance of the required particle size in the case of dispersions, and by the use of surfactants.

In some embodiments, compounds will be administered to the eye including, *e.g.,* by topical administration, intraocular (e.g., intravitreal) injection, or by implant or device. An intravitreal injection can be injected, for example, through the pars plana, 3 mm to 4 mm posterior to the limbus. Pharmaceutical compositions for administration to the eye may formulated in a variety of ways including, for example, eye drops, ophthalmic solutions, ophthalmic suspensions, ophthalmic emulsions, intravitreal injections, sub-Tenon injections, ophthalmic biodrodible implant, and non-bioeordible ophthalmic inserts or depots.

In some embodiments, a therapeutic method of the present disclosure includes administering the pharmaceutical composition systemically, or locally, from an implant or device. Further, the pharmaceutical composition may be encapsulated or injected in a form for delivery to a target tissue site (*e.g.,* bone marrow or muscle). In certain embodiments, compositions of the present disclosure may include a matrix capable of delivering one or more of the agents of the present disclosure to a target tissue site (*e.g.,* bone marrow or muscle), providing a structure for the developing tissue and optimally capable of being resorbed into the body. For example, the matrix may provide slow release of one or more agents of the present disclosure. Such matrices may be formed of materials presently in use for other implanted medical applications.

The choice of matrix material may be based on one or more of: biocompatibility, biodegradability, mechanical properties, cosmetic appearance, and interface properties. The particular application of the subject compositions will define the appropriate formulation. Potential matrices for the compositions may be biodegradable and chemically defined calcium sulfate, tricalciumphosphate, hydroxyapatite, polylactic acid, and polyanhydrides. Other potential materials are biodegradable and biologically well-defined including, for example, bone or dermal collagen. Further matrices are comprised of pure proteins or extracellular matrix components. Other potential matrices are non-biodegradable and chemically defined including, for example, sintered hydroxyapatite, bioglass, aluminates, or other ceramics. Matrices may be comprised of combinations of any of the above mentioned types of material including, for example, polylactic acid and hydroxyapatite or collagen and tricalciumphosphate. The bioceramics may be altered in composition (*e.g*., calcium-aluminate-phosphate) and processing to alter one or more of pore size, particle size, particle shape, and biodegradability.

In certain embodiments, pharmaceutical compositions of present disclosure can be administered topically. "Topical application" or "topically" means contact of the pharmaceutical composition with body surfaces including, for example, the skin, wound sites, and mucous membranes. The topical pharmaceutical compositions can have various application forms and typically comprises a drug-containing layer, which is adapted to be placed near to or in direct contact with the tissue upon topically administering the composition. Pharmaceutical compositions suitable for topical administration may comprise one or more one or more ActRIIB and/or TGFβ antagonists of the disclosure in combination formulated as a liquid, a gel, a cream, a lotion, an ointment, a foam, a paste, a putty, a semi-solid, or a solid. Compositions in the liquid, gel, cream, lotion, ointment, foam, paste, or putty form can be applied by spreading, spraying, smearing, dabbing or rolling the composition on the target tissue. The compositions also may be impregnated into sterile dressings, transdermal patches, plasters, and bandages. Compositions of the putty, semi-solid or solid forms may be deformable. They may be elastic or non-elastic (*e.g.,* flexible or rigid). In certain aspects, the composition forms part of a composite and can include fibers, particulates, or multiple layers with the same or different compositions.

Topical compositions in the liquid form may include pharmaceutically acceptable solutions, emulsions, microemulsions, and suspensions. In addition to the active ingredient(s), the liquid dosage form may contain an inert diluent commonly used in the art including, for example, water or other solvent, a solubilizing agent and/or emulsifier [*e.g*., ethyl alcohol, isopropyl alcohol, ethyl carbonate, ethyl acetate, benzyl alcohol, benzyl benzoate, propylene glycol, or 1,3-butylene glycol, an oil (*e.g.,* cottonseed, groundnut, corn, germ, olive, castor, and sesame oil), glycerol, tetrahydrofuryl alcohol, a polyethylene glycol, a fatty acid ester of sorbitan, and mixtures thereof].

Topical gel, cream, lotion, ointment, semi-solid or solid compositions may include one or more thickening agents, such as a polysaccharide, synthetic polymer or protein-based polymer. In one embodiment of the disclosure, the gelling agent herein is one that is suitably nontoxic and gives the desired viscosity. The thickening agents may include polymers, copolymers, and monomers of: vinylpyrrolidones, methacrylamides, acrylamides N-vinylimidazoles, carboxy vinyls, vinyl esters, vinyl ethers, silicones, polyethyleneoxides, polyethyleneglycols, vinylalcohols, sodium acrylates, acrylates, maleic acids, NN-dimethylacrylamides, diacetone acrylamides, acrylamides, acryloyl morpholine, pluronic, collagens, polyacrylamides, polyacrylates, polyvinyl alcohols, polyvinylenes, polyvinyl silicates, polyacrylates substituted with a sugar (e.g., sucrose, glucose, glucosamines, galactose, trehalose, mannose, or lactose), acylamidopropane sulfonic acids, tetramethoxyorthosilicates, methyltrimethoxyorthosilicates, tetraalkoxyorthosilicates, trialkoxyorthosilicates, glycols, propylene glycol, glycerine, polysaccharides, alginates, dextrans, cyclodextrin, celluloses, modified celluloses, oxidized celluloses, chitosans, chitins, guars, carrageenans, hyaluronic acids, inulin, starches, modified starches, agarose, methylcelluloses, plant gums, hylaronans, hydrogels, gelatins, glycosaminoglycans, carboxymethyl celluloses, hydroxyethyl celluloses, hydroxy propyl methyl celluloses, pectins, low-methoxy pectins, cross-linked dextrans, starch-acrylonitrile graft copolymers, starch sodium polyacrylate, hydroxyethyl methacrylates, hydroxyl ethyl acrylates, polyvinylene, polyethylvinylethers, polymethyl methacrylates, polystyrenes, polyurethanes, polyalkanoates, polylactic acids, polylactates, poly(3-hydroxybutyrate), sulfonated hydrogels, AMPS (2-acrylamido-2-methyl-1-propanesulfonic acid), SEM (sulfoethylmethacrylate), SPM (sulfopropyl methacrylate), SPA (sulfopropyl acrylate), N,N-dimethyl-N-methacryloxyethyl-N-(3-sulfopropyl)ammonium betaine, methacryllic acid amidopropyl-dimethyl ammonium sulfobetaine, SPI (itaconic acid-bis(1-propyl sulfonizacid-3) ester di-potassium salt), itaconic acids, AMBC (3-acrylamido-3-methylbutanoic acid), beta-carboxyethyl acrylate (acrylic acid dimers), and maleic anhydride-methylvinyl ether polymers, derivatives thereof, salts thereof, acids thereof, and combinations thereof. In certain embodiments, pharmaceutical compositions of present disclosure can be administered orally, for example, in the form of capsules, cachets, pills, tablets, lozenges (using a flavored basis such as sucrose and acacia or tragacanth), powders, granules, a solution or a suspension in an aqueous or non-aqueous liquid, an oil-in-water or water-in-oil liquid emulsion, or an elixir or syrup, or pastille (using an inert base, such as gelatin and glycerin, or sucrose and acacia), and/or a mouth wash, each containing a predetermined amount of a compound of the present disclosure and optionally one or more other active ingredients. A compound of the present disclosure and optionally one or more other active ingredients may also be administered as a bolus, electuary, or paste.

In solid dosage forms for oral administration (e.g., capsules, tablets, pills, dragees, powders, and granules), one or more compounds of the present disclosure may be mixed with one or more pharmaceutically acceptable carriers including, for example, sodium citrate, dicalcium phosphate, a filler or extender (*e.g.,* a starch, lactose, sucrose, glucose, mannitol, and silicic acid), a binder (*e.g.* carboxymethylcellulose, an alginate, gelatin, polyvinyl pyrrolidone, sucrose, and acacia), a humectant (*e.g.,* glycerol), a disintegrating agent (*e.g.,* agar-agar, calcium carbonate, potato or tapioca starch, alginic acid, a silicate, and sodium carbonate), a solution retarding agent (*e.g.* paraffin), an absorption accelerator (*e.g.* a quaternary ammonium compound), a wetting agent (*e.g.,* cetyl alcohol and glycerol monostearate), an absorbent (*e.g.,* kaolin and bentonite clay), a lubricant (*e.g.,* a talc, calcium stearate, magnesium stearate, solid polyethylene glycols, sodium lauryl sulfate), a coloring agent, and mixtures thereof. In the case of capsules, tablets, and pills, the pharmaceutical formulation (composition) may also comprise a buffering agent. Solid compositions of a similar type may also be employed as fillers in soft and hard-filled gelatin capsules using one or more excipients including, e.g., lactose or a milk sugar as well as a high molecular-weight polyethylene glycol.

Liquid dosage forms for oral administration of the pharmaceutical composition may include pharmaceutically acceptable emulsions, microemulsions, solutions, suspensions, syrups, and elixirs. In addition to the active ingredient(s), the liquid dosage form may contain an inert diluent commonly used in the art including, for example, water or other solvent, a solubilizing agent and/or emulsifier [*e.g*., ethyl alcohol, isopropyl alcohol, ethyl carbonate, ethyl acetate, benzyl alcohol, benzyl benzoate, propylene glycol, or 1,3-butylene glycol, an oil (*e.g.,* cottonseed, groundnut, corn, germ, olive, castor, and sesame oil), glycerol, tetrahydrofuryl alcohol, a polyethylene glycol, a fatty acid ester of sorbitan, and mixtures thereof]. Besides inert diluents, the oral formulation can also include an adjuvant including, for example, a wetting agent, an emulsifying and suspending agent, a sweetening agent, a flavoring agent, a coloring agent, a perfuming agent, a preservative agent, and combinations thereof.

Suspensions, in addition to the active compounds, may contain suspending agents including, for example, an ethoxylated isostearyl alcohol, polyoxyethylene sorbitol, a sorbitan ester, microcrystalline cellulose, aluminum metahydroxide, bentonite, agar-agar, tragacanth, and combinations thereof.

Prevention of the action and/or growth of microorganisms may be ensured by the inclusion of various antibacterial and antifungal agents including, for example, paraben, chlorobutanol, and phenol sorbic acid.

In certain embodiments, it may be desirable to include an isotonic agent including, for example, a sugar or sodium chloride into the compositions. In addition, prolonged absorption of an injectable pharmaceutical form may be brought about by the inclusion of an agent that delay absorption including, for example, aluminum monostearate and gelatin.

It is understood that the dosage regimen will be determined by the attending physician considering various factors which modify the action of the one or more of the agents of the present disclosure. In the case of a ActRII and/or TGFβ antagonist that promotes red blood cell formation, various factors may include, but are not limited to, the patient's red blood cell count, hemoglobin level, the desired target red blood cell count, the patient's age, the patient's sex, the patient's diet, the severity of any disease that may be contributing to a depressed red blood cell level, the time of administration, and other clinical factors. The addition of other known active agents to the final composition may also affect the dosage. Progress can be monitored by periodic assessment of one or more of red blood cell levels, hemoglobin levels, reticulocyte levels, and other indicators of the hematopoietic process.

In certain embodiments, the present disclosure also provides gene therapy for the *in vivo* production of one or more of the agents of the present disclosure. Such therapy would achieve its therapeutic effect by introduction of the agent sequences into cells or tissues having one or more of the disorders as listed above. Delivery of the agent sequences can be achieved, for example, by using a recombinant expression vector such as a chimeric virus or a colloidal dispersion system. Preferred therapeutic delivery of one or more of agent sequences of the disclosure is the use of targeted liposomes.

Various viral vectors which can be utilized for gene therapy as taught herein include adenovirus, herpes virus, vaccinia, or an RNA virus (*e.g.,* a retrovirus). The retroviral vector may be a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing one or more of the agents of the present disclosure.

Alternatively, tissue culture cells can be directly transfected with plasmids encoding the retroviral structural genes (gag, pol, and env), by conventional calcium phosphate transfection. These cells are then transfected with the vector plasmid containing the genes of interest. The resulting cells release the retroviral vector into the culture medium.

Another targeted delivery system for one or more of the agents of the present disclosure is a colloidal dispersion system. Colloidal dispersion systems include, for example, macromolecule complexes, nanocapsules, microspheres, beads, and lipid-based systems including oil-in-water emulsions, micelles, mixed micelles, and liposomes. In certain embodiments, the preferred colloidal system of this disclosure is a liposome. Liposomes are artificial membrane vesicles which are useful as delivery vehicles *in vitro* and *in vivo.* RNA, DNA, and intact virions can be encapsulated within the aqueous interior and be delivered to cells in a biologically active form [Fraley, et al. (1981) Trends Biochem. Sci., 6:77]. Methods for efficient gene transfer using a liposome vehicle are known in the art [Mannino, et al. (1988) Biotechniques, 6:682, 1988].

The composition of the liposome is usually a combination of phospholipids, which may include a steroid (*e.g.* cholesterol). The physical characteristics of liposomes depend on pH, ionic strength, and the presence of divalent cations. Other phospholipids or other lipids may also be used including, for example a phosphatidyl compound (*e.g.,* phosphatidylglycerol, phosphatidylcholine, phosphatidylserine, phosphatidylethanolamine, a sphingolipid, a cerebroside, and a ganglioside), egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

### EXEMPLIFICATION

The disclosure will be more readily understood by reference to the following examples, which are included merely for purposes of illustration of certain embodiments and embodiments of the present disclosure.

### Example 1: ActRIIa-Fc Fusion Proteins

An ActRIIA fusion protein that has the extracellular domain of human ActRIIa fused to a human or mouse Fc domain with a linker in between was generated. The constructs are referred to as ActRIIA-hFc and ActRIIA-mFc, respectively.

ActRIIA-hFc is shown below as purified from CHO cell lines (SEQ ID NO: 50):

ILGRSETQECLFFNANWEKDRTNQTGVEPCYGDKDKRRHCFATWKNISGSIEI
VKQGCWLDDINCYDRTDCVEKKDSPEVYFCCCEGNMCNEKFSYFPEMEVTQPTSNP
VTPKPPTGGGTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSN
KALPVPIEKTISKAKGQPREPQVYTLPPSREEMTKNQVSLTCLVKGFYPSDIAVEWES

NGQPENNYKTTPPVLDSDGSFFLYSKLTVDKSRWQQGNVFSCSVMHEALHNHYTQK
SLSLSPGK

The ActRIIA-hFc and ActRIIA-mFc proteins were expressed in CHO cell lines. Three different leader sequences were considered:
(i) Honey bee mellitin (HBML): MKFLVNVALVFMVVYISYIYA (SEQ ID NO: 51)
(ii) Tissue plasminogen activator (TPA): MDAMKRGLCCVLLLCGAVFVSP (SEQ ID NO: 52)
(iii) Native: MGAAAKLAFAVFLISCSSGA (SEQ ID NO: 53).

The selected form employs the TPA leader and has the following unprocessed amino acid sequence:

MDAMKRGLCCVLLLCGAVFVSPGAAILGRSETQECLFFNANWEKDRTNQTGVEPCY
GDKDKRRHCFATWKNISGSIEIVKQGCWLDDINCYDRTDCVEKKDSPEVYFCCCEG
NMCNEKFSYFPEMEVTQPTSNPVTPKPPTGGGTHTCPPCPAPELLGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPVPIEKTISKAKGQPREPQVYTLPPSREEMTKN
QVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 54)

This polypeptide is encoded by the following nucleic acid sequence:

ATGGATGCAATGAAGAGAGGGCTCTGCTGTGTGCTGCTGCTGTGTGGAGC
AGTCTTCGTTTCGCCCGGCGCCGCTATACTTGGTAGATCAGAAACTCAGGAGTGT
CTTTTTTTAATGCTAATTGGGAAAAAGACAGAACCAATCAAACTGGTGTTGAACC
GTGTTATGGTGACAAAGATAAACGGCGGCATTGTTTTGCTACCTGGAAGAATATT
TCTGGTTCCATTGAATAGTGAAACAAGGTTGTTGGCTGGATGATATCAACTGCTA
TGACAGGACTGATTGTGTAGAAAAAAAAGACAGCCCTGAAGTATATTTCTGTTGC
TGTGAGGGCAATATGTGTAATGAAAAGTTTTCTTATTTTCCGGAGATGGAAGTCA
CACAGCCCACTTCAAATCCAGTTACACCTAAGCCACCCACCGGTGGTGGAACTCA
CACATGCCCACCGTGCCCAGCACCTGAACTCCTGGGGGGACCGTCAGTCTTCCTC
TTCCCCCCAAAACCCAAGGACACCCTCATGATCTCCCGGACCCCTGAGGTCACAT
GCGTGGTGGTGGACGTGAGCCACGAAGACCCTGAGGTCAAGTTCAACTGGTACG
TGGACGGCGTGGAGGTGCATAATGCCAAGACAAAGCCGCGGGAGGAGCAGTAC
AACAGCACGTACCGTGTGGTCAGCGTCCTCACCGTCCTGCACCAGGACTGGCTGA
ATGGCAAGGAGTACAAGTGCAAGGTCTCCAACAAAGCCCTCCCAGTCCCCATCG
AGAAAACCATCTCCAAAGCCAAAGGGCAGCCCCGAGAACCACAGGTGTACACCC
TGCCCCCATCCCGGGAGGAGATGACCAAGAACCAGGTCAGCCTGACCTGCCTGG

TCAAAGGCTTCTATCCCAGCGACATCGCCGTGGAGTGGGAGAGCAATGGGCAGC
CGGAGAACAACTACAAGACCACGCCTCCCGTGCTGGACTCCGACGGCTCCTTCTT
CCTCTATAGCAAGCTCACCGTGGACAAGAGCAGGTGGCAGCAGGGGAACGTCTT
CTCATGCTCCGTGATGCATGAGGCTCTGCACAACCACTACACGCAGAAGAGCCTC
TCCCTGTCTCCGGGTAAATGAGAATTC (SEQ ID NO: 55)

Both ActRIIA-hFc and ActRIIA-mFc were remarkably amenable to recombinant expression. As shown in Figure 5, the protein was purified as a single, well-defined peak of protein. N-terminal sequencing revealed a single sequence of -ILGRSETQE (SEQ ID NO: 56). Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. The ActRIIA-hFc protein was purified to a purity of >98% as determined by size exclusion chromatography and >95% as determined by SDS PAGE.

ActRIIA-hFc and ActRIIA-mFc showed a high affinity for ligands. GDF-11 or activin A were immobilized on a Biacore^{™} CM5 chip using standard amine-coupling procedure. ActRIIA-hFc and ActRIIA-mFc proteins were loaded onto the system, and binding was measured. ActRIIA-hFc bound to activin with a dissociation constant (K_{D}) of 5 × 10⁻¹² and bound to GDF11 with a K_{D} of 9.96 × 10⁻⁹. See Figure 6. ActRIIA-mFc behaved similarly.

The ActRIIA-hFc was very stable in pharmacokinetic studies. Rats were dosed with 1 mg/kg, 3 mg/kg, or 10 mg/kg of ActRIIA-hFc protein, and plasma levels of the protein were measured at 24, 48, 72, 144 and 168 hours. In a separate study, rats were dosed at 1 mg/kg, 10 mg/kg, or 30 mg/kg. In rats, ActRIIA-hFc had an 11-14 day serum half-life, and circulating levels of the drug were quite high after two weeks (11 µg/ml, 110 µg/ml, or 304 µg/ml for initial administrations of 1 mg/kg, 10 mg/kg, or 30 mg/kg, respectively.) In cynomolgus monkeys, the plasma half-life was substantially greater than 14 days, and circulating levels of the drug were 25 µg/ml, 304 µg/ml, or 1440 µg/ml for initial administrations of 1 mg/kg, 10 mg/kg, or 30 mg/kg, respectively.

A variety of ActRIIA variants that may be used according to the methods described herein are described in the International Patent Application published as WO2006/012627 (see e.g., pp. 55-58), incorporated herein by reference in its entirety. An alternative construct may have a deletion of the C-terminal tail (the final 15 amino acids of the extracellular domain of ActRIIA. The sequence for such a construct is presented below (Fc portion underlined) (SEQ ID NO: 57):

ILGRSETQECLFFNANWEKDRTNQTGVEPCYGDKDKRRHCFATWKNISGSIEIVKQG
CWLDDINCYDRTDCVEKKDSPEVYFCCCEGNMCNEKFSYFPEMTGGGTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPVPIEKTISKAKGQPRE
PQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK

### Example 2: Generation of ActRIIB-Fc fusion proteins

A soluble ActRIIB fusion protein that has the extracellular domain of human ActRIIB fused to a human or mouse Fc domain with a linker in between was constructed. The constructs are referred to as ActRIIB-hFc and ActRIIB-mFc, respectively.

ActRIIB-hFc is shown below as purified from CHO cell lines (SEQ ID NO: 58):

GRGEAETRECIYYNANWELERTNQSGLERCEGEQDKRLHCYASWRNSSGTIELVKK
GCWLDDFNCYDRQECVATEENPQVYFCCCEGNFCNERFTHLPEAGGPEVTYEPPPT
APTGGGTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKAL
PVPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLS
LSPGK

The ActRIIB-hFc and ActRIIB-mFc proteins were expressed in CHO cell lines. Three different leader sequences were considered: (i) Honey bee mellitin (HBML), ii) Tissue plasminogen activator (TPA), and (iii) Native: MGAAAKLAFAVFLISCSSGA (SEQ ID NO: 59).

The selected form employs the TPA leader and has the following unprocessed amino acid sequence (SEQ ID NO: 60):

MDAMKRGLCCVLLLCGAVFVSPGASGRGEAETRECIYYNANWELERTNQSGLERCE
GEQDKRLHCYASWRNSSGTIELVKKGCWLDDFNCYDRQECVATEENPQVYFCCCE
GNFCNERFTHLPEAGGPEVTYEPPPTAPTGGGTHTCPPCPAPELLGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPVPIEKTISKAKGQPREPQVYTLPPSREEMTKNQ
VSLTCLVKGFYPSDIAVEWESNGQPENNYKTTPPVLDSDGSFFLYSKLTVDKSRWQQ
GNVFSCSVMHEALHNHYTQKSLSLSPGK

This polypeptide is encoded by the following nucleic acid sequence (SEQ ID NO: 61):

A TGGATGCAAT GAAGAGAGGG CTCTGCTGTG TGCTGCTGCT GTGTGGAGCA
GTCTTCGTTT CGCCCGGCGC CTCTGGGCGT GGGGAGGCTG AGACACGGGA
GTGCATCTAC TACAACGCCA ACTGGGAGCT GGAGCGCACC AACCAGAGCG
GCCTGGAGCG CTGCGAAGGC GAGCAGGACA AGCGGCTGCA CTGCTACGCC
TCCTGGCGCA ACAGCTCTGG CACCATCGAG CTCGTGAAGA AGGGCTGCTG
GCTAGATGAC TTCAACTGCT ACGATAGGCA GGAGTGTGTG GCCACTGAGG
AGAACCCCCA GGTGTACTTC TGCTGCTGTG AAGGCAACTT CTGCAACGAG
CGCTTCACTC ATTTGCCAGA GGCTGGGGGC CCGGAAGTCA CGTACGAGCC
ACCCCCGACA GCCCCCACCG GTGGTGGAAC TCACACATGC CCACCGTGCC
CAGCACCTGA ACTCCTGGGG GGACCGTCAG TCTTCCTCTT CCCCCCAAAA
CCCAAGGACA CCCTCATGAT CTCCCGGACC CCTGAGGTCA CATGCGTGGT
GGTGGACGTG AGCCACGAAG ACCCTGAGGT CAAGTTCAAC TGGTACGTGG
ACGGCGTGGA GGTGCATAAT GCCAAGACAA AGCCGCGGGA GGAGCAGTAC
AACAGCACGT ACCGTGTGGT CAGCGTCCTC ACCGTCCTGC ACCAGGACTG
GCTGAATGGC AAGGAGTACA AGTGCAAGGT CTCCAACAAA GCCCTCCCAG
TCCCCATCGA GAAAACCATC TCCAAAGCCA AAGGGCAGCC CCGAGAACCA
CAGGTGTACA CCCTGCCCCC ATCCCGGGAG GAGATGACCA AGAACCAGGT
CAGCCTGACC TGCCTGGTCA AAGGCTTCTA TCCCAGCGAC ATCGCCGTGG
AGTGGGAGAG CAATGGGCAG CCGGAGAACA ACTACAAGAC CACGCCTCCC
GTGCTGGACT CCGACGGCTC CTTCTTCCTC TATAGCAAGC TCACCGTGGA
CAAGAGCAGG TGGCAGCAGG GGAACGTCTT CTCATGCTCC GTGATGCATG
AGGCTCTGCA CAACCACTAC ACGCAGAAGA GCCTCTCCCT GTCTCCGGGT
AAATGA

N-terminal sequencing of the CHO-cell-produced material revealed a major sequence of -GRGEAE (SEQ ID NO: 62). Notably, other constructs reported in the literature begin with an -SGR... sequence.

Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange.

A series of mutations were generated in the extracellular domain of ActRIIB and produced these mutant proteins as soluble fusion proteins between extracellular ActRIIB and an Fc domain. The background ActRIIB-Fc fusion has the sequence of SEQ ID NO: 58.

Various mutations, including N- and C-terminal truncations, were introduced into the background ActRIIB-Fc protein. Based on the data presented herien, it is expected that these constructs, if expressed with a TPA leader, will lack the N-terminal serine. Mutations were generated in ActRIIB extracellular domain by PCR mutagenesis. After PCR, fragments were purified through a Qiagen column, digested with SfoI and AgeI and gel purified. These fragments were ligated into expression vector pAID4 (see WO2006/012627) such that upon ligation it created fusion chimera with human IgG1. Upon transformation into E. coli DH5 alpha, colonies were picked and DNAs were isolated. For murine constructs (mFc), a murine IgG2a was substituted for the human IgG1. Sequences of all mutants were verified.
All of the mutants were produced in HEK293T cells by transient transfection. In summary, in a 500ml spinner, HEK293T cells were set up at 6×10⁵ cells/ml in Freestyle (Invitrogen) media in 250ml volume and grown overnight. Next day, these cells were treated with DNA:PEI (1:1) complex at 0.5 ug/ml final DNA concentration. After 4 hrs, 250 ml media was added and cells were grown for 7 days. Conditioned media was harvested by spinning down the cells and concentrated.

Mutants were purified using a variety of techniques, including, for example, a protein A column, and eluted with low pH (3.0) glycine buffer. After neutralization, these were dialyzed against PBS.

Mutants were also produced in CHO cells by similar methodology. Mutants were tested in binding assays and/or bioassays described in WO 2008/097541 and WO 2006/012627 incorporated by reference herein. In some instances, assays were performed with conditioned medium rather than purified proteins. Additional variations of ActRIIB are described in U.S. Patent No. 7,842,663.

An ActRIIB(25-131)-hFc fusion protein, which comprises the human ActRIIB extracellular domain with N-terminal and C-terminal truncations (residues 25-131 of the native protein SEQ ID NO: 1) was fused N-terminally with a TPA leader sequence substituted for the native ActRIIB leader and C-terminally with a human Fc domain via a minimal linker (three glycine residues) (Figure 7; SEQ ID NO: 123). A nucleotide sequence encoding this fusion protein is shown in Figure 8 (SEQ ID NO: 124 coding and SEQ ID NO: 125 complementary strand). The codons were modified and a variant nucleic acid encoding the ActRIIB(25-131)-hFc protein was found that provided substantial improvement in the expression levels of initial transformants (Figure 9; SEQ ID NO: 126 coding and SEQ ID NO: 127 complementary strand).

The mature protein has an amino acid sequence as follows (N-terminus confirmed by N-terminal sequencing)(SEQ ID NO: 63): 
ETRECIYYNA NWELERTNQS GLERCEGEQD KRLHCYASWR NSSGTIELVK
KGCWLDDFNC YDRQECVATE ENPQVYFCCC EGNFCNERFT HLPEAGGPEV
TYEPPPTGGG THTCPPCPAP ELLGGPSVFL FPPKPKDTLM ISRTPEVTCV
VVDVSHEDPE VKFNWYVDGV EVHNAKTKPR EEQYNSTYRV VSVLTVLHQD
WLNGKEYKCK VSNKALPAPI EKTISKAKGQ PREPQVYTLP PSREEMTKNQ
VSLTCLVKGF YPSDIAVEWE SNGQPENNYK TTPPVLDSDG SFFLYSKLTV
DKSRWQQGNV FSCSVMHEAL HNHYTQKSLS LSPGK
 Amino acids 1-107 are derived from ActRIIB.

The expressed molecule was purified using a series of column chromatography steps, including for example, three or more of the following, in any order: Protein A chromatography, Q sepharose chromatography, phenylsepharose chromatography, size exclusion chromatography and cation exchange chromatography. The purification could be completed with viral filtration and buffer exchange.

Affinities of several ligands for ActRIIB(25-131)-hFc and its full-length counterpart ActRIIB(20-134)-hFc were evaluated in vitro with a Biacore^{™} instrument, and the results are summarized in the table below. Kd values were obtained by steady-state affinity fit due to very rapid association and dissociation of the complex, which prevented accurate determination of kₒₙ and k_{off}. ActRIIB(25-131)-hFc bound activin A, activin B, and GDF11 with high affinity.

### Ligand Affinities of ActRIIB-hFc Forms:

| **Fusion Construct** | **Activin A (e-11)** | **Activin B (e-11)** | **GDF11 (e-11)** |
|---|---|---|---|
| ActRIIB(20-134)-hFc | 1.6 | 1.2 | 3.6 |
| ActRIIB(25-131)-hFc | 1.8 | 1.2 | 3.1 |

### Example 3: Generation of a GDF Trap

A GDF trap was constructed as follows. A polypeptide having a modified extracellular domain of ActRIIB (amino acids 20-134 of SEQ ID NO: 1 with an L79D substitution) with greatly reduced activin A binding relative to GDF11 and/or myostatin (as a consequence of a leucine-to-aspartate substitution at position 79 in SEQ ID NO: 1) was fused to a human or mouse Fc domain with a minimal linker (three glycine amino acids) in between. The constructs are referred to as ActRIIB(L79D 20-134)-hFc and ActRIIB(L79D 20-134)-mFc, respectively. Alternative forms with a glutamate rather than an aspartate at position 79 performed similarly (L79E). Alternative forms with an alanine rather than a valine at position 226 with respect to SEQ ID NO: 64, below were also generated and performed equivalently in all respects tested. The aspartate at position 79 (relative to SEQ ID NO: 1, or position 60 relative to SEQ ID NO: 64) is indicated with double underlining below. The valine at position 226 relative to SEQ ID NO: 64 is also indicated by double underlining below.

The GDF trap ActRIIB(L79D 20-134)-hFc is shown below as purified from CHO cell lines (SEQ ID NO: 64).

GRGEAETRECIYYNANWELERTNQSGLERCEGEQDKRLHCYASWRNSSGTIELVKK
GCWDDDFNCYDRQECVATEENPQVYFCCCEGNFCNERFTHLPEAGGPEVTYEPPPT
APTGGGTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKAL
PVPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLS
LSPGK

The ActRIIB-derived portion of the GDF trap has an amino acid sequence set forth below (SEQ ID NO: 65), and that portion could be used as a monomer or as a non-Fc fusion protein as a monomer, dimer, or greater-order complex.

GRGEAETRECIYYNANWELERTNQSGLERCEGEQDKRLHCYASWRNSSGTIELVKK
GCWDDDFNCYDRQECVATEENPQVYFCCCEGNFCNERFTHLPEAGGPEVTYEPPPT
APT (SEQ ID NO: 65)

The GDF trap protein was expressed in CHO cell lines. Three different leader sequences were considered:
(i) Honey bee melittin (HBML), (ii) Tissue plasminogen activator (TPA), and (iii) Native.

The selected form employs the TPA leader and has the following unprocessed amino acid sequence:

MDAMKRGLCCVLLLCGAVFVSPGASGRGEAETRECIYYNANWELERTNQSGLERCE
GEQDKRLHCYASWRNSSGTIELVKKGCWDDDFNCYDRQECVATEENPQVYFCCCE
GNFCNERFTHLPEAGGPEVTYEPPPTAPTGGGTHTCPPCPAPELLGGPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 66)

This polypeptide is encoded by the following nucleic acid sequence (SEQ ID NO: 67):

A TGGATGCAAT GAAGAGAGGG CTCTGCTGTG TGCTGCTGCT GTGTGGAGCA
GTCTTCGTTT CGCCCGGCGC CTCTGGGCGT GGGGAGGCTG AGACACGGGA
GTGCATCTAC TACAACGCCA ACTGGGAGCT GGAGCGCACC AACCAGAGCG
GCCTGGAGCG CTGCGAAGGC GAGCAGGACA AGCGGCTGCA CTGCTACGCC
TCCTGGCGCA ACAGCTCTGG CACCATCGAG CTCGTGAAGA AGGGCTGCTG
GGACGATGAC TTCAACTGCT ACGATAGGCA GGAGTGTGTG GCCACTGAGG
AGAACCCCCA GGTGTACTTC TGCTGCTGTG AAGGCAACTT CTGCAACGAG
CGCTTCACTC ATTTGCCAGA GGCTGGGGGC CCGGAAGTCA CGTACGAGCC
ACCCCCGACA GCCCCCACCG GTGGTGGAAC TCACACATGC CCACCGTGCC
CAGCACCTGA ACTCCTGGGG GGACCGTCAG TCTTCCTCTT CCCCCCAAAA
CCCAAGGACA CCCTCATGAT CTCCCGGACC CCTGAGGTCA CATGCGTGGT
GGTGGACGTG AGCCACGAAG ACCCTGAGGT CAAGTTCAAC TGGTACGTGG
ACGGCGTGGA GGTGCATAAT GCCAAGACAA AGCCGCGGGA GGAGCAGTAC
AACAGCACGT ACCGTGTGGT CAGCGTCCTC ACCGTCCTGC ACCAGGACTG
GCTGAATGGC AAGGAGTACA AGTGCAAGGT CTCCAACAAA GCCCTCCCAG
TCCCCATCGA GAAAACCATC TCCAAAGCCA AAGGGCAGCC CCGAGAACCA
CAGGTGTACA CCCTGCCCCC ATCCCGGGAG GAGATGACCA AGAACCAGGT
CAGCCTGACC TGCCTGGTCA AAGGCTTCTA TCCCAGCGAC ATCGCCGTGG
AGTGGGAGAG CAATGGGCAG CCGGAGAACA ACTACAAGAC CACGCCTCCC
GTGCTGGACT CCGACGGCTC CTTCTTCCTC TATAGCAAGC TCACCGTGGA
CAAGAGCAGG TGGCAGCAGG GGAACGTCTT CTCATGCTCC GTGATGCATG
AGGCTCTGCA CAACCACTAC ACGCAGAAGA GCCTCTCCCT GTCTCCGGGT
AAATGA

Purification could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. In an example of a purification scheme, the cell culture medium is passed over a protein A column, washed in 150 mM Tris/NaCl (pH 8.0), then washed in 50 mM Tris/NaCl (pH 8.0) and eluted with 0.1 M glycine, pH 3.0. The low pH eluate is kept at room temperature for 30 minutes as a viral clearance step. The eluate is then neutralized and passed over a Q-sepharose ion-exchange column and washed in 50 mM Tris pH 8.0, 50 mM NaCl, and eluted in 50 mM Tris pH 8.0, with an NaCl concentration between 150 mM and 300 mM. The eluate is then changed into 50 mM Tris pH 8.0, 1.1 M ammonium sulfate and passed over a phenyl sepharose column, washed, and eluted in 50 mM Tris pH 8.0 with ammonium sulfate between 150 and 300 mM. The eluate is dialyzed and filtered for use.

Additional GDF traps (ActRIIB-Fc fusion proteins modified so as to reduce the ratio of activin A binding relative to myostatin or GDF 11 binding) are described in WO 2008/097541 and WO 2006/012627, incorporated by reference herein.

### Example 4: Bioassay for GDF-11- and Activin-Mediated Signaling

An A-204 reporter gene assay was used to evaluate the effects of ActRIIB-Fc proteins and GDF traps on signaling by GDF-11 and activin A. Cell line: human rhabdomyosarcoma (derived from muscle). Reporter vector: pGL3(CAGA)12 (described in Dennler et al, 1998, EMBO 17: 3091-3100). The CAGA12 motif is present in TGFβ responsive genes (e.g., PAI-1 gene), so this vector is of general use for factors signaling through SMAD2 and 3.

Day 1: Split A-204 cells into 48-well plate.

Day 2: A-204 cells transfected with 10 ug pGL3(CAGA)12 or pGL3(CAGA)12(10 ug) + pRLCMV (1 µg) and Fugene.

Day 3: Add factors (diluted into medium + 0.1 % BSA). Inhibitors need to be preincubated with factors for 1 hr before adding to cells. Six hrs later, cells were rinsed with PBS and lysed.

This is followed by a luciferase assay. In the absence of any inhibitors, activin A showed 10-fold stimulation of reporter gene expression and an ED50 ~ 2 ng/ml. GDF-11: 16 fold stimulation, ED50: ~1.5 ng/ml.

Activity of ActRIIB-Fc proteins and GDF traps was tested in a cell-based assay as described above. Results are summarized in the table below. Some variants were tested in different C-terminal truncation constructs. The GDF traps (L79D and L79E variants) showed substantial loss of activin A inhibition while retaining almost wild-type inhibition of GDF-11.

### Soluble ActRIIB-Fc binding to GDF11 and Activin A:

| ActRIIB-Fc Variations | Portion of ActRIIB (corresponds to amino acids of SEQ ID NO: 1) | GDF11 Inhibition Activity | Activin Inhibition Activity |
|---|---|---|---|
| R64 | 20-134 | +++ (approx. 10⁻⁸ M K_{I}) | +++ (approx. 10⁻⁸ M K_{I}) |
| A64 | 20-134 | + (approx. 10⁻⁶ M K_{I}) | + (approx. 10⁻⁶ M K_{I}) |
| R64 | 20-129 | +++ | +++ |
| R64 K74A | 20-134 | ++++ | ++++ |
| R64 A24N | 20-134 | +++ | +++ |
| R64 A24N | 20-119 | ++ | ++ |
| R64 A24N K74A | 20-119 | + | + |
| R64 L79P | 20-134 | + | + |
| R64 L79P K74A | 20-134 | + | + |
| R64 L79D | 20-134 | +++ | + |
| R64 L79E | 20-134 | +++ | + |
| R64K | 20-134 | +++ | +++ |
| R64K | 20-129 | +++ | +++ |
| R64 P129S P130A | 20-134 | +++ | +++ |
| R64N | 20-134 | + | + |

| | | | |
|---|---|---|---|
| + Poor activity (roughly 1×10⁻⁶ K_{I}) ++ Moderate activity (roughly 1×10⁻⁷ K_{I}) +++ Good (wild-type) activity (roughly 1×10⁻⁸ K_{I}) ++++ Greater than wild-type activity | | | |

### Example 5: Generation of a GDF Trap with Truncated ActRIIB Extracellular Domain

A GDF trap with truncated ActRIIB extracellular domain, referred to as ActRIIB(L79D 25-131)-hFc, was generated by N-terminal fusion of TPA leader to truncated extracellular domain (residues 25-131 in SEQ ID NO:1) containing a leucine-to-aspartate substitution (at residue 79 in SEQ ID NO:1) and C-terminal fusion of human Fc domain with a linker (three glycine residues) (Figure 12, SEQ ID NO: 131). The sequence of the cell purified form of ActRIIB(L79D 25-131)-hFc is presented in Figure 13 (SEQ ID NO: 132) and the mature extracellular domain without the leader, linker or Fc domain is presented in Figure 14 (SEQ ID NO: 133). One nucleotide sequence encoding this fusion protein is shown in Figure 15 (SEQ ID NO: 134) along with its complementary sequence (SEQ ID NO: 135), and an alternative nucleotide sequence encoding exactly the same fusion protein is shown in Figure 16 (SEQ ID NO: 136) and its complementary sequence (SEQ ID NO: 137).

### Example 6: Selective Ligand Binding by GDF Trap with Double-Truncated ActRIIB Extracelluar Domain

The affinity of GDF traps and other ActRIIB-hFc proteins for several ligands was evaluated *in vitro* with a Biacore^{™} instrument. Results are summarized in the table below. Kd values were obtained by steady-state affinity fit due to the very rapid association and dissociation of the complex, which prevented accurate determination of kₒₙ and k_{off}.

### Ligand Selectivity of ActRIIB-hFc Variants:

| **Fusion Construct** | **Activin A (Kd e-11)** | **Activin B (Kd e-11)** | **GDF11 (Kd e-11)** |
|---|---|---|---|
| ActRIIB(L79 20-134)-hFc | 1.6 | 1.2 | 3.6 |
| ActRIIB(L79D 20-134)-hFc | 1350.0 | 78.8 | 12.3 |
| ActRIIB(L79 25-131)-hFc | 1.8 | 1.2 | 3.1 |
| ActRIIB(L79D 25-131)-hFc | 2290.0 | 62.1 | 7.4 |

The GDF trap with a truncated extracellular domain, ActRIIB(L79D 25-131)-hFc, equaled or surpassed the ligand selectivity displayed by the longer variant, ActRIIB(L79D 20-134)-hFc, with pronounced loss of activin A binding, partial loss of activin B binding, and nearly full retention of GDF11 binding compared to ActRIIB-hFc counterparts lacking the L79D substitution. Note that truncation alone (without L79D substitution) did not alter selectivity among the ligands displayed here [compare ActRIIB(L79 25-13 1)-hFc with ActRIIB(L79 20-134)-hFc]. ActRIIB(L79D 25-131)-hFc also retains strong to intermediate binding to the Smad 2/3 signaling ligand GDF8 and the Smad 1/5/8 ligands BMP6 and BMP10.

### Example 7: GDF Trap Derived from ActRIIB5

Others have reported an alternate, soluble form of ActRIIB (designated ActRIIB5), in which exon 4, including the ActRIIB transmembrane domain, has been replaced by a different C-terminal sequence (see, *e.g.,* WO 2007/053775).

The sequence of native human ActRIIB5 without its leader is as follows:

GRGEAETRECIYYNANWELERTNQSGLERCEGEQDKRLHCYASWRNSSGTIELVK

KGCWLDDFNCYDRQECVATEENPQVYFCCCEGNFCNERFTHLPEAGGPEGPWAST

TIPSGGPEATAAAGDQGSALWLCLEGPAHE (SEQ ID NO: 68)

An leucine-to-aspartate substitution, or other acidic substitutions, may be performed at native position 79 (underlined) as described to construct the variant ActRIIB5(L79D), which has the following sequence:

GRGEAETRECIYYNANWELERTNQSGLERCEGEQDKRLHCYASWRNSSGTIELVK

KGCWDDDFNCYDRQECVATEENPQVYFCCCEGNFCNERFTHLPEAGGPEGPWAST

TIPSGGPEATAAAGDQGSGALWLCLEGPAHE (SEQ ID NO: 69)

This variant may be connected to human Fc (double underline) with a TGGG linker (single underline) to generate a human ActRIIB5(L79D)-hFc fusion protein with the following sequence:

GRGEAETRECIYYNANWELERTNQSGLERCEGEQDKRLHCYASWRNSSGTIELVK

KGCWDDDFNCYDRQECVATEENPQVYFCCCEGNFCNERFTHLPEAGGPEGPWAST

TIPSGGPEATAAAGDQGSGALWLCLEGPAHETGGGTHTCPPCPAPELLGGPSVFL

FPPKPKDTLMISRTPEVTCVVVDVSHEDPEVKFNWYVDGVEVHNAKTKPREEQYN

STYRVVSVLTVLHQDWLNGKEYKCKVSNKALPAPIEKTISKAKGQPREPQVYTLP

PSREEMTKNQVSLTCLVKGFYPSDIAVEWESNGQPENNYKTTPPVLDSDGSFFLY

SKLTVDKSRWQQGNVFSCSVMHEALHNHYTQKSLSLSPGK (SEQ ID NO: 70).

This construct may be expressed in CHO cells.

### Example 8: Generation of an ALK4:ActRIIB heterodimer

An ALK4-Fc:ActRIIB-Fc heteromeric complex was constructed comprising the extracellular domains of human ActRIIB and human ALK4, which are each separately fused to an Fc domain with a linker positioned between the extracellular domain and the Fc domain. The individual constructs are referred to as ActRIIB-Fc fusion polypeptide and ALK4-Fc fusion polypeptide, respectively, and the sequences for each are provided below.

A methodology for promoting formation of ALK4-Fc:ActRIIB-Fc heteromeric complexes, as opposed to ActRIIB-Fc or ALK4-Fc homodimeric complexes, is to introduce alterations in the amino acid sequence of the Fc domains to guide the formation of asymmetric heteromeric complexes. Many different approaches to making asymmetric interaction pairs using Fc domains are described in this disclosure.

In one approach, illustrated in the ActRIIB-Fc and ALK4-Fc polypeptide sequences of SEQ ID NOs: 71 and 73 and SEQ ID Nos: 74 and 76, respectively, one Fc domain is altered to introduce cationic amino acids at the interaction face, while the other Fc domain is altered to introduce anionic amino acids at the interaction face. ActRIIB-Fc fusion polypeptide and ALK4-Fc fusion polypeptide each employ the tissue plasminogen activator (TPA) leader.

The ActRIIB-Fc polypeptide sequence (SEQ ID NO: 71) is shown below:

1 MDAMKRGLCC VLLLCGAVFV SPGASGRGEA ETRECIYYNA NWELERTNQS

51 GLERCEGEQD KRLHCYASWR NSSGTIELVK KGCWLDDFNC YDRQECVATE

101 ENPQVYFCCC EGNFCNERFT HLPEAGGPEV TYEPPPTAPT GGGTHTCPPC

151 PAPELLGGPS VFLFPPKPKD TLMISRTPEV TCVVVDVSHE DPEVKFNWYV

201 DGVEVHNAKT KPREEQYNST YRVVSVLTVL HQDWLNGKEY KCKVSNKALP

251 APIEKTISKA KGQPREPQVY TLPPSRKEMT KNQVSLTCLV KGFYPSDIAV

301 EWESNGQPEN NYKTTPPVLK SDGSFFLYSK LTVDKSRWQQ GNVFSCSVMH

351 EALHNHYTQK SLSLSPGK (SEQ ID NO: 71)

The leader (signal) sequence and linker are underlined. To promote formation of ALK4-Fc:ActRIIB-Fc heterodimer rather than either of the possible homodimeric complexes, two amino acid substitutions (replacing acidic amino acids with lysine) can be introduced into the Fc domain of the ActRIIB fusion protein as indicated by double underline above. The amino acid sequence of SEQ ID NO: 71 may optionally be provided with lysine (K) removed from the C-terminus.

This ActRIIB-Fc fusion protein is encoded by the following nucleic acid sequence (SEQ ID NO: 72):

1 ATGGATGCAA TGAAGAGAGG GCTCTGCTGT GTGCTGCTGC TGTGTGGAGC

51 AGTCTTCGTT TCGCCCGGCG CCTCTGGGCG TGGGGAGGCT GAGACACGGG

101 AGTGCATCTA CTACAACGCC AACTGGGAGC TGGAGCGCAC CAACCAGAGC

151 GGCCTGGAGC GCTGCGAAGG CGAGCAGGAC AAGCGGCTGC ACTGCTACGC

201 CTCCTGGCGC AACAGCTCTG GCACCATCGA GCTCGTGAAG AAGGGCTGCT

251 GGCTAGATGA CTTCAACTGC TACGATAGGC AGGAGTGTGT GGCCACTGAG

301 GAGAACCCCC AGGTGTACTT CTGCTGCTGT GAAGGCAACT TCTGCAACGA

351 GCGCTTCACT CATTTGCCAG AGGCTGGGGG CCCGGAAGTC ACGTACGAGC

401 CACCCCCGAC AGCCCCCACC GGTGGTGGAA CTCACACATG CCCACCGTGC

451 CCAGCACCTG AACTCCTGGG GGGACCGTCA GTCTTCCTCT TCCCCCCAAA

501 ACCCAAGGAC ACCCTCATGA TCTCCCGGAC CCCTGAGGTC ACATGCGTGG

551 TGGTGGACGT GAGCCACGAA GACCCTGAGG TCAAGTTCAA CTGGTACGTG

601 GACGGCGTGG AGGTGCATAA TGCCAAGACA AAGCCGCGGG AGGAGCAGTA

651 CAACAGCACG TACCGTGTGG TCAGCGTCCT CACCGTCCTG CACCAGGACT

701 GGCTGAATGG CAAGGAGTAC AAGTGCAAGG TCTCCAACAA AGCCCTCCCA

751 GCCCCCATCG AGAAAACCAT CTCCAAAGCC AAAGGGCAGC CCCGAGAACC

801 ACAGGTGTAC ACCCTGCCCC CATCCCGGAA GGAGATGACC AAGAACCAGG

851 TCAGCCTGAC CTGCCTGGTC AAAGGCTTCT ATCCCAGCGA CATCGCCGTG

901 GAGTGGGAGA GCAATGGGCA GCCGGAGAAC AACTACAAGA CCACGCCTCC

951 CGTGCTGAAG TCCGACGGCT CCTTCTTCCT CTATAGCAAG CTCACCGTGG

1001 ACAAGAGCAG GTGGCAGCAG GGGAACGTCT TCTCATGCTC CGTGATGCAT

1051 GAGGCTCTGC ACAACCACTA CACGCAGAAG AGCCTCTCCC TGTCTCCGGG

1101 TAAA (SEQ ID NO: 72)

A mature ActRIIB-Fc fusion polypeptide (SEQ ID NO: 73) is as follows, and may optionally be provided with lysine (K) removed from the C-terminus.

1   GRGEAETREC IYYNANWELE RTNQSGLERC EGEQDKRLHC YASWRNSSGT

51  IELVKKGCWL DDFNCYDRQE CVATEENPQV YFCCCEGNFC NERFTHLPEA

101 GGPEVTYEPP PTAPTGGGTH TCPPCPAPEL LGGPSVFLFP PKPKDTLMIS

151 RTPEVTCVVV DVSHEDPEVK FNWYVDGVEV HNAKTKPREE QYNSTYRVVS

201 VLTVLHQDWL NGKEYKCKVS NKALPAPIEK TISKAKGQPR EPQVYTLPPS

251 RKEMTKNQVS LTCLVKGFYP SDIAVEWESN GQPENNYKTT PPVLKSDGSF

301 FLYSKLTVDK SRWQQGNVFS CSVMHEALHN HYTQKSLSLS PGK

(SEQ ID NO: 73)

A complementary form of ALK4-Fc fusion polypeptide (SEQ ID NO: 74) is as follows:

1   MDAMKRGLCC VLLLCGAVFV SPGASGPRGV QALLCACTSC LQANYTCETD

51   GACMVSIFNL DGMEHHVRTC IPKVELVPAG KPFYCLSSED LRNTHCCYTD

101  YCNRIDLRVP SGHLKEPEHP SMWGPVETGG GTHTCPPCPA PELLGGPSVF

151  LFPPKPKDTL MISRTPEVTC VVVDVSHEDP EVKFNWYVDG VEVHNAKTKP

201  REEQYNSTYR VVSVLTVLHQ DWLNGKEYKC KVSNKALPAP IEKTISKAKG

251  QPREPQVYTL PPSREEMTKN QVSLTCLVKG FYPSDIAVEW ESNGQPENNY

301  DTTPPVLDSD GSFFLYSDLT VDKSRWQQGN VFSCSVMHEA LHNHYTQKSL

351  SLSPG     (SEQ ID NO: 74)

The leader sequence and linker are underlined. To guide heterodimer formation with the ActRIIB-Fc fusion polypeptide of SEQ ID NOs: 71 and 73 above, two amino acid substitutions (replacing lysines with aspartic acids) can be introduced into the Fc domain of the ALK4-Fc fusion polypeptide as indicated by double underline above. The amino acid sequence of SEQ ID NO: 74 may optionally be provided with lysine (K) added at the C-terminus.

This ALK4-Fc fusion protein is encoded by the following nucleic acid (SEQ ID NO: 75):

1 ATGGATGCAA TGAAGAGAGG GCTCTGCTGT GTGCTGCTGC TGTGTGGAGC

51 AGTCTTCGTT TCGCCCGGCG CCTCCGGGCC CCGGGGGGTC CAGGCTCTGC

101 TGTGTGCGTG CACCAGCTGC CTCCAGGCCA ACTACACGTG TGAGACAGAT

151 GGGGCCTGCA TGGTTTCCAT TTTCAATCTG GATGGGATGG AGCACCATGT

201 GCGCACCTGC ATCCCCAAAG TGGAGCTGGT CCCTGCCGGG AAGCCCTTCT

251 ACTGCCTGAG CTCGGAGGAC CTGCGCAACA CCCACTGCTG CTACACTGAC

301 TACTGCAACA GGATCGACTT GAGGGTGCCC AGTGGTCACC TCAAGGAGCC

351 TGAGCACCCG TCCATGTGGG GCCCGGTGGA GACCGGTGGT GGAACTCACA

401 CATGCCCACC GTGCCCAGCA CCTGAACTCC TGGGGGGACC GTCAGTCTTC

451 CTCTTCCCCC CAAAACCCAA GGACACCCTC ATGATCTCCC GGACCCCTGA

501 GGTCACATGC GTGGTGGTGG ACGTGAGCCA CGAAGACCCT GAGGTCAAGT

551 TCAACTGGTA CGTGGACGGC GTGGAGGTGC ATAATGCCAA GACAAAGCCG

601 CGGGAGGAGC AGTACAACAG CACGTACCGT GTGGTCAGCG TCCTCACCGT

651 CCTGCACCAG GACTGGCTGA ATGGCAAGGA GTACAAGTGC AAGGTCTCCA

701 ACAAAGCCCT CCCAGCCCCC ATCGAGAAAA CCATCTCCAA AGCCAAAGGG

751 CAGCCCCGAG AACCACAGGT GTACACCCTG CCCCCATCCC GGGAGGAGAT

801 GACCAAGAAC CAGGTCAGCC TGACCTGCCT GGTCAAAGGC TTCTATCCCA

851 GCGACATCGC CGTGGAGTGG GAGAGCAATG GGCAGCCGGA GAACAACTAC

901 GACACCACGC CTCCCGTGCT GGACTCCGAC GGCTCCTTCT TCCTCTATAG

951 CGACCTCACC GTGGACAAGA GCAGGTGGCA GCAGGGGAAC GTCTTCTCAT

1001 GCTCCGTGAT GCATGAGGCT CTGCACAACC ACTACACGCA GAAGAGCCTC

1051 TCCCTGTCTC CGGGT (SEQ ID NO: 75)

A mature ALK4-Fc fusion protein sequence (SEQ ID NO: 76) is as follows and may optionally be provided with lysine (K) added at the C-terminus.

1 SGPRGVQALL CACTSCLQAN YTCETDGACM VSIFNLDGME HHVRTCIPKV

51 ELVPAGKPFY CLSSEDLRNT HCCYTDYCNR IDLRVPSGHL KEPEHPSMWG

101 PVETGGGTHT CPPCPAPELL GGPSVFLFPP KPKDTLMISR TPEVTCVVVD

151 VSHEDPEVKF NWYVDGVEVH NAKTKPREEQ YNSTYRVVSV LTVLHQDWLN

201 GKEYKCKVSN KALPAPIEKT ISKAKGQPRE PQVYTLPPSR EEMTKNQVSL

251 TCLVKGFYPS DIAVEWESNG QPENNYDTTP PVLDSDGSFF LYSDLTVDKS

301 RWQQGNVFSC SVMHEALHNH YTQKSLSLSP G (SEQ ID NO: 76)

The ActRIIB-Fc and ALK4-Fc proteins of SEQ ID NO: 73 and SEQ ID NO: 76, respectively, may be co-expressed and purified from a CHO cell line, to give rise to a heteromeric complex comprising ALK4-Fc:ActRIIB-Fc.

In another approach to promote the formation of heteromultimer complexes using asymmetric Fc fusion proteins the Fc domains are altered to introduce complementary hydrophobic interactions and an additional intermolecular disulfide bond as illustrated in the ActRIIB-Fc and ALK4-Fc polypeptide sequences of SEQ ID NOs: 77 and 78 and SEQ ID Nos: 79 and 80, respectively. The ActRIIB-Fc fusion polypeptide and ALK4-Fc fusion polypeptide each employ the tissue plasminogen activator (TPA) leader.

The ActRIIB-Fc polypeptide sequence (SEQ ID NO: 77) is shown below:

1    MDAMKRGLCC VLLLCGAVFV SPGASGRGEA ETRECIYYNA NWELERTNQS

51   GLERCEGEQD KRLHCYASWR NSSGTIELVK KGCWLDDFNC YDRQECVATE

101  ENPQVYFCCC EGNFCNERFT HLPEAGGPEV TYEPPPTAPT GGGTHTCPPC

151  PAPELLGGPS VFLFPPKPKD TLMISRTPEV TCVVVDVSHE DPEVKFNWYV

201  DGVEVHNAKT KPREEQYNST YRVVSVLTVL HQDWLNGKEY KCKVSNKALP

251  APIEKTISKA KGQPREPQVY TLPPCREEMT KNQVSLWCLV KGFYPSDIAV

301  EWESNGQPEN NYKTTPPVLD SDGSFFLYSK LTVDKSRWQQ GNVFSCSVMH

351  EALHNHYTQK SLSLSPGK          (SEQ ID NO: 77)

The leader (signal) sequence and linker are underlined. To promote formation of the ALK4-Fc:ActRIIB-Fc heterodimer rather than either of the possible homodimeric complexes, two amino acid substitutions (replacing a serine with a cysteine and a threonine with a trytophan) can be introduced into the Fc domain of the fusion protein as indicated by double underline above. The amino acid sequence of SEQ ID NO: 77 may optionally be provided with lysine (K) removed from the C-terminus.

A mature ActRIIB-Fc fusion polypeptide is as follows:

1 GRGEAETREC IYYNANWELE RTNQSGLERC EGEQDKRLHC YASWRNSSGT

51 IELVKKGCWL DDFNCYDRQE CVATEENPQV YFCCCEGNFC NERFTHLPEA

101 GGPEVTYEPP PTAPTGGGTH TCPPCPAPEL LGGPSVFLFP PKPKDTLMIS

151 RTPEVTCVVV DVSHEDPEVK FNWYVDGVEV HNAKTKPREE QYNSTYRVVS

201 VLTVLHQDWL NGKEYKCKVS NKALPAPIEK TISKAKGQPR EPQVYTLPPC

251 REEMTKNQVS LWCLVKGFYP SDIAVEWESN GQPENNYKTT PPVLDSDGSF

301 FLYSKLTVDK SRWQQGNVFS CSVMHEALHN HYTQKSLSLS PGK

(SEQ ID NO: 78)

A complementary form of ALK4-Fc fusion polypeptide (SEQ ID NO: 79) is as follows and may optionally be provided with lysine (K) removed from the C-terminus.

1    MDAMKRGLCC VLLLCGAVFV SPGASGPRGV QALLCACTSC LQANYTCETD

51   GACMVSIFNL DGMEHHVRTC IPKVELVPAG KPFYCLSSED LRNTHCCYTD

101  YCNRIDLRVP SGHLKEPEHP SMWGPVETGG GTHTCPPCPA PELLGGPSVF

151  LFPPKPKDTL MISRTPEVTC VVVDVSHEDP EVKFNWYVDG VEVHNAKTKP

201  REEQYNSTYR VVSVLTVLHQ DWLNGKEYKC KVSNKALPAP IEKTISKAKG

251  QPREPQVCTL PPSREEMTKN QVSLSCAVKG FYPSDIAVEW ESNGQPENNY

301  KTTPPVLDSD GSFFLVSKLT VDKSRWQQGN VFSCSVMHEA LHNHYTQKSL

351  SLSPGK    (SEQ ID NO: 79)

The leader sequence and the linker are underlined. To guide heterodimer formation with the ActRIIB-Fc fusion polypeptide of SEQ ID NOs: 77 and 78 above, four amino acid substitutions can be introduced into the Fc domain of the ALK4 fusion polypeptide as indicated by double underline above. The amino acid sequence of SEQ ID NO: 79 may optionally be provided with lysine (K) removed from the C-terminus.

A mature ALK4-Fc fusion protein sequence is as follows and may optionally be provided with lysine (K) removed from the C-terminus.

1 SGPRGVQALL CACTSCLQAN YTCETDGACM VSIFNLDGME HHVRTCIPKV

51 ELVPAGKPFY CLSSEDLRNT HCCYTDYCNR IDLRVPSGHL KEPEHPSMWG

101 PVETGGGTHT CPPCPAPELL GGPSVFLFPP KPKDTLMISR TPEVTCVVVD

151 VSHEDPEVKF NWYVDGVEVH NAKTKPREEQ YNSTYRVVSV LTVLHQDWLN

201 GKEYKCKVSN KALPAPIEKT ISKAKGQPRE PQVCTLPPSR EEMTKNQVSL

251 SCAVKGFYPS DIAVEWESNG QPENNYKTTP PVLDSDGSFF LVSKLTVDKS

301 RWQQGNVFSC SVMHEALHNH YTQKSLSLSP GK (SEQ ID NO: 80)

ActRIIB-Fc and ALK4-Fc proteins of SEQ ID NO: 78 and SEQ ID NO: 80 respectively, may be co-expressed and purified from a CHO cell line, to give rise to a heteromeric complex comprising ALK4-Fc:ActRIIB-Fc.

Purification of various ALK4-Fc:ActRIIB-Fc complexes could be achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange.

In another approach to promote the formation of heteromultimer complexes using asymmetric Fc fusion proteins, the Fc domains are altered to introduce complementary hydrophobic interactions, an additional intermolecular disulfide bond, and electrostatic differences between the two Fc domains for facilitating purification based on net molecular charge, as illustrated in the ActRIIB-Fc and ALK4-Fc polypeptide sequences of SEQ ID NOs: 139-142 and 143-146, respectively. The ActRIIB-Fc fusion polypeptide and ALK4-Fc fusion polypeptide each employ the tissue plasminogen activator (TPA) leader) .

The ActRIIB-Fc polypeptide sequence (SEQ ID NO: 139) is shown below:

1 MDAMKRGLCC VLLLCGAVFV SPGASGRGEA ETRECIYYNA NWELERTNQS
51 GLERCEGEQD KRLHCYASWR NSSGTIELVK KGCWLDDFNC YDRQECVATE
101 ENPQVYFCCC EGNFCNERFT HLPEAGGPEV TYEPPPTAPT GGGTHTCPPC
151 PAPELLGGPS VFLFPPKPKD TLMISRTPEV TCVVVDVSHE DPEVKFNWYV
201 DGVEVHNAKT KPREEQYNST YRVVSVLTVL HQDWLNGKEY KCKVSNKALP
251 APIEKTISKA KGQPREPQVY TLPPCREEMT ENQVSLWCLV KGFYPSDIAV
301 EWESNGQPEN NYKTTPPVLD SDGSFFLYSK LTVDKSRWQQ GNVFSCSVMH
351 EALHNHYTQD SLSLSPG (SEQ ID NO: 139)

The leader sequence and linker are underlined. To promote formation of the ALK4-Fc:ActRIIB-Fc heterodimer rather than either of the possible homodimeric complexes, two amino acid substitutions (replacing a serine with a cysteine and a threonine with a trytophan) can be introduced into the Fc domain of the fusion protein as indicated by double underline above. To facilitate purification of the ALK4-Fc:ActRIIB-Fc heterodimer, two amino acid substitutions (replacing lysines with acidic amino acids) can also be introduced into the Fc domain of the fusion protein as indicated by double underline above. The amino acid sequence of SEQ ID NO: 139 may optionally be provided with a lysine added at the C-terminus.

This ActRIIB-Fc fusion protein is encoded by the following nucleic acid (SEQ ID NO: 140):

1 ATGGATGCAA TGAAGAGAGG GCTCTGCTGT GTGCTGCTGC TGTGTGGAGC
51 AGTCTTCGTT TCGCCCGGCG CCTCTGGGCG TGGGGAGGCT GAGACACGGG
101 AGTGCATCTA CTACAACGCC AACTGGGAGC TGGAGCGCAC CAACCAGAGC
151 GGCCTGGAGC GCTGCGAAGG CGAGCAGGAC AAGCGGCTGC ACTGCTACGC
201 CTCCTGGCGC AACAGCTCTG GCACCATCGA GCTCGTGAAG AAGGGCTGCT
251 GGCTAGATGA CTTCAACTGC TACGATAGGC AGGAGTGTGT GGCCACTGAG
301 GAGAACCCCC AGGTGTACTT CTGCTGCTGT GAAGGCAACT TCTGCAACGA
351 GCGCTTCACT CATTTGCCAG AGGCTGGGGG CCCGGAAGTC ACGTACGAGC
401 CACCCCCGAC AGCCCCCACC GGTGGTGGAA CTCACACATG CCCACCGTGC
451 CCAGCACCTG AACTCCTGGG GGGACCGTCA GTCTTCCTCT TCCCCCCAAA
501 ACCCAAGGAC ACCCTCATGA TCTCCCGGAC CCCTGAGGTC ACATGCGTGG
551 TGGTGGACGT GAGCCACGAA GACCCTGAGG TCAAGTTCAA CTGGTACGTG
601 GACGGCGTGG AGGTGCATAA TGCCAAGACA AAGCCGCGGG AGGAGCAGTA
651 CAACAGCACG TACCGTGTGG TCAGCGTCCT CACCGTCCTG CACCAGGACT
701 GGCTGAATGG CAAGGAGTAC AAGTGCAAGG TCTCCAACAA AGCCCTCCCA
751 GCCCCCATCG AGAAAACCAT CTCCAAAGCC AAAGGGCAGC CCCGAGAACC
801 ACAGGTGTAC ACCCTGCCCC CATGCCGGGA GGAGATGACC GAGAACCAGG
851 TCAGCCTGTG GTGCCTGGTC AAAGGCTTCT ATCCCAGCGA CATCGCCGTG
901 GAGTGGGAGA GCAATGGGCA GCCGGAGAAC AACTACAAGA CCACGCCTCC
951 CGTGCTGGAC TCCGACGGCT CCTTCTTCCT CTATAGCAAG CTCACCGTGG
1001 ACAAGAGCAG GTGGCAGCAG GGGAACGTCT TCTCATGCTC CGTGATGCAT
1051 GAGGCTCTGC ACAACCACTA CACGCAGGAC AGCCTCTCCC TGTCTCCGGG

1101  T        (SEQ ID NO: 140)

The mature ActRIIB-Fc fusion polypeptide is as follows (SEQ ID NO: 141) and may optionally be provided with a lysine added to the C-terminus.

1 GRGEAETREC IYYNANWELE RTNQSGLERC EGEQDKRLHC YASWRNSSGT
51 IELVKKGCWL DDFNCYDRQE CVATEENPQV YFCCCEGNFC NERFTHLPEA
101 GGPEVTYEPP PTAPTGGGTH TCPPCPAPEL LGGPSVFLFP PKPKDTLMIS
151 RTPEVTCVVV DVSHEDPEVK FNWYVDGVEV HNAKTKPREE QYNSTYRVVS
201 VLTVLHQDWL NGKEYKCKVS NKALPAPIEK TISKAKGQPR EPQVYTLPPC
251 REEMTENQVS LWCLVKGFYP SDIAVEWESN GQPENNYKTT PPVLDSDGSF
301 FLYSKLTVDK SRWQQGNVFS CSVMHEALHN HYTQDSLSLS PG
(SEQ ID NO: 141)

This ActRIIB-Fc fusion polypeptide is encoded by the following nucleic acid (SEQ ID NO: 142):

1 GGGCGTGGGG AGGCTGAGAC ACGGGAGTGC ATCTACTACA ACGCCAACTG
51 GGAGCTGGAG CGCACCAACC AGAGCGGCCT GGAGCGCTGC GAAGGCGAGC
101 AGGACAAGCG GCTGCACTGC TACGCCTCCT GGCGCAACAG CTCTGGCACC
151 ATCGAGCTCG TGAAGAAGGG CTGCTGGCTA GATGACTTCA ACTGCTACGA
201 TAGGCAGGAG TGTGTGGCCA CTGAGGAGAA CCCCCAGGTG TACTTCTGCT
251 GCTGTGAAGG CAACTTCTGC AACGAGCGCT TCACTCATTT GCCAGAGGCT
301 GGGGGCCCGG AAGTCACGTA CGAGCCACCC CCGACAGCCC CCACCGGTGG
351 TGGAACTCAC ACATGCCCAC CGTGCCCAGC ACCTGAACTC CTGGGGGGAC
401 CGTCAGTCTT CCTCTTCCCC CCAAAACCCA AGGACACCCT CATGATCTCC
451 CGGACCCCTG AGGTCACATG CGTGGTGGTG GACGTGAGCC ACGAAGACCC
501 TGAGGTCAAG TTCAACTGGT ACGTGGACGG CGTGGAGGTG CATAATGCCA
551 AGACAAAGCC GCGGGAGGAG CAGTACAACA GCACGTACCG TGTGGTCAGC
601 GTCCTCACCG TCCTGCACCA GGACTGGCTG AATGGCAAGG AGTACAAGTG
651 CAAGGTCTCC AACAAAGCCC TCCCAGCCCC CATCGAGAAA ACCATCTCCA
701 AAGCCAAAGG GCAGCCCCGA GAACCACAGG TGTACACCCT GCCCCCATGC
751 CGGGAGGAGA TGACCGAGAA CCAGGTCAGC CTGTGGTGCC TGGTCAAAGG
801 CTTCTATCCC AGCGACATCG CCGTGGAGTG GGAGAGCAAT GGGCAGCCGG
851 AGAACAACTA CAAGACCACG CCTCCCGTGC TGGACTCCGA CGGCTCCTTC
901 TTCCTCTATA GCAAGCTCAC CGTGGACAAG AGCAGGTGGC AGCAGGGGAA
951 CGTCTTCTCA TGCTCCGTGA TGCATGAGGC TCTGCACAAC CACTACACGC

1001 AGGACAGCCT CTCCCTGTCT CCGGGT (SEQ ID NO: 142)

The complementary form of ALK4-Fc fusion polypeptide (SEQ ID NO: 143) is as follows and may optionally be provided with lysine removed from the C-terminus.

1 MDAMKRGLCC VLLLCGAVFV SPGASGPRGV QALLCACTSC LQANYTCETD
51 GACMVSIFNL DGMEHHVRTC IPKVELVPAG KPFYCLSSED LRNTHCCYTD
101 YCNRIDLRVP SGHLKEPEHP SMWGPVETGG GTHTCPPCPA PELLGGPSVF
151 LFPPKPKDTL MISRTPEVTC VVVDVSHEDP EVKFNWYVDG VEVHNAKTKP
201 REEQYNSTYR VVSVLTVLHQ DWLNGKEYKC KVSNKALPAP IEKTISKAKG
251 QPREPQVCTL PPSREEMTKN QVSLSCAVKG FYPSDIAVEW ESRGQPENNY
301 KTTPPVLDSR GSFFLVSKLT VDKSRWQQGN VFSCSVMHEA LHNHYTQKSL
351 SLSPGK (SEQ ID NO: 143)

The leader sequence and the linker are underlined. To guide heterodimer formation with the ActRIIB-Fc fusion polypeptide of SEQ ID NOs: 139 and 141 above, four amino acid substitutions (replacing a tyrosine with a cysteine, a threonine with a serine, a leucine with an alanine, and a tyrosine with a valine) can be introduced into the Fc domain of the ALK4 fusion polypeptide as indicated by double underline above. To facilitate purification of the ALK4-Fc:ActRIIB-Fc heterodimer, two amino acid substitutions (replacing an asparagine with an arginine and an aspartate with an arginine) can also be introduced into the Fc domain of the ALK4-Fc fusion polypeptide as indicated by double underline above. The amino acid sequence of SEQ ID NO: 143 may optionally be provided with lysine removed from the C-terminus.

This ALK4-Fc fusion polypeptide is encoded by the following nucleic acid (SEQ ID NO: 144):

1 ATGGATGCAA TGAAGAGAGG GCTCTGCTGT GTGCTGCTGC TGTGTGGAGC
51 AGTCTTCGTT TCGCCCGGCG CCTCCGGGCC CCGGGGGGTC CAGGCTCTGC
101 TGTGTGCGTG CACCAGCTGC CTCCAGGCCA ACTACACGTG TGAGACAGAT
151 GGGGCCTGCA TGGTTTCCAT TTTCAATCTG GATGGGATGG AGCACCATGT
201 GCGCACCTGC ATCCCCAAAG TGGAGCTGGT CCCTGCCGGG AAGCCCTTCT
251 ACTGCCTGAG CTCGGAGGAC CTGCGCAACA CCCACTGCTG CTACACTGAC
301 TACTGCAACA GGATCGACTT GAGGGTGCCC AGTGGTCACC TCAAGGAGCC
351 TGAGCACCCG TCCATGTGGG GCCCGGTGGA GACCGGTGGT GGAACTCACA
401 CATGCCCACC GTGCCCAGCA CCTGAACTCC TGGGGGGACC GTCAGTCTTC

451 CTCTTCCCCC CAAAACCCAA GGACACCCTC ATGATCTCCC GGACCCCTGA
501 GGTCACATGC GTGGTGGTGG ACGTGAGCCA CGAAGACCCT GAGGTCAAGT
551 TCAACTGGTA CGTGGACGGC GTGGAGGTGC ATAATGCCAA GACAAAGCCG
601 CGGGAGGAGC AGTACAACAG CACGTACCGT GTGGTCAGCG TCCTCACCGT
651 CCTGCACCAG GACTGGCTGA ATGGCAAGGA GTACAAGTGC AAGGTCTCCA
701 ACAAAGCCCT CCCAGCCCCC ATCGAGAAAA CCATCTCCAA AGCCAAAGGG
751 CAGCCCCGAG AACCACAGGT GTGCACCCTG CCCCCATCCC GGGAGGAGAT
801 GACCAAGAAC CAGGTCAGCC TGTCCTGCGC CGTCAAAGGC TTCTATCCCA
851 GCGACATCGC CGTGGAGTGG GAGAGCCGCG GGCAGCCGGA GAACAACTAC
901 AAGACCACGC CTCCCGTGCT GGACTCCCGC GGCTCCTTCT TCCTCGTGAG
951 CAAGCTCACC GTGGACAAGA GCAGGTGGCA GCAGGGGAAC GTCTTCTCAT
1001 GCTCCGTGAT GCATGAGGCT CTGCACAACC ACTACACGCA GAAGAGCCTC
1051 TCCCTGTCTC CGGGTAAA (SEQ ID NO: 144)

The mature ALK4-Fc fusion polypeptide sequence is as follows (SEQ ID NO: 145) and may optionally be provided with lysine removed from the C-terminus.

1 SGPRGVQALL CACTSCLQAN YTCETDGACM VSIFNLDGME HHVRTCIPKV
51 ELVPAGKPFY CLSSEDLRNT HCCYTDYCNR IDLRVPSGHL KEPEHPSMWG
101 PVETGGGTHT CPPCPAPELL GGPSVFLFPP KPKDTLMISR TPEVTCVVVD
151 VSHEDPEVKF NWYVDGVEVH NAKTKPREEQ YNSTYRVVSV LTVLHQDWLN
201 GKEYKCKVSN KALPAPIEKT ISKAKGQPRE PQVCTLPPSR EEMTKNQVSL
251 SCAVKGFYPS DIAVEWESRG QPENNYKTTP PVLDSRGSFF LVSKLTVDKS
301 RWQQGNVFSC SVMHEALHNH YTQKSLSLSP GK (SEQ ID NO: 145)

This ALK4-Fc fusion polypeptide is encoded by the following nucleic acid (SEQ ID NO: 146):

1 TCCGGGCCCC GGGGGGTCCA GGCTCTGCTG TGTGCGTGCA CCAGCTGCCT
51 CCAGGCCAAC TACACGTGTG AGACAGATGG GGCCTGCATG GTTTCCATTT
101 TCAATCTGGA TGGGATGGAG CACCATGTGC GCACCTGCAT CCCCAAAGTG
151 GAGCTGGTCC CTGCCGGGAA GCCCTTCTAC TGCCTGAGCT CGGAGGACCT
201 GCGCAACACC CACTGCTGCT ACACTGACTA CTGCAACAGG ATCGACTTGA
251 GGGTGCCCAG TGGTCACCTC AAGGAGCCTG AGCACCCGTC CATGTGGGGC
301 CCGGTGGAGA CCGGTGGTGG AACTCACACA TGCCCACCGT GCCCAGCACC
351 TGAACTCCTG GGGGGACCGT CAGTCTTCCT CTTCCCCCCA AAACCCAAGG
401 ACACCCTCAT GATCTCCCGG ACCCCTGAGG TCACATGCGT GGTGGTGGAC

451 GTGAGCCACG AAGACCCTGA GGTCAAGTTC AACTGGTACG TGGACGGCGT
501 GGAGGTGCAT AATGCCAAGA CAAAGCCGCG GGAGGAGCAG TACAACAGCA
551 CGTACCGTGT GGTCAGCGTC CTCACCGTCC TGCACCAGGA CTGGCTGAAT
601 GGCAAGGAGT ACAAGTGCAA GGTCTCCAAC AAAGCCCTCC CAGCCCCCAT
651 CGAGAAAACC ATCTCCAAAG CCAAAGGGCA GCCCCGAGAA CCACAGGTGT
701 GCACCCTGCC CCCATCCCGG GAGGAGATGA CCAAGAACCA GGTCAGCCTG
751 TCCTGCGCCG TCAAAGGCTT CTATCCCAGC GACATCGCCG TGGAGTGGGA
801 GAGCCGCGGG CAGCCGGAGA ACAACTACAA GACCACGCCT CCCGTGCTGG
851 ACTCCCGCGG CTCCTTCTTC CTCGTGAGCA AGCTCACCGT GGACAAGAGC
901 AGGTGGCAGC AGGGGAACGT CTTCTCATGC TCCGTGATGC ATGAGGCTCT
951 GCACAACCAC TACACGCAGA AGAGCCTCTC CCTGTCTCCG GGTAAA
(SEQ ID NO: 146)

ActRIIB-Fc and ALK4-Fc proteins of SEQ ID NO: 141 and SEQ ID NO: 145, respectively, may be co-expressed and purified from a CHO cell line, to give rise to a heteromeric complex comprising ALK4-Fc:ActRIIB-Fc.

Purification of various ALK4-Fc:ActRIIB-Fc complexes could be achieved by a series of column chromatography steps, including, for example, three or more of the following, in any order: protein A chromatography, Q sepharose chromatography, phenylsepharose chromatography, size exclusion chromatography, cation exchange chromatography, epitope-based affinity chromatography (e.g., with an antibody or functionally equivalent ligand directed against an epitope on ALK4 or ActRIIB), and multimodal chromatography (e.g., with resin containing both electrostatic and hydrophobic ligands). The purification could be completed with viral filtration and buffer exchange.

### Example 9. Ligand binding profile of ALK4-Fc:ActRIIB-Fc heterodimer compared to ActRIIB-Fc homodimer and ALK4-Fc homodimer

A Biacore^{™}-based binding assay was used to compare ligand binding selectivity of the ALK4-Fc:ActRIIB-Fc heterodimeric complex described above with that of ActRIIB-Fc and ALK4-Fc homodimer complexes. The ALK4-Fc:ActRIIB-Fc heterodimer, ActRIIB-Fc homodimer, and ALK4-Fc homodimer were independently captured onto the system using an anti-Fc antibody. Ligands were injected and allowed to flow over the captured receptor protein. Results are summarized in the table below, in which ligand off-rates (k_{d}) most indicative of effective ligand traps are denoted by gray shading.

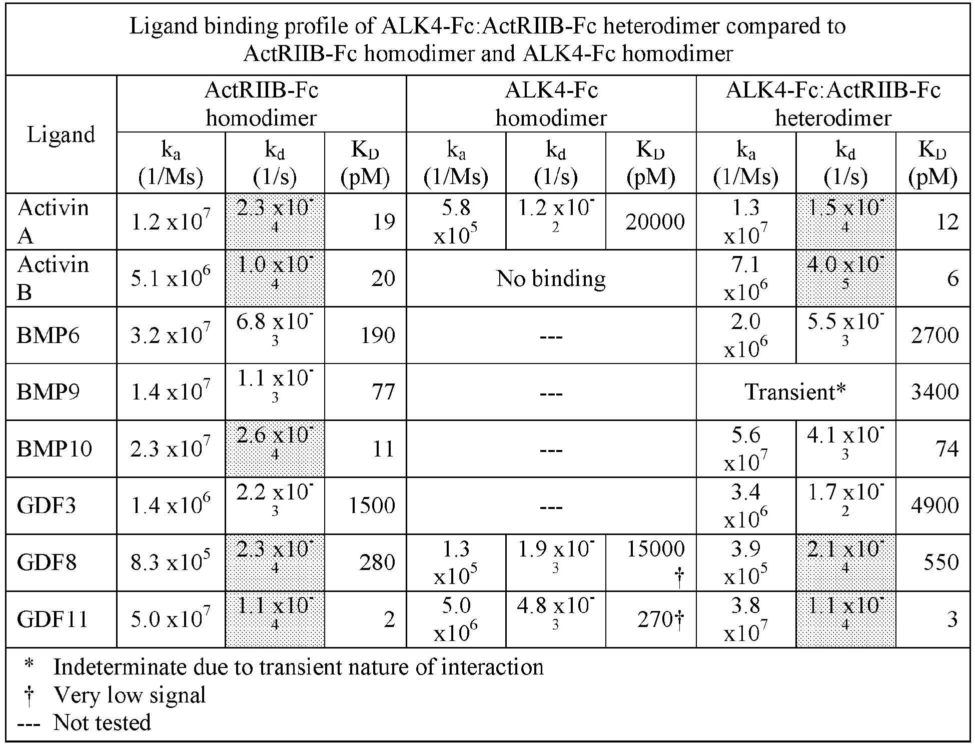

These comparative binding data demonstrate that ALK4-Fc:ActRIIB-Fc heterodimer has an altered binding profile/selectivity relative to either ActRIIB-Fc or ALK4-Fc homodimers. ALK4-Fc:ActRIIB-Fc heterodimer displays enhanced binding to activin B compared with either homodimer, retains strong binding to activin A, GDF8, and GDF11 as observed with ActRIIB-Fc homodimer, and exhibits substantially reduced binding to BMP9, BMP10, and GDF3. In particular, BMP9 displays low or no observable affinity for ALK4-Fc:ActRIIB-Fc heterodimer, whereas this ligand binds strongly to ActRIIB-Fc homodimer. Like the ActRIIB-Fc homodimer, the heterodimer retains intermediate-level binding to BMP6. See Figure 19.

In addition, an A-204 Reporter Gene Assay was used to evaluate the effects of ALK4-Fc:ActRIIB-Fc heterodimer and ActRIIB-Fc: ActRIIB-Fc homodimer on signaling by activin A, activin B, GDF11, GDF8, BMP10, and BMP9. Cell line: Human Rhabdomyosarcoma (derived from muscle). Reporter vector: pGL3(CAGA)12 (as described in Dennler et al, 1998, EMBO 17: 3091-3100). The CAGA12 motif is present in TGFβ responsive genes (PAI-1 gene), so this vector is of general use for factors signaling through Smad2 and 3. An exemplary A-204 Reporter Gene Assay is outlined below.

Day 1: Split A-204 cells into 48-well plate.

Day 2: A-204 cells transfected with 10 ug pGL3(CAGA)12 or pGL3(CAGA)12(10 ug)+pRLCMV (1 ug) and Fugene.

Day 3: Add factors (diluted into medium+0.1% BSA). Inhibitors need to be preincubated with Factors for about one hr before adding to cells. About six hrs later, cells are rinsed with PBS and then lysed.

Following the above steps, a Luciferase assay was performed.

Both the ALK4-Fc:ActRIIB-Fc heterodimer and ActRIIB-Fc:ActRIIB-Fc homodimer were determined to be potent inhibitors of activin A, activin B, GDF11, and GDF8 in this assay. In particular, as can be seen in the comparative homodimer/heterodimer IC₅₀ data illustrated in Figure 19, ALK4-Fc:ActRIIB-Fc heterodimer inhibits activin A, activin B, GDF8, and GDF11 signaling pathways similarly to the ActRIIB-Fc:ActRIIB-Fc homodimer. However, ALK4-Fc:ActRIIB-Fc heterodimer inhibition of BMP9 and BMP10 signaling pathways is significantly reduced compared to the ActRIIB-Fc:ActRIIB-Fc homodimer. This data is consistent with the above-discussed binding data in which it was observed that both the ALK4-Fc:ActRIIB-Fc heterodimer and ActRIIB-Fc:ActRIIB-Fc homodimer display strong binding to activin A, activin B, GDF8, and GDF11, but BMP10 and BMP9 have significantly reduced affinity for the ALK4-Fc:ActRIIB-Fc heterodimer compared to the ActRIIB-Fc:ActRIIB-Fc homodimer.

Together, these data therefore demonstrate that ALK4-Fc:ActRIIB-Fc heterodimer is a more selective antagonist of activin A, activin B, GDF8, and GDF11 compared to ActRIIB-Fc homodimer. Accordingly, an ALK4-Fc:ActRIIB-Fc heterodimer will be more useful than an ActRIIB-Fc homodimer in certain applications where such selective antagonism is advantageous. Examples include therapeutic applications where it is desirable to retain antagonism of one or more of activin A, activin B, activin AC, GDF8, and GDF11 but minimize antagonism of one or more of BMP9, BMP10, GDF3, and BMP6.

### Example 10. Generation of TGFβRII-Fc fusion protein

Human TGFβRII occurs naturally in at least two isoforms - A (long) and B (short) - generated by alternative splicing in the extracellular domain (ECD) (Figures 10 and 11). TGFβRII binds with high affinity to TGFβ1 and TGFβ3. As detailed below, a TGFβRII-Fc fusion protein comprising an extracellular domain of the TGFβRII long isoforms (Tβ3RII_{long}) was generated.

The wild-type hTβRII_{long}(23-184) sequence is shown below (SEQ ID NO: 147), in which the 25 amino-acid insertion is underlined. Note that splicing results in a conservative amino acid substitution (Val→Ile) at the flanking position C-terminal to the insertion. Sequence relationships among several hTβRIIₛₕₒᵣₜ variants and their hTβRII_{long} counterparts are indicated in Figure 24.

1 TIPPHVQKSD VEMEAQKDEI ICPSCNRTAH PLRHINNDMI VTDNNGAVKF
51 PQLCKFCDVR FSTCDNQKSC MSNCSITSIC EKPQEVCVAV WRKNDENITL
101 ETVCHDPKLP YHDFILEDAA SPKCIMKEKK KPGETFFMCS CSSDECNDNI
151 IFSEEYNTSN PD (SEQ ID NO: 147)

A hTβRII_{long}(23-184)-Fc fusion protein was generated in which the hTβII_{long}(23-184) domain was fused at the C-terminus (via a linker) to a human IgG1 Fc domain and fused at the N-terminus to a TPA leader sequence, which has the following amino acid sequence (SEQ ID NO: 148):

1 MDAMKRGLCC VLLLCGAVFV SPGATIPPHV QKSDVEMEAQ KDEIICPSCN
51 RTAHPLRHIN NDMIVTDNNG AVKFPQLCKF CDVRFSTCDN QKSCMSNCSI
101 TSICEKPQEV CVAVWRKNDE NITLETVCHD PKLPYHDFIL EDAASPKCIM
151 KEKKKPGETF FMCSCSSDEC NDNIIFSEEY NTSNPDTGGG THTCPPCPAP
201 ELLGGPSVFL FPPKPKDTLM ISRTPEVTCV VVDVSHEDPE VKFNWYVDGV
251 EVHNAKTKPR EEQYNSTYRV VSVLTVLHQD WLNGKEYKCK VSNKALPAPI
301 EKTISKAKGQ PREPQVYTLP PSREEMTKNQ VSLTCLVKGF YPSDIAVEWE
351 SNGQPENNYK TTPPVLDSDG SFFLYSKLTV DKSRWQQGNV FSCSVMHEAL
401 HNHYTQKSLS LSPGK (SEQ ID NO: 148)

The N-terminal leader sequence and C-terminal Fc domain are represented by a single underline and the linker domain is indicated by double underline. A nucleotide sequence encoding the_hTβRII_{long}(23-184)-Fc fusion protein has the following nucleotide sequence (SEQ ID NO: 149):

1 ATGGATGCAA TGAAGAGAGG GCTCTGCTGT GTGCTGCTGC TGTGTGGAGC
AGTCTTCGTT

61 TCGCCCGGCG CCACGATCCC ACCGCACGTT CAGAAGTCGG ATGTGGAAAT
GGAGGCCCAG
121 AAAGATGAAA TCATCTGCCC CAGCTGTAAT AGGACTGCCC ATCCACTGAG
ACATATTAAT
181 AACGACATGA TAGTCACTGA CAACAACGGT GCAGTCAAGT TTCCACAACT
GTGTAAATTT
241 TGTGATGTGA GATTTTCCAC CTGTGACAAC CAGAAATCCT GCATGAGCAA
CTGCAGCATC
301 ACCTCCATCT GTGAGAAGCC ACAGGAAGTC TGTGTGGCTG TATGGAGAAA
GAATGACGAG
361 AACATAACAC TAGAGACAGT TTGCCATGAC CCCAAGCTCC CCTACCATGA
CTTTATTCTG
421 GAAGATGCTG CTTCTCCAAA GTGCATTATG AAGGAAAAAA AAAAGCCTGG
TGAGACTTTC
481 TTCATGTGTT CCTGTAGCTC TGATGAGTGC AATGACAACA TCATCTTCTC
AGAAGAATAT
541 AACACCAGCA ATCCTGACAC CGGTGGTGGA ACTCACACAT GCCCACCGTG
CCCAGCACCT
601 GAACTCCTGG GGGGACCGTC AGTCTTCCTC TTCCCCCCAA AACCCAAGGA
CACCCTCATG
661 ATCTCCCGGA CCCCTGAGGT CACATGCGTG GTGGTGGACG TGAGCCACGA
AGACCCTGAG
721 GTCAAGTTCA ACTGGTACGT GGACGGCGTG GAGGTGCATA ATGCCAAGAC
AAAGCCGCGG
781 GAGGAGCAGT ACAACAGCAC GTACCGTGTG GTCAGCGTCC TCACCGTCCT
GCACCAGGAC
841 TGGCTGAATG GCAAGGAGTA CAAGTGCAAG GTCTCCAACA AAGCCCTCCC
AGCCCCCATC
901 GAGAAAACCA TCTCCAAAGC CAAAGGGCAG CCCCGAGAAC CACAGGTGTA
CACCCTGCCC
961 CCATCCCGGG AGGAGATGAC CAAGAACCAG GTCAGCCTGA CCTGCCTGGT
CAAAGGCTTC
1021 TATCCCAGCG ACATCGCCGT GGAGTGGGAG AGCAATGGGC AGCCGGAGAA
CAACTACAAG
1081 ACCACGCCTC CCGTGCTGGA CTCCGACGGC TCCTTCTTCC TCTATAGCAA
GCTCACCGTG
1141 GACAAGAGCA GGTGGCAGCA GGGGAACGTC TTCTCATGCT CCGTGATGCA
TGAGGCTCTG
1201 CACAACCACT ACACGCAGAA GAGCCTCTCC CTGTCTCCGG GTAAATGA (SEQ ID
NO: 149)

A processed version of the hTβRII_{long}(23-184)-Fc fusion protein has the following amino acid sequence (SEQ ID NO: 150):

TIPPHV QKSDVEMEAQ KDEIICPSCN
RTAHPLRHIN NDMIVTDNNG AVKFPQLCKF CDVRFSTCDN QKSCMSNCSI
TSICEKPQEV CVAVWRKNDE NITLETVCHD PKLPYHDFIL EDAASPKCIM
KEKKKPGETF FMCSCSSDEC NDNIIFSEEY NTSNPDTGGG THTCPPCPAP
ELLGGPSVFL FPPKPKDTLM ISRTPEVTCV VVDVSHEDPE VKFNWYVDGV
EVHNAKTKPR EEQYNSTYRV VSVLTVLHQD WLNGKEYKCK VSNKALPAPI
EKTISKAKGQ PREPQVYTLP PSREEMTKNQ VSLTCLVKGF YPSDIAVEWE
SNGQPENNYK TTPPVLDSDG SFFLYSKLTV DKSRWQQGNV FSCSVMHEAL
HNHYTQKSLS LSPGK (SEQ ID NO: 150)

The hTβRII_{long}(23-184)-Fc fusion protein was expressed in CHO cells and purified from conditioned media by filtration and protein A chromatography. Purity of samples for reporter gene assays was evaluated by SDS-PAGE and Western blot analysis.

For use in certain animal models described herein, an Fc fusion protein comprising the mature, full-length ECD from the mouse TβRII isoform 1, which is designated herein as mTβRII_{long}-Fc was generated. The mouse TβRII isoform 1 is homologous to the human TβRII isoform A (long form) and thus is a mouse equivalent version of the hTβRII_{long}(23-184)-Fc fusion protein above described. As with the human version, it was determined that mTβRII_{long}-Fc binds with high affinity (picomolar) to TGFβ1 and TGFβ3, but does not bind to TGFβ2. In addition, it was determined that the hTβRII_{long}(23-184)-Fc fusion protein and mTβRII_{long}-Fc are potent inhibitors of TGFβ1 and TGFβ3 activity, but does not inhibit TGFβ2 activity, in a cell-based assay.

### Example 11: Antitumor activity of ActRII and TGFβ antagonists

Potential antitumor activity of ActRIIA-mFc (mouse Fc form of SEQ ID NO: 50), ActRIIB-mFc (mouse Fc form of SEQ ID NO: 58), and TβRII_{long}(23-184)-mFc (mouse Fc form of SEQ ID NO: 150) fusion proteins were investigated in a syngeneic murine leukemia model. Eight-week-old BALB/c mice were randomly assigned to treatment (n = 10 per group) and treated intraperitoneally with ActRIIA-mFc (10 mg/kg), ActRIIB-mFc (10 mg/kg), TβRII_{long}(23-184)-mFc (10 mg/kg), or vehicle (phosphate-buffered saline, PBS, 5 ml/kg) twice weekly beginning two days prior to administration of cancer cells. On day 0, each mouse was inoculated subcutaneously with 1 × 10⁶ RL♂1 (RLmale1) cells suspended in PBS (100 µL). RLmale1 is an x-ray-induced leukemia of BALB/c origin (Sato H et al., 1973, J Exp Med 138:593-606). After inoculation of mice, body weight and tumor volume were measured twice weekly. Tumor volumes were calculated from two-dimensional measurements obtained with calipers: tumor volume (in mm³) = (L × W × W)/2 where L and W are the tumor length and width (in mm), respectively. Complete tumor regression and tumor-free survival were both defined according to Teicher BA (ed) Anticancer Drug Development Guide: Preclinical Screening, Clinical Trials, and Approval; Humana Press, 1997. Per local IACUC regulations, endpoints used for survival analysis were a tumor volume larger than 2000 mm³, loss of body weight greater than 20%, or hind-leg paralysis. The survival curves of different groups were compared by median survival as well as by log-rank (Mantel-Cox) test.

As shown in the following table, both ActRIIA-mFc and ActRIIB-mFc exhibited antitumor activity. However, TβRII_{long}(23-184)-mFc did not demonstrate any appreciable antitumor activity in this model.

| Test article | Strain | n | Dose (mg/kg) | Route | Schedule | % tumor free (day 56) | Median survival (days) |
|---|---|---|---|---|---|---|---|
| Vehicle | BALB/c | 10 | -- | i.p. | biw | 0 | 15 |
| ActRIIA-mFc | BALB/c | 10 | 10 | i.p. | biw | 20 | 21.5 |
| ActRIIB-mFc | BALB/c | 10 | 10 | i.p. | biw | 20 | 32.5 |
| TβRII_{long}(2 3-184)-mFc | BALB/c | 10 | 10 | i.p. | biw | 0 | 17 |

Treatment with ActRIIA-mFc or ActRIIB-mFc led to 2 of 10 mice (20%) with tumor-free status on day 56, compared to none of the vehicle and TβRII_{long}(23-184)-mFc treated mice. Increased median survival and high significance in the log-rank test also indicate that ActRIIA-mFc and ActRIIB-mFc each increased survival of tumor-bearing mice. The initial response to ActRIIB-mFc was particularly robust, as 50% of ActRIIB-mFc-treated mice showed complete tumor regression by day 34 compared to none in the vehicle-treated group. These results show that ActRIIA-mFc and ActRIIB-mFc possess antitumor activity in vivo, indicating that these proteins, as well as other ActRII antagonists, may be useful in the treatment of cancer. In contrast, the data for TβRII_{long}(23-184)-mFc suggests that inhibition of TGFβ1 and TGFβ3 is not sufficient for promoting an antitumor response.

Using the same murine leukemia model, it was then assessed whether ActRIIB-hFc (homodimer of SEQ ID NO: 58) has antitumor activity similar to that of ActRIIB-mFc and whether antitumor activity is dependent on T cell-mediated immunity. Eight-week-old BALB/c mice were randomly assigned to treatment (n = 10 per group) and treated intraperitoneally with ActRIIB-mFc (10 mg/kg), ActRIIB-hFc (10 mg/kg), or vehicle (PBS, 5 ml/kg) twice weekly beginning two days prior to administration of cancer cells. In addition, 7-week-old NCr-nude mice with defective T cell immunity were randomly assigned to treatment (n = 10 per group) and treated intraperitoneally with ActRIIB-mFc (10 mg/kg), ActRIIB-hFc (10 mg/kg), or vehicle (PBS, 5 ml/kg) twice weekly beginning two days prior to administration of cancer cells.. Finally, the four mice that had remained tumor free for approximately 7 weeks during the experiment described above (two mice treated with ActRIIA-mFc and two mice treated with ActRIIB-mFc) were re-challenged with RLmale1 cells to test for antitumor immune memory. On day 0, each mouse was inoculated subcutaneously with 1 × 10⁶ RL♂1 (RLmale1) cells suspended in PBS (100 µL). After mouse inoculation, body weight and tumor volume were measured twice weekly. Per local IACUC regulations, endpoints used for survival analysis were a tumor volume larger than 2000 mm³, loss of body weight greater than 20%, or hind-leg paralysis.

As show in the table below, antitumor effects of ActRIIB-mFc and ActRIIB-hFc were dependent on mouse strain.

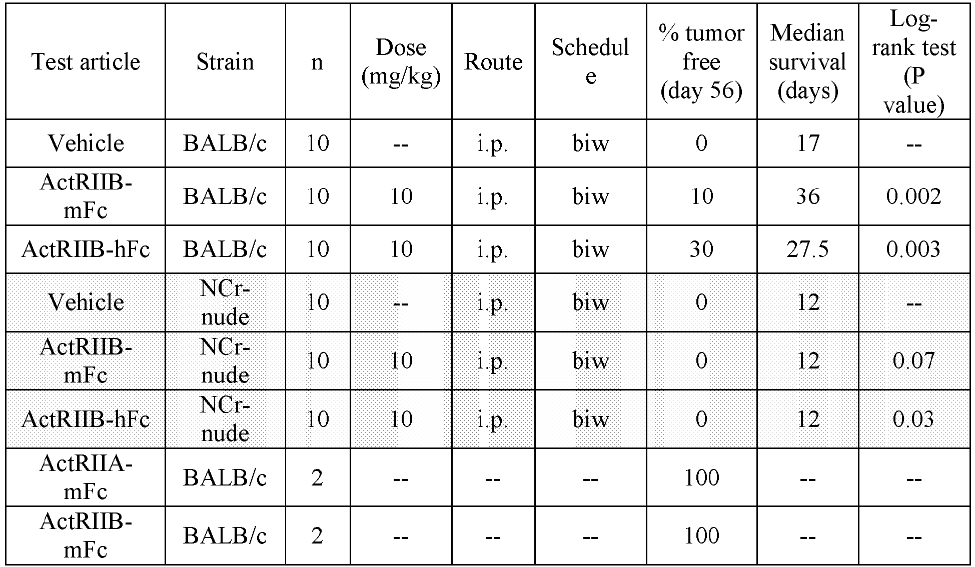

Both ActRIIB-hFc and ActRIIB-mFc exhibited antitumor activity in immunocompetent BALB/c mice, as shown in the following table. Treatment with ActRIIB-mFc or ActRIIB-hFc led to 10% or 30% of mice, respectively, with tumor-free status on day 56, compared to none of the vehicle-treated mice. Increased median survival and high significance in the log-rank test also demonstrate that ActRIIB-mFc and ActRIIB-hFc each promoted survival of tumor-bearing mice. Importantly, the antitumor effects of ActRIIB-mFc and ActRIIB-hFc in NCr-nude mice were absent or markedly blunted compared to BALB/c mice, thereby implicating T cell immunity in the mechanism of action for these inhibitors of ActRIIB ligands. Moreover, all four tumor-free mice carried over from the previous experiment exhibited no detectable tumor growth throughout the present experiment despite a repeat inoculation with RLmale1 tumor cells. These results provide further evidence that immune cells mediate the regression of RLmale1 tumors caused by treatment with ActRIIA-mFc or ActRIIB-mFc on a BALB/c background and that the effective antitumor immune response generated immunologic memory to tumor antigens. Furthermore, these results confirm antitumor activity of ActRIIB-hFc and ActRIIB-mFc in vivo and strongly implicate T cell immunity in this activity. Together, the data suggest that ActRII antagonists may be used to potentiate immune activity in vivo and thus such antagonists may be useful in treating a variety of disorders and conditions wherein increased immune activity is desirable (e.g., immune-oncology applications as well as treatment of a variety of pathogens).

### Example 12: Antitumor activity of ActRII and TGFβ antagonists combination therapy

Using the same murine leukemia model as described in Example 11, Applicants then investigated whether ActRIIB-hFc antitumor activity can be enhanced by combining it with a TGFβ antagonist. For the combination study, a pan-specific TGFβ antibody (one that binds to TGFβ1, TGFβ2, and TGFβ3 with high affinity) was used as the TGFβ antagonist.

Eight-week-old BALB/c mice were randomly assigned to treatment (n = 10 per group) and treated intraperitoneally with ActRIIB-hFc (10 mg/kg), TGFβ antibody (mAb) (10 mg/kg), combination of ActRIIA-hFc and TGFβ mAb (both at 10 mg/kg), or vehicle (phosphate-buffered saline, PBS, 5 ml/kg) twice weekly beginning two days prior to administration of cancer cells. On day 0, each mouse was inoculated subcutaneously with 1 × 10⁶ RL♂1 (RLmale1) cells suspended in PBS (100 µL). RLmalel is an x-ray-induced leukemia of BALB/c origin (Sato H et al., 1973, J Exp Med 138:593-606). After inoculation of mice, body weight and tumor volume were measured twice weekly as described in the previous example. The survival curves of different groups were compared by median survival as well as by log-rank (Mantel-Cox) test.

As shown in the following table, combination therapy with an ActRII antagonist and a TGFβ antagonist exhibited greater antitumor activity than observed for each antagonist alone.

| Test article | Strain | n | Dose (mg/kg) | Route | Schedule | % tumor free (day 56) | Median survival (days) |
|---|---|---|---|---|---|---|---|
| Vehicle | BALB/c | 10 | -- | i.p. | biw | 0 | 15 |
| ActRIIB-hFc | BALB/c | 10 | 10 | i.p. | biw | 30 | 17 |
| TGFβ mAb | BALB/c | 10 | 10 | i.p. | biw | 20 | 15 |
| Combo | BALB/c | 10 | 10 (of each agent) | i.p. | biw | 70 | >31 |

Treatment with ActRIIB-hFc alone or TGFβ mAb alone showed modest effects on tumor regression in this model, 30% and 20% tumor-free status respectively. Combined treatment with ActRIIB-hFc and TGFβ mAb led to a surprising and significant increase in antitumor activity, 70% tumor-free status and approximately doubled the median survival time. Synergy of this type is generally considered evidence that the individual agents are acting through different cellular mechanism. Therefore, while inhibition of either the ActRII or TGFβRII signaling pathway may promote antitumor activity, inhibition of both pathways may be used to synergistically increase antitumor activity in such experimental or clinical situations where increased antitumor activity is desirable. Together, these data indicate that ActRII and TGFβ antagonists can be used alone but particularly in combination to treat cancer.

Furthermore, the antitumor activity of the TGFβ mAb treatment alone is surprising in view of the data above for TβRII_{long}(23-184)-mFc, which was not observed to have any antitumor activity in this model. This may provide further mechanistic insight into the antitumor activity of the TGFβ signaling pathway. As previously described, TβRII_{long}(23-184)-mFc binds to TGFβ1 and TGFβ3 with high affinity and can neutralize TGFβ1 and TGFβ3 signaling in cell-based assays. However, TβRII_{long}(23-184)-mFc does not bind to TGFβ2. In contrast, the TGFβ mAb used in this study binds with high affinity to all three isoforms of TGFβ (TGFβ1, TGFβ2, and TGFβ3). In view of the difference in activity, these data indicate TGFβ2 may be more important for tumor development compared to TGFβ2 and TGFβ3. Therefore TGFβ antagonists that inhibit at least TGFβ2 activity may be useful promoting an antitumor response. Moreover, the data suggest that a TGFβ2 antagonist may be used synergistically with an ActRII antagonist to increase antitumor activity and thus such a combination therapy may be useful in the treatment of cancer.

## Claims

1. A composition for use in treating cancer in a patient comprising administering to the patient in need thereof:
(i) an effective amount of an ActRIIB antagonist, wherein the ActRIIB antagonist is an ActRIIB-Fc fusion protein comprising an amino acid sequence of SEQ ID NO: 58; and
(ii) an effective amount of a TGFβ antagonist, wherein the TGFβ antagonist comprises a pan-specific antibody that binds TGFβ1, TGFβ2, and TGFβ3, and wherein the cancer is leukemia.

2. The composition for use of claim 1, wherein the composition is for use in combination with one or more additional immuno-oncology agents.

3. The composition for use of claim 1 or 2, wherein the polypeptide or fusion protein binds to and/or inhibits one or more ligands selected from the group consisting of: activin A, activin B, GDF11, GDF8, GDF3, BMP6, BMP10, and BMP9.

## Patentansprüche

1. Eine Zusammensetzung zur Verwendung bei der Behandlung von Krebs bei einem Patienten, welche die Verabreichung von:
(i) einer wirksamen Menge eines ActRIIB-Antagonisten, wobei der ActRIIB-Antagonist ein ActRIIB-Fc-Fusionsprotein ist, das eine Aminosäuresequenz der SEQ ID NO: 58 umfasst, und
(ii) einer wirksamen Menge eines TGFβ-Antagonisten, wobei der TGFβ-Antagonist einen pan-spezifischen Antikörper umfasst, der TGFβ1, TGFβ2 und TGFβ3 bindet,
an den Patienten, der dies benötigt, umfasst,
und wobei der Krebs Leukämie ist.

2. Die Zusammensetzung zur Verwendung nach Anspruch 1, wobei die Zusammensetzung zur Verwendung in Kombination mit einem oder mehreren zusätzlichen Immunonkologie-Mitteln dient.

3. Die Zusammensetzung zur Verwendung nach Anspruch 1 oder 2, wobei das Polypeptid oder Fusionsprotein an einen oder mehrere Liganden, ausgewählt aus der Gruppe bestehend aus: Activin A, Activin B, GDF11, GDF8, GDF3, BMP6, BMP10 und BMP9, bindet und/oder einen oder mehrere davon inhibiert.

## Revendications

1. Composition pour utilisation dans le traitement d'un cancer chez un patient, comprenant l'administration, au patient en ayant besoin :
(i) d'une quantité efficace d'un antagoniste d'ActRIIB, dans laquelle l'antagoniste d'ActRIIB est une protéine de fusion ActRIIB-Fc comprenant une séquence d'acides aminés de SEQ ID N° : 58 ; et
(ii) d'une quantité efficace d'un antagoniste de TGFβ, dans laquelle l'antagoniste de TGFβ comprend un anticorps pan-spécifique qui se lie à TGFβ1, TGFβ2 et TGFβ3, et où le cancer est une leucémie.

2. Composition pour utilisation selon la revendication 1, où la composition est pour utilisation en combinaison avec un ou plusieurs agents immuno-oncologiques supplémentaires.

3. Composition pour utilisation selon la revendication 1 ou 2, dans laquelle le polypeptide ou la protéine de fusion se lie à et/ou inhibe un ou plusieurs ligands sélectionnés dans le groupe constitué de : l'activine A, l'activine B, le GDF11, le GDF8, le GDF3, la BMP6, la BMP10 et la BMP9.
